# EUROPÄISCHE PATENTANMELDUNG

(11) **EP 2 374 791 A1**
(43) Veröffentlichungstag der Anmeldung: **12.10.2011**
(21) Anmeldenummer: 11165244.2
(22) Anmeldetag: 01.08.2009
(51) Int. Cl.: C07C 243/22, C07D 231/12, C07D 231/16, C07D 231/18, C07D 231/38, C07D 249/08, C07D 403/10, A01N 43/54, A01N 43/56

(54) **Insektizide 4-Phenyl-1H-pyrazole**

(30) Priorität: 14.08.2008 EP 08162378
(62) Teilanmeldung aus: 09777596.9
(71) Anmelder: Bayer CropScience Aktiengesellschaft, 40789 Monheim Am Rhein (DE)
(72) Erfinder: Werner, Stefan, 40789 Monheim (DE); Müller, Klaus-Helmut, 40593 Düsseldorf (DE); Schwarz, Hans-Georg, 46282 Dorsten (DE); Murata, Tetsuya, Oyama-shi Tochigi 323-0820 (JP); RAMING, Klaus, 51375 Leverkusen (DE); Görgens, Ulrich, 40882 Ratingen (DE); Becker, Angela, 40235 Düsseldorf (DE); Franken, Eva-Maria, 42799 Leichlingen (DE); Shimojo, Eiichi, Oyama-shi Tochigi 323-0820 (JP); Shibuya, Katsuhiko, Shimotuke-shi Tochigi 329-0433 (JP); Ebbinghaus-Kintscher, Ulrich, 44287 Dortmund (DE); Ichihara, Teruyuki, Oyama-shi Tochigi 323-0822 (JP); Ataka, Masashi, Omiya-ku, Saitama-shi Saitama 330-0803 (JP); Malsam, Olga, 51503 Rösrath (DE); Voerste, Arnd, 50674 Köln (DE)

(57) **Zusammenfassung**

Die vorliegende Erfindung betrifft neue 4-Phenyl-1H-pyrazole und ihre Verwendung als Insektizide und/oder Parasitizide sowie Verfahren zu ihrer Herstellung und Mittel, die solche Phenylpyrazole enthalten.

## Beschreibung

Die vorliegende Erfindung betrifft neue 4-Phenyl-1H-pyrazole und ihre Verwendung als Insektizide und/oder Parasitizide sowie Verfahren zu ihrer Herstellung und Mittel, die solche Phenylpyrazole enthalten.

In der EP 846686 werden 4-Phenyl-1H-pyrazole (A) beschrieben, die parasitizide, insektizide und nematizide Wirkung aufweisen. Die Definitionen der Substituenten R³, R⁵ und R⁷ sind wie folgt festgelegt: 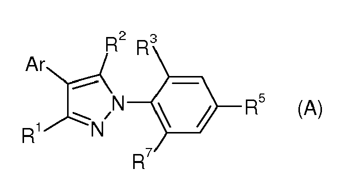 R³ Halogen;
R⁵ H, C₁₋₆-Alkyl, gegebenenfalls substituiert mit einem oder mehreren Halogenatomen, C₁₋₆-Alkoxy, gegebenenfalls substituiert mit einem oder mehreren Halogenatomen, oder SF₅;
R⁷ Halogen.

In der WO 2008/077483 werden Pyrimidinylpyrazole (B) beschrieben, die insektizide und/oder parasitizide Wirkung aufweisen. Die Definitionen der Substituenten R³ und n sind wie folgt festgelegt: 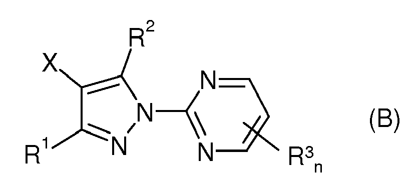 R³ Halogen, Alkyl, Haloalkyl, Alkoxy oder Dialkylamino
n 0 oder 1

Meegalla et al. beschreiben die Synthese und insektizide Aktivität von 3-Thiomethyl-4-(hetero)aryl-5-amino-1-phenylpyrazolen als GABA-Kanal-Blocker (Bioorganic & Medicinal Chemistry Letters (2004), 14, 4949-4953).

In der WO 2007/048734 werden 5-Aminopyrazole (C) zur Bekämpfung von pflanzenpathogenen Schadpilzen beschrieben. Die Definitionen der Substituenten R³, R⁴ und R⁵ sind wie folgt festgelegt: 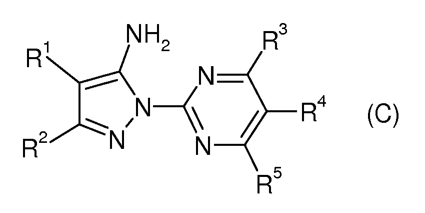 R³, R⁴, R⁵ unabhängig voneinander Wasserstoff, Halogen, Cyano, Hydroxy, Mercapto, C₁-C₁₀-Alkyl, C₁-C₁₀-Halogenalkyl, C₃-C₈-Cycloalkyl, C₂-C₁₀-Alkenyl, C₂-C₁₀-Alkinyl, C₁-C₆-Alkoxy, C₁-C₆-Alkylthio, C₁-C₆-Alkoxy-C₁-C₆-alkyl, NR^{A}R^{B}, Phenyl, Phenoxy oder Phenylthio
R^{A}, R^{B} Wasserstoff oder C₁-C₆-Alkyl

Gegenstand der vorliegenden Erfindung sind neue 4-Phenyl-1H-pyrazole der Formel (I), 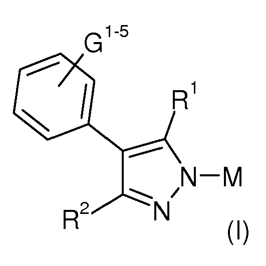 in welcher
M für eine der nachstehend aufgeführten Gruppierungen steht:

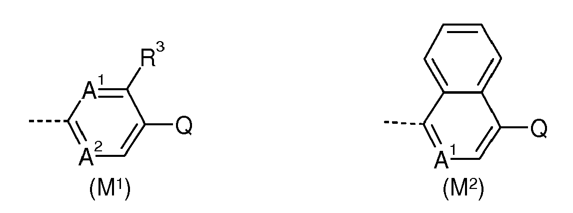

Hieraus ergeben sich 4-Phenyl-1H-pyrazole der Formel (I-A) und (I-B), 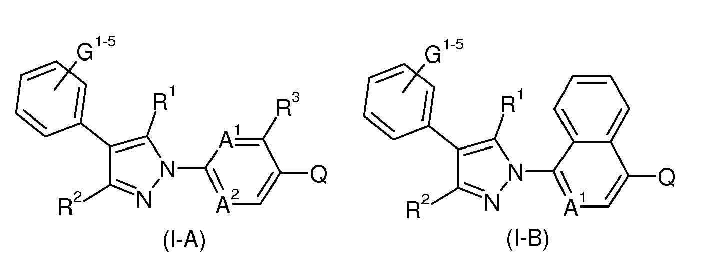 in welcher
A¹ und A² unabhängig voneinander für Stickstoff oder C-R⁴ stehen;
G¹, G², G³, G⁴ und G⁵ für Wasserstoff oder einen Substituenten stehen, unabhängig voneinander ausgewählt aus: Halogen, Alkyl, Alkenyl, Alkinyl, Haloalkyl, SF₅, Hydroxy, Alkoxy, Alkoxyalkyl, Alkoxyalkoxy, Cycloalkyl, Alkenyloxy, Alkinyloxy, Cycloalkylalkoxy, Haloalkoxy, Haloalkoxyalkyl, Alkylsulfanyl, Haloalkylsulfanyl, Alkylsulfinyl, Haloalkylsulfinyl, Alkylsulfonyl, Haloalkylsulfonyl, Cyano, Alkylcarbonyl, Alkoxycarbonyl, Alkoxycarbonylalkyl, Carboxyl, Aminocarbonyl, Aminothiocarbonyl, Alkylaminocarbonyl, Dialkylaminocarbonyl, Trialkylsilyl, Nitro, Amino, Alkylamino, Dialkylamino, Alkylsulfonylamino, Dialkylsulfonylamino, -CR⁵=NO-R⁵, -CR⁵=NO-Haloalkyl oder Formyl, wobei vicinale Alkyl-, Haloalkyl, Alkoxy- und/oder Haloalkoxygruppen zusammen mit den Kohlenstoffatomen, an die sie gebunden sind, ein fünf- bis sechsgliedriges cyclisches System bilden können, das 0 bis 2 Sauerstoffatome enthält, und dessen Alkylanteil gegebenenfalls mit einem oder mehreren Fluoratomen substituiert sein kann;
Q für einen Substituenten steht, ausgewählt aus Q¹, Q², Q³ oder Q⁴;
Q¹ für eine der nachstehend aufgeführten heterocyclischen Gruppierungen steht; 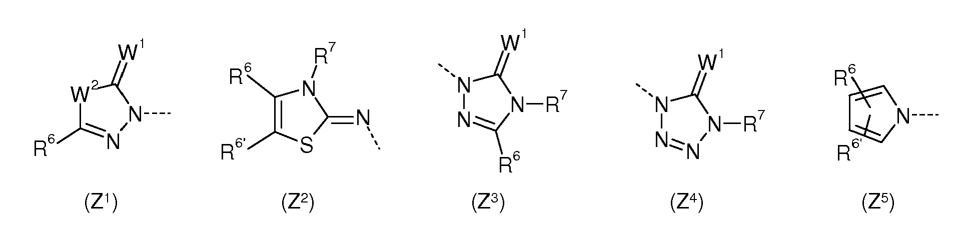 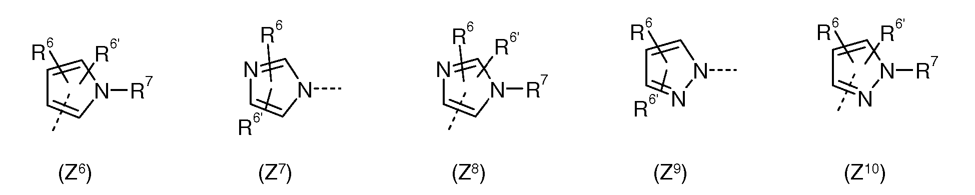 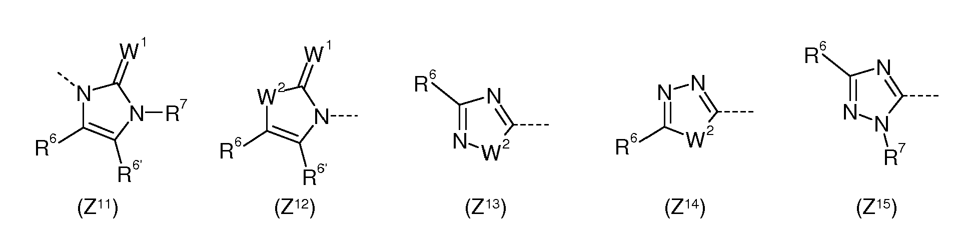 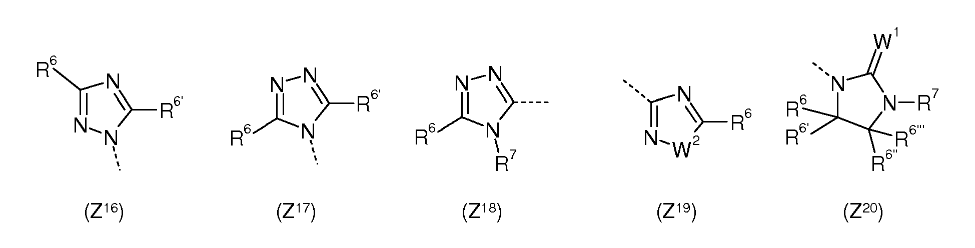 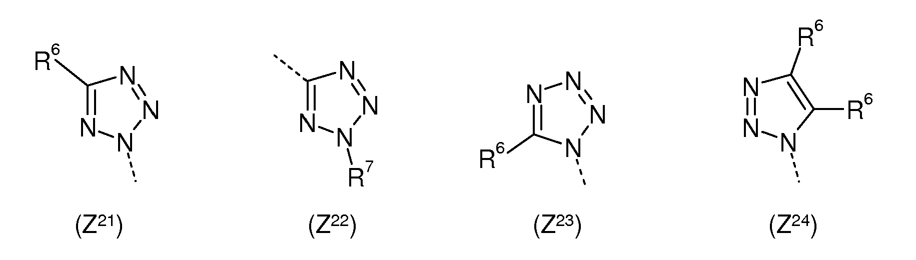 wobei
W¹ und W² unabhängig voneinander für Sauerstoff oder Schwefel stehen,
und
R⁶, R^{6'}, R^{6"}, R^{6"'} und R⁷ die unten stehende Bedeutung haben;
Q² für C(W¹)NR⁸R⁹ steht;
Q³ für C(R¹⁰R¹¹)NR⁸R⁹ steht;
Q⁴ für Cyano (wobei R¹ nicht für Amino steht), Nitro, Amino, COOH, COOR¹², Fluor (falls R³ verschieden von Chlor ist), Chlor (falls R³ verschieden von Chlor, COOH, CH₂CH₂OMe und OMe ist), Brom, Iod, SR¹² (wobei R¹ nicht für Amino steht, falls R¹² für Alkyl steht), S(O)R¹², S(O)₂R¹² oder S(O)₂NR⁸R⁹ steht;
R¹ für Wasserstoff, Halogen, Alkylsulfanyl, Alkylsulfinyl, Alkylsulfonyl, Cyano, Amino, Z¹⁶ oder NR¹³R¹⁴ steht;
R² für Wasserstoff, Halogen, Alkyl, Alkenyl, Alkinyl, Haloalkyl, Cycloalkyl, Hydroxyalkyl, Alkoxyalkyl, Haloalkoxyalkyl, Alkoxyhaloalkyl, Alkylsulfanylalkyl, Alkylsulfinylalkyl, Alkylsulfonylalkyl, Haloalkylsulfanylalkyl, Haloalkylsulfinylalkyl, Haloalkylsulfonylalkyl Cycloalkylalkyl, Phenylalkyl, Heteroarylalkyl, Heterocyclylalkyl, Phenyl, oder auch für Arylhaloalkyl, Haloalkyloxyhaloalkyloxyhaloalkyl, Heterocyclyl, Alkylsulfanyl, Alkylsulfinyl, Alkylsulfonyl oder steht;
wobei ein Phenyl- oder Heteroarylrest gegebenenfalls ein- oder mehrfach substituiert ist durch Halogen, Alkyl, Haloalkyl, Alkoxy, Nitro oder Cyano; ein Heterocyclylrest gegebenenfalls substituiert ist durch -OH / =O, -SH / =S, -NH₂, Halogen, Alkyl, Haloalkyl, Alkoxy, Alkylsulfanyl, Alkylsulfinyl, Alkylsulfonyl, Monoalkylamino, Dialkylamino, Nitro oder Cyano; und ein an Phenyl, Heteroaryl oder Heterocyclyl gebundener Alkandiyl-Rest gegebenenfalls ein- oder mehrfach substituiert ist durch Halogen, Alkyl, Haloalkyl, Alkoxy oder Cyano.
R³ für Halogen, Alkyl, Alkenyl, Alkinyl, Haloalkyl, Cycloalkyl, Alkoxyalkyl, Alkylcarbonyloxyalkyl, Alkylsulfanylalkyl, Alkylsulfinylalkyl, Alkylsulfonylalkyl, Hydroxy, Z¹⁶, Alkoxy, Acylamino, Alkoxycarbonylamino, Alkenyloxy, Alkinyloxy, Cycloalkylalkoxy, Haloalkoxy, Alkylsulfanyl, Haloalkylsulfanyl, Alkylsulfinyl, Haloalkylsulfinyl, Alkylsulfonyl, Haloalkylsulfonyl, Cyano, Alkylcarbonyl, Alkoxycarbonyl, Alkoxycarbonylalkyl, Carboxyl, Aminocarbonyl, Aminothiocarbonyl, Alkylaminocarbonyl, Dialkylaminocarbonyl, Alkylsulfonylaminocarbonyl, Trialkylsilyl, Nitro, Amino, Alkylamino, Dialkylamino, Alkylsulfonylamino, Dialkylsulfonylamino, -CR⁵=NO-R⁵, -CR⁵=NO-Haloalkyl oder Formyl steht;
R⁴ für Wasserstoff, Cyano, Nitro, Alkyl, Haloalkyl, Halogen oder Alkoxy steht;
R⁵ für Wasserstoff oder Alkyl steht;
R⁶, R^{6'}, R^{6"}, R^{6'''} unabhängig voneinander für Wasserstoff, Amino, Hydroxy, Mercapto, Nitro, Cyano, Carboxyl, Halogen, Alkyl, Haloalkyl, Alkenyl, Alkinyl, Cycloalkyl, Alkoxy, Haloalkoxy, Alkenyloxy, Alkinyloxy, Alkoxycarbonyl, Alkylsulfanyl, Haloalkylsulfanyl, Alkylsulfinyl, Haloalkylsulfinyl, Alkylsulfonyl, Haloalkylsulfonyl, Aminocarbonyl, Aminothiocarbonyl, CR⁵=NO-R⁵, -CR⁵=NO-Haloalkyl, Formyl, Alkylamino, Dialkylamino, Phenyl, Heteroaryl, Heteroarylalkyl oder Heterocyclylalkyl stehen;
wobei ein Phenyl- oder Heteroarylrest gegebenenfalls ein- oder mehrfach substituiert ist durch Halogen, Alkyl, Haloalkyl, Alkoxy, Nitro oder Cyano; ein Heterocyclylrest gegebenenfalls substituiert ist durch -OH / =O, -SH / =S, -NH₂, Halogen, Alkyl, Haloalkyl, Alkoxy, Alkylsulfanyl, Alkylsulfinyl, Alkylsulfonyl, Monoalkylamino, Dialkylamino, Nitro oder Cyano; und ein an Phenyl, Heteroaryl oder Heterocyclyl gebundener Alkandiyl-Rest gegebenenfalls ein- oder mehrfach substituiert ist durch Halogen, Alkyl, Haloalkyl, Alkoxy oder Cyano;
R⁷ für Wasserstoff, Amino, Hydroxy, Cyano, Alkyl, Haloalkyl, Alkenyl, Alkinyl, Cycloalkyl, Alkoxy, Alkenyloxy, Alkinyloxy, Alkoxycarbonyl, Alkylcarbonyl, Alkylaminocarbonyl, Dialkylaminocarbonyl, Phenyl, Phenylalkyl, Heteroarylalkyl oder Heterocyclylalkyl steht;
wobei ein Phenyl- oder Heteroarylrest gegebenenfalls ein- oder mehrfach substituiert ist durch Halogen, Alkyl, Haloalkyl, Alkoxy, Nitro oder Cyano; ein Heterocyclylrest gegebenenfalls substituiert ist durch -OH / =O, -SH / =S, -NH₂, Halogen, Alkyl, Haloalkyl, Alkoxy, Alkylsulfanyl, Alkylsulfinyl, Alkylsulfonyl, Monoalkylamino, Dialkylamino, Nitro oder Cyano; und ein an Phenyl, Heteroaryl oder Heterocyclyl gebundener Alkandiyl-Rest gegebenenfalls ein- oder mehrfach substituiert ist durch Halogen, Alkyl, Haloalkyl, Alkoxy oder Cyano und
wobei gegebenenfalls zwei benachbarte Reste aus R⁶, R^{6'}, R^{6"}, R^{6'''} und R⁷ zusammen für gegebenenfalls substituiertes und/oder gegebenenfalls durch Sauerstoff oder Schwefel oder eine Gruppierung aus der Reihe -S(O)-, -SO₂-, -NH- oder -N-Alkyl- am Anfang (bzw. am Ende) oder innerhalb der Kohlenwasserstoffkette unterbrochenes Alkandiyl oder Alkendiyl mit jeweils 2 bis 5 Kohlenstoffatomen stehen;
R⁸ und R⁹ unabhängig voneinander für Wasserstoff, Alkyl, Haloalkyl, Alkenyl (gegebenenfalls substituiert durch Halogen, Alkyl, Haloalkyl oder Cyano), Alkinyl, Cycloalkyl (am Cyclus gegebenenfalls ein- oder mehrfach substituiert durch Halogen, Haloalkyl, Alkyl oder kondensiert an einen Aromaten oder Heteroaromaten), Cycloalkylalkyl (am Cyclus gegebenenfalls ein- oder mehrfach substituiert durch Halogen, Haloalkyl, Alkyl oder kondensiert an einen Aromaten oder Heteroaromaten, am Alkylteil gegebenenfalls ein- oder mehrfach substituiert durch Halogen, Alkyl, Haloalkyl, Alkoxy oder Cyano), Cycloalkylcarbonylheterocyclyl, Alkyloxycarbonylheterocyclyl, Alkylsulfinylalkyl, Alkylsulfanylalkyl, Alkylsulfonylalkyl, Hydroxyalkyl, Heterocyclylcarbonylalkyl, Heteroarylcarbonylaminoalkyl, Haloalkoxyalkyl, Alkylthiocarbonyl, Dialkylaminocarbonyl, Alkylaminocarbonyl, Haloalkylaminocarbonyl, Alkoxy, Alkenyloxy, Alkinyloxy, Alkoxyalkyl, Alkylcarbonylalkyl, C(R¹⁰R¹¹)CR⁵=NO-R⁵, Alkylsulfonyl, Alkylcarbonyl, Haloalkylcarbonyl, Alkenylcarbonyl, Alkinylcarbonyl, Cycloalkylcarbonyl, Alkoxycarbonyl, Alkenyloxycarbonyl, Alkinyloxycarbonyl, Alkoxyalkylcarbonyl, Phenylsulfonyl, Phenyl, Heteroaryl, Heterocyclyl, Phenylalkyl, Heteroarylalkyl, Heterocyclylalkyl, Phenylcarbonyl, Heteroarylcarbonyl, Heterocyclylcarbonyl, Phenylalkylcarbonyl, Heteroarylalkylcarbonyl, Heterocyclylalkylcarbonyl, Phenoxycarbonyl oder Phenylalkoxycarbonyl stehen;
wobei ein Phenyl- oder Heteroarylrest gegebenenfalls ein- oder mehrfach substituiert ist durch Halogen, Alkyl, Haloalkyl, Alkoxy, Nitro oder Cyano; ein Heterocyclylrest gegebenenfalls substituiert ist durch -OH / =O, -SH / =S, -NH₂, Halogen, Alkyl, Haloalkyl, Alkoxy, Alkylsulfanyl, Alkylsulfinyl, Alkylsulfonyl, Monoalkylamino, Dialkylamino, Nitro oder Cyano; und ein an Phenyl, Heteroaryl oder Heterocyclyl gebundener Alkandiyl-Rest gegebenenfalls ein- oder mehrfach substituiert ist durch Halogen, Alkyl, Haloalkyl, Alkoxy oder Cyano und
wobei gegebenenfalls R⁸ / R⁹ zusammen für gegebenenfalls substituiertes und/oder gegebenenfalls durch Sauerstoff oder Schwefel oder eine Gruppierung aus der Reihe -S(O)-, -SO₂-, -NH- oder -N-Alkyl- am Anfang (bzw. am Ende) oder innerhalb der Kohlenwasserstoffkette unterbrochenes Alkandiyl oder Alkendiyl mit jeweils 2 bis 5 Kohlenstoffatomen stehen;
R¹⁰ und R¹¹ unabhängig voneinander für Wasserstoff, Alkyl, Haloalkyl oder Cyano stehen;
R¹² für Alkyl oder Haloalkyl steht;
R¹³ und R¹⁴ unabhängig voneinander für Wasserstoff, Alkyl, Haloalkyl, Alkenyl, Alkinyl, Alkoxyalkyl, Alkylcarbonylalkyl, C(R¹⁰R¹¹)CR⁵=NO-R⁵, Alkylsulfonyl, Alkylcarbonyl, Haloalkylcarbonyl, Alkenylcarbonyl, Alkinylcarbonyl, Cycloalkylcarbonyl, Alkoxycarbonyl, Alkenyloxycarbonyl, Alkinyloxycarbonyl, Alkoxyalkylcarbonyl, Phenylsulfonyl, Phenyl, Heteroaryl, Heterocyclyl, Phenylalkyl, Heteroarylalkyl, Heterocyclylalkyl, Phenylcarbonyl, Heteroarylcarbonyl, Heterocyclylcarbonyl, Phenylalkylcarbonyl, Heteroarylalkylcarbonyl, Heterocyclylalkylcarbonyl, Phenoxycarbonyl oder Phenylalkoxycarbonyl stehen;
wobei ein Phenyl- oder Heteroarylrest gegebenenfalls ein- oder mehrfach substituiert ist durch Halogen, Alkyl, Haloalkyl, Alkoxy, Nitro oder Cyano; ein Heterocyclylrest gegebenenfalls substituiert ist durch -OH / =O, -SH / =S, -NH₂, Halogen, Alkyl, Haloalkyl, Alkoxy, Alkylsulfanyl, Alkylsulfinyl, Alkylsulfonyl, Monoalkylamino, Dialkylamino, Nitro oder Cyano; und ein an Phenyl, Heteroaryl oder Heterocyclyl gebundener Alkandiyl-Rest gegebenenfalls ein- oder mehrfach substituiert ist durch Halogen, Alkyl, Haloalkyl, Alkoxy oder Cyano
oder
R¹³ und R¹⁴ als Imin die Gruppierung =CR⁵-NR¹⁵R¹⁶ oder =CR⁵-OR¹² bilden;
R¹⁵ und R¹⁶ unabhängig voneinander für Alkyl stehen oder
R¹⁵ und R¹⁶ zusammen für gegebenenfalls substituiertes und/oder gegebenenfalls durch Sauerstoff oder Schwefel oder eine Gruppierung aus der Reihe -S(O)-, -SO₂-, -NH- oder -N-Alkyl- am Anfang (bzw. am Ende) oder innerhalb der Kohlenwasserstoffkette unterbrochenes Alkandiyl oder Alkendiyl mit jeweils 2 bis 5 Kohlenstoffatomen stehen;
und die Verbindungen der allgemeinen Formel (I) außerdem N-Oxide, Salze, Tautomere, Diastereomere und optische Isomere umfassen.

Schließlich wurde gefunden, dass die erfindungsgemäßen Verbindungen der Formel (I) sehr gute insektizide und parasitizide Eigenschaften besitzen und sich im Pflanzenschutz, in der Veterinärhygiene, im Haushalt und im Materialschutz zur Bekämpfung unerwünschter Schädlinge, wie Insekten, Endo- oder Ektoparasiten, verwenden lassen.

Durch Halogen substituierte Reste, z.B. Haloalkyl, sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können einfach oder mehrfach substituiert sein, wobei bei Mehrfachsubstitutionen die Substituenten gleich oder verschieden sein können.

Die erfindungsgemäßen 4-Phenyl-1H-pyrazole sind durch Formel (I) allgemein definiert. Bevorzugte, besonders bevorzugte, ganz besonders bevorzugte und insbesonders bevorzugte Restedefinitionen der vorstehenden und nachfolgend genannten Formeln sind im Folgenden angegeben. Diese Definitionen gelten für die Endprodukte der Formel (I) wie für alle Zwischenprodukte gleichermaßen.

A¹ und A² stehen unabhängig voneinander bevorzugt jeweils für Stickstoff, C-H, C-Halogen, C-(C₁-C₆-Haloalkyl), C-(C₁-C₆-Alkoxy), C-Cyano oder C-(C₁-C₆-Alkyl);
A¹ und A² stehen unabhängig voneinander besonders bevorzugt für Stickstoff, C-H, C-Halogen oder C-(C₁-C₄-Haloalkyl);
A¹ und A² stehen unabhängig voneinander ganz besonders bevorzugt für Stickstoff oder C-H;
A¹ und A² stehen insbesonders bevorzugt für C-H; oder
A¹ und A² stehen insbesonders bevorzugt für Stickstoff; oder
A¹ steht insbesonders bevorzugt für Stickstoff und A² insbesonders bevorzugt für C-H; oder
A¹ steht insbesonders bevorzugt für C-H und A² insbesonders bevorzugt für Stickstoff;
G¹, G², G³, G⁴, G⁵ stehen bevorzugt für Wasserstoff oder eine Kombination von Substituenten, unabhängig voneinander ausgewählt aus: Halogen, C₁-C₆-Alkyl, C₂-C₆-Alkenyl, C₂-C₆-Alkinyl, C₁-C₆-Haloalkyl, SF₅, Hydroxy, C₁-C₆-Alkoxy, C₁-C₆-Alkoxy-C₁-C₆-alkyl, C₁-C₆-Alkoxy-C₁-C₆-alkoxy, C₃-C₆-Cycloalkyl, C₂-C₆-Alkenyloxy, C₂-C₆-Alkinyloxy, C₃-C₆-Cycloalkyl-C₁-C₆-alkoxy, C₁-C₆-Haloalkoxy, C₁-C₆-Haloalkoxy-C₁-C₆-alkyl, C₁-C₆-Alkylsulfanyl, C₁-C₆-Haloalkylsulfanyl, C₁-C₆-Alkylsulfinyl, C₁-C₆-Haloalkylsulfinyl, C₁-C₆-Alkylsulfonyl, C₁-C₆-Haloalkylsulfonyl, Cyano, C₁-C₆-Alkylcarbonyl, C₁-C₆-Alkoxycarbonyl, C₁-C₆-Alkoxycarbonyl-C₁-C₆-alkyl, Carboxyl, Aminocarbonyl, Aminothiocarbonyl, C₁-C₆-Alkylaminocarbonyl, C₁-C₆-Dialkylaminocarbonyl, C₁-C₆-Trialkylsilyl, Nitro, Amino, C₁-C₆-Alkylamino, C₁-C₆-Dialkylamino, C₁-C₆-Alkylsulfonylamino, C₁-C₆-Dialkylsulfonylamino, -CR⁵=NO-C₁-C₆-Alkyl, -CR⁵=NO-Halo-C₁-C₆-alkyl oder Formyl, wobei vicinale C₁-C₆-Alkyl-, C₁-C₆-Haloalkyl, C₁-C₆-Alkoxy- und/oder C₁-C₆-Haloalkoxygruppen zusammen mit den Kohlenstoffatomen, an die sie gebunden sind, ein fünf- bis sechsgliedriges cyclisches System bilden können, das 0 bis 2 Sauerstoffatome enthält, und dessen Alkandiyl-Rest gegebenenfalls mit einem oder mehreren Fluoratomen substituiert sein kann;
G¹, G², G³, G⁴, G⁵ stehen besonders bevorzugt für Wasserstoff oder eine Kombination von bis zu drei Substituenten, unabhängig voneinander ausgewählt aus: Halogen, C₁-C₄-Alkyl, C₂-C₄-Alkenyl, C₂-C₄-Alkinyl, C₁-C₄-Haloalkyl, SF₅, Hydroxy, C₁-C₄-Alkoxy, C₁-C₄-Alkoxy-C₁-C₄-alkyl, C₁-C₄-Alkoxy-C₁-C₄-alkoxy, C₃-C₄-Cycloalkyl, C₂-C₄-Alkenyloxy, C₂-C₄-Alkinyloxy, C₃-C₄-Cycloalkyl-C₁-C₄-alkoxy, C₁-C₄-Haloalkoxy, C₁-C₄-Haloalkoxy-C₁-C₄-alkyl, C₁-C₄-Alkylsulfanyl, C₁-C₄-Haloalkylsulfanyl, C₁-C₄-Alkylsulfinyl, C₁-C₄-Haloalkylsulfinyl, C₁-C₄-Alkylsulfonyl, C₁-C₄-Haloalkylsulfonyl, Cyano, C₁-C₄-Alkylcarbonyl, C₁-C₄-Alkoxycarbonyl, C₁-C₄-Alkoxycarbonyl-C₁-C₄-alkyl, Carboxyl, Aminocarbonyl, Aminothiocarbonyl, C₁-C₄-Alkylaminocarbonyl, C₁-C₄-Dialkylaminocarbonyl, C₁-C₄-Trialkylsilyl, Nitro, Amino, C₁-C₄-Alkylamino, C₁-C₄-Dialkylamino, C₁-C₄-Alkylsulfonylamino, C₁-C₄-Dialkylsulfonylamino, -CR⁵=NO-C₁-C₄-Alkyl, -CR⁵=NO-Halo-C₁-C₄-alkyl oder Formyl, wobei vicinale C₁-C₄-Alkyl-, C₁-C₄-Haloalkyl, C₁-C₄-Alkoxy- und/oder C₁-C₄-Haloalkoxygruppen zusammen mit den Kohlenstoffatomen, an die sie gebunden sind, ein fünf- bis sechsgliedriges cyclisches System bilden können, das 0 bis 2 Sauerstoffatome enthält, und dessen Alkandiyl-Rest gegebenenfalls mit einem oder mehreren Fluoratomen substituiert sein kann;
G¹, G², G, G⁴, G⁵ stehen ganz besonders bevorzugt für eine 3-Substitution, 4-Substitution, 3,4-Disubstitution, 3,5-Disubstitution oder 3,4,5-Trisubstitution, wobei die Substituenten G¹, G², G³, G⁴ und G⁵ unabhängig voneinander ausgewählt werden aus: Wasserstoff, Halogen, C₁-C₂-Alkyl, C₁-C₂-Haloalkyl, C₁-C₂-Alkoxy, C₁-C₂-Alkoxy-C₁-C₂-alkyl, C₁-C₂-Alkoxy-C₁-C₂-alkoxy, C₃-C₆-Cycloalkyl-C₁-C₂-alkoxy, C₁-C₂-Haloalkoxy, C₁-C₂-Alkylsulfanyl, C₁-C₂-Haloalkylsulfanyl, C₁-C₂-Alkylsulfinyl, C₁-C₂-Haloalkylsulfinyl, C₁-C₂-Alkylsulfonyl, C₁-C₂-Haloalkylsulfonyl, Cyano, C₁-C₂-Alkylcarbonyl, C₁-C₂-Alkoxycarbonyl, Carboxyl, Aminocarbonyl, Aminothiocarbonyl, C₁-C₂-Alkylaminocarbonyl, C₁-C₂-Dialkylaminocarbonyl, Nitro, Amino, C₁-C₂-Alkylamino, C₁-C₂-Dialkylamino, C₁-C₂-Alkylsulfonylamino, C₁-C₂-Dialkylsulfonylamino;
G¹, G², G³, G⁴, G⁵ stehen insbesonders bevorzugt für eine 2-Chlorsubstitution, 3-Chlorsubstitution, 3-Bromsubstitution, 3-Fluorsubstitution, 4-Bromsubstitution, 4-Chlorsubstitution, 3,4-Dichlorsubstitution, 3,4-Difluorsubstitution, 3-Chlor-4-fluorsubstitution, 3-Fluor-4-chlorsubstitution, 3-Fluor-5-chlorsubstitution, 3-Chlor-5-trifluormethylsubstitution, 3,5-Dichlorsubstitution, 3,5-Dimethylsubstitution oder 3,5-Bistrifluormethylsubstitution;
Q¹ steht bevorzugt für eine der nachstehend aufgeführten heterocyclischen Gruppierungen; 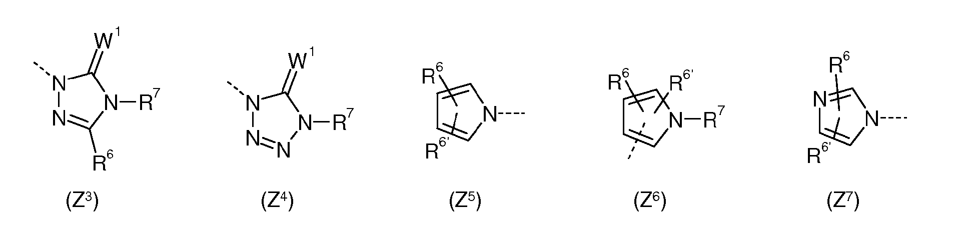 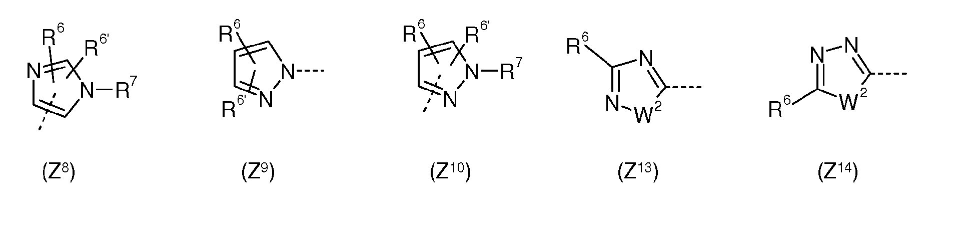 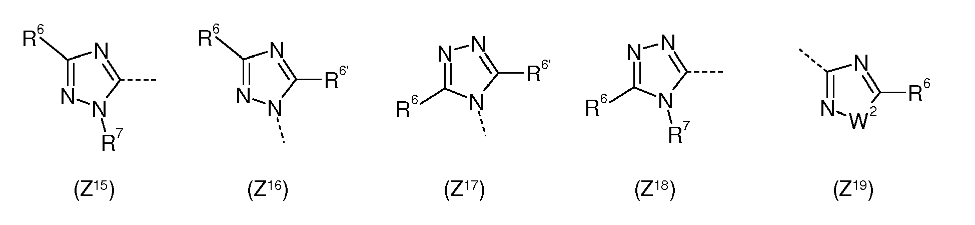 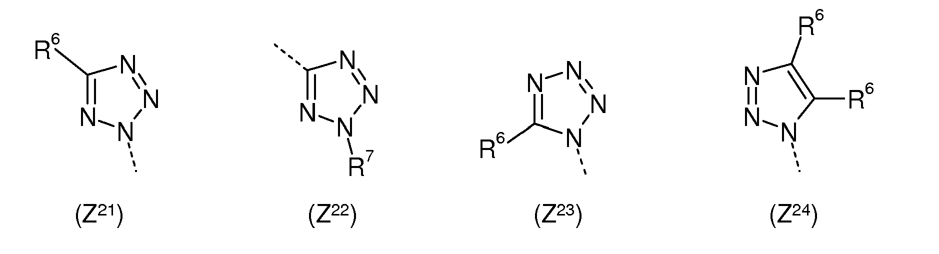 Q¹ steht besonders bevorzugt für eine der nachstehend aufgeführten heterocyclischen Gruppierungen; 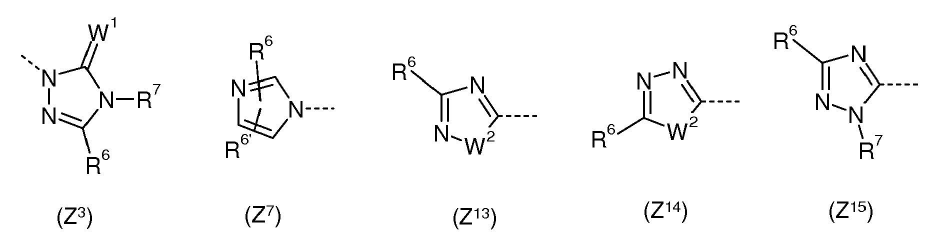 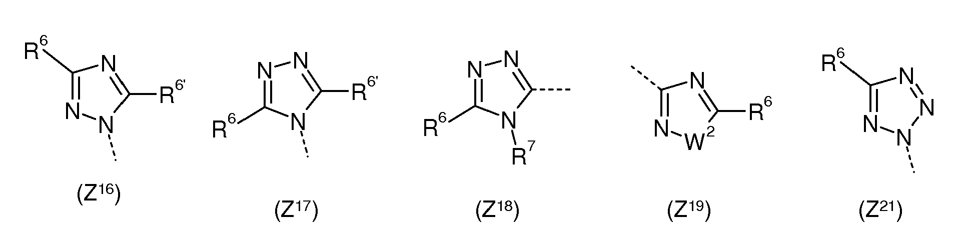 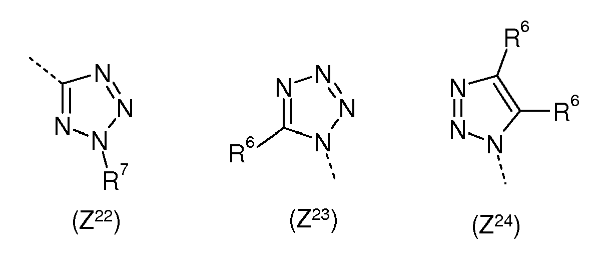 Q¹ steht ganz besonders bevorzugt für eine der nachstehend aufgeführten heterocyclischen Gruppierungen; 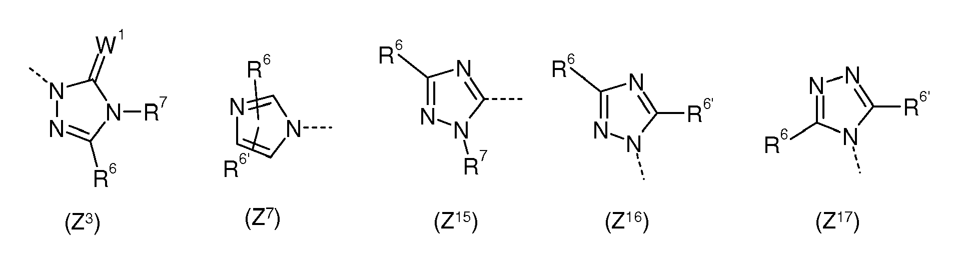 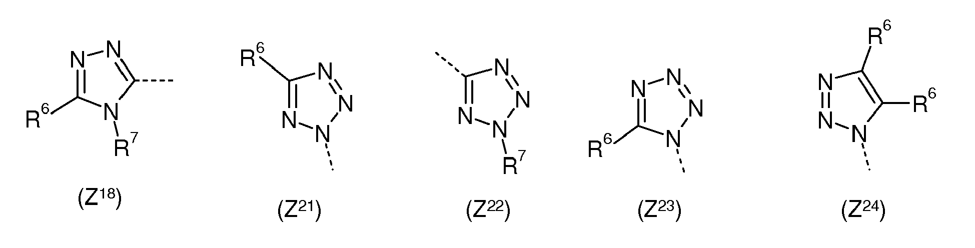 Q¹ steht insbesonders bevorzugt für eine der nachstehend aufgeführten heterocyclischen Gruppierungen; 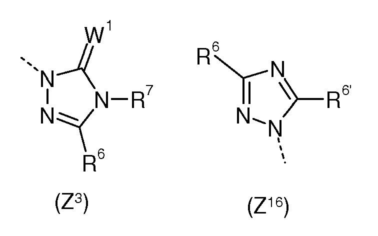 wobei
W¹ bevorzugt für Sauerstoff oder Schwefel steht;
W¹ ganz besonders bevorzugt für Sauerstoff steht;
W² bevorzugt für Sauerstoff oder Schwefel steht;
W² ganz besonders bevorzugt für Sauerstoff steht; und
R⁶, R^{6'}, R^{6"}, R^{6'''} und R⁷ die unten stehende Bedeutung haben;
Q² steht bevorzugt für C(O)NR⁸R⁹;
Q³ steht bevorzugt für C(R¹⁰R¹¹)NR⁸R⁹;
Q⁴ steht bevorzugt für Cyano (wobei R¹ nicht für Amino steht), COOH, COOR¹², Fluor (falls R³ verschieden von Chlor ist), Chlor (falls R³ verschieden von Chlor, COOH, CH₂CH₂OMe und OMe ist), Brom, Iod, SR¹² (wobei R¹ nicht für Amino steht, falls R¹² für Alkyl steht), S(O)R¹², S(O)₂R¹² oder S(O)₂NR⁸R⁹;
Q⁴ steht besonders bevorzugt für Cyano (wobei R¹ nicht für Amino steht), COOH, COOMe, COOEt, Fluor (falls R³ verschieden von Chlor ist ), Chlor (falls R³ verschieden von Chlor, COOH, CH₂CH₂OMe und OMe ist), Brom, Iod, SR¹² (wobei R¹ nicht für Amino steht, falls R¹² für Alkyl steht), S(O)R¹², S(O)₂R¹² oder S(O)₂NR⁸R⁹;
Q⁴ steht ganz besonders bevorzugt für Cyano (wobei R¹ nicht für Amino steht), COOH, COOMe, COOEt, Fluor (falls R³ verschieden von Chlor ist), Brom, Iod, SR¹² (wobei R¹ nicht für Amino steht, falls R¹² für Alkyl steht), S(O)R¹², S(O)₂R¹² oder S(O)₂NR⁸R⁹;
Q⁴ steht insbesonders bevorzugt für Cyano (wobei R¹ nicht für Amino steht), COOH, COOMe, COOEt, Fluor (falls R³ verschieden von Chlor ist), Brom, Iod, SR¹² (wobei R¹ nicht für Amino steht, falls R¹² für Alkyl steht), S(O)R¹² oder S(O)₂R¹²;
R¹ steht bevorzugt für Wasserstoff, Halogen, C₁-C₄-Alkylsulfanyl, C₁-C₄-Alkylsulfinyl, C₁-C₄-Alkylsulfonyl, Z¹⁶, Amino oder NR¹³R¹⁴;
R¹ steht besonders bevorzugt für Wasserstoff, Fluor, Chlor, Brom, Iod, Z¹⁶, Amino oder NR¹³R¹⁴;
R¹ steht ganz besonders bevorzugt für Chlor, Brom, Iod, Z¹⁶, Amino oder NR¹³R¹⁴;
R¹ steht insbesonders bevorzugt für Amino oder NR¹³R¹⁴;
R² steht bevorzugt für Wasserstoff, Halogen, C₁-C₆-Alkyl, C₁-C₆-Haloalkyl, C₁-C₆-Alkoxy-C₁-C₆-alkyl, C₁-C₆-Haloalkoxy-C₁-C₆-alkyl, C₁-C₆-Alkoxy-C₁-C₆-haloalkyl, C₁-C₆-Alkylsulfanyl-C₁-C₆-alkyl, C₁-C₆-Alkylsulfinyl-C₁-C₆-alkyl, C₁-C₆-Alkylsulfonyl-C₁-C₆-alkyl, C₁-C₆-Haloalkylsulfanyl-C₁-C₆-alkyl, C₁-C₆-Haloalkylsulfinyl-C₁-C₆-alkyl, C₁-C₆-Haloalkylsulfonyl-C₁-C₆-alkyl, C₃-C₆-Cycloalkyl-C₁-C₆-alkyl, Phenyl-C₁-C₆-alkyl, Heteroaryl-C₁-C₆-alkyl, Hetero-C₃-C₆-cyclyl-C₁-C₆-alkyl, Aryl-C₁-C₆-haloalkyl, C₁-C₆-Haloalkyloxy-C₁-C₆-haloalkyloxy-C₁-C₆-haloalkyl, C₃-C₆-Heterocyclyl, C₁-C₆-Alkylsulfanyl, C₁-C₆-Alkylsulfinyl, C₁-C₆-Alkylsulfonyl oder Phenyl;
wobei ein Phenyl- oder Heteroarylrest gegebenenfalls ein- oder mehrfach substituiert ist durch Halogen, C₁-C₆-Alkyl, C₁-C₆-Haloalkyl, C₁-C₆-Alkoxy, Nitro oder Cyano; ein Heterocyclylrest gegebenenfalls substituiert ist durch -OH / =O, -SH / =S, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, C₁-C₆-Monoalkylamino, C₁-C₆-Dialkylamino, Nitro oder Cyano; und ein an Phenyl, Heteroaryl oder Heterocyclyl gebundener Alkandiyl-Rest gegebenenfalls ein- oder mehrfach substituiert ist durch Halogen, C₁-C₆-Alkyl, C₁-C₆-Haloalkyl, C₁-C₆-Alkoxy oder Cyano;
R² steht besonders bevorzugt für Wasserstoff, Halogen, C₁-C₄-Alkyl, C₁-C₄-Haloalkyl, C₁-C₄-Alkoxy-C₁-C₄-alkyl, C₁-C₄-Haloalkoxy-C₁-C₄-alkyl, C₁-C₄-Alkoxy-C₁-C₄-haloalkyl, C₁-C₄-Alkylsulfanyl-C₁-C₄-alkyl, C₁-C₄-Alkylsulfinyl-C₁-C₄-alkyl, C₁-C₄-Alkylsulfonyl-C₁-C₄-alkyl, C₁-C₄-Haloalkylsulfanyl-C₁-C₄-alkyl, C₁-C₄-Haloalkylsulfinyl-C₁-C₄-alkyl, C₁-C₄-Haloalkylsulfonyl-C₁-C₄-alkyl, C₃-C₄-Cycloalkyl-C₁-C₄-alkyl, Phenyl-C₁-C₄-alkyl, Heteroaryl-C₁-C₄-alkyl, Hetero-C₃-C₄-cyclyl-C₁-C₄-alkyl, Aryl-C₁-C₄-haloalkyl, C₁-C₄-Haloalkyloxy-C₁-C₄-haloalkyloxy-C₁-C₄-haloalkyl, C₃-C₄-Heterocyclyl, C₁-C₄-Alkylsulfanyl, C₁-C₄-Alkylsulfinyl, C₁-C₄-Alkylsulfonyl oder Phenyl;
wobei ein Phenyl- oder Heteroarylrest gegebenenfalls ein- oder mehrfach substituiert ist durch Halogen, C₁-C₄-Alkyl, C₁-C₄-Haloalkyl, C₁-C₄-Alkoxy, Nitro oder Cyano; ein Heterocyclylrest gegebenenfalls substituiert ist durch -OH / =O, -SH / =S, -NH₂, Halogen, C₁-C₄-Alkyl, C₁-C₄-Haloalkyl, C₁-C₄-Alkoxy, C₁-C₄-Alkylsulfanyl, C₁-C₄-Alkylsulfinyl, C₁-C₄-Alkylsulfonyl, C₁-C₄-Monoalkylamino, C₁-C₄-Dialkylamino, Nitro oder Cyano; und ein an Phenyl, Heteroaryl oder Heterocyclyl gebundener Alkandiyl-Rest gegebenenfalls einoder mehrfach substituiert ist durch Halogen, C₁-C₄-Alkyl, C₁-C₄-Haloalkyl, C₁-C₄-Alkoxy oder Cyano.
R² steht ganz besonders bevorzugt für Fluor, Chlor, Brom, Iod, C₁-C₄-Alkoxy-C₁-C₄-alkyl, C₁₋₄-Alkyl, Aryl-C₁-C₄-haloalkyl, C₁-C₆-Alkylsulfanyl, C₁-C₆-Alkylsulfinyl, C₁-C₆-Alkylsulfonyl oder C₁₋₄-Haloalkyl;
R² steht insbesonders bevorzugt für C₁₋₂-Haloalkyl;
R³ steht bevorzugt für Halogen, C₁-C₆-Alkyl, C₂-C₆-Alkenyl, C₂-C₆-Alkinyl, C₁-C₆-Haloalkyl, C₃-C₆-Cycloalkyl, C₁-C₆-Alkoxy-C₁-C₆-alkyl, C₁-C₆-Alkylcarbonyloxy-C₁-C₆-alkyl, C₁-C₆-Alkylsulfanyl-C₁-C₆-alkyl, C₁-C₆-Alkylsulfinyl-C₁-C₆-alkyl, C₁-C₆-Alkylsulfonyl-C₁-C₆-alkyl, Hydroxy, C₁-C₆-Alkoxy, C₁-C₆-Acylamino, C₁-C₆-Alkoxycarbonylamino, C₂-C₆-Alkenyloxy, C₂-C₆-Alkinyloxy, C₃-C₆-Cycloalkyl-C₁-C₆-alkoxy, C₁-C₆-Haloalkoxy, C₁-C₆-Alkylsulfanyl, C₁-C₆-Haloalkylsulfanyl, C₁-C₆-Alkylsulfinyl, C₁-C₆-Haloalkylsulfinyl, C₁-C₆-Alkylsulfonyl, C₁-C₆-Haloalkylsulfonyl, Cyano, C₁-C₆-Alkylcarbonyl, C₁-C₆-Alkoxycarbonyl, C₁-C₆-Alkoxycarbonyl-C₁-C₆-alkyl, Carboxyl, Aminocarbonyl, Aminothiocarbonyl, C₁-C₆-Alkylaminocarbonyl, C₁-C₆-Dialkylaminocarbonyl, C₁-C₆-Alkylsulfonylaminocarbonyl, C₁-C₆-Trialkylsilyl, Nitro, Amino, C₁-C₆-Alkylamino, C₁-C₆-Dialkylamino, C₁-C₆-Alkylsulfonylamino, C₁-C₆-Dialkylsulfonylamino, - CR⁵=NO-R⁵, -CR⁵=NO-Halo-C₁-C₆-alkyl, Z¹⁶ oder Formyl;
R³ steht besonders bevorzugt für Halogen, C₁-C₄-Alkyl, C₂-C₄-Alkenyl, C₂-C₄-Alkinyl, C₁-C₄-Haloalkyl, C₃-C₄-Cycloalkyl, C₁-C₄-Alkoxy-C₁-C₄-alkyl, C₁-C₄-Alkylcarbonyloxy-C₁-C₄-alkyl, C₁-C₄-Alkylsulfanyl-C₁-C₄-alkyl, C₁-C₄-Alkylsulfinyl-C₁-C₄-alkyl, C₁-C₄-Alkylsulfonyl-C₁-C₄-alkyl, Hydroxy, C₁-C₄-Alkoxy, C₁-C₄-Acylamino, C₁-C₄-Alkoxycarbonylamino, C₂-C₄-Alkenyloxy, C₂-C₄-Alkinyloxy, C₃-C₄-Cycloalkyl-C₁-C₄-alkoxy, C₁-C₄-Haloalkoxy, C₁-C₄-Alkylsulfanyl, C₁-C₄-Haloalkylsulfanyl, C₁-C₄-Alkylsulfinyl, C₁-C₄-Haloalkylsulfinyl, C₁-C₄-Alkylsulfonyl, C₁-C₄-Haloalkylsulfonyl, Cyano, C₁-C₄-Alkylcarbonyl, C₁-C₄-Alkoxycarbonyl, C₁-C₄-Alkoxycarbonyl-C₁-C₄-alkyl, Carboxyl, Aminocarbonyl, Aminothiocarbonyl, C₁-C₄-Alkylaminocarbonyl, C₁-C₄-Dialkylaminocarbonyl, C₁-C₄-Alkylsulfonylaminocarbonyl, C₁-C₄-Trialkylsilyl, Nitro, Amino, C₁-C₄-Alkylamino, C₁-C₄-Dialkylamino, C₁-C₄-Alkylsulfonylamino, C₁-C₄-Dialkylsulfonylamino, -CR⁵=NO-R⁵, -CR⁵=NO-Halo-C₁-C₄-alkyl, Z¹⁶ oder Formyl;
R³ steht ganz besonders bevorzugt für Halogen, C₁-C₂-Alkyl, C₁-C₂-Haloalkyl, Cyclopropyl, C₁-C₂-Alkoxy-C₁-C₂-alkyl, C₁-C₂-Alkylcarbonyloxy-C₁-C₂-alkyl, C₁-C₂-Alkylsulfanyl-C₁-C₂-alkyl, C₁-C₂-Alkylsulfinyl-C₁-C₂-alkyl, C₁-C₄-Alkylsulfonyl-C₁-C₂-alkyl, C₁-C₂-Alkoxy, C₁-C₂-Acylamino, C₁-C₂-Alkoxycarbonylamino, Cyclopropyl-C₁-C₂-alkoxy, C₁-C₂-Haloalkoxy, C₁-C₂-Alkylsulfanyl, C₁-C₂-Haloalkylsulfanyl, C₁-C₂-Alkylsulfinyl, C₁-C₂-Haloalkylsulfinyl, C₁-C₂-Alkylsulfonyl, C₁-C₂-Haloalkylsulfonyl, Cyano, C₁-C₂-Alkylcarbonyl, C₁-C₂-Alkoxycarbonyl, C₁-C₂-Alkoxycarbonyl-C₁-C₂-alkyl, Carboxyl, Aminocarbonyl, Aminothiocarbonyl, C₁-C₂-Alkylaminocarbonyl, C₁-C₂-Dialkylaminocarbonyl, C₁-C₂-Alkylsulfonylaminocarbonyl, C₁-C₂-Trialkylsilyl, Nitro, Amino, C₁-C₂-Alkylamino, C₁-C₂-Dialkylamino, C₁-C₂-Alkylsulfonylamino, C₁-C₂-Dialkylsulfonylamino, -CR⁵=NO-R⁵, -CR⁵=NO-Halo-C₁-C₂-alkyl, Z¹⁶ oder Formyl;
R³ steht insbesonders bevorzugt für Fluor, Chlor, Brom, Iod, C₁-C₂-Alkyl, C₁-C₂-Haloalkyl, C₁-C₂-Haloalkylsulfanyl, C₁-C₂-Haloalkylsulfinyl, C₁-C₂-Haloalkylsulfonyl oder Cyano;
R⁴ steht bevorzugt für Wasserstoff, Cyano, Nitro, C₁-C₆-Alkyl, C₁-C₆-Haloalkyl, Halogen oder C₁-C₆-Alkoxy;
R⁴ steht besonders bevorzugt für Wasserstoff, Cyano, Nitro, C₁-C₄-Alkyl, C₁-C₄-Haloalkyl, Halogen oder C₁-C₄-Alkoxy;
R⁴ steht ganz besonders bevorzugt für Wasserstoff, Fluor, Chlor, Brom oder Iod;
R⁴ steht insbesonders bevorzugt für Wasserstoff;
R⁵ steht bevorzugt für Wasserstoff oder C₁₋₆-Alkyl;
R⁵ steht besonders bevorzugt für Wasserstoff oder C₁₋₄-Alkyl;
R⁵ steht ganz besonders bevorzugt für Wasserstoff oder C₁₋₃-Alkyl;
R⁵ steht insbesonders bevorzugt für Wasserstoff, Methyl oder Ethyl;
R⁶, R^{6'}, R^{6"}, R^{6'''} stehen unabhängig voneinander bevorzugt für Wasserstoff, Amino, Hydroxy, Mercapto, Nitro, Cyano, Carboxyl, Halogen, C₁-C₆-Alkyl, C₁-C₆-Haloalkyl, C₂-C₆-Alkenyl, C₂-C₆-Alkinyl, C₃-C₆-Cycloalkyl, C₁-C₆-Alkoxy, C₁-C₆-Haloalkoxy, C₂-C₆-Alkenyloxy, C₂-C₆-Alkinyloxy, C₁-C₆-Alkoxycarbonyl, C₁-C₆-Alkylsulfanyl, C₁-C₆-Haloalkylsulfanyl, C₁-C₆-Alkylsulfinyl, C₁-C₆-Haloalkylsulfinyl, C₁-C₆-Alkylsulfonyl, C₁-C₆-Haloalkylsulfonyl, Aminocarbonyl, Aminothiocarbonyl, CR⁵=NO-C₁-C₆-alkyl, -CR⁵=NO-Halo-C₁-C₆-alkyl, Formyl, C₁-C₆-Alkylamino, C₁-C₆-Dialkylamino, Phenyl, Heteroaryl, Heteroaryl-C₁-C₆-alkyl oder Hetero-C₃-C₆-cyclyl-C₁-C₆-alkyl;
wobei ein Phenyl- oder Heteroarylrest gegebenenfalls ein- oder mehrfach substituiert ist durch Halogen, C₁-C₆-Alkyl, C₁-C₆-Haloalkyl, C₁-C₆-Alkoxy, Nitro oder Cyano; ein Heterocyclylrest gegebenenfalls substituiert ist durch -OH / =O, -SH / =S, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, C₁-C₆-Monoalkylamino, C₁-C₆-Dialkylamino, Nitro oder Cyano; und ein an Phenyl, Heteroaryl oder Heterocyclyl gebundener Alkandiyl-Rest gegebenenfalls einoder mehrfach substituiert ist durch Halogen, C₁-C₆-Alkyl, C₁-C₆-Haloalkyl, C₁-C₆-Alkoxy oder Cyano.
R⁶, R^{6'}, R^{6"}, R^{6'''} stehen unabhängig voneinander besonders bevorzugt für Wasserstoff, Amino, Hydroxy, Mercapto, Nitro, Cyano, Carboxyl, Halogen, C₁-C₄-Alkyl, C₁-C₄-Haloalkyl, C₂-C₄-Alkenyl, C₂-C₄-Alkinyl, C₃-C₄-Cycloalkyl, C₁-C₄-Alkoxy, C₁-C₄-Haloalkoxy, C₂-C₄-Alkenyloxy, C₂-C₄-Alkinyloxy, C₁-C₄-Alkoxycarbonyl, C₁-C₄-Alkylsulfanyl, C₁-C₄-Haloalkylsulfanyl, C₁-C₄-Alkylsulfinyl, C₁-C₄-Haloalkylsulfinyl, C₁-C₄-Alkylsulfonyl, C₁-C₄-Haloalkylsulfonyl, Aminocarbonyl, Aminothiocarbonyl, CR⁵=NO-C₁-C₄-alkyl, -CR⁵=NO-Halo-C₁-C₄-alkyl, Formyl, C₁-C₄-Alkylamino, C₁-C₄-Dialkylamino, Phenyl, Heteroaryl, Heteroaryl-C₁-C₄-alkyl oder Hetero-C₃-C₄-cyclyl-C₁-C₄-alkyl;
wobei ein Phenyl- oder Heteroarylrest gegebenenfalls ein- oder mehrfach substituiert ist durch Halogen, C₁-C₄-Alkyl, C₁-C₄-Haloalkyl, C₁-C₄-Alkoxy, Nitro oder Cyano; ein Heterocyclylrest gegebenenfalls substituiert ist durch -OH / =O, -SH / =S, -NH₂, Halogen, C₁-C₄-Alkyl, C₁-C₄-Haloalkyl, C₁-C₄-Alkoxy, C₁-C₄-Alkylsulfanyl, C₁-C₄-Alkylsulfinyl, C₁-C₄-Alkylsulfonyl, C₁-C₄-Monoalkylamino, C₁-C₄-Dialkylamino, Nitro oder Cyano; und ein an Phenyl, Heteroaryl oder Heterocyclyl gebundener Alkandiyl-Rest gegebenenfalls einoder mehrfach substituiert ist durch Halogen, C₁-C₄-Alkyl, C₁-C₄-Haloalkyl, C₁-C₄-Alkoxy oder Cyano.
R⁶, R^{6'}, R^{6"}, R^{6'''} stehen unabhängig voneinander ganz besonders bevorzugt für Wasserstoff, Amino, Cyano, Fluor, Chlor, Brom, Iod, C₁-C₂-Alkyl, C₁-C₂-Haloalkyl, C₁-C₂-Alkoxy, C₁-C₂-Haloalkoxy, Phenyl oder Heteroaryl;
wobei ein Phenyl- oder Heteroarylrest gegebenenfalls ein- oder mehrfach substituiert ist durch Halogen, C₁-C₂-Alkyl, C₁-C₂-Haloalkyl, C₁-C₂-Alkoxy, Nitro oder Cyano.
R⁶, R^{6'}, R^{6"}, R^{6'''} stehen unabhängig voneinander insbesonders bevorzugt für Wasserstoff, Amino, Cyano, Fluor, Chlor, Methyl, Ethyl, C₁-C₂-Haloalkyl, Methoxy, Ethoxy oder C₁-C₂-Haloalkoxy;
R⁷ steht bevorzugt für Wasserstoff, Amino, Hydroxy, Cyano, C₁-C₆-Alkyl, C₁-C₆-Haloalkyl, C₂-C₆-Alkenyl, C₂-C₆-Alkinyl, C₃-C₆-Cycloalkyl, C₁-C₆-Alkoxy, C₂-C₆-Alkenyloxy, C₂-C₆-Alkinyloxy, C₁-C₆-Alkoxycarbonyl, C₁-C₆-Alkylcarbonyl, C₁-C₆-Alkylaminocarbonyl, C₁-C₆-Dialkylaminocarbonyl, Phenyl, Phenyl-C₁-C₆-alkyl, Heteroaryl-C₁-C₆-alkyl oder Hetero-C₃-C₆-cyclyl-C₁-C₆-alkyl;
wobei ein Phenyl- oder Heteroarylrest gegebenenfalls ein- oder mehrfach substituiert ist durch Halogen, C₁-C₆-Alkyl, C₁-C₆-Haloalkyl, C₁-C₆-Alkoxy, Nitro oder Cyano; ein Heterocyclylrest gegebenenfalls substituiert ist durch -OH / =O, -SH / =S, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, C₁-C₆-Monoalkylamino, C₁-C₆-Dialkylamino, Nitro oder Cyano; und ein an Phenyl, Heteroaryl oder Heterocyclyl gebundener Alkandiyl-Rest gegebenenfalls ein-oder mehrfach substituiert ist durch Halogen, C₁-C₆-Alkyl, C₁-C₆-Haloalkyl, C₁-C₆-Alkoxy oder Cyano und
wobei gegebenenfalls zwei benachbarte Reste aus R⁶, R^{6'}, R^{6"}, R^{6'''} und R⁷ zusammen für gegebenenfalls substituiertes und/oder gegebenenfalls durch Sauerstoff oder Schwefel oder eine Gruppierung aus der Reihe -S(O)-, -SO₂-, -NH- oder -N-C₁-C₆-Alkyl- am Anfang (bzw. am Ende) oder innerhalb der Kohlenwasserstoffkette unterbrochenes Alkandiyl oder Alkendiyl mit jeweils 2 bis 5 Kohlenstoffatomen stehen;
R⁷ steht besonders bevorzugt für Wasserstoff, Amino, Hydroxy, Cyano, C₁-C₄-Alkyl, C₁-C₄-Haloalkyl, C₂-C₄-Alkenyl, C₂-C₄-Alkinyl, C₃-C₄-Cycloalkyl, C₁-C₄-Alkoxy, C₂-C₄-Alkenyloxy, C₂-C₄-Alkinyloxy, C₁-C₄-Alkoxycarbonyl, C₁-C₄-Alkylcarbonyl, C₁-C₄-Alkylaminocarbonyl, C₁-C₄-Dialkylaminocarbonyl, Phenyl, Phenyl-C₁-C₄-alkyl, Heteroaryl-C₁-C₄-alkyl oder Hetero-C₃-C₄-cyclyl-C₁-C₄-alkyl;
wobei ein Phenyl- oder Heteroarylrest gegebenenfalls ein- oder mehrfach substituiert ist durch Halogen, C₁-C₄-Alkyl, C₁-C₄-Haloalkyl, C₁-C₄-Alkoxy, Nitro oder Cyano; ein Heterocyclylrest gegebenenfalls substituiert ist durch -OH / =O, -SH / =S, -NH₂, Halogen, C₁-C₄-Alkyl, C₁-C₄-Haloalkyl, C₁-C₄-Alkoxy, C₁-C₄-Alkylsulfanyl, C₁-C₄-Alkylsulfinyl, C₁-C₄-Alkylsulfonyl, C₁-C₄-Monoalkylamino, C₁-C₄-Dialkylamino, Nitro oder Cyano; und ein an Phenyl, Heteroaryl oder Heterocyclyl gebundener Alkandiyl-Rest gegebenenfalls ein- oder mehrfach substituiert ist durch Halogen, C₁-C₄-Alkyl, C₁-C₄-Haloalkyl, C₁-C₄-Alkoxy oder Cyano und
wobei gegebenenfalls zwei benachbarte Reste aus R⁶, R^{6'}, R^{6"}, R^{6'''} und R⁷ zusammen für gegebenenfalls substituiertes und/oder gegebenenfalls durch Sauerstoff oder Schwefel oder eine Gruppierung aus der Reihe -S(O)-, -SO₂-, -NH- oder -N- C₁-C₄-Alkyl- am Anfang (bzw. am Ende) oder innerhalb der Kohlenwasserstoffkette unterbrochenes Alkandiyl oder Alkendiyl mit jeweils 2 bis 5 Kohlenstoffatomen stehen;
R⁷ steht ganz besonders bevorzugt für Wasserstoff, Amino, Hydroxy, Cyano, C₁-C₂-Alkyl, C₁-C₂-Haloalkyl, Cyclopropyl, C₁-C₂-Alkoxy, C₁-C₂-Alkoxycarbonyl, C₁-C₂-Alkylcarbonyl, C₁-C₂-Alkylaminocarbonyl, C₁-C₂-Dialkylaminocarbonyl, Phenyl, Phenyl-C₁-C₂-alkyl oder Heteroaryl-C₁-C₂-alkyl;
wobei ein Phenyl- oder Heteroarylrest gegebenenfalls ein- oder mehrfach substituiert ist durch Halogen, C₁-C₂-Alkyl, C₁-C₂-Haloalkyl, C₁-C₂-Alkoxy, Nitro oder Cyano; und ein an Phenyl oder Heteroaryl gebundener Alkandiyl-Rest gegebenenfalls ein- oder mehrfach substituiert ist durch Halogen, C₁-C₂-Alkyl, C₁-C₂-Haloalkyl, C₁-C₂-Alkoxy oder Cyano und wobei gegebenenfalls zwei benachbarte Reste R⁶ / R⁶, R⁶ / R⁷ oder R⁷ / R⁷ zusammen für gegebenenfalls substituiertes und/oder gegebenenfalls durch Sauerstoff oder Schwefel oder eine Gruppierung aus der Reihe -S(O)-, -SO₂-, -NH- oder -N- C₁-C₂-Alkyl- am Anfang (bzw. am Ende) oder innerhalb der Kohlenwasserstoffkette unterbrochenes Alkandiyl oder Alkendiyl mit jeweils 2 bis 4 Kohlenstoffatomen stehen;
R⁷ steht insbesonders bevorzugt für Wasserstoff, Amino, Cyano, C₁-C₂-Alkyl, C₁-C₂-Haloalkyl oder C₁-C₂-Alkoxy;
R⁸ und R⁹ stehen bevorzugt und unabhängig voneinander für Wasserstoff, C₁-C₆-Alkyl, C₁-C₆-Haloalkyl, C₂-C₆-Alkenyl (gegebenenfalls substituiert durch Halogen, C₁-C₆-Alkyl, C₁-C₆-Haloalkyl oder Cyano), C₂-C₆-Alkinyl, C₃-C₆-Cycloalkyl (am Cyclus gegebenenfalls ein- oder mehrfach substituiert durch Halogen, C₁-C₆-Haloalkyl, C₁-C₆-Alkyl oder kondensiert an einen Aromaten oder Heteroaromaten), C₃-C₆-Cycloalkyl-C₁-C₆-alkyl (am Cyclus gegebenenfalls ein-oder mehrfach substituiert durch Halogen, C₁-C₆-Haloalkyl, C₁-C₆-Alkyl oder kondensiert an einen Aromaten oder Heteroaromaten, am C₁-C₆-Alkylteil gegebenenfalls ein- oder mehrfach substituiert durch Halogen, C₁-C₆-Alkyl, C₁-C₆-Haloalkyl, C₁-C₆-Alkoxy oder Cyano), C₃-C₆-Cycloalkylcarbonyl-C₃-C₆-heterocyclyl, C₁-C₆-Alkyloxycarbonyl-C₃-C₆-heterocyclyl, C₁-C₆-Alkylsulfinyl-C₁-C₆-alkyl, C₁-C₆-Alkylsulfanyl-C₁-C₆-alkyl, C₁-C₆-Alkylsulfonyl-C₁-C₆-alkyl, Hydroxy-C₁-C₆-alkyl, C₃-C₆-Heterocyclylcarbonyl-C₁-C₆-alkyl, Heteroarylcarbonylamino-C₁-C₆-alkyl, C₁-C₆-Haloalkoxy-C₁-C₆-alkyl, C₁-C₆-Alkylthiocarbonyl, C₁-C₆-Dialkylaminocarbonyl, C₁-C₆-Alkylaminocarbonyl, C₁-C₆-Haloalkylaminocarbonyl, C₁-C₆-Alkoxy, C₂-C₆-Alkenyloxy, C₂-C₆-Alkinyloxy, C₁-C₆-Alkoxy-C₁-C₆-alkyl, C₁-C₆-Alkylcarbonyl-C₁-C₆-alkyl, C(R¹⁰R¹¹)CR⁵=NO-C₁-C₆-Alkyl, C₁-C₆-Alkylsulfonyl, C₁-C₆-Alkylcarbonyl, C₁-C₆-Haloalkylcarbonyl, C₂-C₆-Alkenylcarbonyl, C₂-C₆-Alkinylcarbonyl, C₃-C₆-Cycloalkylcarbonyl, C₁-C₆-Alkoxycarbonyl, C₂-C₆-Alkenyloxycarbonyl, C₂-C₆-Alkinyloxycarbonyl, C₁-C₆-Alkoxyalkylcarbonyl, Phenylsulfonyl, Phenyl, Heteroaryl, Hetero-C₃-C₆-cyclyl, Phenyl-C₁-C₆-alkyl, Heteroaryl-C₁-C₆-alkyl, Hetero-C₃-C₆-cyclyl-C₁-C₆-alkyl, Phenylcarbonyl, Heteroarylcarbonyl, Hetero-C₃-C₆-cyclylcarbonyl, Phenyl-C₁-C₆-alkylcarbonyl, Heteroaryl-C₁-C₆-alkylcarbonyl, Hetero-C₃-C₆-cyclyl-C₁-C₆-alkylcarbonyl, Phenoxycarbonyl oder Phenyl-C₁-C₆-alkoxycarbonyl;
wobei ein Phenyl- oder Heteroarylrest gegebenenfalls ein- oder mehrfach substituiert ist durch Halogen, C₁-C₆-Alkyl, C₁-C₆-Haloalkyl, C₁-C₆-Alkoxy, Nitro oder Cyano; ein Heterocyclylrest gegebenenfalls substituiert ist durch -OH / =O, -SH / =S, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, C₁-C₆-Monoalkylamino, C₁-C₆-Dialkylamino, Nitro oder Cyano; und ein an Phenyl, Heteroaryl oder Heterocyclyl gebundener Alkandiyl-Rest gegebenenfalls einoder mehrfach substituiert ist durch Halogen, C₁-C₆-Alkyl, C₁-C₆-Haloalkyl, C₁-C₆-Alkoxy oder Cyano und
wobei gegebenenfalls R⁸ / R⁹ zusammen für gegebenenfalls substituiertes und/oder gegebenenfalls durch Sauerstoff oder Schwefel oder eine Gruppierung aus der Reihe -S(O)-, -SO₂-, -NH- oder -N-C₁-C₆-Alkyl- am Anfang (bzw. am Ende) oder innerhalb der Kohlenwasserstoffkette unterbrochenes Alkandiyl oder Alkendiyl mit jeweils 2 bis 5 Kohlenstoffatomen stehen;
R⁸ und R⁹ stehen besonders bevorzugt und unabhängig voneinander für Wasserstoff, C₁-C₄-Alkyl, C₁-C₄-Haloalkyl, C₂-C₄-Alkenyl (gegebenenfalls substituiert durch Halogen, C₁-C₄-Alkyl, C₁-C₄-Haloalkyl oder Cyano), C₂-C₄-Alkinyl, C₃-C₅-Cycloalkyl (am Cyclus gegebenenfalls einoder mehrfach substituiert durch Halogen, C₁-C₄-Haloalkyl, C₁-C₄-Alkyl oder kondensiert an einen Aromaten oder Heteroaromaten), C₃-C₅-Cycloalkyl-C₁-C₄-alkyl (am Cyclus gegebenenfalls einoder mehrfach substituiert durch Halogen, C₁-C₄-Haloalkyl, C₁-C₄-Alkyl oder kondensiert an einen Aromaten oder Heteroaromaten, am C₁-C₄-Alkylteil gegebenenfalls ein- oder mehrfach substituiert durch Halogen, C₁-C₄-Alkyl, C₁-C₄-Haloalkyl, C₁-C₄-Alkoxy oder Cyano), C₁-C₄-Alkylsulfinyl-C₁-C₄-alkyl, C₁-C₄-Alkylsulfanyl-C₁-C₄-alkyl, C₁-C₄-Alkylsulfonyl-C₁-C₄-alkyl, C₁-C₄-Haloalkoxy-C₁-C₄-alkyl, C₁-C₄-Alkylthiocarbonyl, C₁-C₄-Dialkylaminocarbonyl, C₁-C₄-Alkylaminocarbonyl, C₁-C₄-Haloalkylaminocarbonyl, C₁-C₄-Alkoxy, C₂-C₄-Alkenyloxy, C₂-C₄-Alkinyloxy, C₁-C₆-Alkoxy-C₁-C₄-alkyl, C₁-C₄-Alkylcarbonyl-C₁-C₄-alkyl, C(R¹⁰R¹¹)CR⁵=NO-C₁-C₄-Alkyl, C₁-C₄-Alkylsulfonyl, C₁-C₄-Alkylcarbonyl, C₁-C₄-Haloalkylcarbonyl, C₂-C₄-Alkenylcarbonyl, C₂-C₄-Alkinylcarbonyl, C₃-C₄-Cycloalkylcarbonyl, C₁-C₄-Alkoxycarbonyl, C₂-C₄-Alkenyloxycarbonyl, C₂-C₄-Alkinyloxycarbonyl, C₁-C₄-Alkoxyalkylcarbonyl, Phenylsulfonyl, Phenyl, Heteroaryl, Hetero-C₃-C₄-cyclyl, Phenyl-C₁-C₄-alkyl, Heteroaryl-C₁-C₄-alkyl, Hetero-C₃-C₄-cyclyl-C₁-C₄-alkyl, Phenylcarbonyl, Heteroarylcarbonyl, Hetero-C₃-C₄-cyclylcarbonyl, Phenyl-C₁-C₄-alkylcarbonyl, Heteroaryl-C₁-C₄-alkylcarbonyl, Hetero-C₃-C₄-cyclyl-C₁-C₄-alkylcarbonyl, Phenoxycarbonyl oder Phenyl-C₁-C₄-alkoxycarbonyl;
wobei ein Phenyl- oder Heteroarylrest gegebenenfalls ein- oder mehrfach substituiert ist durch Halogen, C₁-C₄-Alkyl, C₁-C₄-Haloalkyl, C₁-C₄-Alkoxy, Nitro oder Cyano; ein Heterocyclylrest gegebenenfalls substituiert ist durch -OH / =O, -SH / =S, -NH₂, Halogen, C₁-C₄-Alkyl, C₁-C₄-Haloalkyl, C₁-C₄-Alkoxy, C₁-C₄-Alkylsulfanyl, C₁-C₄-Alkylsulfinyl, C₁-C₄-Alkylsulfonyl, C₁-C₄-Monoalkylamino, C₁-C₄-Dialkylamino, Nitro oder Cyano; und ein an Phenyl, Heteroaryl oder Heterocyclyl gebundener Alkandiyl-Rest gegebenenfalls ein-oder mehrfach substituiert ist durch Halogen, C₁-C₄-Alkyl, C₁-C₄-Haloalkyl, C₁-C₄-Alkoxy oder Cyano und
wobei gegebenenfalls R⁸ / R⁹ zusammen für gegebenenfalls substituiertes und/oder gegebenenfalls durch Sauerstoff oder Schwefel oder eine Gruppierung aus der Reihe -S(O)-, -SO₂-, -NH- oder -N-C₁-C₄-Alkyl- am Anfang (bzw. am Ende) oder innerhalb der Kohlenwasserstoffkette unterbrochenes Alkandiyl oder Alkendiyl mit jeweils 2 bis 5 Kohlenstoffatomen stehen;
R⁸ und R⁹ stehen ganz besonders bevorzugt und unabhängig voneinander für Wasserstoff, C₁-C₄-Alkyl, C₁-C₄-Haloalkyl, C₃-C₅-Cycloalkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten), C₃-C₅-Cycloalkyl-C₁-C₂-alkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten, am C₁-C₂-Alkylteil gegebenenfalls ein- oder mehrfach substituiert durch Halogen, C₁-C₂-Alkyl, C₁-C₂-Haloalkyl, C₁-C₂-Alkoxy oder Cyano), C₁-C₂-Alkylsulfinyl-C₁-C₃-alkyl, C₁-C₂-Alkylsulfanyl-C₁-C₃-alkyl, C₁-C₂-Alkylsulfonyl-C₁-C₃-alkyl, Phenyl-C₁-C₄-alkyl, Heteroaryl-C₁-C₄-alkyl, C₁-C₄-Dialkylaminocarbonyl, C₁-C₄-Alkylaminocarbonyl, C₁-C₄-Haloalkylaminocarbonyl, C₁-C₂-Alkylsulfonyl, C₁-C₂-Alkylcarbonyl, C₁-C₂-Haloalkylcarbonyl, C₃-C₅-Cycloalkylcarbonyl, C₁-C₂-Alkoxycarbonyl, C₁-C₂-Alkoxy-C₁-C₂-alkylcarbonyl, Phenylsulfonyl, Phenyl, Heteroaryl, Hetero-C₃-C₅-cyclyl, Phenylcarbonyl, Heteroarylcarbonyl, Hetero-C₃-C₅-cyclylcarbonyl, Phenyl-C₁-C₂-alkylcarbonyl, Heteroaryl-C₁-C₂-alkylcarbonyl, Hetero-C₃-C₅-cyclyl-C₁-C₂-alkylcarbonyl, Phenoxycarbonyl oder Phenyl-C₁-C₂-alkoxycarbonyl stehen;
wobei ein Phenyl- oder Heteroarylrest gegebenenfalls ein- oder mehrfach substituiert ist durch Halogen, C₁-C₂-Alkyl, C₁-C₂-Haloalkyl, C₁-C₂-Alkoxy, Nitro oder Cyano; und ein an Phenyl oder Heteroaryl gebundener Alkandiy-Rrest gegebenenfalls ein- oder mehrfach substituiert ist durch Halogen, C₁-C₄-Alkyl, C₁-C₄-Haloalkyl, C₁-C₂-Alkoxy oder Cyano und wobei gegebenenfalls R⁸ / R⁹ zusammen für gegebenenfalls substituiertes und/oder gegebenenfalls durch Sauerstoff oder Schwefel oder eine Gruppierung aus der Reihe -S(O)-, -SO₂-, -NH- oder -N-C₁-C₄-Alkyl- am Anfang (bzw. am Ende) oder innerhalb der Kohlenwasserstoffkette unterbrochenes Alkandiyl oder Alkendiyl mit jeweils 2 bis 5 Kohlenstoffatomen stehen;
R⁸ und R⁹ stehen insbesonders bevorzugt und unabhängig voneinander für Wasserstoff, C₁-C₄-Alkyl, C₁-C₄-Haloalkyl, C₃-C₅-Cycloalkyl, C₃-C₅-Cycloalkyl-C₁-C₂-alkyl, C₃-C₅-Cyclohalo-alkyl-C₁-C₂-alkyl, C₃-C₄-Halocycloalkyl, Methylsulfanyl-C₁-C₃-alkyl, Methylsulfinyl-C₁-C₃-alkyl, Methylsulfonyl-C₁-C₃-alkyl, Phenylmethyl (am Aromaten gegebenenfalls ein- oder mehrfach substituiert durch Fluor, Chlor, Brom, Methyl, Ethyl, Trifluormethyl, Methoxy, Cyano oder Nitro, am Methylteil gegebenenfalls ein- oder mehrfach substituiert durch Methyl, Ethyl, Propyl, iso-Propyl, Methoxy oder Trifluormethyl), Pyridylmethyl (am Pyridyl gegebenenfalls ein- oder mehrfach substituiert durch Fluor, Chlor, Brom, Methyl, Ethyl, Trifluormethyl, Methoxy, Cyano oder Nitro, am Methylteil gegebenenfalls ein- oder mehrfach substituiert durch Methyl, Ethyl, Propyl, iso-Propyl, Methoxy oder Trifluormethyl) oder Pyrimidylmethyl (am Pyrimidyl gegebenenfalls ein- oder mehrfach substituiert durch Fluor, Chlor, Brom, Methyl, Ethyl, Trifluormethyl, Methoxy, Cyano oder Nitro, am Methylteil gegebenenfalls ein- oder mehrfach substituiert durch Methyl, Ethyl, Propyl, iso-Propyl, Methoxy oder Trifluormethyl);
R¹⁰ und R¹¹ stehen bevorzugt und unabhängig voneinander für Wasserstoff, C₁-C₆-Alkyl, C₁-C₆-Haloalkyl oder Cyano;
R¹⁰ und R¹¹ stehen besonders bevorzugt und unabhängig voneinander für Wasserstoff, C₁-C₄-Alkyl, C₁-C₄-Haloalkyl oder Cyano;
R¹⁰ und R¹¹ stehen ganz besonders bevorzugt und unabhängig voneinander für Wasserstoff, C₁-C₂-Alkyl oder C₁-C₂-Haloalkyl;
R¹⁰ und R¹¹ stehen insbesonders bevorzugt für Wasserstoff;
R¹² steht bevorzugt für C₁-C₆-Alkyl oder C₁-C₆-Haloalkyl;
R¹² steht besonders bevorzugt für C₁-C₄-Alkyl oder C₁-C₄-Haloalkyl;
R¹² steht ganz besonders bevorzugt für C₁-C₄-Alkyl;
R¹² steht insbesonders bevorzugt für Methyl oder Ethyl;
R¹³ und R¹⁴ stehen bevorzugt und unabhängig voneinander für Wasserstoff, C₁-C₆-Alkyl, C₁-C₆-Haloalkyl, C₂-C₆-Alkenyl, C₂-C₆-Alkinyl, C₁-C₆-Alkoxy-C₁-C₆-alkyl, C₁-C₆-Alkylcarbonyl-C₁-C₆-alkyl, C(R¹⁰R¹¹)CR⁵=NO-C₁-C₆-Alkyl, C₁-C₆-Alkylsulfonyl, C₁-C₆-Alkylcarbonyl, C₁-C₆-Haloalkylcarbonyl, C₂-C₆-Alkenylcarbonyl, C₂-C₆-Alkinylcarbonyl, C₃-C₆-Cycloalkylcarbonyl, C₁-C₆-Alkoxycarbonyl, C₂-C₆-Alkenyloxycarbonyl, C₂-C₆-Alkinyloxycarbonyl, C₁-C₆-Alkoxy-C₁-C₆-alkylcarbonyl, Phenylsulfonyl, Phenyl, Heteroaryl, Hetero-C₃-C₆-cyclyl, Phenyl-C₁-C₆-alkyl, Heteroaryl-C₁-C₆-alkyl, Hetero-C₃-C₆-cyclyl-C₁-C₆-alkyl, Phenylcarbonyl, Heteroarylcarbonyl, Hetero-C₃-C₆-cyclylcarbonyl, Phenyl- C₁-C₆-alkylcarbonyl, Heteroaryl-C₁-C₆-alkylcarbonyl, Hetero-C₃-C₆-cyclyl-C₁-C₆-alkylcarbonyl, Phenoxycarbonyl oder Phenyl-C₁-C₆-alkoxycarbonyl;
wobei ein Phenyl- oder Heteroarylrest gegebenenfalls ein- oder mehrfach substituiert ist durch Halogen, C₁-C₆-Alkyl, C₁-C₆-Haloalkyl, C₁-C₆-Alkoxy, Nitro oder Cyano; ein Heterocyclylrest gegebenenfalls substituiert ist durch -OH / =O, -SH / =S, -NH₂, Halogen, C₁-C₆-Alkyl, C₁-C₆-Haloalkyl, C₁-C₆-Alkoxy, C₁-C₆-Alkylsulfanyl, C₁-C₆-Alkylsulfinyl, C₁-C₆-Alkylsulfonyl, C₁-C₆-Monoalkylamino, C₁-C₆-Dialkylamino, Nitro oder Cyano; und ein an Phenyl, Heteroaryl oder Heterocyclyl gebundener Alkandiyl-Rest gegebenenfalls einoder mehrfach substituiert ist durch Halogen, C₁-C₆-Alkyl, C₁-C₆-Haloalkyl, C₁-C₆-Alkoxy oder Cyano
oder
R¹³ und R¹⁴ bilden als Imin die Gruppierung =CR⁵-NR¹⁵R¹⁶ oder =CR⁵-OR¹²;
R¹⁵ und R¹⁶ stehen unabhängig voneinander für Alkyl oder
R¹⁵ und R¹⁶ stehen zusammen für gegebenenfalls substituiertes und/oder gegebenenfalls durch Sauerstoff oder Schwefel oder eine Gruppierung aus der Reihe -S(O)-, -SO₂-, -NH- oder -N-Alkyl- am Anfang (bzw. am Ende) oder innerhalb der Kohlenwasserstoffkette unterbrochenes Alkandiyl oder Alkendiyl mit jeweils 2 bis 5 Kohlenstoffatomen;
R¹³ und R¹⁴ stehen besonders bevorzugt und unabhängig voneinander für Wasserstoff, C₁-C₄-Alkyl, C₁-C₄-Haloalkyl, C₂-C₄-Alkenyl, C₂-C₄-Alkinyl, C₁-C₄-Alkoxy-C₁-C₄-alkyl, C₁-C₄-Alkylcarbonyl-C₁-C₄-alkyl, C(R¹⁰R¹¹)CR⁵=NO-C₁-C₄-Alkyl, C₁-C₄-Alkylsulfonyl, C₁-C₄-Alkylcarbonyl, C₁-C₄-Haloalkylcarbonyl, C₂-C₄-Alkenylcarbonyl, C₂-C₄-Alkinylcarbonyl, C₃-C₄-Cycloalkylcarbonyl, C₁-C₄-Alkoxycarbonyl, C₂-C₄-Alkenyloxycarbonyl, C₂-C₄-Alkinyloxycarbonyl, C₁-C₄-Alkoxy-C₁-C₄-alkylcarbonyl, Phenylsulfonyl, Phenyl, Heteroaryl, Hetero-C₃-C₄-cyclyl, Phenyl-C₁-C₄-alkyl, Heteroaryl-C₁-C₄-alkyl, Hetero-C₃-C₄-cyclyl-C₁-C₄-alkyl, Phenylcarbonyl, Heteroarylcarbonyl, Hetero-C₃-C₄-cyclylcarbonyl, Phenyl-C₁-C₄-alkylcarbonyl, Heteroaryl-C₁-C₄-alkylcarbonyl, Hetero-C₃-C₄-cyclyl-C₁-C₄-alkylcarbonyl, Phenoxycarbonyl oder Phenyl-C₁-C₄-alkoxycarbonyl stehen;
wobei ein Phenyl- oder Heteroarylrest gegebenenfalls ein- oder mehrfach substituiert ist durch Halogen, C₁-C₄-Alkyl, C₁-C₄-Haloalkyl, C₁-C₄-Alkoxy, Nitro oder Cyano; ein Heterocyclylrest gegebenenfalls substituiert ist durch -OH / =O, -SH / =S, -NH₂, Halogen, C₁-C₄-Alkyl, C₁-C₄-Haloalkyl, C₁-C₄-Alkoxy, C₁-C₄-Alkylsulfanyl, C₁-C₄-Alkylsulfinyl, C₁-C₄-Alkylsulfonyl, C₁-C₄-Monoalkylamino, C₁-C₄-Dialkylamino, Nitro oder Cyano; und ein an Phenyl, Heteroaryl oder Heterocyclyl gebundener Alkandiyl-Rest gegebenenfalls ein-oder mehrfach substituiert ist durch Halogen, C₁-C₄-Alkyl, C₁-C₄-Haloalkyl, C₁-C₄-Alkoxy oder Cyano
oder
R¹³ und R¹⁴ bilden als Imin die Gruppierung =CR⁵-NR¹⁵R¹⁶ oder =CR⁵-OR¹²;
R¹⁵ und R¹⁶ stehen unabhängig voneinander für Alkyl oder
R¹⁵ und R¹⁶ stehen zusammen für gegebenenfalls substituiertes und/oder gegebenenfalls durch Sauerstoff oder Schwefel oder eine Gruppierung aus der Reihe -S(O)-, -SO₂-, -NH- oder -N-Alkyl- am Anfang (bzw. am Ende) oder innerhalb der Kohlenwasserstoffkette unterbrochenes Alkandiyl oder Alkendiyl mit jeweils 2 bis 5 Kohlenstoffatomen;
R¹³ und R¹⁴ stehen ganz besonders bevorzugt und unabhängig voneinander für Wasserstoff, C₁-C₂-Alkyl, C₁-C₂-Haloalkyl, C₁-C₂-Alkoxy-C₁-C₂-alkyl, C₁-C₂-Alkylsulfonyl, C₁-C₂-Alkylcarbonyl, C₁-C₂-Haloalkylcarbonyl, C₁-C₂-Alkoxycarbonyl, C₁-C₂-Alkoxy-C₁-C₂-alkylcarbonyl, Phenylsulfonyl, Phenyl, Heteroaryl, Phenyl-C₁-C₂-alkyl, Heteroaryl-C₁-C₂-alkyl, Phenylcarbonyl, Heteroarylcarbonyl, Phenyl-C₁-C₂-alkylcarbonyl, Phenoxycarbonyl oder Phenyl-C₁-C₂-alkoxycarbonyl;
wobei ein Phenyl- oder Heteroarylrest gegebenenfalls ein- oder mehrfach substituiert ist durch Halogen, C₁-C₂-Alkyl, C₁-C₂-Haloalkyl, C₁-C₂-Alkoxy, Nitro oder Cyano; und ein an Phenyl oder Heteroaryl gebundener Alkandiyl-Rest gegebenenfalls ein- oder mehrfach substituiert ist durch Halogen, C₁-C₂-Alkyl, C₁-C₂-Haloalkyl, C₁-C₂-Alkoxy oder Cyano
oder
R¹³ und R¹⁴ bilden als Imin die Gruppierung =CR⁵-NR¹⁵R¹⁶ oder =CR⁵-OR¹²;
R¹⁵ und R¹⁶ stehen unabhängig voneinander für Alkyl oder
R¹⁵ und R¹⁶ stehen zusammen für gegebenenfalls substituiertes und/oder gegebenenfalls durch Sauerstoff oder Schwefel oder eine Gruppierung aus der Reihe -S(O)-, -SO₂-, -NH- oder -N-Alkyl- am Anfang (bzw. am Ende) oder innerhalb der Kohlenwasserstoffkette unterbrochenes Alkandiyl oder Alkendiyl mit jeweils 2 bis 5 Kohlenstoffatomen;
R¹³ und R¹⁴ stehen insbesonders bevorzugt und unabhängig voneinander für Wasserstoff, Methyl, Ethyl, Propyl, iso-Propyl, Methylcarbonyl, Methoxycarbonyl, Trifluormethylcarbonyl, Methylsulfanyl, Trifluormethylsulfanyl, Benzyl oder Pyridylmethyl.

Die Verbindungen der Formel (I) lassen sich in Abhängigkeit von ihrem Substituenten R¹ in die Verbindungen der Formel (II), (III), (IV) und (V) unterteilen: 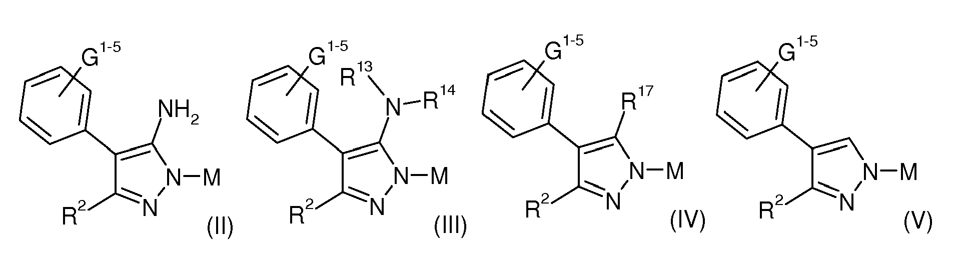 wobei
G¹⁻⁵, R², R¹³, R¹⁴, und M die oben angegebenen Bedeutungen haben, R¹³ und R¹⁴ aber nicht gleichzeitig für Wasserstoff stehen und
R¹⁷ für Halogen, Alkylsulfanyl, Alkylsulfinyl, Alkylsulfonyl oder Cyano steht.

Hieraus ergeben sich 4-Phenyl-1H-pyrazole der Formeln (II-A), (II-B), (III-A), (III-B), (IV-A), (IV-B), (V-A) und (V-B), 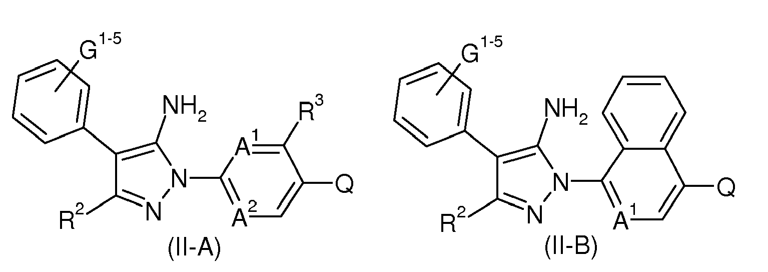 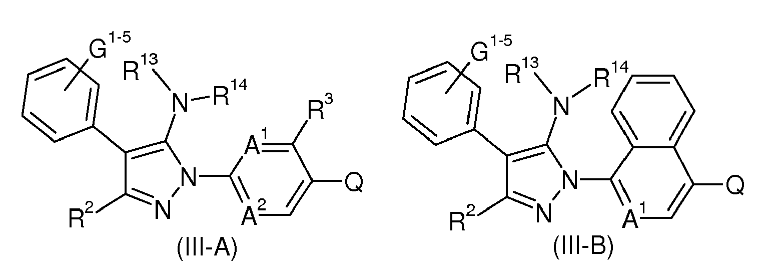 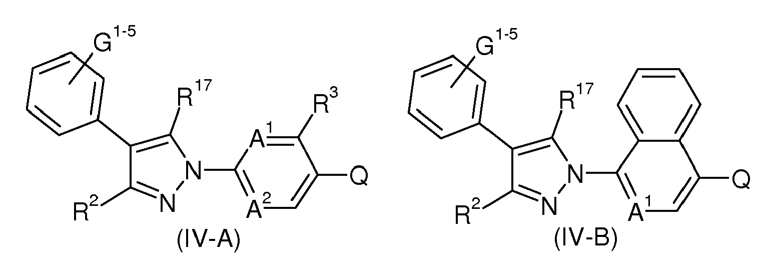 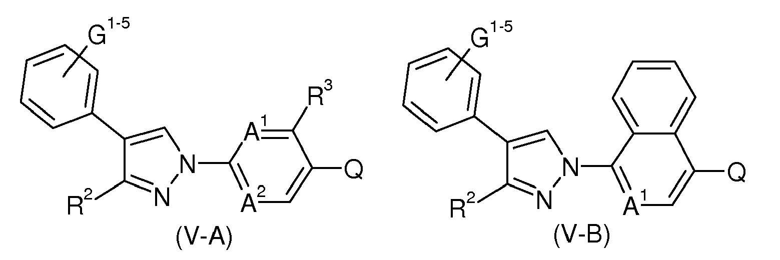 wobei
G¹⁻⁵, R², R³, R¹³, R¹⁴, A¹⁻² und Q die oben angegebenen Bedeutungen haben, R¹³ und R¹⁴ aber nicht gleichzeitig für Wasserstoff stehen und
R¹⁷ für Halogen, Alkylsulfanyl, Alkylsulfinyl, Alkylsulfonyl oder Cyano steht.

Die Erfindung betrifft in einer Ausführungsform 1 Verbindungen der Formel (II-A) 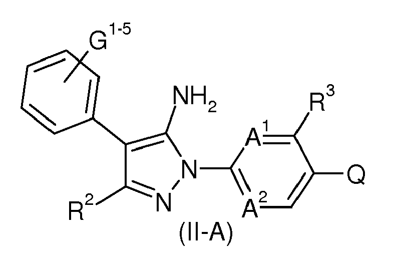 wobei
G¹, G² und G³ unabhängig voneinander für Wasserstoff, Halogen, Methyl oder CF₃ stehen;
G⁴ und G⁵ für Wasserstoff stehen;
R² für Fluor, Chlor, Brom, Iod, C₁-C₄-Alkoxy-C₁-C₄-Alkyl, C₁-C₄-Alkyl, Aryl-C₁-C₄-haloalkyl, C₁-C₆-Alkylsulfanyl, C₁-C₆-Alkylsulfinyl, C₁-C₆-Alkylsulfonyl oder C₁-C₄ Haloalkyl steht;
A¹ und A² unabhängig voneinander für N oder CH stehen;
R³ für Fluor, Chlor, Brom, Iod, C₁₋₂-Alkyl, C₁₋₂-Haloalkyl, C₁-C₂-Haloalkylsulfanyl, C₁-C₂-Haloalkylsulfonyl, C₁-C₂-Haloalkylsulfinyl oder Cyano steht und
Q für Q¹, Q², Q³ oder Q⁴ steht.

In einer Ausführungsform 2 betrifft die Erfindung Verbindungen gemäß Ausführungsform 1, in denen Q für Q¹ steht.

In einer Ausführungsform 3 betrifft die Erfindung Verbindungen gemäß Ausführungsform 2, in denen Q¹ für Z³, Z⁷, Z¹⁵, Z¹⁶, Z¹⁷, Z¹⁸, Z²¹, Z²², Z²³ oder Z²⁴ steht; R⁶, R^{6'}, R^{6"}, R^{6'"} für Wasserstoff, Amino, Cyano, Fluor, Chlor, Methyl, Ethyl, C₁-C₂-Haloalkyl, Methoxy, Ethoxy oder C₁-C₂-Haloalkoxy stehen; und R⁷ für Wasserstoff, Amino, Cyano, C₁-C₂-Alkyl, C₁-C₂-Haloalkyl oder C₁-C₂-Alkoxy steht.

In einer Ausführungsform 4 betrifft die Erfindung Verbindungen gemäß Ausführungsform 3, in denen Q¹ für Z¹⁶ oder Z³ steht.

In einer Ausführungsform 5 betrifft die Erfindung Verbindungen gemäß Ausführungsform 1, in denen Q für Q² steht.

In einer Ausführungsform 6 betrifft die Erfindung Verbindungen gemäß Ausführungsform 5, in denen Q² für C(O)NR⁸R⁹ steht und R⁸ und R⁹ unabhängig voneinander für Wasserstoff, C₁-C₄-Alkyl, C₁-C₄-Haloalkyl, C₃-C₅-Cycloalkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten), C₃-C₅-Cycloalkyl-C₁-C₂-alkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten, am C₁-C₂-Alkylteil gegebenenfalls ein- oder mehrfach substituiert durch Halogen, C₁-C₂-Alkyl, C₁-C₂-Haloalkyl, C₁-C₂-Alkoxy oder Cyano), C₁-C₂-Alkylsulfinyl-C₁-C₃-alkyl, C₁-C₃-Alkylsulfanyl-C₁-C₃-alkyl, C₁-C₂-Alkylsulfonyl-C₁-C₃-alkyl, Phenyl-C₁-C₄-alkyl oder Heteroaryl -C₁-C₄-alkyl stehen; wobei ein Phenyl- oder Heteroarylrest gegebenenfalls einoder mehrfach substituiert ist durch Halogen, C₁-C₂-Alkyl, C₁-C₂-Haloalkyl, C₁-C₂-Alkoxy, Nitro oder Cyano; und ein an Phenyl oder Heteroaryl gebundener Alkandiyl-Rest gegebenenfalls einoder mehrfach substituiert ist durch Halogen, C₁-C₄-Alkyl, C₁-C₄-Haloalkyl, C₁-C₂-Alkoxy oder Cyano.

In einer Ausführungsform 7 betrifft die Erfindung Verbindungen gemäß Ausführungsform 6, in denen R⁸ und R⁹ unabhängig voneinander für Wasserstoff, C₁-C₄-Alkyl, C₁-C₄-Haloalkyl, C₃-C₄-Cycloalkyl, C₃-C₄-Halocycloalkyl, C₃-C₅-Cyloalkyl-C₁-C₂-alkyl, C₃-C₅-Halocycloalkyl-C₁-C₂-alkyl, Methylsulfinyl-C₁-C₃-alkyl, Methylsulfanyl-C₁-C₃-alkyl, Methylsulfonyl-C₁-C₃-alkyl, Phenylmethyl (am Aromaten gegebenenfalls ein- oder mehrfach substituiert durch Fluor, Chlor, Brom, Methyl, Ethyl, Trifluormethyl, Methoxy, Cyano oder Nitro, am Methylteil gegebenenfalls ein- oder mehrfach substituiert durch Methyl, Ethyl, Propyl, iso-Propyl, Methoxy oder Trifluormethyl), Pyridylmethyl (am Pyridyl gegebenenfalls ein- oder mehrfach substituiert durch Fluor, Chlor, Brom, Methyl, Ethyl, Trifluormethyl, Methoxy, Cyano oder Nitro, am Methylteil gegebenenfalls ein- oder mehrfach substituiert durch Methyl, Ethyl, Propyl, iso-Propyl, Methoxy oder Trifluormethyl) oder Pyrimidylmethyl (am Pyrimidyl gegebenenfalls ein- oder mehrfach substituiert durch Fluor, Chlor, Brom, Methyl, Ethyl, Trifluormethyl, Methoxy, Cyano oder Nitro, am Methylteil gegebenenfalls ein- oder mehrfach substituiert durch Methyl, Ethyl, Propyl, iso-Propyl, Methoxy oder Trifluormethyl) stehen.

In einer Ausführungsform 8 betrifft die Erfindung Verbindungen gemäß Ausführungsform 1, in denen Q für Q³ steht.

In einer Ausführungsform 9 betrifft die Erfindung Verbindungen gemäß Ausführungsform 8, in denen Q³ für C(R¹⁰R¹¹)NR⁸R⁹ steht; R¹⁰ und R¹¹ unabhängig voneinander für Wasserstoff, C₁-C₂-Alkyl oder C₁-C₂-Haloalkyl stehen und R⁸ und R⁹ unabhängig voneinander für Wasserstoff, C₁-C₄-Alkyl, C₁-C₄-Haloalkyl, C₃-C₅-Cycloalkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten), C₃-C₅-Cycloalkyl-C₁-C₂-alkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten, am C₁-C₂-Alkylteil gegebenenfalls ein- oder mehrfach substituiert durch Halogen, C₁-C₂-Alkyl, C₁-C₂-Haloalkyl, C₁-C₂-Alkoxy oder Cyano), C₁-C₂-Alkylsulfinyl-C₁-C₃-alkyl, C₁-C₂-Alkylsulfanyl-C₁-C₃-alkyl, C₁-C₂-Alkylsulfonyl-C₁-C₃-alkyl, Phenyl-C₁-C₄-alkyl, Heteroaryl-C₁-C₄-alkyl, C₁-C₄-Dialkylaminocarbonyl, C₁-C₄-Alkylaminocarbonyl, C₁-C₄-Haloalkylaminocarbonyl, C₁-C₂-Alkylsulfonyl, C₁-C₂-Alkylcarbonyl, C₁-C₂-Haloalkylcarbonyl, C₃-C₅-Cycloalkylcarbonyl, C₁-C₂-Alkoxycarbonyl, C₁-C₂-Alkoxy-C₁-C₂-alkylcarbonyl, Phenylsulfonyl, Phenyl, Heteroaryl, Hetero-C₃-C₅-cyclyl, Phenylcarbonyl, Heteroarylcarbonyl, Hetero-C₃-C₅-cyclylcarbonyl, Phenyl-C₁-C₂-alkylcarbonyl, Heteroaryl-C₁-C₂-alkylcarbonyl, Hetero-C₃-C₅-cyclyl-C₁-C₂-alkylcarbonyl, Phenoxycarbonyl oder Phenyl-C₁-C₂-alkoxycarbonyl stehen; wobei ein Phenyl- oder Heteroarylrest gegebenenfalls ein- oder mehrfach substituiert ist durch Halogen, C₁-C₂-Alkyl, C₁-C₂-Haloalkyl, C₁-C₂-Alkoxy, Nitro oder Cyano; und ein an Phenyl oder Heteroaryl gebundener Alkandiyl-Rest gegebenenfalls ein- oder mehrfach substituiert ist durch Halogen, C₁-C₄-Alkyl, C₁-C₄-Haloalkyl, C₁-C₂-Alkoxy oder Cyano.

In einer Ausführungsform 10 betrifft die Erfindung Verbindungen gemäß Ausführungsform 9, in denen R⁸ und R⁹ unabhängig voneinander für Wasserstoff, C₁-C₂-Alkyl, C₁-C₂-Haloalkyl, C₃-C₅-Cycloalkyl, C₃-C₅-Cycloalkyl-C₁-C₂-alkyl, C₃-C₅-Halocycloalkyl, C₃-C₅-Halocycloalkyl-C₁-C₂-alkyl, Methylsulfinyl-C₁-C₃-alkyl, Methylsulfanyl-C₁-C₃-alkyl, Methylsulfonyl-C₁-C₃-alkyl, Phenyl-C₁-C₂-alkyl, Heteroaryl-C₁-C₂-alkyl, C₁-C₂-Dialkylaminocarbonyl, C₁-C₂-Alkylaminocarbonyl, C₁-C₂-Haloalkylaminocarbonyl, C₁-C₂-Alkylsulfonyl, C₁-C₂-Alkylcarbonyl, C₁-C₂-Haloalkylcarbonyl, C₃-C₅-Cycloalkylcarbonyl, C₁-C₂-Alkoxycarbonyl, Phenylsulfonyl, Phenylcarbonyl, Heteroarylcarbonyl, Hetero-C₃-C₅-cyclylcarbonyl, Phenyl-C₁-C₂-alkylcarbonyl, Heteroaryl-C₁-C₂-alkylcarbonyl, Hetero-C₃-C₅-cyclyl-C₁-C₂-alkylcarbonyl oder Phenoxycarbonyl stehen; wobei ein Phenyl- oder Heteroarylrest gegebenenfalls ein- oder mehrfach substituiert ist durch Halogen, C₁-C₂-Alkyl, C₁-C₂-Haloalkyl, C₁-C₂-Alkoxy, Nitro oder Cyano; und ein an Phenyl oder Heteroaryl gebundener Alkandiyl-Rest gegebenenfalls ein- oder mehrfach substituiert ist durch Halogen, C₁-C₄-Alkyl, C₁-C₄-Haloalkyl, C₁-C₂-Alkoxy oder Cyano.

In einer Ausführungsform 11 betrifft die Erfindung Verbindungen gemäß Ausführungsform 1, in denen Q für Q⁴ steht.

In einer Ausführungsform 12 betrifft die Erfindung Verbindungen gemäß Ausführungsform 11, in denen Q⁴ für Cyano (wobei R¹ nicht für Amino steht), Nitro, Amino, COOH, COOR¹², Fluor (falls R³ verschieden von Chlor ist), Chlor (falls R³ verschieden von Chlor, COOH, CH₂CH₂OMe und OMe ist), Brom, Iod, SR¹² (wobei R¹ nicht für Amino steht, falls R¹² für Alkyl steht), S(O)R¹² oder S(O)₂R¹² steht und R¹² für C₁-C₄-Alkyl oder C₁-C₄-Haloalkyl steht.

In einer Ausführungsform 13 betrifft die Erfindung Verbindungen gemäß Ausführungsform 12, in denen Q⁴ für Cyano (wobei R¹ nicht für Amino steht), COOH, COOMe, COOEt, Fluor (falls R³ verschieden von Chlor ist), Brom, Iod, SR¹² (wobei R¹ nicht für Amino steht, falls R¹² für Alkyl steht), S(O)R¹² oder S(O)₂R¹² steht.

In einer Ausführungsform 14 betrifft die Erfindung Verbindungen der Formel (III-A) 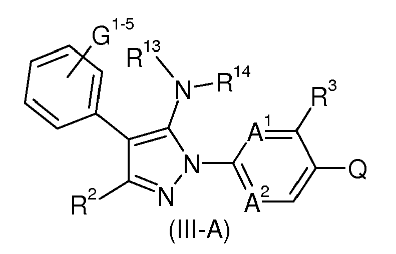 wobei
G¹, G² und G³ unabhängig voneinander für Wasserstoff, Halogen, Methyl oder CF₃ stehen;
G⁴ und G⁵ für Wasserstoff stehen;
R² für Fluor, Chlor, Brom, Iod, C₁-C₄-Alkoxy-C₁-C₄-Alkyl, C₁-C₄-Alkyl, Aryl-C₁-C₄-haloalkyl, C₁-C₆-Alkylsulfanyl, C₁-C₆-Alkylsulfinyl, C₁-C₆-Alkylsulfonyl oder C₁-C₄ Haloalkyl steht;
A¹ und A² unabhängig voneinander für N oder CH stehen;
R³ für Fluor, Chlor, Brom, Iod, C₁₋₂-Alkyl, C₁₋₂-Haloalkyl, C₁-C₂-Haloalkylsulfanyl, C₁-C₂-Haloalkylsulfonyl, C₁-C₂-Haloalkylsulfinyl oder Cyano steht;
R¹³ und R¹⁴ unabhängig voneinander für Wasserstoff, Methyl, Ethyl, Propyl, iso-Propyl, Methylcarbonyl, Methoxycarbonyl, Trifluormethylcarbonyl, Methylsulfanyl, Trifluormethylsulfanyl, Benzyl oder Pyridylmethyl stehen, R¹³ und R¹⁴ aber nicht gleichzeitig für Wasserstoff stehen, und
Q für Q¹, Q², Q³ oder Q⁴ steht.

In einer Ausführungsform 15 betrifft die Erfindung Verbindungen gemäß Ausführungsform 14, in denen Q für Q¹ steht.

In einer Ausführungsform 16 betrifft die Erfindung Verbindungen gemäß Ausführungsform 15, in denen Q¹ für Z³, Z⁷, Z¹⁵, Z¹⁶, Z¹⁷, Z¹⁸, Z²¹, Z²², Z²³ oder Z²⁴ steht; R⁶, R^{6'}, R^{6"}, R^{6"'} für Wasserstoff, Amino, Cyano, Fluor, Chlor, Methyl, Ethyl, C₁-C₂-Haloalkyl, Methoxy, Ethoxy oder C₁-C₂-Haloalkoxy stehen und R⁷ für Wasserstoff, Amino, Cyano, C₁-C₂-Alkyl, C₁-C₂-Haloalkyl oder C₁-C₂-Alkoxy steht.

In einer Ausführungsform 17 betrifft die Erfindung Verbindungen gemäß Ausführungsform 16, in denen Q¹ für Z¹⁶ oder Z³ steht.

In einer Ausführungsform 18 betrifft die Erfindung Verbindungen gemäß Ausführungsform 14, in denen Q für Q² steht.

In einer Ausführungsform 19 betrifft die Erfindung Verbindungen gemäß Ausführungsform 18, in denen Q² für C(O)NR⁸R⁹ steht; und R⁸ und R⁹ unabhängig voneinander für Wasserstoff, C₁-C₄-Alkyl, C₁-C₄-Haloalkyl, C₃-C₅-Cycloalkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten), C₃-C₅-Cycloalkyl-C₁-C₂-alkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten, am C₁-C₂-Alkylteil gegebenenfalls ein- oder mehrfach substituiert durch Halogen, C₁-C₂-Alkyl, C₁-C₂-Haloalkyl, C₁-C₂-Alkoxy oder Cyano), C₁-C₂-Alkylsulfinyl-C₁-C₃-alkyl, C₁-C₂-Alkylsulfanyl-C₁-C₃-alkyl, C₁-C₂-Alkylsulfonyl-C₁-C₃-alkyl, Phenyl-C₁-C₄-alkyl oder Heteroaryl -C₁-C₄-alkyl stehen; wobei ein Phenyl- oder Heteroarylrest gegebenenfalls ein-oder mehrfach substituiert ist durch Halogen, C₁-C₂-Alkyl, C₁-C₂-Haloalkyl, C₁-C₂-Alkoxy, Nitro oder Cyano; und ein an Phenyl oder Heteroaryl gebundener Alkandiyl-Rest gegebenenfalls ein-oder mehrfach substituiert ist durch Halogen, C₁-C₄-Alkyl, C₁-C₄-Haloalkyl, C₁-C₂-Alkoxy oder Cyano.

In einer Ausführungsform 20 betrifft die Erfindung Verbindungen gemäß Ausführungsform 19, in denen R⁸ und R⁹ unabhängig voneinander für Wasserstoff, C₁-C₄-Alkyl, C₁-C₄-Haloalkyl, C₃-C₄-Cycloalkyl, C₃-C₄-Halocyloalkyl, C₃-C₅-Cycloalkyl-C₁-C₂-alkyl, C₃-C₅-Halocycloalkyl-C₁-C₂-alkyl, Methylalkylsulfinyl-C₁-C₃-alkyl, Methylalkylsulfanyl-C₁-C₃-alkyl, Methylalkylsulfonyl-C₁-C₃-alkyl, Phenylmethyl (am Aromaten gegebenenfalls ein- oder mehrfach substituiert durch Fluor, Chlor, Brom, Methyl, Ethyl, Trifluormethyl, Methoxy, Cyano oder Nitro, am Methylteil gegebenenfalls ein- oder mehrfach substituiert durch Methyl, Ethyl, Propyl, iso-Propyl, Methoxy oder Trifluormethyl), Pyridylmethyl (am Pyridyl gegebenenfalls ein- oder mehrfach substituiert durch Fluor, Chlor, Brom, Methyl, Ethyl, Trifluormethyl, Methoxy, Cyano oder Nitro, am Methylteil gegebenenfalls ein- oder mehrfach substituiert durch Methyl, Ethyl, Propyl, iso-Propyl, Methoxy oder Trifluormethyl) oder Pyrimidylmethyl (am Pyrimidyl gegebenenfalls ein- oder mehrfach substituiert durch Fluor, Chlor, Brom, Methyl, Ethyl, Trifluormethyl, Methoxy, Cyano oder Nitro, am Methylteil gegebenenfalls ein- oder mehrfach substituiert durch Methyl, Ethyl, Propyl, iso-Propyl, Methoxy oder Trifluormethyl) stehen.

In einer Ausführungsform 21 betrifft die Erfindung Verbindungen gemäß Ausführungsform 14, in denen Q für Q³ steht.

In einer Ausführungsform 22 betrifft die Erfindung Verbindungen gemäß Ausführungsform 21, in denen Q³ für C(R¹⁰R¹¹)NR⁸R⁹ steht; R¹⁰ und R¹¹ unabhängig voneinander für Wasserstoff, C₁-C₂-Alkyl oder C₁-C₂-Haloalkyl stehen und R⁸ und R⁹ unabhängig voneinander für Wasserstoff, C₁-C₄-Alkyl, C₁-C₄-Haloalkyl, C₃-C₄-Cycloalkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten), C₃-C₄-Cycloalkyl-C₁-C₂-alkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten, am C₁-C₂-Alkylteil gegebenenfalls ein- oder mehrfach substituiert durch Halogen, C₁-C₂-Alkyl, C₁-C₂-Haloalkyl, C₁-C₂-Alkoxy oder Cyano), C₁-C₂-Alkylsulfinyl-C₁-C₂-alkyl, C₁-C₂-Alkylsulfanyl-C₁-C₂-alkyl, C₁-C₂-Alkylsulfonyl-C₁-C₂-alkyl, Phenyl-C₁-C₄-alkyl, Heteroaryl-C₁-C₄-alkyl, C₁-C₄-Dialkylaminocarbonyl, C₁-C₄-Alkylaminocarbonyl, C₁-C₄-Haloalkylaminocarbonyl, C₁-C₂-Alkylsulfonyl, C₁-C₂-Alkylcarbonyl, C₁-C₂-Haloalkylcarbonyl, C₃-C₅-Cycloalkylcarbonyl, C₁-C₂-Alkoxycarbonyl, C₁-C₂-Alkoxy-C₁-C₂-alkylcarbonyl, Phenylsulfonyl, Phenyl, Heteroaryl, Hetero-C₃-C₅-cyclyl, Phenylcarbonyl, Heteroarylcarbonyl, Hetero-C₃-C₅-cyclylcarbonyl, Phenyl-C₁-C₂-alkylcarbonyl, Heteroaryl-C₁-C₂-alkylcarbonyl, Hetero-C₃-C₅-cyclyl-C₁-C₂-alkylcarbonyl, Phenoxycarbonyl oder Phenyl-C₁-C₂-alkoxycarbonyl stehen; wobei ein Phenyl- oder Heteroarylrest gegebenenfalls ein- oder mehrfach substituiert ist durch Halogen, C₁-C₂-Alkyl, C₁-C₂-Haloalkyl, C₁-C₂-Alkoxy, Nitro oder Cyano; und ein an Phenyl oder Heteroaryl gebundener Alkandiyl-Rest gegebenenfalls ein- oder mehrfach substituiert ist durch Halogen, C₁-C₄-Alkyl, C₁-C₄-Haloalkyl, C₁-C₂-Alkoxy oder Cyano.

In einer Ausführungsform 23 betrifft die Erfindung Verbindungen gemäß Ausführungsform 22, in denen R⁸ und R⁹ unabhängig voneinander für Wasserstoff, C₁-C₂-Alkyl, C₁-C₂-Haloalkyl, C₃-C₅-Cycloalkyl, C₃-C₅-Cycloalkyl-C₁-C₂-alkyl, C₃-C₅-Halocycloalkyl, C₃-C₅-Halocycloalkyl-C₁-C₂-alkyl, Methylsulfinyl-C₁-C₃-alkyl, Methylsulfanyl-C₁-C₃-alkyl, Methylsulfonyl-C₁-C₃-alkyl, Phenyl-C₁-C₂-alkyl, Heteroaryl-C₁-C₂-alkyl, C₁-C₂-Dialkylaminocarbonyl, C₁-C₂-Alkylaminocarbonyl, C₁-C₂-Haloalkylaminocarbonyl, C₁-C₂-Alkylsulfonyl, C₁-C₂-Alkylcarbonyl, C₁-C₂-Haloalkylcarbonyl, C₃-C₅-Cycloalkylcarbonyl, C₁-C₂-Alkoxycarbonyl, Phenylsulfonyl, Phenylcarbonyl, Heteroarylcarbonyl, Hetero-C₃-C₅-cyclylcarbonyl, Phenyl-C₁-C₂-alkylcarbonyl, Heteroaryl-C₁-C₂-alkylcarbonyl, Hetero-C₃-C₅-cyclyl-C₁-C₂-alkylcarbonyl oder Phenoxycarbonyl stehen; wobei ein Phenyl- oder Heteroarylrest gegebenenfalls ein- oder mehrfach substituiert ist durch Halogen, C₁-C₂-Alkyl, C₁-C₂-Haloalkyl, C₁-C₂-Alkoxy, Nitro oder Cyano; und ein an Phenyl oder Heteroaryl gebundener Alkandiyl-Rest gegebenenfalls ein- oder mehrfach substituiert ist durch Halogen, C₁-C₄-Alkyl, C₁-C₄-Haloalkyl, C₁-C₂-Alkoxy oder Cyano. In einer Ausführungsform 24 betrifft die Erfindung Verbindungen gemäß Ausführungsform 14, in denen Q für Q⁴ steht.

In einer Ausführungsform 25 betrifft die Erfindung Verbindungen gemäß Ausführungsform 24, in denen Q⁴ für Cyano (wobei R¹ nicht für Amino steht), Nitro, Amino, COOH, COOR¹², Fluor (falls R³ verschieden von Chlor ist), Chlor (falls R³ verschieden von Chlor, COOH, CH₂CH₂OMe und OMe ist), Brom, Iod, SR¹² (wobei R¹ nicht für Amino steht, falls R¹² für Alkyl steht), S(O)R¹² oder S(O)₂R¹² steht und R¹² für C₁-C₄-Alkyl oder C₁-C₄-Haloalkyl steht.

In einer Ausführungsform 26 betrifft die Erfindung Verbindungen gemäß Ausführungsform 25, in denen Q⁴ für Cyano (wobei R¹ nicht für Amino steht), COOH, COOMe, COOEt, Fluor (falls R³ verschieden von Chlor ist), Brom, Iod, SR¹² (wobei R¹ nicht für Amino steht, falls R¹² für Alkyl steht), S(O)R¹² oder S(O)₂R¹² steht.

In einer Ausführungsform 27 betrifft die Erfindung Verbindungen der Formel (IV-A) 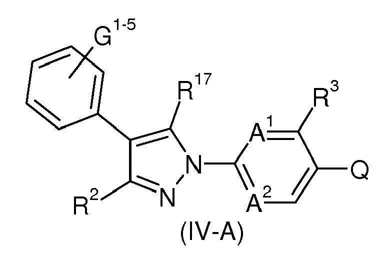 wobei
G¹, G² und G³ unabhängig voneinander für Wasserstoff, Halogen, Methyl oder CF₃ stehen;
G⁴ und G⁵ für Wasserstoff stehen;
R² für Fluor, Chlor, Brom, Iod, C₁-C₄-Alkoxy-C₁-C₄-Alkyl, C₁-C₄-Alkyl, Aryl-C₁-C₄-haloalkyl, C₁-C₆-Alkylsulfanyl, C₁-C₆-Alkylsulfinyl, C₁-C₆-Alkylsulfonyl oder C₁-C₄ Haloalkyl steht;
A¹ und A² unabhängig voneinander für N oder CH stehen;
R³ für Fluor, Chlor, Brom, Iod, C₁₋₂-Alkyl, C₁₋₂-Haloalkyl, C₁-C₂-Haloalkylsulfanyl, C₁-C₂-Haloalkylsulfonyl, C₁-C₂-Haloalkylsulfinyl oder Cyano steht;
R¹⁷ für Halogen, Alkylsulfanyl, Alkylsulfinyl, Alkylsulfonyl oder Cyano steht und
Q für Q¹, Q², Q³ oder Q⁴ steht.

In einer Ausführungsform 28 betrifft die Erfindung Verbindungen gemäß Ausführungsform 27, in denen Q für Q¹ steht.

In einer Ausführungsform 29 betrifft die Erfindung Verbindungen gemäß Ausführungsform 28, in denen Q¹ für Z³, Z⁷, Z¹⁵, Z¹⁶, Z¹⁷, Z¹⁸, Z²¹, Z²², Z²³ oder Z²⁴ steht; R⁶, R^{6'}, R^{6"}, R^{6"'} für Wasserstoff, Amino, Cyano, Fluor, Chlor, Methyl, Ethyl, C₁-C₂-Haloalkyl, Methoxy, Ethoxy oder C₁-C₂-Haloalkoxy stehen und R⁷ für Wasserstoff, Amino, Cyano, C₁-C₂-Alkyl, C₁-C₂-Haloalkyl oder C₁-C₂-Alkoxy steht.

In einer Ausführungsform 30 betrifft die Erfindung Verbindungen gemäß Ausführungsform 29, in denen Q¹ für Z¹⁶ oder Z³ steht.

In einer Ausführungsform 31 betrifft die Erfindung Verbindungen gemäß Ausführungsform 27, in denen Q für Q² steht.

In einer Ausführungsform 32 betrifft die Erfindung Verbindungen gemäß Ausführungsform 31, in denen Q² für C(O)NR⁸R⁹ steht und R⁸ und R⁹ unabhängig voneinander für Wasserstoff, C₁-C₄-Alkyl, C₁-C₄-Haloalkyl, C₃-C₅-Cycloalkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten), C₃-C₅-Cycloalkyl-C₁-C₂-alkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten, am C₁-C₂-Alkylteil gegebenenfalls ein- oder mehrfach substituiert durch Halogen, C₁-C₂-Alkyl, C₁-C₂-Haloalkyl, C₁-C₂-Alkoxy oder Cyano), C₁-C₂-Alkylsulfinyl-C₁-C₃-alkyl, C₁-C₂-Alkylsulfanyl-C₁-C₃-alkyl, C₁-C₂-Alkylsulfonyl-C₁-C₃-alkyl, Phenyl-C₁-C₄-alkyl oder Heteroaryl -C₁-C₄-alkyl stehen; wobei ein Phenyl- oder Heteroarylrest gegebenenfalls einoder mehrfach substituiert ist durch Halogen, C₁-C₂-Alkyl, C₁-C₂-Haloalkyl, C₁-C₂-Alkoxy, Nitro oder Cyano; und ein an Phenyl oder Heteroaryl gebundener Alkandiyl-Rest gegebenenfalls einoder mehrfach substituiert ist durch Halogen, C₁-C₄-Alkyl, C₁-C₄-Haloalkyl, C₁-C₂-Alkoxy oder Cyano.

In einer Ausführungsform 33 betrifft die Erfindung Verbindungen gemäß Ausführungsform 32, in denen R⁸ und R⁹ unabhängig voneinander für Wasserstoff, C₁-C₄-Alkyl, C₁-C₄-Haloalkyl, C₃-C₄-Cycloalkyl, C₃-C₄-Halocyloalkyl, C₃-C₅-Cycloalkyl-C₁-C₂-alkyl, C₃-C₅-Halocycloalkyl-C₁-C₂-alkyl, Methylalkylsulfinyl-C₁-C₃-alkyl, Methylalkylsulfanyl-C₁-C₃-alkyl, Methylalkylsulfonyl-C₁-C₃-alkyl, Phenylmethyl (am Aromaten gegebenenfalls ein- oder mehrfach substituiert durch Fluor, Chlor, Brom, Methyl, Ethyl, Trifluormethyl, Methoxy, Cyano oder Nitro, am Methylteil gegebenenfalls ein- oder mehrfach substituiert durch Methyl, Ethyl, Propyl, iso-Propyl, Methoxy oder Trifluormethyl), Pyridylmethyl (am Pyridyl gegebenenfalls ein- oder mehrfach substituiert durch Fluor, Chlor, Brom, Methyl, Ethyl, Trifluormethyl, Methoxy, Cyano oder Nitro, am Methylteil gegebenenfalls ein- oder mehrfach substituiert durch Methyl, Ethyl, Propyl, iso-Propyl, Methoxy oder Trifluormethyl) oder Pyrimidylmethyl (am Pyrimidyl gegebenenfalls ein- oder mehrfach substituiert durch Fluor, Chlor, Brom, Methyl, Ethyl, Trifluormethyl, Methoxy, Cyano oder Nitro, am Methylteil gegebenenfalls ein- oder mehrfach substituiert durch Methyl, Ethyl, Propyl, iso-Propyl, Methoxy oder Trifluormethyl) stehen.

In einer Ausführungsform 34 betrifft die Erfindung Verbindungen gemäß Ausführungsform 27, in denen Q für Q³ steht.

In einer Ausführungsform 35 betrifft die Erfindung Verbindungen gemäß Ausführungsform 34, in denen Q³ für C(R¹⁰R¹¹)NR⁸R⁹ steht; R¹⁰ und R¹¹ unabhängig voneinander für Wasserstoff, C₁-C₂-Alkyl oder C₁-C₂-Haloalkyl stehen und R⁸ und R⁹ unabhängig voneinander für Wasserstoff, C₁-C₄-Alkyl, C₁-C₄-Haloalkyl, C₃-C₄-Cycloalkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten), C₃-C₄-Cycloalkyl-C₁-C₂-alkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten, am C₁-C₂-Alkylteil gegebenenfalls ein- oder mehrfach substituiert durch Halogen, C₁-C₂-Alkyl, C₁-C₂-Haloalkyl, C₁-C₂-Alkoxy oder Cyano), C₁-C₂-Alkylsulfinyl-C₁-C₂-alkyl, C₁-C₂-Alkylsulfanyl-C₁-C₂-alkyl, C₁-C₂-Alkylsulfonyl-C₁-C₂-alkyl, Phenyl-C₁-C₄-alkyl, Heteroaryl-C₁-C₄-alkyl, C₁-C₄-Dialkylaminocarbonyl, C₁-C₄-Alkylaminocarbonyl, C₁-C₄-Haloalkylaminocarbonyl, C₁-C₂-Alkylsulfonyl, C₁-C₂-Alkylcarbonyl, C₁-C₂-Haloalkylcarbonyl, C₃-C₅-Cycloalkylcarbonyl, C₁-C₂-Alkoxycarbonyl, C₁-C₂-Alkoxy-C₁-C₂-alkylcarbonyl, Phenylsulfonyl, Phenyl, Heteroaryl, Hetero-C₃-C₅-cyclyl, Phenylcarbonyl, Heteroarylcarbonyl, Hetero-C₃-C₅-cyclylcarbonyl, Phenyl-C₁-C₂-alkylcarbonyl, Heteroaryl-C₁-C₂-alkylcarbonyl, Hetero-C₃-C₅-cyclyl-C₁-C₂-alkylcarbonyl, Phenoxycarbonyl oder Phenyl-C₁-C₂-alkoxycarbonyl stehen; wobei ein Phenyl- oder Heteroarylrest gegebenenfalls ein- oder mehrfach substituiert ist durch Halogen, C₁-C₂-Alkyl, C₁-C₂-Haloalkyl, C₁-C₂-Alkoxy, Nitro oder Cyano; und ein an Phenyl oder Heteroaryl gebundener Alkandiyl-Rest gegebenenfalls ein- oder mehrfach substituiert ist durch Halogen, C₁-C₄-Alkyl, C₁-C₄-Haloalkyl, C₁-C₂-Alkoxy oder Cyano.

In einer Ausführungsform 36 betrifft die Erfindung Verbindungen gemäß Ausführungsform 35, in denen R⁸ und R⁹ unabhängig voneinander für Wasserstoff, C₁-C₂-Alkyl, C₁-C₂-Haloalkyl, C₃-C₅-Cycloalkyl, C₃-C₅-Cycloalkyl-C₁-C₂-alkyl, C₃-C₅-Halocycloalkyl, C₃-C₅-Halocycloalkyl-C₁-C₂-alkyl, Methylsulfinyl-C₁-C₃-alkyl, Methylsulfanyl-C₁-C₃-alkyl, Methylsulfonyl-C₁-C₃-alkyl, Phenyl-C₁-C₂-alkyl, Heteroaryl-C₁-C₂-alkyl, C₁-C₂-Dialkylaminocarbonyl, C₁-C₂-Alkylaminocarbonyl, C₁-C₂-Haloalkylaminocarbonyl, C₁-C₂-Alkylsulfonyl, C₁-C₂-Alkylcarbonyl, C₁-C₂-Haloalkylcarbonyl, C₃-C₅-Cycloalkylcarbonyl, C₁-C₂-Alkoxycarbonyl, Phenylsulfonyl, Phenylcarbonyl, Heteroarylcarbonyl, Hetero-C₃-C₅-cyclylcarbonyl, Phenyl-C₁-C₂-alkylcarbonyl, Heteroaryl-C₁-C₂-alkylcarbonyl, Hetero-C₃-C₅-cyclyl-C₁-C₂-alkylcarbonyl oder Phenoxycarbonyl stehen; wobei ein Phenyl- oder Heteroarylrest gegebenenfalls ein- oder mehrfach substituiert ist durch Halogen, C₁-C₂-Alkyl, C₁-C₂-Haloalkyl, C₁-C₂-Alkoxy, Nitro oder Cyano; und ein an Phenyl oder Heteroaryl gebundener Alkandiyl-Rest gegebenenfalls ein- oder mehrfach substituiert ist durch Halogen, C₁-C₄-Alkyl, C₁-C₄-Haloalkyl, C₁-C₂-Alkoxy oder Cyano.

In einer Ausführungsform 37 betrifft die Erfindung Verbindungen gemäß Ausführungsform 27, in denen Q für Q⁴ steht.

In einer Ausführungsform 38 betrifft die Erfindung Verbindungen gemäß Ausführungsform 37, in denen Q⁴ für Cyano (wobei R¹ nicht für Amino steht), Nitro, Amino, COOH, COOR¹², Fluor (falls R³ verschieden von Chlor ist), Chlor (falls R³ verschieden von Chlor, COOH, CH₂CH₂OMe und OMe ist), Brom, Iod, SR¹² (wobei R¹ nicht für Amino steht, falls R¹² für Alkyl steht), S(O)R¹² oder S(O)₂R¹² steht und R¹² für C₁-C₄-Alkyl oder C₁-C₄-Haloalkyl steht.

In einer Ausführungsform 39 betrifft die Erfindung Verbindungen gemäß Ausführungsform 38, in denen Q⁴ für Cyano (wobei R¹ nicht für Amino steht), COOH, COOMe, COOEt, Fluor (falls R³ verschieden von Chlor ist), Brom, Iod, SR¹² (wobei R¹ nicht für Amino steht, falls R¹² für Alkyl steht), S(O)R¹² oder S(O)₂R¹² steht.

In einer Ausführungsform 40 betrifft die Erfindung Verbindungen der Formel (V-A) 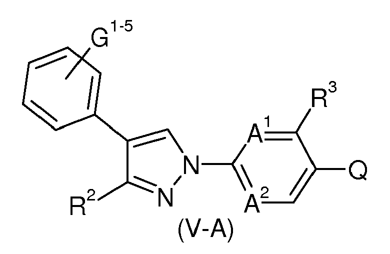 wobei
G¹, G² und G³ unabhängig voneinander für Wasserstoff, Halogen, Methyl oder CF₃ stehen;
G⁴ und G⁵ für Wasserstoff stehen;
R² für Fluor, Chlor, Brom, Iod, C₁-C₄-Alkoxy-C₁-C₄-Alkyl, C₁-C₄-Alkyl, Aryl-C₁-C₄-haloalkyl, C₁-C₆-Alkylsulfanyl, C₁-C₆-Alkylsulfinyl, C₁-C₆-Alkylsulfonyl oder C₁-C₄ Haloalkyl steht;
A¹ und A² unabhängig voneinander für N oder CH stehen;
R³ für Fluor, Chlor, Brom, Iod, C₁₋₂-Alkyl, C₁₋₂-Haloalkyl, C₁-C₂-Haloalkylsulfanyl, C₁-C₂-Haloalkylsulfonyl, C₁-C₂-Haloalkylsulfinyl oder Cyano steht und
Q für Q¹, Q², Q³ oder Q⁴ steht.

In einer Ausführungsform 41 betrifft die Erfindung Verbindungen gemäß Ausführungsform 40, in denen Q für Q¹ steht.

In einer Ausführungsform 42 betrifft die Erfindung Verbindungen gemäß Ausführungsform 41, in denen Q¹ für Z³, Z⁷, Z¹⁵, Z¹⁶, Z¹⁷, Z¹⁸, Z²¹, Z²², Z²³ oder Z²⁴ steht; R⁶, R^{6'}, R^{6"}, R^{6"'} für Wasserstoff, Amino, Cyano, Fluor, Chlor, Methyl, Ethyl, C₁-C₂-Haloalkyl, Methoxy, Ethoxy oder C₁-C₂-Haloalkoxy stehen und R⁷ für Wasserstoff, Amino, Cyano, C₁-C₂-Alkyl, C₁-C₂-Haloalkyl oder C₁-C₂-Alkoxy steht.

In einer Ausführungsform 43 betrifft die Erfindung Verbindungen gemäß Ausführungsform 42, in denen Q¹ für Z¹⁶ oder Z³ steht.

In einer Ausführungsform 44 betrifft die Erfindung Verbindungen gemäß Ausführungsform 40, in denen Q für Q² steht.

In einer Ausführungsform 45 betrifft die Erfindung Verbindungen gemäß Ausführungsform 44, in denen Q² für C(O)NR⁸R⁹ steht und R⁸ und R⁹ unabhängig voneinander für Wasserstoff, C₁-C₄-Alkyl, C₁-C₄-Haloalkyl, C₃-C₅-Cycloalkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten), C₃-C₅-Cycloalkyl-C₁-C₂-alkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten, am C₁-C₂-Alkylteil gegebenenfalls ein- oder mehrfach substituiert durch Halogen, C₁-C₂-Alkyl, C₁-C₂-Haloalkyl, C₁-C₂-Alkoxy oder Cyano), C₁-C₂-Alkylsulfinyl-C₁-C₃-alkyl, C₁-C₂-Alkylsulfanyl-C₁-C₃-alkyl, C₁-C₂-Alkylsulfonyl-C₁-C₃-alkyl, Phenyl-C₁-C₄-alkyl oder Heteroaryl -C₁-C₄-alkyl stehen; wobei ein Phenyl- oder Heteroarylrest gegebenenfalls einoder mehrfach substituiert ist durch Halogen, C₁-C₂-Alkyl, C₁-C₂-Haloalkyl, C₁-C₂-Alkoxy, Nitro oder Cyano; und ein an Phenyl oder Heteroaryl gebundener Alkandiyl-Rest gegebenenfalls einoder mehrfach substituiert ist durch Halogen, C₁-C₄-Alkyl, C₁-C₄-Haloalkyl, C₁-C₂-Alkoxy oder Cyano.

In einer Ausführungsform 46 betrifft die Erfindung Verbindungen gemäß Ausführungsform 45, in denen R⁸ und R⁹ unabhängig voneinander für Wasserstoff, C₁-C₄-Alkyl, C₁-C₄-Haloalkyl, C₃-C₄-Cycloalkyl, C₃-C₄-Halocycloalkyl, C₃-C₅-Cycloalkyl-C₁-C₂-alkyl, C₃-C₅-Halocycloalkyl-C₁-C₂alkyl, Methylalkylsulfinyl-C₁-C₃-alkyl, Methylalkylsulfanyl-C₁-C₃-alkyl, Methylalkylsulfonyl-C₁-C₃-alkyl, Phenylmethyl (am Aromaten gegebenenfalls ein- oder mehrfach substituiert durch Fluor, Chlor, Brom, Methyl, Ethyl, Trifluormethyl, Methoxy, Cyano oder Nitro, am Methylteil gegebenenfalls ein- oder mehrfach substituiert durch Methyl, Ethyl, Propyl, iso-Propyl, Methoxy oder Trifluormethyl), Pyridylmethyl (am Pyridyl gegebenenfalls ein- oder mehrfach substituiert durch Fluor, Chlor, Brom, Methyl, Ethyl, Trifluormethyl, Methoxy, Cyano oder Nitro, am Methylteil gegebenenfalls ein- oder mehrfach substituiert durch Methyl, Ethyl, Propyl, iso-Propyl, Methoxy oder Trifluormethyl) oder Pyrimidylmethyl (am Pyrimidyl gegebenenfalls ein- oder mehrfach substituiert durch Fluor, Chlor, Brom, Methyl, Ethyl, Trifluormethyl, Methoxy, Cyano oder Nitro, am Methylteil gegebenenfalls ein- oder mehrfach substituiert durch Methyl, Ethyl, Propyl, iso-Propyl, Methoxy oder Trifluormethyl) stehen.

In einer Ausführungsform 47 betrifft die Erfindung Verbindungen gemäß Ausführungsform 40, in denen Q für Q³ steht.

In einer Ausführungsform 48 betrifft die Erfindung Verbindungen gemäß Ausführungsform 47, in denen Q³ für C(R¹⁰R¹¹)NR⁸R⁹ steht; R¹⁰ und R¹¹ unabhängig voneinander für Wasserstoff, C₁-C₂-Alkyl oder C₁-C₂-Haloalkyl stehen und R⁸ und R⁹ unabhängig voneinander für Wasserstoff, C₁-C₄-Alkyl, C₁-C₄-Haloalkyl, C₃-C₄-Cycloalkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten), C₃-C₄-Cycloalkyl-C₁-C₂-alkyl (am Cyclus gegebenenfalls ein- oder mehrfach substituiert durch Halogen, C₁-C₂-Haloalkyl, C₁-C₂-Alkyl oder kondensiert an einen Aromaten oder Heteroaromaten, am C₁-C₂-Alkylteil gegebenenfalls ein- oder mehrfach substituiert durch Halogen, C₁-C₂-Alkyl, C₁-C₂-Haloalkyl, C₁-C₂-Alkoxy oder Cyano), C₁-C₂-Alkylsulfinyl-C₁-C₂-alkyl, C₁-C₂-Alkylsulfanyl-C₁-C₂-alkyl, C₁-C₂-Alkylsulfonyl-C₁-C₂-alkyl, Phenyl-C₁-C₄-alkyl, Heteroaryl-C₁-C₄-alkyl, C₁-C₄-Dialkylaminocarbonyl, C₁-C₄-Alkylaminocarbonyl, C₁-C₄-Haloalkylaminocarbonyl, C₁-C₂-Alkylsulfonyl, C₁-C₂-Alkylcarbonyl, C₁-C₂-Haloalkylcarbonyl, C₃-C₅-Cycloalkylcarbonyl, C₁-C₂-Alkoxycarbonyl, C₁-C₂-Alkoxy-C₁-C₂-alkylcarbonyl, Phenylsulfonyl, Phenyl, Heteroaryl, Hetero-C₃-C₅-cyclyl, Phenylcarbonyl, Heteroarylcarbonyl, Hetero-C₃-C₅-cyclylcarbonyl, Phenyl-C₁-C₂-alkylcarbonyl, Heteroaryl-C₁-C₂-alkylcarbonyl, Hetero-C₃-C₅-cyclyl-C₁-C₂-alkylcarbonyl, Phenoxycarbonyl oder Phenyl-C₁-C₂-alkoxycarbonyl stehen; wobei ein Phenyl- oder Heteroarylrest gegebenenfalls ein- oder mehrfach substituiert ist durch Halogen, C₁-C₂-Alkyl, C₁-C₂-Haloalkyl, C₁-C₂-Alkoxy, Nitro oder Cyano; und ein an Phenyl oder Heteroaryl gebundener Alkandiyl-Rest gegebenenfalls ein- oder mehrfach substituiert ist durch Halogen, C₁-C₄-Alkyl, C₁-C₄-Haloalkyl, C₁-C₂-Alkoxy oder Cyano.

In einer Ausführungsform 49 betrifft die Erfindung Verbindungen gemäß Ausführungsform 48, in denen R⁸ und R⁹ unabhängig voneinander für Wasserstoff, C₁-C₂-Alkyl, C₁-C₂-Haloalkyl, C₃-C₅-Cycloalkyl, C₃-C₅-Cycloalkyl-C₁-C₂-alkyl, C₃-C₅-Halocycloalkyl, C₃-C₅-Halocycloalkyl-C₁-C₂-alkyl, Methylsulfinyl-C₁-C₃-alkyl, Methylsulfanyl-C₁-C₃-alkyl, Methylsulfonyl-C₁-C₃-alkyl, Phenyl-C₁-C₂-alkyl, Heteroaryl-C₁-C₂-alkyl, C₁-C₂-Dialkylaminocarbonyl, C₁-C₂-Alkylaminocarbonyl, C₁-C₂-Haloalkylaminocarbonyl, C₁-C₂-Alkylsulfonyl, C₁-C₂-Alkylcarbonyl, C₁-C₂-Haloalkylcarbonyl, C₃-C₅-Cycloalkylcarbonyl, C₁-C₂-Alkoxycarbonyl, Phenylsulfonyl, Phenylcarbonyl, Heteroarylcarbonyl, Hetero-C₃-C₅-cyclylcarbonyl, Phenyl-C₁-C₂-alkylcarbonyl, Heteroaryl-C₁-C₂-alkylcarbonyl, Hetero-C₃-C₅-cyclyl-C₁-C₂-alkylcarbonyl oder Phenoxycarbonyl stehen; wobei ein Phenyl- oder Heteroarylrest gegebenenfalls ein- oder mehrfach substituiert ist durch Halogen, C₁-C₂-Alkyl, C₁-C₂-Haloalkyl, C₁-C₂-Alkoxy, Nitro oder Cyano; und ein an Phenyl oder Heteroaryl gebundener Alkandiyl-Rest gegebenenfalls ein- oder mehrfach substituiert ist durch Halogen, C₁-C₄-Alkyl, C₁-C₄-Haloalkyl, C₁-C₂-Alkoxy oder Cyano.

In einer Ausführungsform 50 betrifft die Erfindung Verbindungen gemäß Ausführungsform 40, in denen Q für Q⁴ steht.

In einer Ausführungsform 51 betrifft die Erfindung Verbindungen gemäß Ausführungsform 50, in denen Q⁴ für Cyano (wobei R¹ nicht für Amino steht), Nitro, Amino, COOH, COOR¹², Fluor (falls R³ verschieden von Chlor ist), Chlor (falls R³ verschieden von Chlor, COOH, CH₂CH₂OMe und OMe ist), Brom, Iod, SR¹² (wobei R¹ nicht für Amino steht, falls R¹² für Alkyl steht), S(O)R¹² oder S(O)₂R¹² steht und R¹² für C₁-C₄-Alkyl oder C₁-C₄-Haloalkyl steht.

In einer Ausführungsform 52 betrifft die Erfindung Verbindungen gemäß Ausführungsform 51, in denen Q⁴ für Cyano (wobei R¹ nicht für Amino steht), COOH, COOMe, COOEt, Fluor (falls R³ verschieden von Chlor ist), Brom, Iod, SR¹² (wobei R¹ nicht für Amino steht, falls R¹² für Alkyl steht), S(O)R¹² oder S(O)₂R¹² steht.

Gegenstand der vorliegenden Erfindung ist auch die Verwendung der Verbindungen der Formel (I) zur Bekämpfung tierischer Schädlinge, wobei die Reste wie oben definiert sind.

Gegenstand der vorliegenden Erfindung ist auch die Verwendung der Verbindungen der Formel (I) zur Bekämpfung tierischer Schädlinge, wobei M für M¹ steht, Q für Q⁴ steht, Q⁴ für Wasserstoff steht und alle anderen Reste wie oben definiert sind, mit der Maßgabe, dass A¹ und A² nicht gleichzeitig für Stickstoff stehen.

Gegenstand der vorliegenden Erfindung ist auch die Verwendung der Verbindungen der Formel (I) zur Bekämpfung tierischer Schädlinge, wobei wobei M für M¹ steht, R³ für Wasserstoff steht und alle anderen Reste wie oben definiert sind, mit der Maßgabe, dass A¹ und A² nicht gleichzeitig für Stickstoff stehen.

Die erfindungsgemäßen Verbindungen können in unten genannter oder hierzu analoger Weise hergestellt werden.

### Verfahren (A)

Verbindungen der Formel (II) werden z.B. nach folgendem Verfahren (A) synthetisiert: 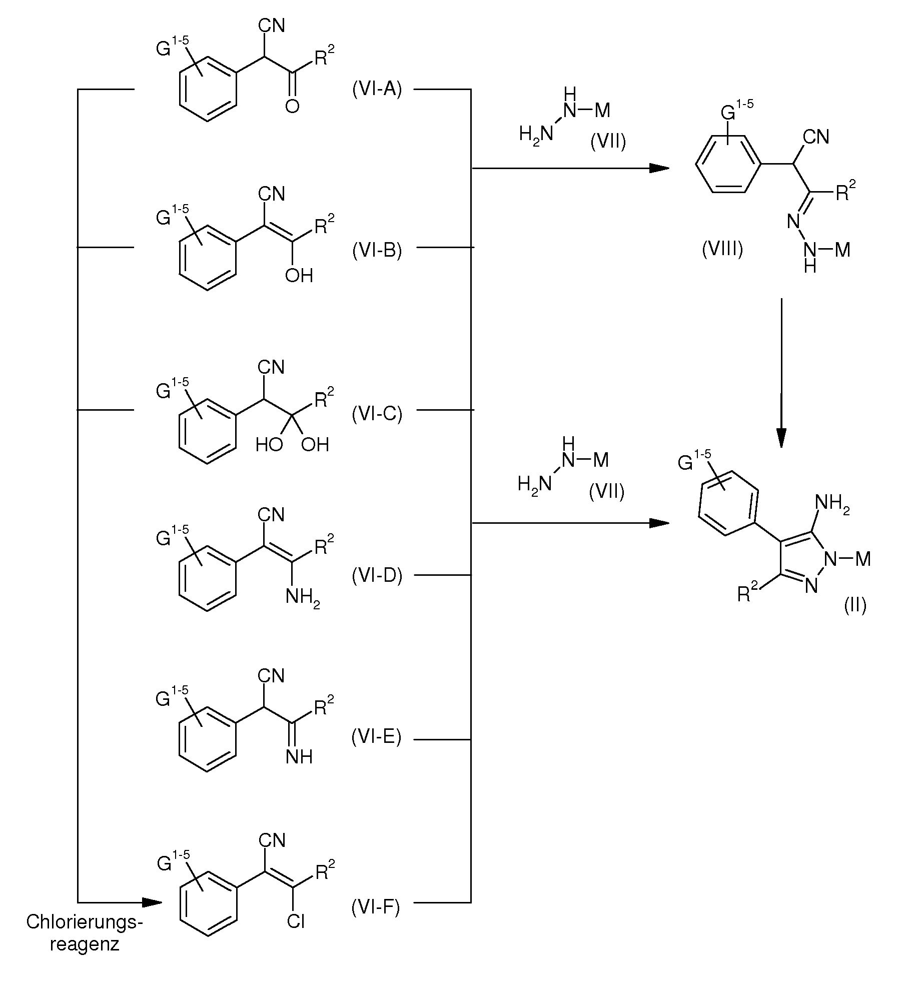 wobei
G¹⁻⁵, R² und M die oben angegebenen Bedeutungen haben;
Beim Verfahren (A) zur Herstellung der Verbindungen der Formel (II) werden Ketonitrile, deren Tautomere oder Hydrate der Formeln (VI-A), (VI-B) bzw. (VI-C) oder Aminoacrylonitrile und deren Tautomere der Formeln (VI-D) und (IV-E) mit Aryl- oder Heteroaryl-Hydrazinen der Formel (VII) kondensiert, wobei zunächst Hydrazone der Formel (VIII) als Intermediat entstehen und bei längerer Reaktionszeit und höherer Temperatur der Ringschluss zum Aminopyrazol der Formel (II) erfolgt. Dabei können Säuren als Katalysator zugesetzt werden, wobei anorganische Säuren wie Salzsäure und organische Säuren wie Sulfonsäuren oder Essigsäure geeignet sein können.

Alternativ werden Ketonitrile, deren Tautomere oder Hydrate der Formeln (VI-A), (VI-B) bzw. (VI-C) zunächst mit einem Chlorierungsmittel, z.B. Phosphorylchlorid, Phosphorpentachlorid, Thionylchlorid, Phosgen, Chlor oder Oxalylchlorid, gegebenenfalls in einem inerten organischen Lösungsmittel verdünnt, zu Chloracrylnitrilen (VI-F) umgesetzt, wobei die Reaktion im Temperaturbereich von -20 °C bis 120°C durchgeführt werden kann. In einem anschließenden Schritt erfolgt die Kondensation mit Aryl oder Heteroaryl-Hydrazinen der Formel (VII) in einem geeigneten organischen Lösungsmittel in Gegenwart von basischen Hilfsreagentien, z.B. Alkoholaten oder Stickstoffbasen, wobei die Reaktion im Temperaturbereich von -20 °C bis 120°C durchgeführt werden kann.

Synthesemethoden strukturverwandter Aminopyrazolen ist bekannt und wird beispielsweise in J. Med. Chem. 2006, 14(6), 1785-1791; Eur. J. Med. Chem. 2005, 40, 922-927; J. Chem. Res. Synopses 1985, 5, 198-199; J. Mol. Cat. A 2006, 258(1-2), 371-375; J. Med. Chem. 2006, 49, 3332-3344; Bioorg. Med. Chem. 2006, 14(6), 1785-1791; Eur. J. Med. Chem. 2005, 40(9), 922-927; Tetrahedron 2004, 60(4), 901-906; J. Heterocyclic Chem. 1975, 12(5), 899-901, Molecular Div. 2003, 7(2-4), 161-164; DE -A-2643640; und US 3,041,342 beschrieben.

Die Ketonitrile können in den tautomeren Formen (VI-A) und (VI-B) und als Hydrat (VI-C) vorliegen. Die Ausgangsverbindungen können auch in Form ihrer Salze eingesetzt werden, z.B. können die Ketonitrile in Form ihrer Alkalisalze verwendet werden.

Die Aminoacrylnitrile können in den tautomeren Formen (VI-D) und (VI-E) vorliegen. Die Ausgangsverbindungen können auch in Form ihrer Salze eingesetzt werden, z.B. können die Aminoacrylnitrile in Form ihrer Alkalisalze verwendet werden.

Ketonitrile der Formel (VI-A, VI-B und VI-C) lassen sich nach bekannten Methoden herstellen. Solche Methoden sind beschrieben in: J. Amer. Chem. Soc. 1950, 72, 5409-5413; Tetrahedron 2007, 63(47), 11626-11635, Org. Lett. 2007, 9(18), 3575-3578, J. Med. Chem. 2007, 50(2), 399-403, Bioorg. Med. Chem. Lett. 2006, 16(3), 695-700.

Acrylonitrile der Formel (VI-D und VI-E) lassen sich nach bekannten Methoden herstellen. Solche Methoden beschrieben in: J. Med. Chem. 2006, 49, 3332-3344.

Chloracrylnitrile der Formel (VI-F) lassen sich nach bekannten Methoden herstellen. Solche Methoden beschrieben in: J. Org Chem (UdSSR) 1981, 1441, JP 08-208620, J. Med. Chem. 2005, 48(22), 6843-6854, Nucleosides, Nucleotides Nucleic Acids 2004, 23(5), 805-812, J. Med. Chem. 2001, 44(3), 350-361.

Aryl-, Pyridyl- und Pyrimidinylhydrazine der Formel (VII) sind zum Teil kommerziell verfügbar oder lassen sich nach dem Fachmann bekannten Methoden, wie z.B. im Verfahren (H) beschrieben, herstellen.

Die zum Aufbau von Q¹⁻³ benötigten Amidierungs-, Acylierungs- und Substitutionsreaktionen werden nach dem Fachmann bekannten Methoden, wie z.B. in den Verfahren (F), (G) und (J) beschrieben, durchgeführt und können entweder am Ende der Synthesefrequenz oder bevorzugt auf der Stufe von geeigneten Intermediaten erfolgen.

### Verfahren (B)

Erfindungsgemäße Verbindungen der Formel (I) werden z.B. nach folgendem Verfahren (B) synthetisiert: 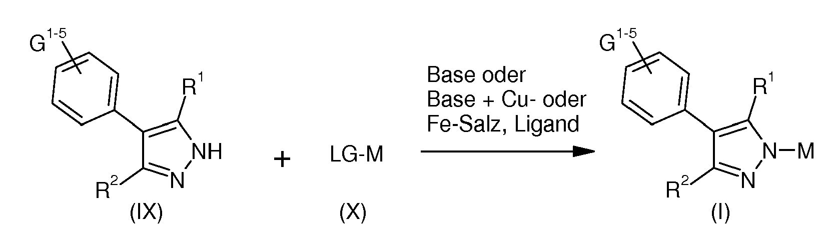 wobei
LG = Halogen, Alkylsulfonyl, Boronsäure oder Boronsäureester

Die Zwischenprodukte (IX) werden nach Verfahren (A) mit Hydrazinhydrat hergestellt:

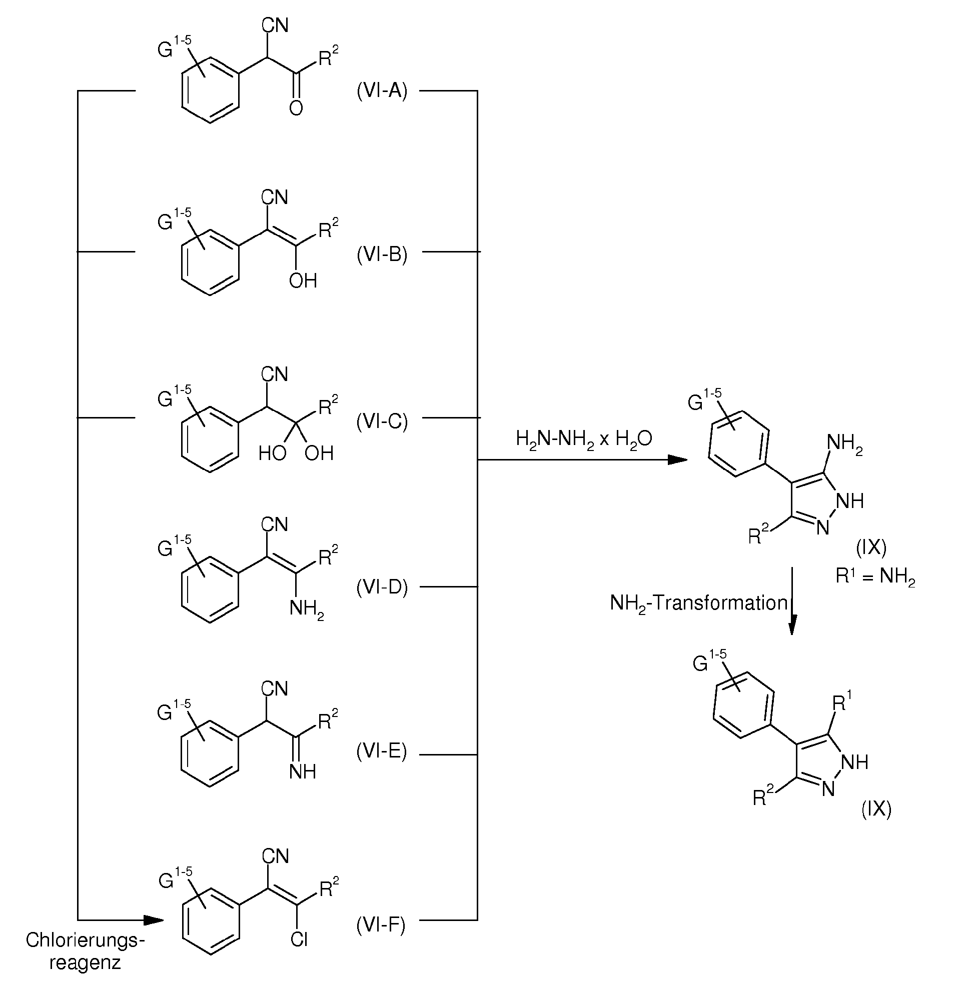

Beim Verfahren (B) werden 1-H-Arninopyrazole der Formel (IX) mit Arylhalogeniden, Heteroarylhalogeniden, Arylalkylsulfonen Heteroarylalkylsulfonen, Arylboronsäuren, Heteroarylboronsäuren, Arylboronsäureestern oder Heteroarylboronsäureestern (LG-M) in Gegenwart einer Base und eventuell eines Kupfer- oder Eisensalzes und eines Liganden in einem geeigneten organischen Lösungsmitteln umgesetzt, wobei bevorzugt ein bestimmtes Isomer, das Aminopyrazol der Formel (I), gebildet wird.

Die Synthese strukturverwandter Aminopyrazolen der Formel (IX) ist allgemein bekannt und wird beschrieben in: DE-A 2643640, Bioorg. Med. Chem. Lett. 2004, 14, 3669, Bioorg. Med. Chem. 2000, 8(1), 181-190, J. Comb. Chem. 2007, 9(3), 507-512, J. Med. Chem. 2004, 47(24), 5872-5893, EP-A-269859, J. Prakt. Chem. 1979, 321(2), 341-344,

Die Cu- und Fe-katalysierte Arylierung von Pyrazolen ist bekannt und wird beschrieben in: J. Org. Chem. 2004, 69(17), 5578-5587, Angew. Chem. Int. Ed. 2007, 46, 934-936, Bioorg. Med. Chem. Lett. 2007, 17(15), 4303-4307, IN 178903, Bioorg. Med. Chem. 2007, 17(2), 354-357, J. Med. Chem. 2004, 47(19), 4645-4648.

Die Synthese von Aminopyrazolen durch nukleophile Aromaten- und Heteroaromatensubstitution ist bekannt und wird beschrieben in: J. Med. Chem. 2004, 47, 2995-3008, JP 2007-169575, Synth. Commun. 2006, 36(11), 1601-1612, Can. J. Chem. 1988, 66(3), 420-428.

Die Ketonitrile können in den tautomeren Formen (VI-A) und (VI-B) und als Hydrat (VI-C) vorliegen. Die Ausgangsverbindungen können auch in Form ihrer Salze eingesetzt werden, z.B. können die Ketonitrile in Form ihrer Alkalisalze verwendet werden.

Die Aminoacrylnitrile können in den tautomeren Formen (VI-D) und (VI-E) vorliegen. Die Ausgangsverbindungen können auch in Form ihrer Salze eingesetzt werden, z.B. können die Aminoacrylnitrile in Form ihrer Alkalisalze verwendet werden.

Die Ketonitrile der Formel (VI-A, VI-B und VI-C) lassen sich nach bekannten Methoden herstellen: J. Amer. Chem. Soc. 1950, 72, 5409-5413; Tetrahedron 2007, 63(47), 11626-11635; Org. Lett. 2007, 9(18), 3575-3578; J. Med. Chem. 2007, 50(2), 399-403, Bioorg. Med. Chem. Lett. 2006, 16(3), 695-700.

Die Acrylonitrile der Formel (VI-D und VI-E) lassen sich nach bekannten Methoden herstellen: J. Med. Chem. 2006, 49, 3332-3344

Die Chloracrylnitrile der Formel (VI-F) lassen sich nach bekannten Methoden herstellen: J. Org. Chem. (UdSSR) 1981, 1441 JP 08208620 , J. Med. Chem., 2005, 48(22), 6843-6854, Nucleosides, Nucleotides and Nucleic Acids, 2004, 23(5), 805-812, J. Med. Chem., 2001, 44(3), 350-361

Die Aryl- und Heteroarylverbindungen LG-M sind zum Teil kommerziell verfügbar oder lassen sich nach dem Fachmann bekannten Methoden herstellen.

Die zum Aufbau von Q¹⁻³ benötigten Amidierungs-, Acylierungs und Substitutionsreaktionen werden nach dem Fachmann bekannten Methoden, wie z.B. in den Verfahren (F), (G) und (I) beschrieben, durchgeführt und können entweder am Ende der Synthesefrequenz oder bevorzugt auf der Stufe von geeigneten Intermediaten erfolgen.

Gegenstand der vorliegenden Erfindung sind auch Verbindungen der Formel (IX) 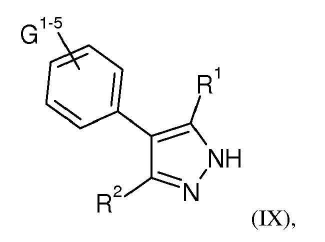 wobei G¹, G², G³, G⁴, G⁵, R¹ und R² wie oben definiert sind, mit der Maßgabe, dass R¹ nicht für Amino steht.

### Verfahren (C)

Erfindungsgemäße Verbindungen der Formel (IIIa, IIIb, IIIc und IIId) lassen sich nach dem Verfahren (C) synthetisieren: 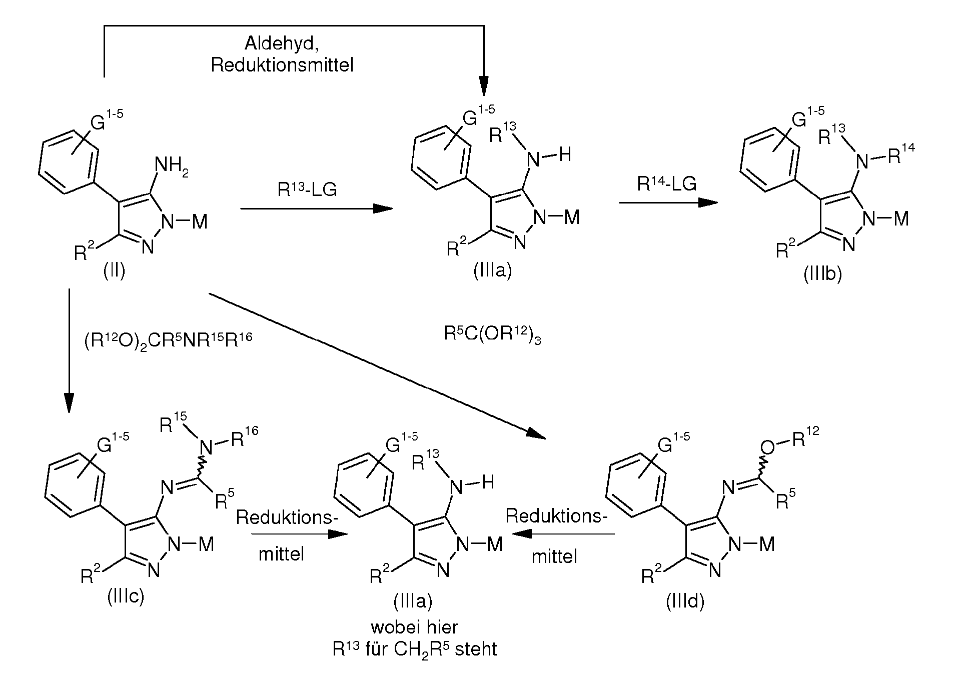 wobei
G¹⁻⁵, R², R¹³, R¹⁴ und M die oben angegebenen Bedeutungen haben können, R¹³ und R¹⁴ aber nicht beide gleichzeitig für Wasserstoff stehen;
LG für Halogen oder Alkylsulfonyl steht.

Beim erfindungsgemäßen Verfahren (C) zur Herstellung der Verbindungen der Formel (IIIa) und (IIIb) werden Verbindungen der Formel (II) mit einem oder zwei Alkylierungsmitteln, Acylierungsmitteln oder Sulfonylierungsmitteln R13-LG bzw. R¹⁴-LG umgesetzt, wobei durch Monosubstitution und Disubstitution Aminopyrazole der Formel (IIIa) bzw. (IIIb) entstehen. Geeignete Alkylierungsmittel sind Alkylbromide, Alkyldibromide, Alkyliodide, Alkyldiiodide, Dialkylsulfate und Alkylsulfonate. Als Acylierungsmittel werden Carbonsäureanhydride und Carbonsäurechloride, als Sulfonylierungsmittel Sulfonylchloride verwendet. Alternativ können die einfach N-substituierten Aminopyrazole der Formel (IIIa) durch reduktive Aminierung aus den Aminopyrazolen der Formel (II), einem Aldehyd und einem Reduktionsmittel, z.B. Wasserstoff in Gegenwart eines Hydrierkatalysators, Alkaliborhydriden oder Boran erhalten werden.

Alternativ können Verbindungen der Formel (IIIa) durch Umsetzung von Verbindungen der Formel (II) zu Amidinen der Formel (IIIc) oder Imidoestern der Formel (IIId), gefolgt von einem Reduktionsschritt, erhalten werden.

Die Alkylierung von strukturverwandten Aminopyrazolen wird beschrieben in:, WO 2006/000312, WO 2005/09312, WO 2005/023761, WO 2004/099156

Die reduktive Aminierung von strukturverwandten Aminopyrazolen wird beschrieben in: J. Med. Chem. 2002, 45(14), 2994-3008, WO 2002/010153, Tetrahedron Lett. 2001, 42(21), 3587-3590

Die Acylierung von strukturverwandten Aminopyrazolen wird beschrieben in:, J. Med. Chem. 2006, 49(11), 3332-3344, J. Med. Chem. 2007, 50(21), 5161-5167, Bioorg. Med. Chem. 2006, 14(22), 7501-7511, Org. Biomol. Chem. 2004, 2(11), 1603-1611.

Die Sulfonylierung von strukturverwandten Aminopyrazolen wird beschrieben in:, Acta Poloniae Pharmaceutica 2003, 60(1), 51-60, Biochemistry 2003, 42(21), 6363-6369, J. Med. Chem. 2001, 44(22), 3622-3631.

Die Synthese von strukturverwandten Iminen der Formel (IIIc) wird beschrieben in:, Tetrahedron 2006, 62(24), 5617-5625, Tetrahedron 1987, 53(18), 4185-4193.

Die Synthese von strukturverwandten Amidinen (IIIc) und Imidoestern (IIId) und deren Reduktion wird beschrieben in: WO 2005/060749.

### Verfahren (D)

Erfindungsgemäße Verbindungen der Formel (III) und (IVa bis IVe) werden z.B. nach Verfahren (D) synthetisiert: 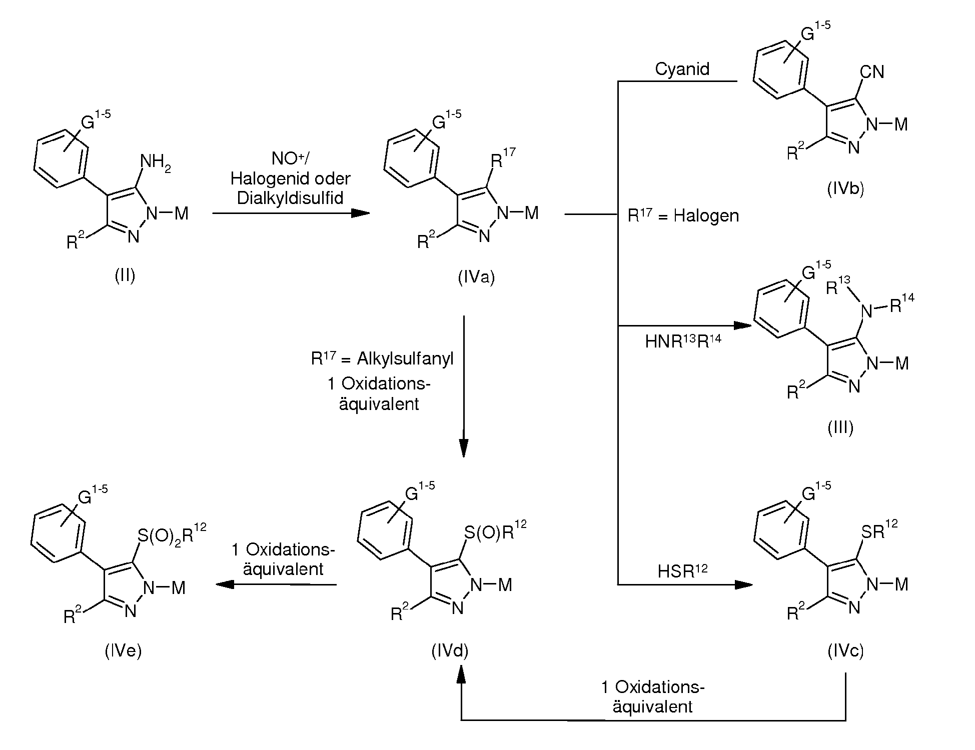 wobei
G¹⁻⁵, R², R¹², R¹³, R¹⁴ und M die oben beschriebenen Bedeutungen haben;
R¹⁷ für Halogen oder Alkylsulfanyl steht.

Beim erfindungsgemäßen Verfahren (D) zur Herstellung der Verbindungen der Formel (III) und (IV) werden Aminopyrazole der Formel (II) mit Nitrosylspezies in Gegenwart von geeigneten Halogeniden umgesetzt, wobei nach der Bildung eines Diazo-Intermediates ein 5-Halogenpyrazol der Formel (IVa, R¹⁷ = Halogen) entsteht. Geeignete Quellen für Nitrosylspezies sind Alkalinitrite plus Säuren sowie Ester der salpetrigen Säure, z.B. Butylnitrit und tert-Butylnitrit. Als Halogenide können Metallhalogenide sowie organische Halogenide, z.B. Bromoform oder Iodoform verwendet werden. Diese 5-Halogenpyrazole können mit Cyaniden, z.B. CuCN in geeigneten Lösungsmitteln, z.B. NMP unter Hitzezufuhr in 5-Cyanopyrazole der Formel (IVb) oder mit Aminen in geeigneten Lösungsmitteln in 5-Aminopyrazole der Formel (III), umgewandelt werden, wobei R¹³ oder R¹⁴ ungleich Wasserstoff ist. Die Umsetzung der Diazo-Intermediate mit Alkylsulfanylquellen, z.B. Dialkyldisulfiden führt zu 5-Alkylsulfanylpyrazolen der Formel (IVa, R¹⁷ = Alkylsulfanyl). Diese Thioether können in Gegenwart eines geeigneten Oxidationsmittels, z.B. mit H₂O₂, Natriumperiodat, *tert*.-Butylhypochlorit, Calciumhypochlorit Ca(OCl)₂, Natriumchlorit NaClO₂, Natriumhypochlorit NaOCl, Persäuren oder O₂ und katalytischem Cerammoniumnitrat zu 5-Alkylsulfinylpyrazolen der Formel (IVd) und 5-Alkylsulfonylpyrazolen der Formel (IVe) oxidiert werden.

Die Synthese von strukturverwandten 5-Halogen- und 5-Alkylsulfanylpyrazolen wird beschrieben in, WO 2003/074492, J. Heterocycl. Chem. 1987, 24, 267-270, GB 2149402A, J. Med. Chem. 2006, 49, 1562-1575.

Die Synthese von strukturverwandten 5-Cyanopyrazolen wird beschrieben in:, J. Med. Chem. 2006, 49, 1562-1575

Die Synthese von N-substituierten Aminopyrazolen der Formel (III) wird beschrieben in:, DE 3520327, WO 2005/023776

Die Oxidation von Thioethern wird beschrieben in :, J. March "Advanced Organic Chemistry" 4. Auflage, John Wiley & Sons, 1992, S. 1202 und die dort zitierte Literatur

### Verfahren (E)

Erfindungsgemäße Verbindungen der Formel (I) werden z.B. auch nach folgendem Verfahren (E) synthetisiert: 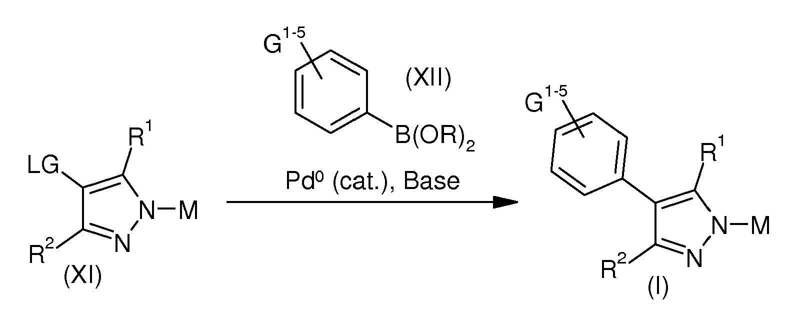 wobei G¹⁻⁵, R¹, R² und M die oben angegebenen Bedeutungen haben und LG für Brom, Iod oder Alkylsulfonyl steht.

Beim erfindungsgemäßen Verfahren (E) zur Herstellung der Verbindungen der Formel (I) werden Bromide oder Iodide der Formel (XI) mit Boronsäuren oder Boronsäureestern der Formel (XII) in Gegenwart von geeigneten Palladium-Katalysatoren, Liganden und Basen (Suzuki-Reaktion) im Temperaturbereich von -20 °C bis 120°C in geeigneten Lösungsmitteln zur Reaktion gebracht.

Die Bromide oder Iodide der Formel (XI) lassen sich nach bekannten Methoden herstellen, beschrieben z.B. in:, WO 2005/11292 , Chemistry of Heterocyclic Compounds (New York, NY, United States), 2005, 41(1), 105-110, Bioorg. & Med. Chem. 2004, 12(12), 3345-3355, Bioorg. Med. Chem. Lett. 2004, 14, 4949, J. Med. Chem. 1977, 20(12), 1562-1569

Die Boronsäuren oder Boronsäureester der Formel (XII) sind zum Teil kommerziell verfügbar oder sie sind nach bekannten Methoden leicht herstellbar. Dies ist beispielsweise beschrieben in:, WO 1999/64428.

Gegenstand der vorliegenden Erfindung sind auch Verbindungen der Formeln (XI-A) oder (XI-B) 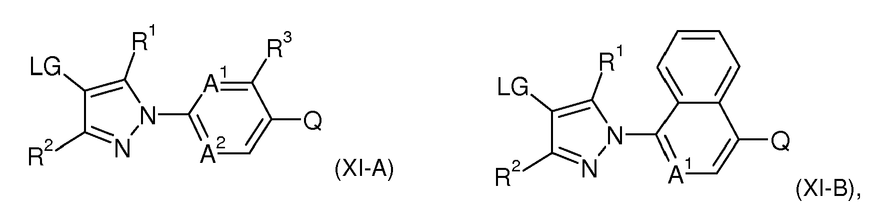 wobei R¹, R², R³, A¹, A² und Q wie oben definiert sind und LG für Chlor, Brom, Iod oder Alkylsulfonyl steht.

### Verfahren (F)

Erfindungsgemäße Verbindungen der Formel (I-A) und (I-B) mit Q = Q¹ werden z.B. nach folgendem Verfahren (F) synthetisiert: 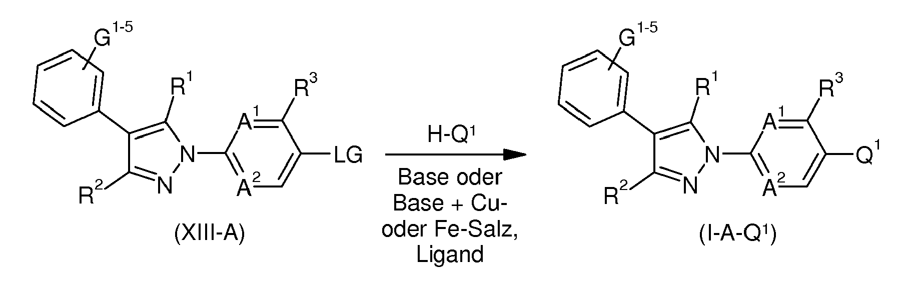 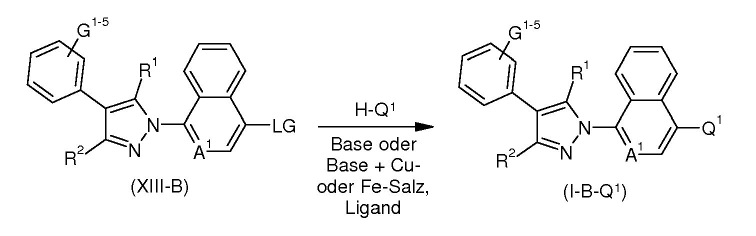 wobei
G¹⁻⁵, R¹, R², R³, A¹ und A² die oben angegebenen Bedeutungen haben und LG = Halogen, Alkylsulfonyl, Boronsäure oder Boronsäureester bedeutet.

Beim erfindungsgemäßen Verfahren (F) zur Herstellung der Verbindungen der Formel (I-A-Q1) bzw. (I-B-Q1) werden Verbindungen der Formel (XIII-A) bzw. (XIII-B) mit N-Heterocyclen in Gegenwart einer Base und eventuell eines Kupfer- oder Eisensalzes und eines Liganden in einem geeigneten organischen Lösungsmittel umgesetzt wobei eine Verbindung der Formel (I-A-Q1) bzw. (I-B-Q1) gebildet wird.

Die Cu -katalysierte Arylierung von Heterocyclen wie Triazolen wird beschrieben in: J. Org. Chem. 2007, 72(22), 8535, J. Am. Chem. Soc. 2007, 129 (45), 13879, J. Org. Chem. 2007, 72 (8), 2737, Angew. Chem. 2007, 46 (6), 934.

Die Arylierung von Heterocyclen wie Triazolen durch nukleophile Substitution wird beschrieben in: Bioorg. Med. Chem. Lett. 2007, 17(24), 6707, J. Heterocycl. Chem. 2007, 44 (6), 1323, Chem. Pharm. Bull. 2005, 53 (7), 764.

### Verfahren (G)

Erfindungsgemäße Verbindungen der Formel (I-A) und (I-B) mit Q = Q² werden z.B. nach folgendem Verfahren (G) synthetisiert: 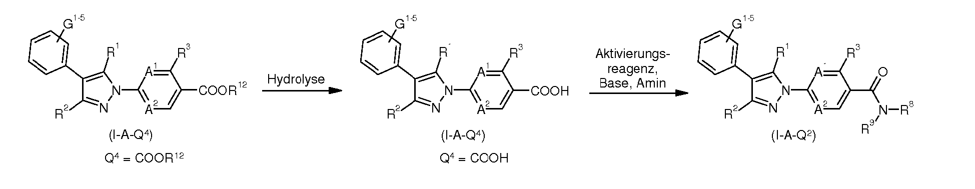 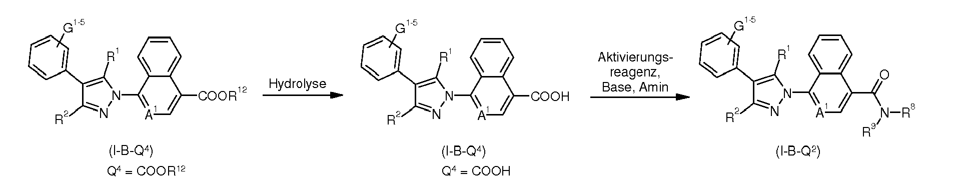 wobei
G¹⁻⁵, R¹, R², R³, R⁸, R⁹, R¹², A¹ und A² die oben angegebenen Bedeutungen haben.

Beim erfindungsgemäßen Verfahren (G) zur Herstellung der Verbindungen (I-A-Q2) bzw. (I-B-Q2) werden Verbindungen der Formel (I-A-Q4) bzw. (I-B-Q4) [Q4 = Ester] nach dem Fachmann bekannten Methoden zu (I-A-Q4) bzw. (I-B-Q4) [Q4 = COOH] hydrolysiert und dann mit Hilfe eines Aktivierungsreagenzes mit einem primären oder sekundären Amin HNR⁸R⁹ in Amide der Formeln (I-A-Q2) bzw. (I-B-Q2) umgewandelt. Die Aktivierung kann über Säurechloride, gemischte Anhydride, Pentafluorphenylester oder mit Hilfe von substituierten Carbodiimiden, z. B. DCC (N, N'-Dicyclohexylcarbodiimid), DIC (Diisopropylcarbodiimid) oder EDC (N-Ethyl-N'-(3-dimethylaminopropyl)carbodiimid HCl), und einem Benzotriazol wie HOBt (1-Hydroxybenzotriazol) oder Azabenzotriazol wie HOAt (7-Aza-1-hydroxybenxotriazol) erfolgen.

### Verfahrenswege zur Herstellung von Intermediaten

### Verfahren (H)

Zwischenprodukte der Formel (VII) werden z.B. nach folgendem Verfahren (H) synthetisiert: 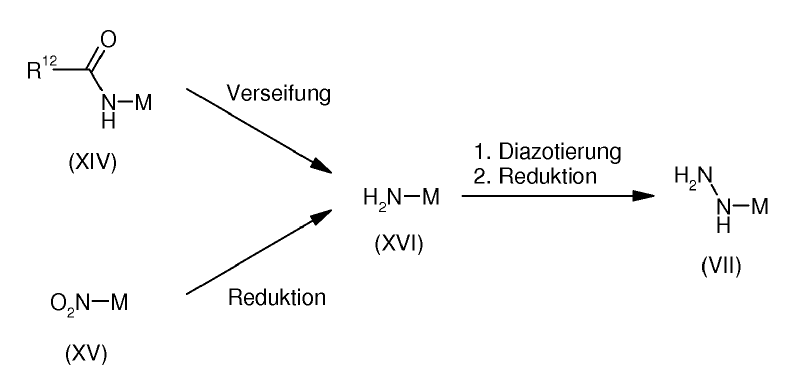 wobei
R¹² und M die oben angegebenen Bedeutungen haben;
Beim erfindungsgemäßen Verfahren (H) zur Herstellung der Hydrazine (VII) werden die Amine (XVI) nach dem Fachmann bekannten Methoden, z. B. in einer Sequenz aus Diazotierung und Reduktion, in die entsprechenden Hydrazine umgewandelt (vgl. z.B. J. Med. Chem 1993, 36 (11) S. 1529-1538 und WO 2006/081034). Hydrazine der allgemeinen Formel (VII) sind darüberhinaus zum Teil kommerziell verfügbar (z.B. Ethyl-4-hydrazinylbenzolcarboxylat oder Methyl-4-hydrazinyl-2-methoxybenzolcarboxylat). Amine der Formel (XVI) sind zum Teil kommerziell verfügbar oder lassen sich nach dem Fachmann bekannten Methoden herstellen, z.B. durch Verseifung von Verbindungen der Formel (XIV) oder Reduktion von Verbindungen der Formel (XV).

Gegenstand der vorliegenden Erfidnung sind auch Verbindungen der Formel (VII-A) oder (VII-B) 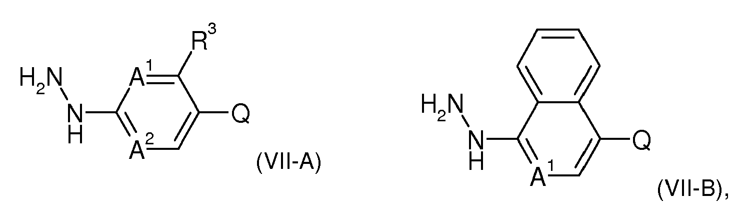 wobei A¹, A², R³ und Q wie oben definiert sind.

### Verfahren (J)

Zwischenprodukte der Formel (X-A)/(X-B) und (XVI-A)/(XVI-B) werden z.B. nach folgendem Verfahren (J) synthetisiert: 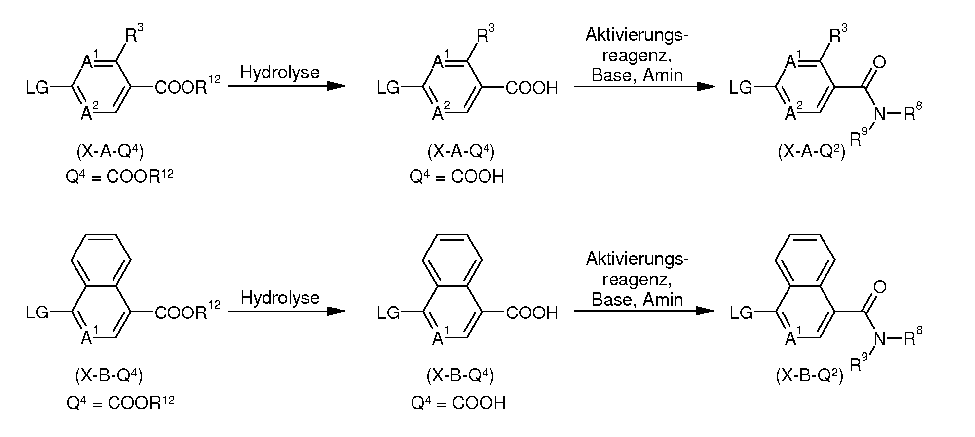 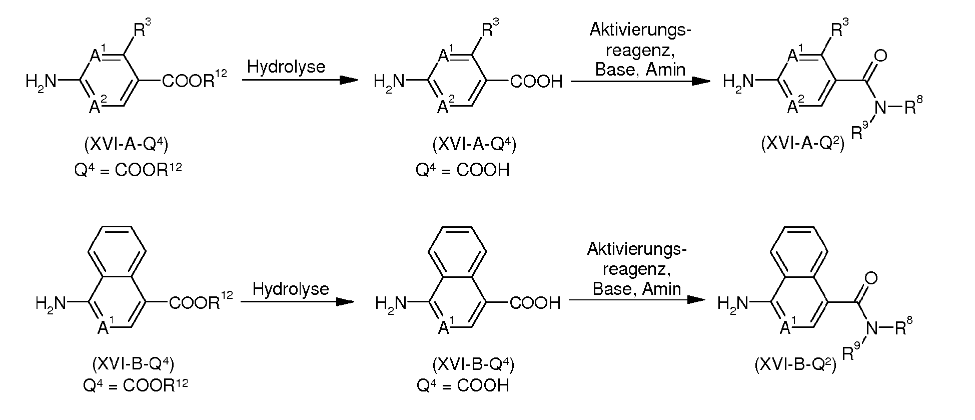 wobei
R³, R⁸, R⁹, R¹², A¹ und A² die oben angegebenen Bedeutungen haben;

Beim erfindungsgemässen Verfahren (J) zur Herstellung der Verbindungen (X-A)/(X-B)) und (XVI-A)/(XVI-B) werden Verbindungen der Formel (X-A-Q4/X-B-Q4) bzw. (XVI-A-Q4)/(XVI-B-Q4) (Q⁴ = Ester) nach dem Fachmann bekannten Methoden hydrolysiert (Q⁴ = COOH) und dann mit Hilfe eines Aktivierungsreagenzes mit einem primären oder sekundären Amin HNR⁸R⁹ in Amide der Formeln (X-A-Q2)/(X-B-Q2) und (XVI-A-Q2)/(XVI-B-Q2) umgewandelt. Die Aktivierung kann über Säurechloride, gemischte Anhydride, Pentafluorphenylester oder mit Hilfe von substituierten Carbodiimiden, z. B. DCC (N, N'-Dicyclohexylcarbodiimid), DIC (Diisopropylcarbodiimid) oder EDC (N-Ethyl-N'-(3-dimethylaminopropyl)carbodiimid HCl), und einem Benzotriazol wie HOBt (1-Hydroxybenzotriazol) oder Azabenzotriazol wie HOAt (7-Aza-1-hydroxybenzotriazol) erfolgen.

Gegenstand der vorliegenden Erfindung sind auch Verbindungen der Formeln (X-A) oder (X-B) 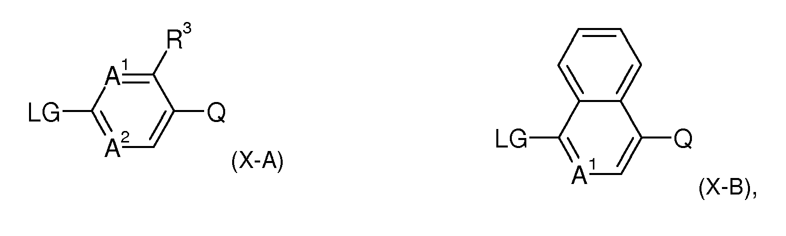 wobei A¹, A², R³ und Q wie oben definiert sind und LG für Halogen steht.

### Synthesevorschrift zur Herstellung von Verbindungen der Formel (I-AB-Q1 mit R¹ = NH₂) mittels nukleophiler Substitution

### 5-[5-Amino-4-(3-chlor-5-trifluormethylphenyl)-3-trifluormethylpyrazol-1-yl]-2-fluorbenzonitril

### (I-A-Q4-009)

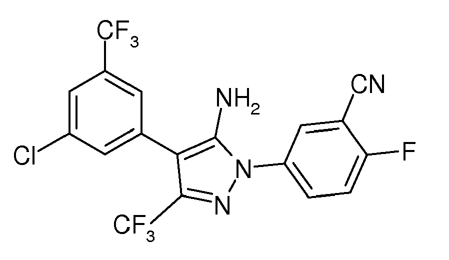

Zu einer Lösung von 2-(3-Chlor-5-trifluormethylphenyl)-4,4,4-trifluor-3-oxobutyronitril (250 mg, 0,79 mmol) in Toluol (5 mL) wird 2-Fluor-5-hydrazinobenzonitril (120 mg, 0,79 mmol) und eine Spatelspitze p-Toluolsulfonsäure hinzugefügt. Das Reaktionsgefäß wird verschlossen und in einem CEM Discover Mikrowellenreaktor 12 min bei 170 °C gerührt. Das Lösungsmittel wird im Vakuum entfernt. Reinigung mittels Chromatographie über Kieselgel mit Cyclohexan/Ethylacetat 8/1 liefert 5-[5-Amino-4-(3-chlor-5-trifluormethylphenyl)-3-trifluormethylpyrazol-1-yl]-2-fluorbenzonitril (265 mg, 0,59 mmol, 72 %).
log P (HCOOH): 4,54
¹H NMR (DMSO-d6): 8,18, (dd, 1 H, J = 5,6 und 2,8 Hz), 8,01 (ddd, 1 H, J = 9,2 und 4,8 und 2,8 Hz), 7,78 (s, 1 H), 7,71 (d, 1 H, J = 9,2 Hz), 7,67 (s, 1 H), 7,59 (s, 1 H), 5,86 (s, 2 H)

### 5-[5-Amino-4-(3-chlor-5-trifluormethylphenyl)-3-trifluormethylpyrazol-1-yl]-2-[1,2,4]triazol-1-ylbenzonitril (I-A-Q1-001)

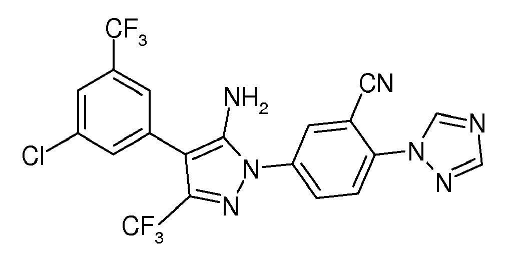

Zu einer Lösung von 5-[5-Amino-4-(3-chlor-5-trifluormethylphenyl)-3-trifluormethylpyrazol-1-yl]-2-fluorbenzonitril (100 mg, 0,22 mmol) in DMF (2 mL) werden 1,2,4-Triazol (18 mg, 0,27 mmol) und Kaliumcarbonat (37 mg, 0,27 mmol) hinzugefügt. Das Reaktionsgemisch wird 15 min bei 90 °C gerührt, auf Raumtemperatur abgekühlt, auf Wasser (5 mL) gegossen und mit Ethylacetat (5 mL) extrahiert. Die organische Phase wird mit Wasser (5 mL) gewaschen, über Natriumsulfat getrocknet und einrotiert. Reinigung mittels Chromatographie über Kieselgel mit Cyclohexan/Ethylacetat 2/1 bis 1/1 liefert 5-[5-Amino-4-(3-chlor-5-trifluormethylphenyl)-3-trifluormethylpyrazol-1-yl]-2-[1,2,4]triazol-1-ylbenzonitril (50 mg, 0,10 mmol, 45 %).
log P(HCOOH): 3,92
¹H NMR (DMSO-d6): 9,19 (s, 1 H), 8,36 (s, 1 H), 8,33 (d, 1 H, J = 2,4 Hz), 8,15 (dd, 1 H, J = 8,8 und 2,4 Hz), 8,05 (d, 1 H, J = 8,8 Hz), 7,80 (s, 1 H), 7,69 (s, 1 H), 7,61 (s, 1 H), 5,98 (s, 2 H)

### 5-[5-Amino-4-(3-chlor-5-trifluormethylphenyl)-3-trifluormethylpyrazol-1-yl]-2-(5-oxo-4,5-dihydro[1,2,4]triazol-1-yl)benzonitril (I-A-Q1-002)

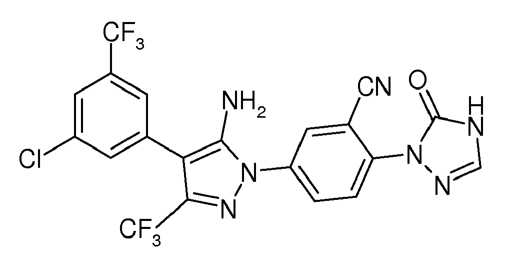

Zu einer Lösung von 5-[5-Amino-4-(3-chlor-5-trifluormethylphenyl)-3-trifluormethylpyrazol-1-yl]-2-fluorbenzonitril (50 mg, 0,11 mmol) in DMF (2 mL) werden 2,4-Dihydro[1,2,4]triazol-3-on (11 mg, 0,13 mmol) und Kaliumcarbonat (18 mg, 0,13 mmol) hinzugefügt. Das Reaktionsgemisch wird 15 min bei 80 °C gerührt, auf Raumtemperatur abgekühlt, auf Wasser (5 mL) gegossen und mit Ethylacetat (5 mL) extrahiert. Die organische Phase wird mit Wasser (5 mL) gewaschen, über Natriumsulfat getrocknet und einrotiert. Reinigung mittels Chromatographie über Kieselgel mit Cyclohexan/Ethylacetat 1/2 bis 1/3 liefert 5-[5-Amino-4-(3-chlor-5-trifluormethylphenyl)-3-trifluormethylpyrazol-1-yl]-2-(5-oxo-4,5-dihydro[1,2,4]triazol-1-yl)benzonitril (8 mg, 0,016 mmol, 12 %).
log P (HCOOH): 3,45
¹H NMR (DMSO-d6): 8,28 (d, 1 H, J = 2,3 Hz), 8,24 (d, 1 H, J = 1,2 Hz), 8,12 (dd, 1 H, J = 8,8 und 2,4 Hz), 7,87 (d, 1 H, J = 8,8 Hz), 7,79 (s, 1 H), 7,69 (s, 1 H), 7,60 (s, 1 H), 5,96 (s, 2 H)

### Synthesevorschrift zur Herstellung von Verbindungen der Formel (I-A/B-Q1 mit R¹ = NH₂) mittels Hydrazinen

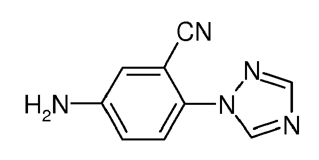

### 5-Amino-2-[1,2,4]triazol-1-yl-benzonitril

[1,2,4]Triazol (10,1 g, 73,5 mmol) wird in NMP (50 mL) vorgelegt und mit Kaliumcarbonat (30,5 g, 220 mmol) versetzt. Nachdem 10 min bei Raumtemperatur gerührt wurde, gibt man 5-Amino-2-fluorbenzonitril (10,0 g, 73,5 mmol) hinzu und rührt 5h bei 170 °C nach. Das Reaktionsgemisch wird abgekühlt und in Wasser (800 mL) gegossen. Absaugen des Niederschlages und Nachwaschen mit Wasser liefert 5-Amino-2-[1,2,4]triazol-1-yl-benzonitril (7,50 g, 40,5 mmol, 53 %), das ohne weitere Reinigung im nächsten Schritt eingesetzt wird.
log P (HCOOH): 1,01
1H NMR (DMSO-d6): 8,82 (s, 1 H), 8,16 (s, 1 H), 7,37 (d, 1 H, J = 8,4 Hz), 7,03 (d, 1 H, J = 2,8 Hz), 6,97 (dd, 1 H, J = 8,8 und 2,4 Hz), 5,85 (s, 2 H)

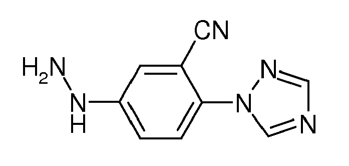

### 5-Hydrazino-2-[1,2,4]triazol-1-yl-benzonitril (VII-A-103)

5-Amino-2-[1,2,4]triazol-1-yl-benzonitril (5,0 g, 27,0 mmol) wird in Wasser (25 mL) vorgelegt, mit konzentrierter Salzsäure (50 mL) versetzt und mit mittlerer Rührgeschwindigkeit bei Raumtemperatur gerührt. Das Reaktionsgemisch wird auf 0 °C abgekühlt und mit einer Lösung von Natriumnitrit (3,73 g, 54,0 mmol) in Wasser (25 mL) versetzt. Es wird 1 h bei 0 °C nachgerührt, bevor eine Lösung von Zinn(II)chloriddihydrat (18,3 g, 81,0 mmol) in konzentrierter Salzsäure (100 mL) hinzugetropft wird. Nach vollständiger Zugabe wird 30 min bei 0 °C gerührt, auf Raumtemperatur erwärmt und weitere 1,5 h gerührt. Das Reaktionsgemisch wird unter Eiskühlung mit konzentrierter Natronlauge alkalisch gestellt, mit Ethylacetat versetzt und über Celite abgesaugt. Die organische Phase wird über Natriumsulfat getrocknet und einrotiert. Kristallisation des Rückstandes aus Methanol liefert 5-Hydrazino-2-[1,2,4]triazol-1-yl-benzonitril (1,2 g, 6,0 mmol, 22 %).
¹H NMR (DMSO-d6): 8,84 (s, 1 H), 8,17 (s, 1 H), 7,46 (s, 1 H), 7,43 (d, 1 H, J = 8,8 Hz), 7,23 (d, 1 H, J = 2,4 Hz), 7,13 (dd, 1 H, J = 9,2 und 2,8 Hz), 4,20 (, 2 H)

### Synthesevorschrift zur Herstellung von Verbindungen der Formel (I-A/B-Q2 mit R¹ = NH₂) mittels Hydrazinen

### 4-Hydrazino-2-methylbenzoesäuremethylester (VII-A-008)

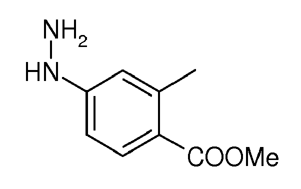

4-Acetamido-2-methylbenzoesäure (46 g, 238 mmol) wird in Methanol (460 mL) vorgelegt und mit konzentrierter Schwefelsäure (63 g, 643 mmol) versetzt. Das Reaktionsgemisch wird 3 h zum Refluxieren erhitzt, abgekühlt, vorsichtig auf Eiswasser gegossen und mit 5N Natronlauge alkalisch gestellt. Extraktion mit Ethylacetat, Trocknen der organischen Phase über Natriumsulfat und Entfernen des Lösungsmittels im Vakuum liefert 4-Amino-2-methylbenzoesäureethylester (38,3 g, 232 mmol, 94 %), der ohne weitere Reinigung im nächsten Schritt eingesetzt wird.

4-Amino-2-methylbenzoesäuremethylester (5,0 g, 30 mmol) wird in Wasser (25 mL) vorgelegt, mit konzentrierter Salzsäure (50 mL) versetzt und 30 min bei Raumtemperatur gerührt. Das Reaktionsgemisch wird auf 0 °C abgekühlt und eine Lösung von Natriumnitrit (2,72 g, 39,3 mmol) in Wasser (25 mL) wird langsam zugetropft. Man rührt eine Stunde bei 0 °C nach und tropft dann bei 0 °C Zinn(II)chlorid-Dihydrat (20,5 g, 90,8 mmol) in konzentrierter Salzsäure (100 mL) hinzu.

Es wird zunächst 30 min bei 0 °C und dann 1,5 h bei Raumtemperatur nachgerührt. Das Reaktionsgemisch wird mit Wasser (50 mL) verdünnt, der Niederschlag wird abfiltriert und mit wenig Wasser gewaschen. Das Filtrat wird in Eiswasser gegossen, mit halbkonzentrierter Natronlauge auf pH 8-9 eingestellt und zweimal mit Dichlormethan extrahiert. Die organischen Phasen werden vereint und über Natriumsulfat getrocknet. Entfernen des Lösungsmittels im Vakuum liefert 4-Hydrazino-2-methylbenzoesäuremethylester (4,1 g, 22,8 mmol, 70 %), der ohne weitere Reingung im nächsten Schritt eingesetzt wird.
log P (HCOOH): 0,40
¹H NMR (DMSO-d6): 7,67 (d, 1 H, J = 8,8 Hz), 7,19 (s, 1 H), 6,61-6,58 (m, 2 H), 4,03 (s, 2 H), 3,71 (s, 3 H), 2,44 (s, 3 H)

### 4-[5-Amino-4-(3,5-dichlorphenyl)-3-trifluormethylpyrazol-1-yl]-2-methylbenzoesäuremethylester

### (I-A-Q4-011)

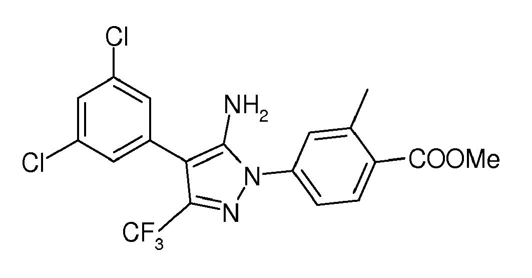

Eine Lösung von 3-Chlor-2-(3,5-dichlorphenyl)-4,4,4-trifluorbut-2-ennitril (100 mg, 0,24 mmol) in Ethanol (3 mL) wird mit 4-Hydrazino-2-methylbenzoesäuremethylester (43 mg, 0,24 mmol) und Triethylamin (24 mg, 0,24 mmol) versetzt und über Nacht bei 80 °C gerührt. Das Reaktionsgemisch wird auf Raumtemperatur abgekühlt, auf Wasser (5 mL) gegossen und mit Ethylacetat (5 mL) extrahiert. Die organische Phase wird mit Wasser (5 mL) gewaschen, über Natriumsulfat getrocknet und einrotiert. Reinigung mittels Chromatographie über Kieselgel mit Cyclohexan/Ethylacetat 6/1 liefert 4-[5-Amino-4-(3,5-dichlorphenyl)-3-trifluormethylpyrazol-1-yl]-2-methylbenzoesäuremethylester (55 mg, 0,12 mmol, 52 %).
log P (HCOOH): 5,08
¹H NMR (DMSO-d6): 7,96 (d, 1 H, J = 8,4 Hz), 7,59-7,55 (m, 2 H), 7,53 (t, 1 H, J = 2,0 Hz), 7,34 (d, 2 H, J = 2,0 Hz), 5,70 (s, 2 H), 3,87 (s, 3 H), 2,60 (s, 3 H)

### 4-[5-Amino-4-(3,5-dichlorphenyl)-3-trifluormethylpyrazol-1-yl]-2-methylbenzoesäure (I-A-Q4-010)

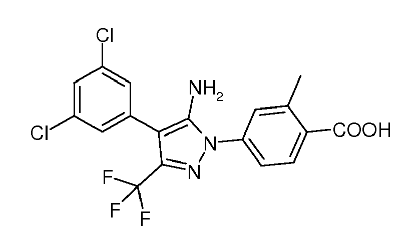

4-[5-Amino-4-(3,5-dichlorphenyl)-3-trifluormethylpyrazol-1-yl]-2-methylbenzoesäuremethylester (300 mg, 0,51 mmol) wird in Wasser (10 mL) und Ethanol (10 mL) vorgelegt und mit Natriumhydroxid (200 mg, 5,1 mmol) versetzt. Das Reaktionsgemisch wird zwei Tage bei Raumtemperatur gerührt, in Wasser (50 mL) gegossen, mit 2N Salzsäure sauer gestellt (pH3-4), zweimal mit Ethylacetat extrahiert und über Natriumsulfat getrocknet. Einrotieren des Lösungsmittels liefert 4-[5-Amino-4-(3,5-dichlorphenyl)-3-trifluormethylpyrazol-1-yl]-2-methyl-benzoesäure in quantitativer Ausbeute.
log P (HCOOH): 4,04
¹H NMR (DMSO-d6): 7,97 (d, 1 H, J = 8,4 Hz), 7,55-7,52 (m, 3 H), 7,34 (d, 2 H, J = 2,0 Hz), 5,68 (s, 2 H), 2,61 (s, 3 H)

### 4-[5-Amino-4-(3,5-dichlorphenyl)-3-trifluormethylpyrazol-1-yl]-2-methyl-N-pyridin-2-ylmethylbenzamid (I-A-Q2-012)

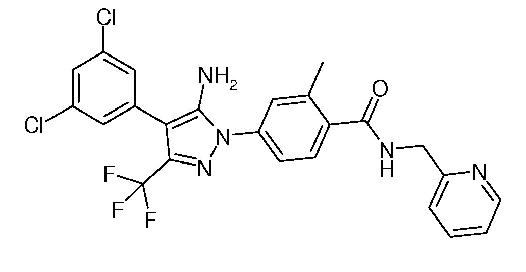

Eine Lösung von 2-Aminomethylpyridin (25 mg, 0,23 mmol) in DMF (1 mL) wird mit einer Lösung aus 4-[5-Amino-4-(3,5-dichlorphenyl)-3-trifluormethylpyrazol-1-yl]-2-methylbenzoesäure (100 mg, 0,23 mmol), 1-Ethyl-3-[3-dimethylaminopropyl]carbodiimid Hydrochlorid (58 mg, 0,30 mmol), 1-Hydroxy-1H-benzotriazol (41 mg, 0,30 mmol) und Triethylamin (28 mg, 0,28 mmol) in DMF (4 mL) versetzt und über Nacht bei Raumtemperatur gerührt. Das Reaktionsgemisch wird mit Wasser (5 mL) versetzt, zweimal mit Ethylacetat (je 5 mL) extrahiert, mit NaHCO₃-Lsg (5 mL) und NaCl-Lsg (5 mL) gewaschen, über Natriumsulfat getrocknet und einrotiert. Reinigung durch Säulenchromatographie über Kieselgel mit Cyclohexan/Ethylacetat 1/2 bis 1/3 liefert 4-[5-Amino-4-(3,5-dichlorphenyl)-3-trifluormethylpyrazol-1-yl]-2-methyl-N-pyridin-2-ylmethylbenzamid (75 mg, 0,14 mmol, 61 %).
log P (HCOOH): 3,35
¹H NMR (DMSO-d6): 8,71 (t, 1 H, J = 5,6 Hz), 8,52 (d, 1 H, J = 4,0 Hz), 7,78 (td, 1 H, J = 8,0 und 2,0 Hz), 7,60 (d, 1 H, J = 8,0 Hz), 7,53 (t, 1 H, J = 2,0 Hz), 7,50-7,47 (m, 2 H), 7,40 (d, 1 H, J = 7,6 Hz), 7,34 (d, 2 H, J = 2,0 Hz), 7,26 (dd, 1 H, J = 7,6 und 5,6 Hz), 5,59 (s, 2 H), 4,58 (d, 2 H, J = 6,0 Hz), 2,46 (s, 3 H)

### Synthesevorschrift zur Herstellung von Verbindungen der Formel (I-A/B-Q2 mit R¹ = NH₂) mittels N-Arylierung von Pyrazolen

### 4-[5-Amino-4-(3,4-dichlorphenyl)-3-(trifluormethyl)-1H-pyrazol-1-yl]-2-methylbenzoesäure-ethylester (I-A-Q4-013)

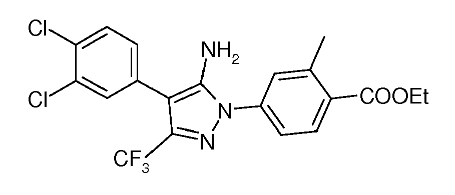

4-(3,4-Dichlorphenyl)-3-(trifluormethyl)-1H-pyrazol-5-amin (70 %ig) (1,94 g, 6,55 mmol) werden in Toluol (39 mL) unter Argon vorgelegt, mit Ethyl-4-iod-2-methylbenzoat (90 %ig) (1,90 g), Kaliumcarbonat (3,10 g), Kupfer(I)iodid (62 mg) und trans-Dimethylamino-cyclohexan (186 mg) versetzt und 16 Stunden bei 110°C unter Argon gerührt.

Das Reaktionsgemisch wird einrotiert, in Wasser (20 mL) und Ethylacetat (39 mL) aufgenommen, mit konz. HCl auf pH= 1 gestellt, die org. Phase abgetrennt, die wässrige Phase nochmals mit Ethylacetat (30 mL) extrahiert, die vereinigten organischen Phasen über Natriumsulfat getrocknet und einrotiert. Nach Extraktion von organischen Verunreinigungen mit Hexan verbleibt ein brauner Feststoff. Reinigung mittels HPLC liefert 4-[5-Amino-4-(3,4-dichlorphenyl)-3-(trifluormethyl)-1H-pyrazol-1-yl]-2-methylbenzoesäureethylester (630 mg, 24 %)) log P (HCOOH): 5,24
¹H NMR (DMSO-d6): 1.35 (t, 3 H); 2.56 (s, 3 H); 4.34 (q, 2 H); 5.62 (br. s, 2 H); 7.32 (dd, 1 H); 7.54-7.59 (m, 3 H); 7.67 (d, 1 H); 7.95 (d, 1 H)

### 4-[5-Amino-4-(3,4-dichlorphenyl)-3-(trifluormethyl)-1H-pyrazol-1-yl]-2-methylbenzoesäure

### (I-A-Q4-012)

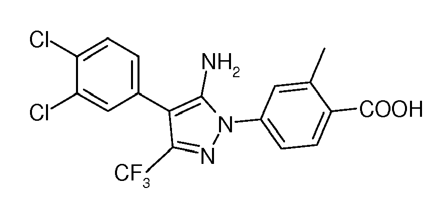

4-[5-Amino-4-(3,4-dichlorphenyl)-3-(trifluormethyl)-1H-pyrazol-1-yl]--2-methylbenzoesäureethylester (600 mg) werden in Isopropanol (9 mL) und 1N -Natronlauge (6 mL) zwei Tage bei 20 °C gerührt.

Das Isopropanol wird im Vakuum abrotiert, die wässrige Phase mit konzentrierter Salzsäure auf pH = 1 gestellt. Der ausgefallene Niederschlag wird abgesaugt, mit Wasser gewaschen und getrocknet.

Man erhält 4-[5-Amino-4-(3,4-dichlorphenyl)-3-(trifluormethyl)-1H-pyrazol-1-yl]-2-methylbenzol-carbonsäure (495 mg, 86 %) als gelblichen Feststoff, Gehalt HPLC: 98.8 %.
log P (HCOOH): 3,92
¹H NMR (DMSO-d6): 2.61 (s, 3 H); 5.61 (br. S, 2 H); 7.32 (dd, 1 H); 7.52-7.56 (m, 3 H); 7.67 (d, 1 H); 7.96 (d, 1 H)

### Synthesevorschrift zur Herstellung von Verbindungen der Formel (I-A/B-Q2 mit R¹ = I)

### 4-[4-(3,5-Dichlorphenyl)-5-iod-3-trifluormethylpyrazol-1-yl]-2-methyl-N-pyridin-2-ylmethyl-benzamid (I-A-Q2-025)

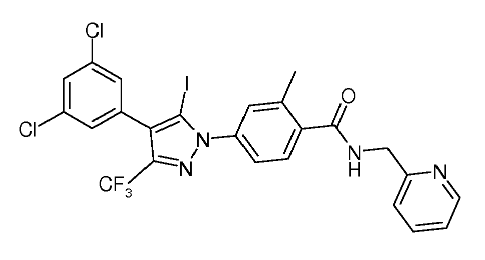

Eine Lösung von 4-[5-Amino-4-(3,5-dichlorphenyl)-3-trifluormethylpyrazol-1-yl]-2-methyl-N-pyridin-2-ylmethylbenzamid (80 mg, 0,15 mmol) in Chloroform (1 mL) wird mit Iodoform (121 mg, 0,31 mmol) versetzt. Zu dem Reaktionsgemisch wird langsam tert-Butylnitrit (35 mg, 0,34 mmol) hinzugetropft und es wird über Nacht bei Raumtemperatur rühren gelassen. Reinigung mittels Chromatographie über Kieselgel mit Cyclohexan/Ethylacetat 1/1 liefert 4-[4-(3,5-Dichlorphenyl)-5-iod-3-trifluormethylpyrazol-1-yl]-2-methyl-N-pyridin-2-ylmethylbenzamid (45 mg, 0,071 mmol, 46%). Leichte Verunreinigungen an 4-[4-(3,5-Dichlorphenyl)-3-trifluor-methylpyrazol-1-yl]-2-methyl-N-pyridin-2-ylmethylbenzamid können mittels HPLC entfernt werden.
log P (HCOOH): 4,29
¹H NMR (DMSO-d6): 8,85 (t, 1 H, J = 5,8 Hz), 8,52 (d, 1 H, J = 4,0 Hz), 7,78 (td, 1 H, J = 8,0 und 2,0 Hz), 7,71 (t, 1 H, J = 2,0 Hz), 7,64 (d, (1 H, J = 8,8 Hz), 7,53-7,50 (m, 2 H), 7,45 (d, 2 H, J = 1,6 Hz), 7,42 (d, 1 H, J = 8,0 Hz), 7,27 (dd, 1 H, J = 6,4 und 5,2 Hz), 4,58 (d, 2 H, J = 6,0 Hz), Me unter DMSO-d6 Signal

### Synthesevorschrift zur Herstellung von Verbindungen der Formel (I-A/B-Q2 mit R¹ = Br)

### 4-[5-Brom-4-(3,5-dichlorphenyl)-3-trifluormethylpyrazol-1-yl]-2-methyl-N-(2,2,2-trifluor-1-methylethyl)benzamid (I-A-Q2-024)

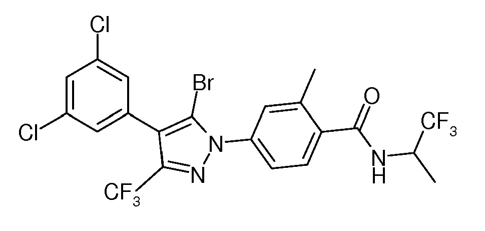

Zu einer Lösung von 4-[5-Amino-4-(3,5-dichlorphenyl)-3-trifluormethylpyrazol-1-yl]-2-methyl-N-(2,2,2-trifluor-1-methylethyl)benzamid (200 mg, 0,38 mmol) in Bromoform (0,5 mL) wird langsam tert-Butylnitrit (86 mg, 0,84 mmol) hinzugetropft und es wird über Nacht bei Raumtemperatur rühren gelassen. Reinigung mittels Chromatographie über Kieselgel mit Cyclohexan/Ethylacetat 1/1 liefert 4-[5-Brom-4-(3,5-dichlorphenyl)-3-trifluormethylpyrazol-1-yl]-2-methyl-N-(2,2,2-trifluor-1-methylethyl)benzamid (62 mg, 0,11 mmol, 24 %).
log P (HCOOH): 5,53
¹H NMR (DMSO-d6): 8,90 (d, 1 H, J = 8,8 Hz), 7,72 (t, 1 H, J = 2,0 Hz), 7,57-7,54 (m, 3 H), 7,50 (d, 2 H, J = 2,0 Hz), 4,84-4,78 (m, 1 H), 2,43 (s, 3 H), 1,34 (d, 3 H, J = 6,4 Hz)

### Allgemeine Synthesevorschrift zur Herstellung von Verbindungen der Formel (1-A/B-Q2 mit R¹ = Cl)

### 4-[5-Chlor-4-(3,5-dichlorphenyl)-3-trifluormethylpyrazol-1-yl]-2-methyl-N-(2,2,2-trifluor-1-methylethyl)benzamid (I-A-Q2-022)

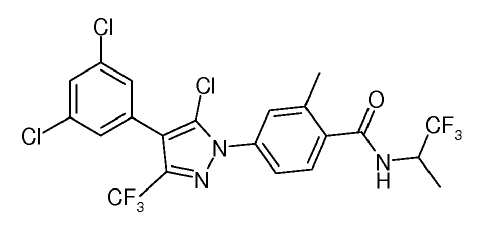

Eine Lösung von tert-Butylnitrit (24 mg, 0,23 mmol) in Acetonitril (2 mL) wird mit Kupfer(II)chlorid (31 mg, 0,23 mmol) und (4-[5-Amino-4-(3,5-dichlorphenyl)-3-trifluormethylpyrazol-1-yl]-2-methyl-N-(2,2,2-trifluor-1-methylethyl)benzamid (80 mg, 0,15 mmol) versetzt und 30 min bei 80 °C rühren gelassen und dann einrotiert. Reinigung mittels Chromatoographie über Kieselgel mit Cyclohexan/Ethylacetat 1/1 Iliefert 4-[5-Chlor-4-(3,5-dichlorphenyl)-3-trifluormethylpyrazol-1-yl] -2-methyl-N-(2,2,2-trifluor-1-methylethyl)benzamid (30 mg, 0,055 mmol, 33 %).
log P (HCOOH): 5,59
¹H NMR (DMSO-d6): 8,88 (d, 1 H, J = 8,4 Hz), 7,72 (t, 1 H, J = 1,8 Hz), 7,59-7,55 (m, 3 H), 7,52 (d, 2 H, J = 2,0 Hz), 4,84-4,78 (m, 1 H), 2,43 (s, 3 H), 1,34 (d, 3 H, J = 6,4 Hz)

### Synthesevorschrift zur Herstellung von Verbindungen der Formel (I-A/B-Q2 mit R¹ = SMe und SOMe)

### 4-[4-(3,5-Dichlorphenyl)-5-methylsulfanyl-3-trifluormethylpyrazol-1-yl]-2-methyl-N-(2,2,2-trifluor-1-methylethyl)benzamid (I-A-Q2-027)

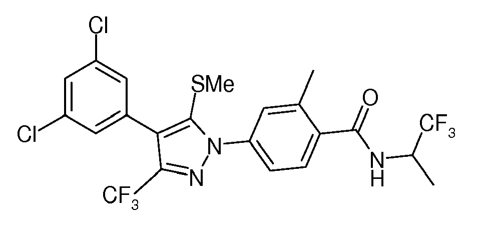

Zu einer Lösung von 4-[5-Amino-4-(3,5-dichlorphenyl)-3-trifluormethylpyrazol-1-yl]-2-methyl-N-(2,2,2-trifluor-1-methylethyl)benzamid (100 mg, 0,19 mmol) in Chloroform (1 mL) wird Dimethyldisulfid (36 mg, 0,38 mmol) hinzugefügt. Das Reaktionsgemisch wird langsam mit tert-Butylnitrit (29 mg, 0,29 mmol), 3 min zum Refluxieren gebracht, abgekühlt, auf Wasser (5 mL) gegossen und mit Ethylacetat (5 mL) extrahiert. Die organische Phase wird mit Wasser (5 mL) gewaschen, über Natriumsulfat getrocknet und einrotiert. Reinigung mittels Chromatographie über Kieselgel mit Cyclohexan/Ethylacetat 8/1 liefert 4-[4-(3,5-Dichlorphenyl)-5-methylsulfanyl-3-trifluormethylpyrazol-1-yl]-2-methyl-N-(2,2,2-trifluor-1-methylethyl)benzamid (43 mg, 0,77 mmol, 39 %).
log P (HCOOH): 5,48
¹H NMR (DMSO-d6): 8,87 (d, 1 H, J = 8,8 Hz), 7,69 (t, 1 H, J = 1,8 Hz), 7,58-7,51 (m, 3 H), 7,50 (d, 2 H, J = 2,0 Hz), 4,84-4,77 (m, 1 H), 2,43 (s, 3 H), 2,04 (s, 3 H), 1,34 (d, 3 H, J = 6,4 Hz)

### 4-[4-(3,5-Dichlorphenyl)-5-methansulfinyl-3-trifluormethylpyrazol-1-yl]-2-methyl-N-pyridin-2-ylmethylbenzamid (I-A-Q2-063)

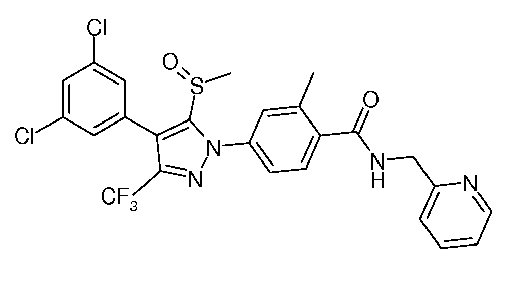

Zu einer Lösung von 4-[4-(3,5-Dichlorphenyl)-5-methansulfanyl-3-trifluormethylpyrazol-1-yl]-2-methyl-N-pyridin-2-ylmethylbenzamid (50 mg, 0,09 mmol) in Dichlormethan (1 mL) gibt man m-Chlorperbenzoesäure (29 mg, 0,12 mmol) und rührt über Nacht. Das Reaktionsgemisch wird mit Wasser gewaschen, die organische Phase über Natriumsulfat getrocknet und einrotiert. Reinigung mittels Chromatographie über Kieselgel mit Cyclohexan/Ethylacetat 9/1 liefert 4-[4-(3,5-Dichlorphenyl)-5-methansulfanyl-3-trifluormethylpyrazol-1-yl]-2-methyl-N-pyridin-2-ylmethylbenzamid (20 mg, 0,035 mmol, 39%).
log P (HCOOH): 3,11
¹H NMR (DMSO-d6): 8,54 (d, 1 H, J = 4,8 Hz), 7,77 (td, 1 H, J = 7,6 und 2,0 Hz), 7,66-7,48 (m, 7 H), 7,41 (d, 1 H, J = 8,0 Hz), 7,26 (dd, 7,2 und 4,8 Hz), 4,66 (d, 2 H, J = 6,0 Hz), 2,65 (s, 3 H), 2,50 (s, 3 H)

### Synthesevorschrift zur Herstellung von Verbindungen der Formel (I-AB-Q2 mit R¹ = NH₂ und A¹= A² = N)

### 2-[5-Amino-4-(3,5-dichlorphenyl)-3-trifluormethylpyrazol-1-yl]-4-trifluormethylpyrimidin-5-carbonsäure(pyridin-2-ylmethyl)amid (I-A-Q2-020)

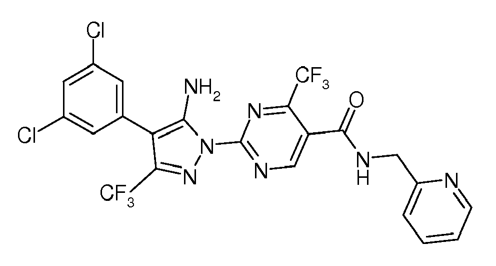

Eine Lösung von 2-Chlor-4-trifluormethylpyrimidin-5-carbonsäurechlorid (300 mg, 1,23 mmol) in Dichlormethan (3 mL) wird mit N-Ethyldiisopropylamin (174 mg, 1,35 mmol) und 2-Aminomethylpyridin (140 mg, 1,29 mmol) in THF (2 mL) versetzt und über Nacht bei Raumtemperatur gerührt. Entfernen des Lösungsmittels im Vakuum liefert 650 mg Rohprudukt, wovon 95 mg in DMF (2 mL) gelöst und mit 4-(3,5-Dichlorphenyl)-5-trifluormethyl-2H-pyrazol-3-ylamin (107 mg, 0,36 mmol) und Kaliumcarbonat (58 mg, 0,42 mmol) versetzt werden. Es wird 1,5 h bei 100 °C gerührt, auf Raumtemperatur abgekühlt, auf Wasser (5 mL) gegossen und mit Ethylacetat (5 mL) extrahiert. Die organische Phase wird mit Wasser (5 mL) gewaschen, über Natriumsulfat getrocknet und einrotiert. Reinigung mittels Chromatographie über Kieselgel mit Cyclohexan/Ethylacetat 3/1 liefert 2-[5-Amino-4-(3,5-dichlorphenyl)-3-trifluormethylpyrazol-1-yl]-4-trifluormethylpyrimidin-5-carbonsäure(pyridin-2-ylmethyl)amid (55 mg, 0,095 mmol, 53 %).
log P (HCOOH): 3,54
¹H NMR (DMSO-d6): 9,30 (s, 1 H), 9,27 (t, 1 H, J = 6,0 Hz), 8,54 (d, 1 H, J = 4,8 Hz), 7,80 (td, 1 H, J = 7,8 und 2,0 Hz), 7,57 (t, 1 H, J = 1,8 Hz), 7,42 (d, 1 H, J = 7,6 Hz), 7,37 (d, 2 H, J = 1,6 Hz), 7,30 (dd, 1 H, J = 6,4 und 3,6 Hz), 7,00 (s, 2 H), 4,62 (d, 2 H, J = 5,6 Hz)

### Synthesevorschrift zur Herstellung von Verbindungen der Formel (I-AB-Q2 mit R¹ = H) 4-(4-Iod-3-trifluormethylpyrazol-1-yl)-2-trifluormethylbenzoesäureethylester (XI-A-001)

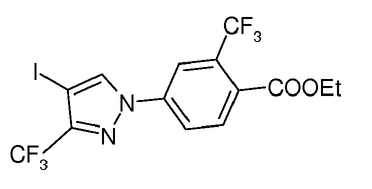

Eine Lösung von 4-Fluor-2-trifluormethylbenzoesäureethylester (2,00 g, 8,47 mmol) in DMF (30 mL) wird mit 4-Iod-3-trifluormethyl-1H-pyrazol (2,22 g, 8,47 mmol) und Kaliumcarbonat (1,40 g, 10,2 mmol) versetzt und 1 h bei 60 °C gerührt. Das Reaktionsgemisch wird mit Ethylacetat verdünnt, mit Wasser und gesättigter Kochsalzlösung gewaschen, über Magnesiumsulfat getrocknet und im Vakuum eingeengt. Reinigung mittels Chromatographie über Kieselgel liefert 4-(4-Iod-3-trifluormethylpyrazol-1-yl)-2-trifluormethylbenzoesäureethylester (3,65 g, 7,63 mmol, 90%).
¹H NMR (CDC13): 1,41 (t, 3 H, J = 7,1 Hz), 4,43 (q, 2 H, J = 7,1 Hz), 7,92-7,99 (m, 2 H), 8,08 (s, 1 H), 8,14-8,14 (m, 1 H).

### 4-[4-(3,5-Dichlorphenyl)-3-trifluormethylpyrazol-1-yl]-2-trifluormethylbenzoesäure-ethylester

### (I-A-Q4-001)

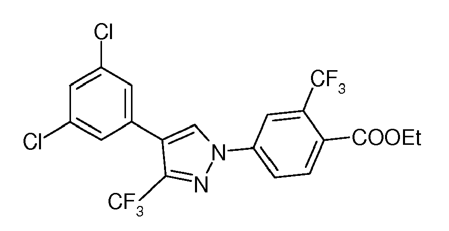

Eine Lösung von 4-(4-Iod-3-trifluormethylpyrazol-1-yl)-2-trifluormethylbenzoesäureethylester (1,00 g, 2,09 mmol) in DME (10 mL) wird mit 3,5-Dichlorphenylboronsäure (0,60 g, 3,14 mmol), und Natriumcarbonat (0,69 g, 6,48 mmol) in Wasser (2 mL) versetzt. Das Reaktionsgefäss wird entgasst und mit Stickstoff befüllt. Tetrakis(triphenylphosphin)palladium (0,73 g, 0,63 mmol) wird hinzugefügt und das Reaktionsgemisch wird 9 h bei 85 °C gerührt. Nach dem Abkühlen wird es auf Wasser gegossen und mit Ethylacetat extrahiert. Die organische Phase wird mit Wasser und gesättigter Kochsalzlösung gewaschen, über Magnesiumsulfat getrocknet und einrotiert. Reinigung mittels Chromatographie über Kieselgel liefert 4-[4-(3,5-Dichlorphenyl)-3-trifluormethylpyrazol-1-yl]-2-trifluormethylbenzoesäureethylester (0,30 g, 0,60 mmol, 29%).
¹H NMR (CDC13): 1,42 (t, 3 H, J = 7,1 Hz), 4,44 (q, 2 H, J = 7,1 Hz), 7,37-7,42 (m, 3 H), 8,00-8,01 (m, 2 H), 8,14-8,15 (m, 2 H)

### 4-[4-(3,5-Dichlorphenyl)-3-trifluormethylpyrazol-1-yl]-2-trifluormethylbenzoesäure

### (I-A-Q4-002)

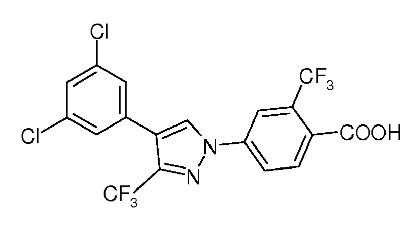

Eine Lösung von 4-[4-(3,5-Dichlorphenyl)-3-trifluormethylpyrazol-1-yl]-2-trifluormethyl-benzoesäureethylester (300 mg, 0,60 mmol) in Ethanol (10 mL) wird mit Natriumhydroxid (48 mg, 1,2 mmol) in Wasser (10 mL) versetzt und über Nacht bei Raumtemperatur gerührt. Das organische Lösungsmittel wird im Vakuum entfernt und der wässrige Rückstand mit verdünnter Salzsäure behandelt. Abfiltrieren des gebildeten Niederschlags und Trocknen bei 50 °C unter vermindertem Druck liefert 4-[4-(3,5-Dichlorphenyl)-3-trifluormethylpyrazol-1-yl]-2-trifluor-methylbenzoesäure (259 mg, 0,53 mmol, 88 %).

### 4-[4-(3,5-Dichlorphenyl)-3-trifluormethylpyrazol-1-yl]-N-(2,2,2-trifluorethyl)-2-trifluormethylbenzamid (I-A-Q2-002)

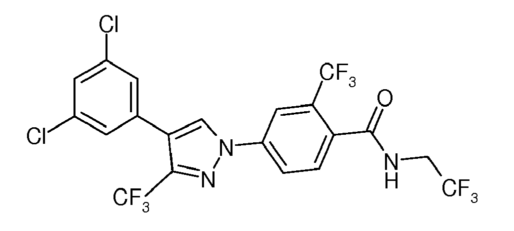

Eine Lösung von 4-[4-(3,5-Dichlorphenyl)-3-trifluormethylpyrazol-1-yl]-2-trifluormethyl-benzoesäure (130 mg, 0,28 mmol) in DMF (10 mL) wird mit 2,2,2-Trifluorethylamin (40 mg, 0,41 mmol), und 1-Ethyl-3-[3-dimethylaminopropyl]carbodiimid Hydrochlorid (60 mg, 0,33 mmol) versetzt. Das Reaktionsgemisch wird 8 h bei Raumtemperatur gerührt, mit tert-Butylmethylether verdünnt, mit Wasser und gesättigter Kochsalzlösung gewaschen, über Magnesiumsulfat getrocknet und das Lösungsmittel im Vakuum entfernt. Reinigung mittels Chromatographie über Kieselgel liefert 4-[4-(3,5-Dichlorphenyl)-3-trifluormethylpyrazol-1-yl]-N-(2,2,2-trifluorethyl)-2-trifluormethylbenz-amid (100 mg, 0,18 mmol, 66 %).
log P (pH2): 5.16
log P (pH7,5): 5.14
¹H NMR (CDC13): 4,10-4,14 (m, 2 H), 6,33-6,35 (m, 1 H), 7,34-7,40 (m, 3 H), 7,59-7,67 (m, 1 H), 7,86-8,15 (m, 3 H).

Die Bestimmung der angegebenen log P-Werte erfolgte gemäß EEC-Direktive 79/831 Annex V.A8 durch HPLC (High Performance Liquid Chromatography) an einer Phasenumkehrsäule (C18).

Agilent 1100 LC-System; 50*4,6 Zorbax Eclipse Plus C18 1,8 microm; Eluent A: Acetonitril (0,1 % Ameisensäure); Eluent B: Wasser (0,09 % Ameisensäure); linearer Gradient von 10 % Acetonitril bis 95 % Acetonitril in 4,25 min, dann 95% Acetonitril für weitere 1,25 min; Ofentemperatur 55 °C; Fluß:2,0 mL/min

Beim Einsatz der erfindungsgemäßen Wirkstoffe als Insektizide und Akarizide können die Aufwandmengen je nach Applikationsart innerhalb eines größeren Bereiches variiert werden. Die Aufwandmenge der erfindungsgemäßen Wirkstoffe beträgt bei der Behandlung von Pflanzenteilen, z.B. Blättern von 0,1 bis 10.000 g/ha, bevorzugt von 10 bis 1.000 g/ha, besonders bevorzugt von 50 bis 300g/ha (bei Anwendung durch Gießen oder Tropfen kann die Aufwandmenge sogar verringert werden, vor allem wenn inerte Substrate wie Steinwolle oder Perlit verwendet werden); bei der Saatgutbehandlung von 2 bis 200 g pro 100 kg Saatgut, bevorzugt von 3 bis 150 g pro 100 kg Saatgut, besonders bevorzugt von 2,5 bis 25 g pro 100 kg Saatgut, ganz besonders bevorzugt von 2,5 bis 12,5 g pro 100 kg Saatgut; bei der Bodenbehandlung von 0,1 bis 10.000 g/ha, bevorzugt von 1 bis 5.000 g/ha. Diese Aufwandmengen seien nur beispielhaft und nicht limitierend im Sinne der Erfindung genannt.

Die erfindungsgemäßen Wirkstoffe können eingesetzt werden, um Pflanzen innerhalb eines gewissen Zeitraumes nach der Behandlung gegen den Befall durch die genannten tierischen Schädlinge zu schützen. Der Zeitraum, innerhalb dessen Schutz herbeigeführt wird, erstreckt sich im Allgemeinen auf 1 bis 28 Tage, bevorzugt auf 1 bis 14 Tage, besonders bevorzugt auf 1 bis 10 Tage, ganz besonders bevorzugt auf 1 bis 7 Tage nach der Behandlung der Pflanzen mit den Wirkstoffen bzw. auf bis zu 200 Tage nach einer Saatgutbehandlung.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp, Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können gegebenenfalls in bestimmten Konzentrationen bzw. Aufwandmengen auch als Herbizide, Safener, Wachstumsregulatoren oder Mittel zur Verbesserung der Pflanzeneigenschaften oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Der erfindungsgemäße Wirkstoff kann in seinen handelsüblichen Formulierungen sowie in den aus diesen Formulierungen bereiteten Anwendungsformen in Mischung mit anderen Wirkstoffen wie Insektiziden, Lockstoffen, Sterilantien, Bakteriziden, Akariziden, Nematiziden, Fungiziden, wachstumsregulierenden Stoffen, Herbiziden, Safenern, Düngemitteln, Semiochemicals oder auch mit Mitteln zur Verbesserung der Pflanzeneigenschaften vorliegen.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Gesamtwirkstoffkonzentration oder die Wirkstoffkonzentration der Einzelwirkstoffe der Anwendungsformen liegt im Bereich von 0,00000001 bis 97 Gew.-% Wirkstoff, vorzugsweise im Bereich von 0,0000001 bis 97 Gew.-%, besonders bevorzugt im Bereich von 0,000001 bis 83 Gew.-% oder 0,000001 bis 5 Gew.-% und ganz besonders bevorzugt im Bereich von 0,0001 bis 1 Gew. %.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Als Pflanzen, welche erfindungsgemäß behandelt werden können, seien folgende erwähnt: Baumwolle, Flachs, Weinrebe, Obst, Gemüse, wie Rosaceae sp. (beispielsweise Kernfrüchte wie Apfel und Birne, aber auch Steinfrüchte wie Aprikosen, Kirschen, Mandeln und Pfirsiche und Beerenfrüchte wie Erdbeeren), Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp. (beispielsweise Bananenbäume und -plantagen), Rubiaceae sp. (beispielsweise Kaffee), Theaceae sp., Sterculiceae sp., Rutaceae sp. (beispielsweise Zitronen, Organen und Grapefruit); Solanaceae sp. (beispielsweise Tomaten), Liliaceae sp., Asteraceae sp. (beispielsweise Salat), Umbelliferae sp., Cruciferae sp., Chenopodiaceae sp., Cucurbitaceae sp. (beispielsweise Gurke), Alliaceae sp. (beispielsweise Lauch, Zwiebel), Papilionaceae sp. (beispielsweise Erbsen); Hauptnutzpflanzen, wie Gramineae sp. (beispielsweise Mais, Rasen, Getreide wie Weizen, Roggen, Reis, Gerste, Hafer, Hirse und Triticale), Asteraceae sp. (beispielsweise Sonnenblume), Brassicaceae sp. (beispielsweise Weißkohl, Rotkohl, Brokkoli, Blumenkohl, Rosenkohl, Pak Choi, Kohlrabi, Radieschen sowie Raps, Senf, Meerrettich und Kresse), Fabacae sp. (beispielsweise Bohne, Erdnüsse), Papilionaceae sp. (beispielsweise Sojabohne), Solanaceae sp. (beispielsweise Kartoffeln), Chenopodiaceae sp. (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen.

Insbesondere eignen sich die erfindungsgemäßen Wirkstoffe zur Behandlung von Saatgut. Bevorzugt sind dabei die vorstehend als bevorzugt oder besonders bevorzugt genannten erfindungsgemäßen Wirkstoffe zu nennen. So entsteht ein großer Teil des durch Schädlinge verursachten Schadens an Kulturpflanzen bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die Bekämpfung von Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln nach der Saat oder nach dem Auflaufen der Pflanzen überflüssig machen. Es ist weiterhin erstrebenswert, die Menge des eingesetzten Wirkstoffs dahingehend zu optimieren, dass das Saatgut und die keimende Pflanze vor dem Befall durch Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden Eigenschaften transgener Pflanzen einbeziehen, um einen optimalen Schutz des Saatguts und auch der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen Wirkstoff behandelt wird. Die Erfindung bezieht sich ebenfalls auf die Verwendung der erfindungsgemäßen Wirkstoffe zur Behandlung von Saatgut zum Schutz des Saatguts und der daraus entstehenden Pflanze vor Schädlingen. Weiterhin bezieht sich die Erfindung auf Saatgut, welches zum Schutz vor Schädlingen mit einem erfindungsgemäßen Wirkstoff behandelt wurde. Die Erfindung bezieht sich auch auf Saatgut, bei dem ein Wirkstoff der Formel I als Bestandteil einer Umhüllung oder als weitere Schicht oder weitere Schichten zusätzlich zu einer Umhüllung aufgebracht sind.

Einer der Vorteile der vorliegenden Erfindung ist es, dass aufgrund der besonderen systemischen Eigenschaften einiger der erfindungsgemäßen Wirkstoffe die Behandlung des Saatguts mit diesen Wirkstoffen nicht nur das Saatgut selbst, sondern auch die daraus hervorgehenden Pflanzen nach dem Auflaufen vor Schädlingen schützt. Auf diese Weise kann die unmittelbare Behandlung der Kultur zum Zeitpunkt der Aussaat oder kurz danach entfallen.

Ebenso ist es als vorteilhaft anzusehen, dass die erfindungsgemäßen Wirkstoffe insbesondere auch bei transgenem Saatgut eingesetzt werden können, wobei die aus diesem Saatgut hervorgehenden Pflanzen zur Expression eines gegen Schädlinge gerichteten Proteins befähigt sind. Durch die Behandlung solchen Saatguts mit den erfindungsgemäßen Wirkstoffen können bestimmte Schädlinge bereits durch die Expression des z.B. insektiziden Proteins kontrolliert werden, und zusätzlich durch die erfindungsgemäßen Wirkstoffen vor Schäden bewahrt werden.

Die erfindungsgemäßen Wirkstoffe eignen sich zum Schutz von Saatgut jeglicher Pflanzensorte wie bereits vorstehend genannt, die in der Landwirtschaft, im Gewächshaus, in Forsten oder im Gartenbau eingesetzt wird. Insbesondere handelt es sich dabei um Saatgut von Mais, Erdnuss, Canola, Raps, Mohn, Soja, Baumwolle, Rübe (z.B. Zuckerrübe und Futterrübe), Reis, Hirse, Weizen, Gerste, Hafer, Roggen, Sonnenblume, Tabak, Kartoffeln oder Gemüse (z.B. Tomaten, Kohlgewächs). Die erfindungsgemäßen Wirkstoffe eignen sich ebenfalls zur Behandlung des Saatguts von Obstpflanzen und Gemüse wie vorstehend bereits genannt. Besondere Bedeutung kommt der Behandlung des Saatguts von Mais, Soja, Baumwolle, Weizen und Canola oder Raps zu.

Wie vorstehend bereits erwähnt, kommt auch der Behandlung von transgenem Saatgut mit einem erfindungsgemäßen Wirkstoff eine besondere Bedeutung zu. Dabei handelt es sich um das Saatgut von Pflanzen, die in der Regel zumindest ein heterologes Gen enthalten, das die Expression eines Polypeptids mit insbesondere insektiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt und dessen Genprodukt Wirksamkeit gegen Maiszünsler und/oder Maiswurzel-Bohrer zeigt. Besonders bevorzugt handelt es sich dabei um ein heterologes Gen, das aus Bacillus thuringiensis stammt.

Im Rahmen der vorliegenden Erfindung wird der erfindungsgemäße Wirkstoff alleine oder in einer geeigneten Formulierung auf das Saatgut aufgebracht. Vorzugsweise wird das Saatgut in einem Zustand behandelt, in dem so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde.

Im Allgemeinen muss bei der Behandlung des Saatguts darauf geachtet werden, dass die Menge des auf das Saatgut aufgebrachten erfindungsgemäßen Wirkstoffs und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die erfindungsgemäß verwendbaren Wirkstoffe können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutylnaphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Das erfindungsgemäße Behandlungsverfahren kann für die Behandlung von genetisch modifizierten Organismen (GMOs), z. B. Pflanzen oder Samen, verwendet werden. Genetisch modifizierte Pflanzen (oder transgene Pflanzen) sind Pflanzen, bei denen ein heterologes Gen stabil in das Genom integriert worden ist. Der Begriff "heterologes Gen" bedeutet im wesentlichen ein Gen, das außerhalb der Pflanze bereitgestellt oder assembliert wird und das bei Einführung in das Zellkerngenom, das Chloroplastengenom oder das Hypochondriengenom der transformierten Pflanze dadurch neue oder verbesserte agronomische oder sonstige Eigenschaften verleiht, daß es ein interessierendes Protein oder Polypeptid exprimiert oder daß es ein anderes Gen, das in der Pflanze vorliegt bzw. andere Gene, die in der Pflanze vorliegen, herunterreguliert oder abschaltet (zum Beispiel mittels Antisense-Technologie, Cosuppressionstechnologie oder RNAi-Technologie [RNA Interference]). Ein heterologes Gen, das im Genom vorliegt, wird ebenfalls als Transgen bezeichnet. Ein Transgen, das durch sein spezifisches Vorliegen im Pflanzengenom definiert ist, wird als Transformations- bzw. transgenes Event bezeichnet.

Zu Pflanzen und Pflanzensorten, die vorzugsweise erfindungsgemäß behandelt werden, zählen alle Pflanzen, die über Erbgut verfügen, das diesen Pflanzen besonders vorteilhafte, nützliche Merkmale verleiht (egal, ob dies durch Züchtung und/oder Biotechnologie erzielt wurde).

Pflanzen und Pflanzensorten, die ebenfalls vorzugsweise erfindungsgemäß behandelt werden, sind gegen einen oder mehrere biotische Streßfaktoren resistent, d. h. diese Pflanzen weisen eine verbesserte Abwehr gegen tierische und mikrobielle Schädlinge wie Nematoden, Insekten, Milben, phytopathogene Pilze, Bakterien, Viren und/oder Viroide auf.

Pflanzen und Pflanzensorten, die ebenfalls erfindungsgemäß behandelt werden können, sind solche Pflanzen, die gegen einen oder mehrere abiotische Streßfaktoren resistent sind. Zu den abiotischen Streßbedingungen können zum Beispiel Dürre, Kälte- und Hitzebedingungen, osmotischer Streß, Staunässe, erhöhter Bodensalzgehalt, erhöhtes Ausgesetztsein an Mineralien, Ozonbedingungen, Starklichtbedingungen, beschränkte Verfügbarkeit von Stickstoffnährstoffen, beschränkte Verfügbarkeit von Phosphornährstoffen oder Vermeidung von Schatten zählen.

Pflanzen und Pflanzensorten, die ebenfalls erfindungsgemäß behandelt werden können, sind solche Pflanzen, die durch erhöhte Ertragseigenschaften gekennzeichnet sind. Ein erhöhter Ertrag kann bei diesen Pflanzen z. B. auf verbesserter Pflanzenphysiologie, verbessertem Pflanzenwuchs und verbesserter Pflanzenentwicklung, wie Wasserverwertungseffizienz, Wasserhalteeffizienz, verbesserter Stickstoffverwertung, erhöhter Kohlenstoffassimilation, verbesserter Photosynthese, verstärkter Keimkraft und beschleunigter Abreife beruhen. Der Ertrag kann weiterhin durch eine verbesserte Pflanzenarchitektur (unter Streß- und nicht-Streß-Bedingungen) beeinflußt werden, darunter frühe Blüte, Kontrolle der Blüte für die Produktion von Hybridsaatgut, Keimpflanzenwüchsigkeit, Pflanzengröße, Internodienzahl und -abstand, Wurzelwachstum, Samengröße, Fruchtgröße, Schotengröße, Schoten- oder Ährenzahl, Anzahl der Samen pro Schote oder Ähre, Samenmasse, verstärkte Samenfüllung, verringerter Samenausfall, verringertes Schotenplatzen sowie Standfestigkeit. Zu weiteren Ertragsmerkmalen zählen Samenzusammensetzung wie Kohlenhydratgehalt, Proteingehalt, Ölgehalt und Ölzusammensetzung, Nährwert, Verringerung der nährwidrigen Verbindungen, verbesserte Verarbeitbarkeit und verbesserte Lagerfähigkeit.

Pflanzen, die erfindungsgemäß behandelt werden können, sind Hybridpflanzen, die bereits die Eigenschaften der Heterosis bzw. des Hybrideffekts exprimieren, was im allgemeinen zu höherem Ertrag, höherer Wüchsigkeit, besserer Gesundheit und besserer Resistenz gegen biotische und abiotische Streßfaktoren führt. Solche Pflanzen werden typischerweise dadurch erzeugt, daß man eine ingezüchtete pollensterile Elternlinie (den weiblichen Kreuzungspartner) mit einer anderen ingezüchteten pollenfertilen Elternlinie (dem männlichen Kreuzungspartner) kreuzt. Das Hybridsaatgut wird typischerweise von den pollensterilen Pflanzen geerntet und an Vermehrer verkauft. Pollensterile Pflanzen können manchmal (z. B. beim Mais) durch Entfahnen (d. h. mechanischem Entfernen der männlichen Geschlechtsorgane bzw. der männlichen Blüten), produziert werden; es ist jedoch üblicher, daß die Pollensterilität auf genetischen Determinanten im Pflanzengenom beruht. In diesem Fall, insbesondere dann, wenn es sich bei dem gewünschten Produkt, da man von den Hybridpflanzen ernten will, um die Samen handelt, ist es üblicherweise günstig, sicherzustellen, daß die Pollenfertilität in Hybridpflanzen, die die für die Pollensterilität verantwortlichen genetischen Determinanten enthalten, völlig restoriert wird. Dies kann erreicht werden, indem sichergestellt wird, daß die männlichen Kreuzungspartner entsprechende Fertilitätsrestorergene besitzen, die in der Lage sind, die Pollenfertilität in Hybridpflanzen, die die genetischen Determinanten, die für die Pollensterilität verantwortlich sind, enthalten, zu restorieren. Genetische Determinanten für Pollensterilität können im Cytoplasma lokalisiert sein. Beispiele für cytoplasmatische Pollensterilität (CMS) wurden zum Beispiel für Brassica-Arten beschrieben (WO 1992/005251, WO 1995/009910, WO 1998/27806, WO 2005/002324, WO 2006/021972 und US 6,229,072). Genetische Determinanten für Pollensterilität können jedoch auch im Zellkerngenom lokalisiert sein. Pollensterile Pflanzen können auch mit Methoden der pflanzlichen Biotechnologie, wie Gentechnik, erhalten werden. Ein besonders günstiges Mittel zur Erzeugung von pollensterilen Pflanzen ist in WO 89/10396 beschrieben, wobei zum Beispiel eine Ribonuklease wie eine Barnase selektiv in den Tapetumzellen in den Staubblättern exprimiert wird. Die Fertilität kann dann durch Expression eines Ribonukleasehemmers wie Barstar in den Tapetumzellen restoriert werden (z. B. WO 1991/002069).

Pflanzen oder Pflanzensorten (die mit Methoden der Pflanzenbiotechnologie, wie der Gentechnik, erhalten werden), die erfindungsgemäß behandelt werden können, sind herbizidtolerante Pflanzen, d. h. Pflanzen, die gegenüber einem oder mehreren vorgegebenen Herbiziden tolerant gemacht worden sind. Solche Pflanzen können entweder durch genetische Transformation oder durch Selektion von Pflanzen, die eine Mutation enthalten, die solch eine Herbizidtoleranz verleiht, erhalten werden.

Herbizidtolerante Pflanzen sind zum Beispiel glyphosatetolerante Pflanzen, d. h. Pflanzen, die gegenüber dem Herbizid Glyphosate oder dessen Salzen tolerant gemacht worden sind. So können zum Beispiel glyphosatetolerante Pflanzen durch Transformation der Pflanze mit einem Gen, das für das Enzym 5-Enolpyruvylshikimat-3-phosphatsynthase (EPSPS) kodiert, erhalten werden. Beispiele für solche EPSPS-Gene sind das AroA-Gen (Mutante CT7) des Bakterium Salmonella typhimurium (Comai et al., Science (1983), 221, 370-371), das CP4-Gen des Bakteriums Agrobacterium sp. (Barry et al., Curr. Topics Plant Physiol. (1992), 7, 139-145), die Gene, die für eine EPSPS aus der Petunie (Shah et al., Science (1986), 233, 478-481), für eine EPSPS aus der Tomate (Gasser et al., J. Biol. Chem. (1988), 263, 4280-4289) oder für eine EPSPS aus Eleusine (WO 2001/66704) kodieren. Es kann sich auch um eine mutierte EPSPS handeln, wie sie zum Beispiel in EP-A 0837944, WO 2000/066746, WO 2000/066747 oder WO 2002/026995 beschrieben ist. Glyphosatetolerante Pflanzen können auch dadurch erhalten werden, daß man ein Gen exprimiert, das für ein Glyphosate-Oxidoreduktase-Enzym, wie es in US 5,776,760 und US 5,463,175 beschrieben ist, kodiert. Glyphosatetolerante Pflanzen können auch dadurch erhalten werden, daß man ein Gen exprimiert, das für ein Glyphosate-acetyltransferase-Enzym, wie es in z. B. WO 2002/036782, WO 2003/092360, WO 2005/012515 und WO 2007/024782 beschrieben ist, kodiert. Glyphosatetolerante Pflanzen können auch dadurch erhalten werden, daß man Pflanzen, die natürlich vorkommende Mutationen der oben erwähnten Gene, wie sie zum Beispiel in WO 2001/024615 oder WO 2003/013226 beschrieben sind, enthalten, selektiert.

Sonstige herbizidresistente Pflanzen sind zum Beispiel Pflanzen, die gegenüber Herbiziden, die das Enzym Glutaminsynthase hemmen, wie Bialaphos, Phosphinotricin oder Glufosinate, tolerant gemacht worden sind. Solche Pflanzen können dadurch erhalten werden, daß man ein Enzym exprimiert, das das Herbizid oder eine Mutante des Enzyms Glutaminsynthase, das gegenüber Hemmung resistent ist, entgiftet. Solch ein wirksames entgiftendes Enzym ist zum Beispiel ein Enzym, das für ein Phosphinotricin-acetyltransferase kodiert (wie zum Beispiel das bar- oder pat-Protein aus Streptomyces-Arten). Pflanzen, die eine exogene Phosphinotricin-acetyltransferase exprimieren, sind zum Beispiel in US 5,561,236; US 5,648,477; US 5,646,024; US 5,273,894; US 5,637,489; US 5,276,268; US 5,739,082; US 5,908,810 und US 7,112,665 beschrieben.

Weitere herbizidtolerante Pflanzen sind auch Pflanzen, die gegenüber den Herbiziden, die das Enzym Hydroxyphenylpyruvatdioxygenase (HPPD) hemmen, tolerant gemacht worden sind. Bei den Hydroxyphenylpyruvatdioxygenasen handelt es sich um Enzyme, die die Reaktion, in der para-Hydroxyphenylpyruvat (HPP) zu Homogentisat umgesetzt wird, katalysieren. Pflanzen, die gegenüber HPPD-Hemmern tolerant sind, können mit einem Gen, das für ein natürlich vorkommendes resistentes HPPD-Enzym kodiert oder einem Gen, das für ein mutiertes HPPD-Enzym gemäß WO 1996/038567, WO 1999/024585 und WO 1999/024586 kodiert, transformiert werden. Eine Toleranz gegenüber HPPD-Hemmern kann auch dadurch erzielt werden, daß man Pflanzen mit Genen transformiert, die für gewisse Enzyme kodieren, die die Bildung von Homogentisat trotz Hemmung des nativen HPPD-Enzyms durch den HPPD-Hemmer ermöglichen. Solche Pflanzen und Gene sind in WO 1999/034008 und WO 2002/36787 beschrieben. Die Toleranz von Pflanzen gegenüber HPPD-Hemmern kann auch dadurch verbessert werden, daß man Pflanzen zusätzlich zu einem Gen, das für ein HPPD-tolerantes Enzym kodiert, mit einem Gen transformiert, das für ein Prephenatdehydrogenase-Enzym kodiert, wie dies in WO 2004/024928 beschrieben ist.

Weitere herbizidresistente Pflanzen sind Pflanzen, die gegenüber Acetolactatsynthase (ALS)-Hemmern tolerant gemacht worden sind. Zu bekannten ALS-Hemmern zählen zum Beispiel Sulfonylharnstoff, Imidazolinon, Triazolopyrimidine, Pyrimidinyloxy(thio)benzoate und/oder Sulfonylaminocarbonyltriazolinon-Herbizide. Es ist bekannt, daß verschiedene Mutationen im Enzym ALS (auch als Acetohydroxysäure-Synthase, AHAS, bekannt) eine Toleranz gegenüber unterschiedlichen Herbiziden bzw. Gruppen von Herbiziden verleihen, wie dies zum Beispiel bei Tranel und Wright, Weed Science (2002), 50, 700-712, jedoch auch in US 5,605,011, US 5,378,824, US 5,141,870 und US 5,013,659, beschrieben ist. Die Herstellung von sulfonylharnstofftoleranten Pflanzen und imidazolinontoleranten Pflanzen ist in US 5,605,011; US 5,013,659; US 5,141,870; US 5,767,361; US 5,731,180; US 5,304,732; US 4,761,373; US 5,331,107; US 5,928,937; und US 5,378,824; sowie in der internationalen Veröffentlichung WO 1996/033270 beschrieben. Weitere imidazolinontolerante Pflanzen sind auch in z. B. WO 2004/040012, WO 2004/106529, WO 2005/020673, WO 2005/093093, WO 2006/007373, WO 2006/015376, WO 2006/024351 und WO 2006/060634 beschrieben. Weitere sulfonylharnstoff-und imidazolinontolerante Pflanzen sind auch in z.B. WO 2007/024782 beschrieben.

Weitere Pflanzen, die gegenüber Imidazolinon und/oder Sulfonylharnstoff tolerant sind, können durch induzierte Mutagenese, Selektion in Zellkulturen in Gegenwart des Herbizids oder durch Mutationszüchtung erhalten werden, wie dies zum Beispiel für die Sojabohne in US 5,084,082, für Reis in WO 1997/41218, für die Zuckerrübe in US 5,773,702 und WO 1999/057965, für Salat in US 5,198,599 oder für die Sonnenblume in WO 2001/065922 beschrieben ist.

Pflanzen oder Pflanzensorten (die nach Methoden der pflanzlichen Biotechnologie, wie der Gentechnik, erhalten wurden), die ebenfalls erfindungsgemäß behandelt werden können, sind insektenresistente transgene Pflanzen, d.h. Pflanzen, die gegen Befall mit gewissen Zielinsekten resistent gemacht wurden. Solche Pflanzen können durch genetische Transformation oder durch Selektion von Pflanzen, die eine Mutation enthalten, die solch eine Insektenresistenz verleiht, erhalten werden.

Der Begriff "insektenresistente transgene Pflanze" umfaßt im vorliegenden Zusammenhang jegliche Pflanze, die mindestens ein Transgen enthält, das eine Kodiersequenz umfaßt, die für folgendes kodiert:
1) ein insektizides Kristallprotein aus Bacillus thuringiensis oder einen insektiziden Teil davon, wie die insektiziden Kristallproteine, die von Crickmore et al., Microbiology and Molecular Biology Reviews (1998), 62, 807-813, von Crickmore et al. (2005) in der Bacillus thuringiensis-Toxinnomenklatur aktualisiert, online bei:
   http://www.lifesci.sussex.ac.uk/Home/Neil_Crickmore/Bt/), zusammengestellt wurden, oder insektizide Teile davon, z.B. Proteine der Cry-Proteinklassen Cry1Ab, Cry1Ac, Cry1F, Cry2Ab, Cry3Ae oder Cry3Bb oder insektizide Teile davon; oder
2) ein Kristallprotein aus Bacillus thuringiensis oder einen Teil davon, der in Gegenwart eines zweiten, anderen Kristallproteins als Bacillus thuringiensis oder eines Teils davon insektizid wirkt, wie das binäre Toxin, das aus den Kristallproteinen Cy34 und Cy35 besteht (Moellenbeck et al., Nat. Biotechnol. (2001), 19, 668-72; Schnepf et al., Applied Environm. Microb. (2006), 71, 1765-1774); oder
3) ein insektizides Hybridprotein, das Teile von zwei unterschiedlichen insektiziden Kristallproteinen aus Bacillus thuringiensis umfaßt, wie zum Beispiel ein Hybrid aus den Proteinen von 1) oben oder ein Hybrid aus den Proteinen von 2) oben, z. B. das Protein Cry1A.105, das von dem Mais-Event MON98034 produziert wird (WO 2007/027777); oder
4) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier-DNA während der Klonierung oder Transformation induziert wurden, wie das Protein Cry3Bb1 in Mais-Events MON863 oder MON88017 oder das Protein Cry3A im Mais-Event MIR 604; oder
5) ein insektizides sezerniertes Protein aus Bacillus thuringiensis oder Bacillus cereus oder einen insektiziden Teil davon, wie die vegetativ wirkenden insektentoxischen Proteine (vegetative insekticidal proteins, VIP), die unter http://www.lifesci.sussex.ac.uk/Home/Neil_Crickmore/Bt/vip.html angeführt sind, z. B. Proteine der Proteinklasse VIP3Aa; oder
6) ein sezerniertes Protein aus Bacillus thuringiensis oder Bacillus cereus, das in Gegenwart eines zweiten sezernierten Proteins aus Bacillus thuringiensis oder B. cereus insektizid wirkt, wie das binäre Toxin, das aus den Proteinen VIP1A und VIP2A besteht (WO 1994/21795); oder
7) ein insektizides Hybridprotein, das Teile von verschiedenen sezernierten Proteinen von Bacillus thuringiensis oder Bacillus cereus umfaßt, wie ein Hybrid der Proteine von 1) oder ein Hybrid der Proteine von 2) oben; oder
8) ein Protein gemäß einem der Punkte 1) bis 3) oben, in dem einige, insbesondere 1 bis 10, Aminosäuren durch eine andere Aminosäure ersetzt wurden, um eine höhere insektizide Wirksamkeit gegenüber einer Zielinsektenart zu erzielen und/oder um das Spektrum der entsprechenden Zielinsektenarten zu erweitern und/oder wegen Veränderungen, die in die Kodier-DNA während der Klonierung oder Transformation induziert wurden (wobei die Kodierung für ein insektizides Protein erhalten bleibt), wie das Protein VIP3Aa im Baumwoll-Event COT 102.

Natürlich zählt zu den insektenresistenten transgenen Pflanzen im vorliegenden Zusammenhang auch jegliche Pflanze, die eine Kombination von Genen umfaßt, die für die Proteine von einer der oben genannten Klassen 1 bis 8 kodieren. In einer Ausführungsform enthält eine insektenresistente Pflanze mehr als ein Transgen, das für ein Protein nach einer der oben genannten 1 bis 8 kodiert, um das Spektrum der entsprechenden Zielinsektenarten zu erweitern oder um die Entwicklung einer Resistenz der Insekten gegen die Pflanzen dadurch hinauszuzögern, daß man verschiedene Proteine einsetzt, die für dieselbe Zielinsektenart insektizid sind, jedoch eine unterschiedliche Wirkungsweise, wie Bindung an unterschiedliche Rezeptorbindungsstellen im Insekt, aufweisen.

Pflanzen oder Pflanzensorten (die nach Methoden der pflanzlichen Biotechnologie, wie der Gentechnik, erhalten wurden), die ebenfalls erfindungsgemäß behandelt werden können, sind gegenüber abiotischen Streßfaktoren tolerant. Solche Pflanzen können durch genetische Transformation oder durch Selektion von Pflanzen, die eine Mutation enthalten, die solch eine Streßresistenz verleiht, erhalten werden. Zu besonders nützlichen Pflanzen mit Streßtoleranz zählen folgende:
a. Pflanzen, die ein Transgen enthalten, das die Expression und/oder Aktivität des Gens für die Poly(ADP-ribose)polymerase (PARP) in den Pflanzenzellen oder Pflanzen zu reduzieren vermag, wie dies in WO 2000/004173 oder EP 04077984.5 oder EP 06009836.5 beschrieben ist.
b. Pflanzen, die ein streßtoleranzförderndes Transgen enthalten, das die Expression und/oder Aktivität der für PARG kodierenden Gene der Pflanzen oder Pflanzenzellen zu reduzieren vermag, wie dies z.B. in WO 2004/090140 beschrieben ist;
c. Pflanzen, die ein streßtoleranzförderndes Transgen enthalten, das für ein in Pflanzen funktionelles Enzym des Nicotinamidadenindinukleotid-Salvage-Biosynthesewegs kodiert, darunter Nicotinamidase, Nicotinatphosphoribosyltransferase, Nicotinsäuremono-nukleotidadenyltransferase, Nicotinamidadenindinukleotidsynthetase oder Nicotinamid-phosphoribosyltransferase, wie dies z. B. in EP 04077624.7 oder WO 2006/133827 oder PCT/EP07/002433 beschrieben ist.

Pflanzen oder Pflanzensorten (die nach Methoden der pflanzlichen Biotechnologie, wie der Gentechnik, erhalten wurden), die ebenfalls erfindungsgemäß behandelt werden können, weisen eine veränderte Menge, Qualität und/oder Lagerfähigkeit des Ernteprodukts und/oder veränderte Eigenschaften von bestimmten Bestandteilen des Ernteprodukts auf, wie zum Beispiel:
1) Transgene Pflanzen, die eine modifizierte Stärke synthetisieren, die bezüglich ihrer chemisch-physikalischen Eigenschaften, insbesondere des Amylosegehalts oder des Amylose/Amylopektin-Verhältnisses, des Verzweigungsgrads, der durchschnittlichen Kettenlänge, der Verteilung der Seitenketten, des Viskositätsverhaltens, der Gelfestigkeit, der Stärkekorngröße und/oder Stärkekornmorphologie im Vergleich mit der synthetisierten Stärke in Wildtyppflanzenzellen oder -pflanzen verändert ist, so daß sich diese modifizierte Stärke besser für bestimmte Anwendungen eignet. Diese transgenen Pflanzen, die eine modifizierte Stärke synthetisieren, sind zum Beispiel in EP 0571427, WO 1995/004826, EP 0719338, WO 1996/15248, WO 1996/19581, WO 1996/27674, WO 1997/11188, WO 1997/26362, WO 1997/32985, WO 1997/42328, WO 1997/44472, WO 1997/45545, WO 1998/27212, WO 1998/40503, WO 99/58688, WO 1999/58690, WO 1999/58654, WO 2000/008184, WO 2000/008185, WO 2000/28052, WO 2000/77229, WO 2001/12782, WO 2001/12826, WO 2002/101059, WO 2003/071860, WO 2004/056999, WO 2005/030942, WO 2005/030941, WO 2005/095632, WO 2005/095617, WO 2005/095619, WO 2005/095618, WO 2005/123927, WO 2006/018319, WO 2006/103107, WO 2006/108702, WO 2007/009823, WO 2000/22140, WO 2006/063862, WO 2006/072603, WO 2002/034923, EP 06090134.5, EP 06090228.5, EP 06090227.7, EP 07090007.1, EP 07090009.7, WO 2001/14569, WO 2002/79410, WO 2003/33540, WO 2004/078983, WO 2001/19975, WO 1995/26407, WO 1996/34968, WO 1998/20145, WO 1999/12950, WO 1999/66050, WO 1999/53072, US 6,734,341, WO 2000/11192, WO 1998/22604, WO 1998/32326, WO 2001/98509, WO 2001/98509, WO 2005/002359, US 5,824,790, US 6,013,861, WO 1994/004693, WO 1994/009144, WO 1994/11520, WO 1995/35026 bzw. WO 1997/20936 beschrieben.
2) Transgene Pflanzen, die Nichtstärkekohlenhydratpolymere synthetisieren, oder Nichtstärkekohlenhydratpolymere, deren Eigenschaften im Vergleich zu Wildtyppflanzen ohne genetische Modifikation verändert sind. Beispiele sind Pflanzen, die Polyfructose, insbesondere des Inulin- und Levantyps, produzieren, wie dies in EP 0663956, WO 1996/001904, Wo 1996/021023, WO 1998/039460 und WO 1999/024593 beschrieben ist, Pflanzen, die alpha-1,4-Glucane produzieren, wie dies in WO 1995/031553, US 2002/031826, US 6,284,479, US 5,712,107, WO 1997/047806, WO 1997/047807, WO 1997/047808 und WO 2000/14249 beschrieben ist, Pflanzen, die alpha-1,6-verzweigte alpha-1,4-Glucane produzieren, wie dies in WO 2000/73422 beschrieben ist, und Pflanzen, die Alternan produzieren, wie dies in WO 2000/047727, EP 06077301.7, US 5,908,975 und EP 0728213 beschrieben ist.
3) Transgene Pflanzen, die Hyaluronan produzieren, wie dies zum Beispiel in WO 2006/032538, WO 2007/039314, WO 2007/039315, WO 2007/039316, JP 2006/304779 und WO 2005/012529 beschrieben ist.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Baumwollpflanzen mit veränderten Fasereigenschaften. Solche Pflanzen können durch genetische Transformation oder durch Selektion von Pflanzen, die eine Mutation enthalten, die solche veränderten Fasereigenschaften verleiht, erhalten werden; dazu zählen:
a) Pflanzen wie Baumwollpflanzen, die eine veränderte Form von Cellulosesynthasegenen enthalten, wie dies in WO 1998/000549 beschrieben ist,
b) Pflanzen wie Baumwollpflanzen, die eine veränderte Form von rsw2- oder rsw3-homologen Nukleinsäuren enthalten, wie dies in WO 2004/053219 beschrieben ist;
c) Pflanzen wie Baumwollpflanzen mit einer erhöhten Expression der Saccharosephosphatsynthase, wie dies in WO 2001/017333 beschrieben ist;
d) Pflanzen wie Baumwollpflanzen mit einer erhöhten Expression der Saccharosesynthase, wie dies in WO 02/45485 beschrieben ist;
e) Pflanzen wie Baumwollpflanzen bei denen der Zeitpunkt der Durchlaßsteuerung der Plasmodesmen an der Basis der Faserzelle verändert ist, z. B. durch Herunterregulieren der faserselektiven β-1,3-Glucanase, wie dies in WO 2005/017157 beschrieben ist;
f) Pflanzen wie Baumwollpflanzen mit Fasern mit veränderter Reaktivität, z. B. durch Expression des N-Acetylglucosamintransferasegens, darunter auch nodC, und von Chitinsynthasegenen, wie dies in WO 2006/136351 beschrieben ist.

Pflanzen oder Pflanzensorten (die nach Methoden der pflanzlichen Biotechnologie, wie der Gentechnik, erhalten wurden), die ebenfalls erfindungsgemäß behandelt werden können, sind Pflanzen wie Raps oder verwandte Brassica-Pflanzen mit veränderten Eigenschaften der Ölzusammensetzung. Solche Pflanzen können durch genetische Transformation oder durch Selektion von Pflanzen, die eine Mutation enthalten, die solche veränderten Öleigenschaften verleiht, erhalten werden; dazu zählen:
a) Pflanzen wie Rapspflanzen, die Öl mit einem hohen Ölsäuregehalt produzieren, wie dies zum Beispiel in US 5,969,169, US 5,840,946 oder US 6,323,392 oder US 6,063, 947 beschrieben ist;
b) Pflanzen wie Rapspflanzen, die Öl mit einem niedrigen Linolensäuregehalt produzieren, wie dies in US 6,270828, US 6,169,190 oder US 5,965,755 beschrieben ist.
c) Pflanzen wie Rapspflanzen, die Öl mit einem niedrigen gesättigten Fettsäuregehalt produzieren, wie dies z. B. in US 5,434,283 beschrieben ist.

Besonders nützliche transgene Pflanzen, die erfindungsgemäß behandelt werden können, sind Pflanzen mit einem oder mehreren Genen, die für ein oder mehrere Toxine kodieren, sind die transgenen Pflanzen, die unter den folgenden Handelsbezeichnungen angeboten werden: YIELD GARD® (zum Beispiel Mais, Baumwolle, Sojabohnen), KnockOut® (zum Beispiel Mais), BiteGard® (zum Beispiel Mais), BT-Xtra® (zum Beispiel Mais), StarLink® (zum Beispiel Mais), Bollgard® (Baumwolle), Nucotn® (Baumwolle), Nucotn 33B® (Baumwolle), NatureGard® (zum Beispiel Mais), Protecta® und NewLeaf® (Kartoffel). Herbizidtolerante Pflanzen, die zu erwähnen sind, sind zum Beispiel Maissorten, Baumwollsorten und Sojabohnensorten, die unter den folgenden Handelsbezeichnungen angeboten werden: Roundup Ready® (Glyphosatetoleranz, zum Beispiel Mais, Baumwolle, Sojabohne), Liberty Link® (Phosphinotricintoleranz, zum Beispiel Raps), IMI® (Imidazolinontoleranz) und SCS® (Sylfonylharnstofftoleranz), zum Beispiel Mais. Zu den herbizidresistenten Pflanzen (traditionell auf Herbizidtoleranz gezüchtete Pflanzen), die zu erwähnen sind, zählen die unter der Bezeichnung Clearfield® angebotenen Sorten (zum Beispiel Mais).

Besonders nützliche transgene Pflanzen, die erfindungsgemäß behandelt werden können, sind Pflanzen, die Transformations-Events, oder eine Kombination von Transformations-Events, enthalten und die zum Beispiel in den Dateien von verschiedenen nationalen oder regionalen Behörden angeführt sind (siehe zum Beispiel http://gmoinfo.jrc.it/gmp_browse.aspx und http://www.agbios.com/dbase.php).

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:

Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neonicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Synthesebeispiele

Folgende Abkürzungen werden in den nachfolgenden Tabellen verwendet:

| | | | |
|---|---|---|---|
| Me = Methyl | Et = Ethyl | Pr = Propyl | iPr = iso-Propyl |
| tBu = tert-Butyl | Pen = Pentyl | Ph = Phenyl | tBu = tert-Butyl |
| Cypr = Cyclopropyl | Cybu = Cyclobutyl | Cypen = Cyclopentyl | Cyhex = Cyclohexyl |
| Ac = C(O)Me | | | |

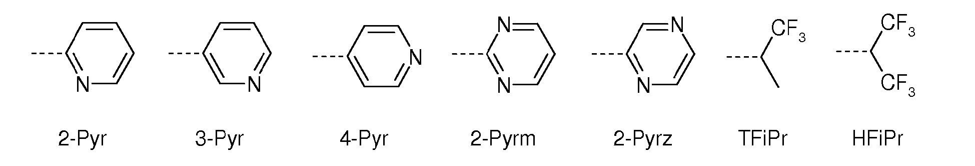

¹H-NMR-Daten (400 MHz., interner Standard: Tetramethylsilan δ = 0.00 ppm; s = Singulett, br. s = breites Singulett, d = Dublett, dd = Doppeldublett, m = Multiplett, q = Quartett, t = Triplett)

**Tabelle 1**

| | | | | | | | | | | |
|---|---|---|---|---|---|---|---|---|---|---|
| Gemäß den oben angegebenen allgemeinen Methoden lassen sich die in der folgenden Tabelle angegebenen Verbi ndungen der Formel (I-A mit Q = Q¹) herstellen. | | | | | | | | | | |
| 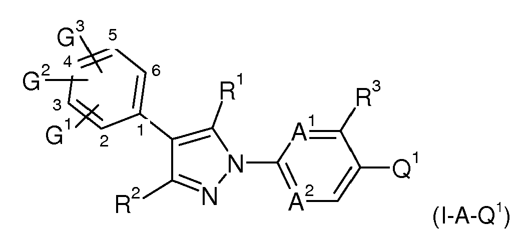 | | | | | | | | | | |

| **Tabelle 1 Beispiel Nr.** | **G¹** | **G²** | **G³** | **R¹** | **R²** | **R³** | **A¹** | **A²** | **Q¹** | **Phys. Chem. Daten** |
|---|---|---|---|---|---|---|---|---|---|---|
| I-A-Q1-001 | 3-CF₃ | H | 5-Cl | NH₂ | CF₃ | CN | CH | CH | 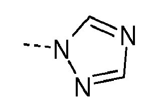 | log P |
| | | | | | | | | | | ¹H-NMR |
| I-A-Q1-002 | 3-CF₃ | H | 5-Cl | NH₂ | CF₃ | CN | CH | CH | 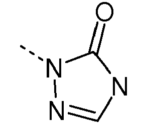 | log P |
| | | | | | | | | | | ¹H-NMR |
| I-A-Q1-003 | 3-Cl | H | 5-Cl | H | CF₃ | Me | CH | CH | 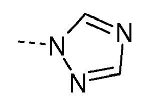 | |
| I-A-Q1-004 | 3-CF₃ | H | 5-CF₃ | H | CF₃ | CN | CH | CH | 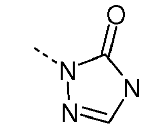 | |
| I-A-Q1-005 | 3-CF₃ | H | H | NH₂ | CF₃ | NO₂ | CH | CH | 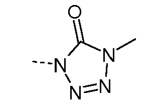 | |
| I-A-Q1-006 | 3-Br | H | 5-Br | NH₂ | CF₃ | CF₃ | CH | CH | 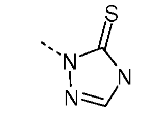 | |
| I-A-Q1-007 | 3-Cl | 4-Cl | 5-Cl | Cl | CF₃ | Br | CH | CH | 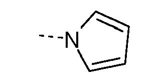 | |
| I-A-Q1-008 | 3-Br | 4-Cl | 5-Br | Cl | C₂F₅ | Cl | CH | CH | 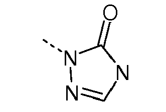 | |
| I-A-Q1-009 | 3-CF₃ | 4-Cl | 5-Cl | Br | C₂F₅ | CH₂OMe | CH | CH | 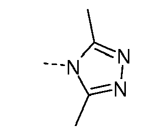 | |
| I-A-Q1-010 | 3-CF₃ | 4-F | H | Br | C₂F₅ | CH₂NHMe | CH | CH | 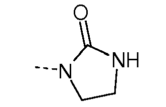 | |
| I-A-Q1-011 | 3-Cl | 4-CF₃ | 5-Cl | I | C₂F₅ | Me | CH | CH | 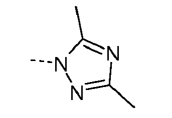 | |
| I-A-Q1-012 | 3-CF₃ | H | 5-Cl | I | CF₃ | CN | CH | CH | 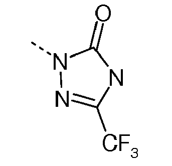 | |
| I-A-Q1-013 | 3-Cl | 4-CF₃ | 5-Cl | CN | CF₃ | NO₂ | N | N | 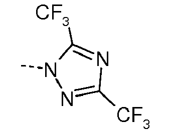 | |
| I-A-Q1-014 | 3-Cl | 4-Br | 5-Cl | CN | CF₃ | CF₃ | N | N | 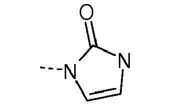 | |
| I-A-Q1-015 | 3-F | 4-F | 5-F | NHCH₂Ph | C₂F₅ | Br | CH | CH | 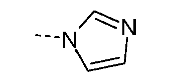 | |
| I-A-Q1-016 | 2-Cl | 3-CF₃ | 4-Cl | NHAc | CF₃ | Cl | CH | CH | 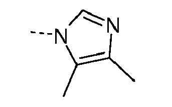 | |
| I-A-Q1-017 | 3-Cl | 4-CF₃ | H | NAc₂ | CF₃ | CH₂OAc | CH | CH | 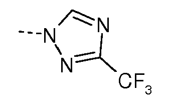 | |
| I-A-Q1-018 | 3-Br | 4-NH₂ | 5-Br | NHMe | CF₂Ph | CH₂NHAc | CH | CH | 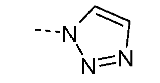 | |
| I-A-Q1-019 | 3-Br | H | H | NMe₂ | CF₂OMe | Me | CH | CH | 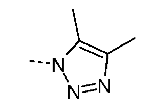 | |
| I-A-Q1-020 | 3-Cl | 4-Cl | H | SMe | CF₂OPh | CN | CH | CH | 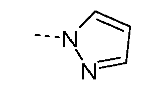 | |
| I-A-Q1-021 | 2-Cl | 3-Cl | 4-Cl | SOMe | CF₂(2-Pyr) | NO2 | N | CH | 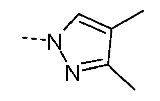 | |
| I-A-Q1-022 | 3-NO₂ | H | H | S0₂Me | CF₂O(2-Pyr) | CF₃ | CH | N | 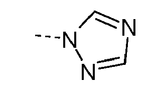 | |
| I-A-Q1-023 | 3-Cl | H | 5-CF₃ | NH₂ | CHMe(OMe) | CN | CH | CH | 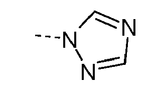 | |
| I-A-Q1-024 | 3-Cl | H | 5-Cl | NH₂ | CF₃ | CN | CH | CH | 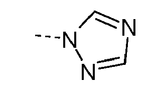 | log P |
| I-A-Q1-025 | 3-Cl | 4-Cl | H | NH₂ | Me | CN | CH | CH | 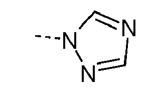 | log P |
| I-A-Q1-026 | 3-Cl | H | 5-Cl | NH₂ | CF₃ | CN | CH | CH | 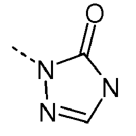 | log P |
| I-A-Q1-027 | 3-CF3 | H | 5-CF3 | NH₂ | CF₃ | CN | CH | CH | 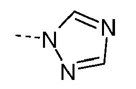 | log P |
| I-A-Q1-028 | 2-Cl | H | 5-Cl | NH₂ | Me | CN | CH | CH | 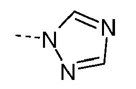 | log P |
| I-A-Q1-029 | H | 4-Cl | H | NH₂ | Me | CN | CH | CH | 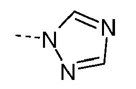 | log P |
| I-A-Q1-030 | H | 4-Cl | H | NH₂ | CHF₂ | CN | CH | CH | 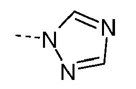 | log P |
| I-A-Q1-031 | 3-Cl | 4-Cl | H | NH₂ | CF₃ | CN | CH | CH | 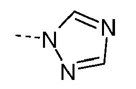 | log P |
| I-A-Q1-032 | 3-F | 4-Cl | H | NH₂ | CF₃ | CN | CH | CH | 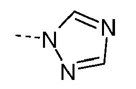 | log P |
| I-A-Q1-033 | H | 4-Cl | H | NH₂ | CF₃ | CN | CH | CH | 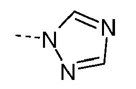 | log P |
| I-A-Q1-034 | 2-F | 4-F | 6-F | NH₂ | CF₃ | CN | CH | CH | 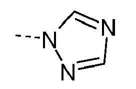 | log P |
| I-A-Q1-035 | 2-Cl | H | H | NH₂ | CF₃ | CN | CH | CH | 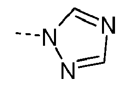 | log P |
| I-A-Q1-036 | 3-Cl | 4-F | H | NH₂ | CF₃ | CN | CH | CH | 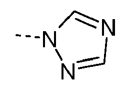 | log P |
| I-A-Q1-037 | 3-F | H | 5-Cl | NH₂ | CF₃ | CN | CH | CH | 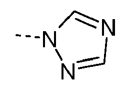 | log P |
| I-A-Q1-038 | 3-Me | H | 5-Me | NH₂ | CF₃ | CN | CH | CH | 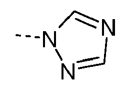 | log P |
| I-A-Q1-039 | 2-Me | H | H | NH₂ | CF₃ | CN | CH | CH | 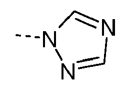 | log P |
| I-A-Q1-040 | H | 4-Br | H | NH₂ | CF₃ | CN | CH | CH | 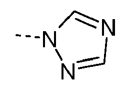 | log P |
| I-A-Q1-041 | 3-F | 4-F | 5-F | NH₂ | CF₃ | CN | CH | CH | 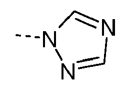 | log P |
| I-A-Q1-042 | 3-F | H | H | NH₂ | CF₃ | CN | CH | CH | 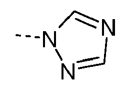 | log P |
| I-A-Q1-043 | H | 4-I | H | NH₂ | CF₃ | CN | CH | CH | 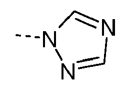 | log P |
| I-A-Q1-044 | 3-F | 4-F | H | NH₂ | CF₃ | CN | CH | CH | 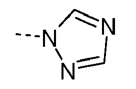 | log P |
| I-A-Q1-045 | 2-Cl | 4-Cl | H | NH₂ | CF₃ | CN | CH | CH | 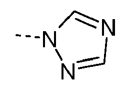 | log P |
| I-A-Q1-046 | 3-Br | H | H | NH₂ | CF₃ | CN | CH | CH | 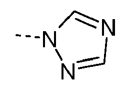 | log P |
| I-A-Q1-047 | H | 4-CN | H | NH₂ | CF₃ | CN | CH | CH | 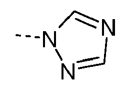 | log P |
| I-A-Q1-048 | 2-F | 4-F | H | NH₂ | CHF₂ | CN | CH | CH | 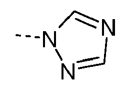 | log P |
| I-A-Q1-049 | 3-Cl | 5-CF₃ | H | NH₂ | CF₂Cl | CN | CH | CH | 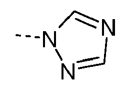 | log P |
| I-A-Q1-050 | 3-CF₃ | H | 5-CF₃ | NH₂ | C₂F₅ | CN | CH | CH | 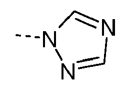 | log P |
| I-A-Q1-051 | 2-Cl | H | 6-F | NH₂ | Me | CN | CH | CH | 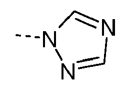 | log P |
| I-A-Q1-052 | 2-Cl | 4-F | H | NH₂ | Me | CN | CH | CH | 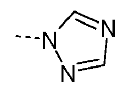 | log P |
| I-A-Q1-053 | 3-Cl | H | H | NH₂ | Me | CN | CH | CH | 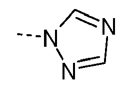 | log P |
| I-A-Q1-054 | 2-F | H | 6-F | NH₂ | Me | CN | CH | CH | 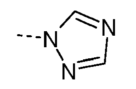 | log P |
| I-A-Q1-055 | 2-Cl | H | 6-Cl | NH₂ | Me | CN | CH | CH | 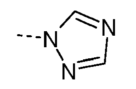 | log P |
| I-A-Q1-056 | 2-Cl | 4-Cl | H | NH₂ | Me | CN | CH | CH | 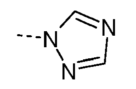 | log P |
| I-A-Q1-057 | 2-F | 4-F | H | NH₂ | Me | CN | CH | CH | 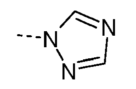 | log P |
| I-A-Q1-058 | H | 4-MeO | H | NH₂ | Me | CN | CH | CH | 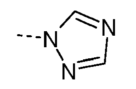 | log P |
| I-A-Q1-059 | H | 4-F | H | NH₂ | Me | CN | CH | CH | 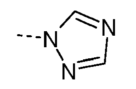 | log P |
| I-A-Q1-060 | 2-Cl | H | H | NH₂ | Me | CN | CH | CH | 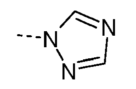 | log P |
| I-A-Q1-061 | 2-F | H | 5-F | NH₂ | Me | CN | CH | CH | 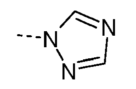 | log P |
| I-A-Q1-062 | H | 4-Cl | H | NH₂ | Et | CN | CH | CH | 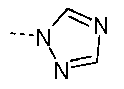 | log P |
| I-A-Q1-063 | H | 4-Cl | H | NH₂ | Ph | CN | CH | CH | 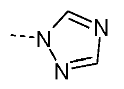 | log P |
| I-A-Q1-064 | H | 4-Cl | H | NH₂ | Cypr | CN | CH | CH | 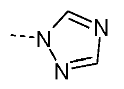 | log P |
| I-A-Q1-065 | H | 4-Cl | H | NH₂ | Pr | CN | CH | CH | 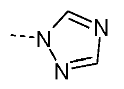 | log P |
| I-A-Q1-066 | H | H | H | NH₂ | Me | CN | CH | CH | 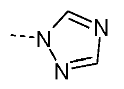 | log P |
| I-A-Q1-067 | 3-Cl | H | 5-Cl | NH₂ | CF₃ | H | CBr | CH | 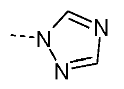 | log P |
| I-A-Q1-068 | 3-Cl | H | 5-Cl | NH₂ | CF₃ | CF₃ | CH | CH | 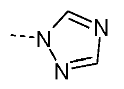 | log P |
| I-A-Q1-069 | 3-Cl | H | 5-Cl | NH₂ | CF₃ | Cl | CH | CH | 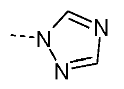 | log P |
| I-A-Q1-070 | 3-Cl | H | 5-Cl | NH₂ | CF₃ | H | CCF₃ | CH | 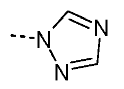 | log P |
| I-A-Q1-071 | 3-Cl | H | 5-CF₃ | NH₂ | (4-ClC₆H₄)OCF₂ | CN | CH | CH | 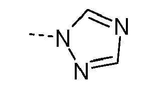 | log P |
| I-A-Q1-072 | 3-Cl | H | 5-CF₃ | NH₂ | (3-ClC₆H₄)OCF₂ | CN | CH | CH | 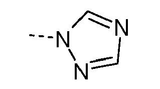 | log P |
| I-A-Q1-073 | 3-Cl | H | 5-CF₃ | NH₂ | (2-ClC₆H₄)OCF₂ | CN | CH | CH | 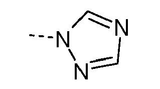 | log P |
| I-A-Q1-074 | 3-Cl | H | 5-CF₃ | NH₂ | PhOCF₂ | CN | CH | CH | 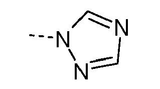 | log P |
| I-A-Q1-075 | 3-Cl | H | 5-Cl | NH₂ | CF₃ | 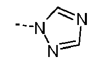 | CH | CH | 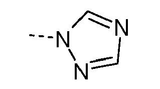 | log P |
| I-A-Q1-076 | 3-Cl | H | 5-Cl | NH₂ | CF₃ | CN | CH | CH | 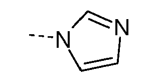 | log P |
| I-A-Q1-077 | 3-Cl | H | 5-Cl | NH₂ | CF₃ | CN | CH | CH | 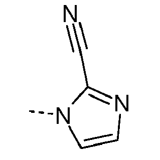 | log P |
| I-A-Q1-078 | 3-Cl | H | 5-Cl | NH₂ | CF₃ | CN | CH | CH | 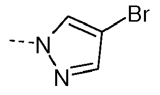 | log P |
| I-A-Q1-079 | 3-Cl | H | 5-Cl | NH₂ | CF₃ | CN | CH | CH | 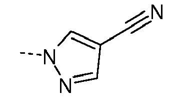 | log P |
| I-A-Q1-080 | 3-Cl | H | 5-Cl | NH₂ | CF₃ | CN | CH | CH | 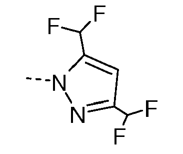 | log P |
| I-A-Q1-081 | 3-Cl | H | 5-Cl | NH₂ | CF₃ | CN | CH | CH | 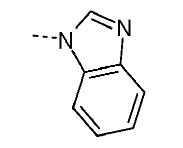 | log P |
| 1-A-QI-082 | 3-Cl | H | 5-Cl | NH₂ | CF₃ | CN | CH | CH | 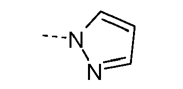 | log P |
| I-A-Q1-083 | 3-Cl | H | 5-Cl | NH₂ | CF₃ | CN | CH | CH | 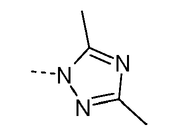 | log P |
| I-A-Q1-084 | 3-Cl | H | 5-Cl | NH₂ | CF₃ | CN | CH | CH | 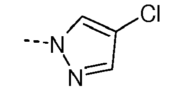 | log P |
| I-A-Q1-085 | 3-Cl | H | 5-Cl | NH₂ | CF₃ | CN | CH | CH | 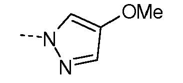 | log P |
| I-A-Q1-086 | 3-Cl | H | 5-Cl | NH₂ | CF₃ | CN | CH | CH | 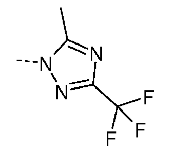 | log P |
| I-A-Q1-087 | 3-Cl | H | 5-Cl | NH₂ | CF₃ | CN | CH | CH | 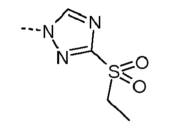 | log P |
| I-A-Q1-088 | 3-Cl | H | 5-Cl | NH₂ | CF₃ | CN | CH | CH | 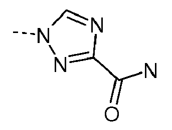 | log P |
| I-A-Q1-089 | 3-Cl | H | 5-Cl | NH₂ | CF₃ | CN | CH | CH | 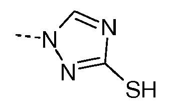 | log P |
| I-A-Q1-090 | 3-Cl | H | 5-Cl | NH₂ | CF₃ | CN | CH | CH | 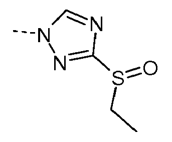 | log P |
| I-A-Q1-091 | 3-Cl | H | 5-Cl | NH₂ | CF₃ | CN | CH | CH | 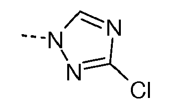 | log P |
| I-A-Q1-092 | 3-Cl | H | 5-Cl | NH₂ | CF₃ | CN | CH | CH | 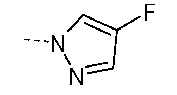 | log P |
| I-A-Q1-093 | 3-Cl | H | 5-Cl | NH₂ | CF₃ | CN | CH | CH | 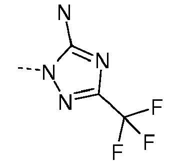 | log P |

### Charakteristische Daten für Synthesebeispiele:

| | | | | | |
|---|---|---|---|---|---|
| **I-A-Q1-001:** | spektroskopische Daten siehe Protokoll unter allgemeinen Synthesevorschriften | | | | |
| | | | | | |
| **I-A-Q1-002:** | spektroskopische Daten siehe Protokoll unter allgemeinen Synthesevorschriften | | | | |
| | | | | | |
| **I-A-Q1-023:** | log P (HCOOH): 2,76 | **I-A-Q1-024:** | log P (HCOOH): 3,90 | **I-A-Q1-025:** | log P (HCOOH): 3,02 |
| | | | | | |
| **I-A-Q1-026:** | log P (HCOOH): 3,37 | **I-A-Q1-027:** | log P (HCOOH): 4,07 | **I-A-Q1-028:** | log P (HCOOH): 2,65 |
| | | | | | |
| **I-A-Q1-029:** | log P (HCOOH): 2,65 | **I-A-Q1-030:** | log P (HCOOH): 2,92 | **I-A-Q1-031:** | log P (HCOOH): 3,60 |
| | | | | | |
| **I-A-Q1-032:** | log P (HCOOH): 3,28 | **I-A-Q1-033:** | log P (HCOOH): 3,30 | **I-A-Q1-034:** | log P (HCOOH): 3,10 |
| | | | | | |
| **I-A-Q1-035:** | log P (HCOOH): 2,80 | **I-A-Q1-036:** | log P (HCOOH): 3,24 | **I-A-Q1-037:** | log P (HCOOH): 3,33 |
| **I-A-Q1-038:** | log P (HCOOH): 3,33 | **I-A-Q1-039:** | log P (HCOOH): 2,89 | **I-A-Q1-040:** | log P (HCOOH): 3,44 |
| | | | | | |
| **I-A-Q1-041:** | log P (HCOOH): 3,19 | **I-A-Q1-042:** | log P (HCOOH): 2,84 | **I-A-Q1-043:** | log P (HCOOH): 3,61 |
| | | | | | |
| **I-A-Q1-044:** | log P (HCOOH): 3,10 | **I-A-Q1-045:** | log P (HCOOH): 2,64 | **I-A-Q1-046:** | log P (HCOOH): 3,24 |
| | | | | | |
| **I-A-Q1-047:** | log P (HCOOH): 2,64 | **I-A-Q1-048:** | log P (HCOOH): 2,39 | **I-A-Q1-049:** | log P (HCOOH): 4,11 |
| | | | | | |
| **I-A-Q1-050:** | log P (HCOOH): 4,34 | **I-A-Q1-051:** | log P (HCOOH): 2,27 | **I-A-Q1-052:** | log P (HCOOH): 2,43 |
| | | | | | |
| **I-A-Q1-053:** | log P (HCOOH): 2,66 | **I-A-Q1-054:** | log P (HCOOH): 2,18 | **I-A-Q1-055:** | log P (HCOOH): 2,47 |
| | | | | | |
| **I-A-Q1-056:** | log P (HCOOH): 2,80 | **I-A-Q1-057:** | log P (HCOOH): 2,35 | **I-A-Q1-058:** | log P (HCOOH): 2,11 |
| | | | | | |
| **I-A-Q1-059:** | log P (HCOOH): 2,30 | **I-A-Q1-060:** | log P (HCOOH): 2,27 | **I-A-Q1-061:** | log P (HCOOH): 2,29 |
| | | | | | |
| **I-A-Q1-062:** | log P (HCOOH): 3,09 | **I-A-Q1-063:** | log P (HCOOH): 3,53 | **I-A-Q1-064:** | log P (HCOOH): 3,31 |
| | | | | | |
| **I-A-Q1-065:** | log P (HCOOH): 3,33 | **I-A-Q1-066:** | log P (HCOOH): 2,14 | **I-A-Q1-067:** | log P (HCOOH): 3,97 |
| | | | | | |
| **I-A-Q1-068:** | log P (HCOOH): 4,32 | **I-A-Q1-069:** | log P (HCOOH): 4,14 | **I-A-Q1-070:** | log P (HCOOH): 4,10 |
| | | | | | |
| **I-A-Q1-071:** | log P (HCOOH): 4,84 | **I-A-Q1-072:** | log P (HCOOH): 4,82 | **I-A-Q1-073:** | log P (HCOOH): 4,51 |
| | | | | | |
| **I-A-Q1-074:** | log P (HCOOH): 4,33 | **I-A-Q1-075:** | log P (HCOOH): 3,28 | **I-A-Q1-076:** | log P (HCOOH): 2,98 |
| | | | | | |
| **I-A-Q1-077:** | log P (HCOOH): 4,05 | **I-A-Q1-078:** | log P (HCOOH): 4,83 | **I-A-Q1-079:** | log P (HCOOH): 4,28 |
| **I-A-Q1-080:** | log P (HCOOH): 4,64 | **I-A-Q1-081:** | log P (HCOOH): 4,28 | **I-A-Q1-082:** | log P (HCOOH): 4,34 |
| | | | | | |
| **I-A-Q1-083:** | log P (HCOOH): 3,84 | **I-A-Q1-084:** | log P (HCOOH): 4,70 | **I-A-Q1-085:** | log P (HCOOH): 4,45 |
| | | | | | |
| **I-A-Q1-086:** | log P (HCOOH): 4,57 | **I-A-Q1-087:** | log P (HCOOH): 3,78 | **I-A-Q1-088:** | log P (HCOOH): 3,06 |
| | | | | | |
| **I-A-Q1-089:** | log P (HCOOH): 3,53 | **I-A-Q1-090:** | log P (HCOOH): 3,47 | **I-A-Q1-091:** | log P (HCOOH): 4,17 |
| | | | | | |
| **I-A-Q1-092:** | log P (HCOOH): 4,40 | **I-A-Q1-093:** | log P (HCOOH): 4,00 | | |

**Tabelle 2**

| | | | | | | | | | | | | |
|---|---|---|---|---|---|---|---|---|---|---|---|---|
| Gemäß den oben angegebenen allgemeinen Methoden lassen sich die in der folgenden Tabelle angegebenen Verbindungen der Formel (I-A mit Q = Q²) herstellen. | | | | | | | | | | | | |
| 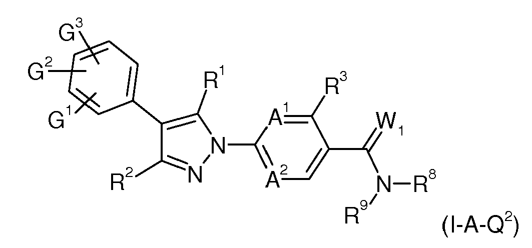 | | | | | | | | | | | | |

| **Tabelle 2 Beispiel Nr.** | **G¹** | **G²** | **G³** | **R¹** | **R²** | **R³** | **A¹** | **A²** | **W¹** | **R⁸** | **R⁹** | **Phys. Chem. Daten** |
|---|---|---|---|---|---|---|---|---|---|---|---|---|
| I-A-Q2-001 | 3-Cl | H | 5-Cl | H | CF₃ | CF₃ | CH | CH | O | H | CH₂-2-Pyr | log P |
| | | | | | | | | | | | | ¹H-NMR |
| I-A-Q2-002 | 3-Cl | H | 5-Cl | H | CF₃ | CF₃ | CH | CH | O | H | CH₂CF₃ | log P |
| | | | | | | | | | | | | ¹H-NMR |
| I-A-Q2-003 | 3-Cl | H | 5-Cl | H | CF₃ | NO₂ | CH | CH | O | H | CH₂-2-Pyr | ¹H-NMR |
| I-A-Q2-004 | 3-Cl | H | 5-Cl | H | CF₃ | NO₂ | CH | CH | O | H | CH₂CF₃ | ¹H-NMR |
| I-A-Q2-005 | 3-Cl | H | 5-Cl | H | CF₃ | CF₃ | CH | CH | O | H | CHMeCF₃ | ¹H-NMR |
| I-A-Q2-006 | 3-Cl | H | 5-Cl | H | H | CF₃ | CH | CH | O | H | CHMeCF₃ | ¹H-NMR |
| I-A-Q2-007 | 3-Cl | H | 5-Cl | H | H | CF₃ | CH | CH | O | H | CH₂CF₃ | |
| I-A-Q2-008 | 3-Cl | H | 5-Cl | H | H | CF₃ | CH | CH | O | H | CH₂-2-Pyr | ¹H-NMR |
| I-A-Q2-009 | 3-CF₃ | H | 5-CF₃ | H | CF₃ | CF₃ | CH | CH | O | H | CH₂-2-Pyr | ¹H-NMR |
| I-A-Q2-010 | 3-CF₃ | H | 5-CF₃ | H | CF₃ | CF₃ | CH | CH | O | H | CH₂CF₃ | ¹H-NMR |
| I-A-Q2-011 | 3-Cl | H | 5-Cl | NH₂ | CF₃ | Me | CH | CH | O | H | TFiPr | log P |
| | | | | | | | | | | | | ¹H-NMR |
| I-A-Q2-012 | 3-Cl | H | 5-Cl | NH₂ | CF₃ | Me | CH | CH | O | H | CH₂-2-Pyr | log P |
| | | | | | | | | | | | | ¹H-NMR |
| I-A-Q2-013 | 3-Cl | H | 5-Cl | NH₂ | CF₃ | Me | CH | CH | O | H | Me | log P |
| I-A-Q2-014 | 3-Cl | H | 5-Cl | NH₂ | CF₃ | Me | CH | CH | O | H | CH₂CF₃ | log P |
| | | | | | | | | | | | | ¹H-NMR |
| I-A-Q2-015 | 3-Cl | H | 5-Cl | NH₂ | CF₃ | Me | CH | CH | O | H | CHMe-2-Pyr | log P |
| | | | | | | | | | | | | ¹H-NMR |
| I-A-Q2-016 | 3-Cl | H | 5-Cl | NH₂ | CF₃ | Me | CH | CH | O | H | CHMePh | log P |
| | | | | | | | | | | | | ¹H-NMR |
| I-A-Q2-017 | 3-Cl | H | 5-Cl | NH₂ | CF₃ | Me | CH | CH | O | H | CH₂CHF₂ | log P |
| | | | | | | | | | | | | ¹H-NMR |
| I-A-Q2-018 | 3-Cl | H | 5-Cl | NH₂ | CF₃ | Me | CH | CH | O | H | CH₂-2-Pyrm | log P |
| | | | | | | | | | | | | ¹H-NMR |
| I-A-Q2-019 | 3-Cl | 4-Cl | H | NH₂ | CF₃ | Me | CH | CH | O | H | TFiPr | log P |
| | | | | | | | | | | | | ¹H-NMR |
| I-A-Q2-020 | 3-Cl | H | 5-Cl | NH₂ | CF₃ | CF₃ | N | N | O | H | CH₂-2-Pyr | log P |
| | | | | | | | | | | | | ¹H-NMR |
| I-A-Q2-021 | 3-Cl | H | 5-Cl | NH₂ | CF₃ | CF₃ | N | N | O | H | CHMe-2-Pyr | log P |
| | | | | | | | | | | | | ¹H-NMR |
| I-A-Q2-022 | 3-Cl | H | 5-Cl | Cl | CF₃ | Me | CH | CH | O | H | TFiPr | log P |
| | | | | | | | | | | | | ¹H-NMR |
| I-A-Q2-023 | 3-Cl | H | 5-Cl | Br | CF₃ | Me | CH | CH | O | H | CH₂-2-Pyr | log P |
| | | | | | | | | | | | | ¹H-NMR |
| I-A-Q2-024 | 3-Cl | H | 5-Cl | Br | CF₃ | Me | CH | CH | O | H | TFiPr | log P |
| | | | | | | | | | | | | ¹H-NMR |
| I-A-Q2-025 | 3-Cl | H | 5-Cl | I | CF₃ | Me | CH | CH | O | H | CH₂-2-Pyr | log P |
| | | | | | | | | | | | | ¹H-NMR |
| I-A-Q2-026 | 3-Cl | H | 5-Cl | I | CF₃ | Me | CH | CH | O | H | TFiPr | log P |
| | | | | | | | | | | | | ¹H-NMR |
| I-A-Q2-027 | 3-Cl | H | 5-Cl | SMe | CF₃ | Me | CH | CH | O | H | TFiPr | log P |
| | | | | | | | | | | | | ¹H-NMR |
| I-A-Q2-028 | 3-Cl | H | 5-Cl | SMe | CF₃ | Me | CH | CH | O | H | CH₂-2-Pyr | log P |
| | | | | | | | | | | | | ¹H-NMR |
| I-A-Q2-029 | 3-Cl | H | 5-Cl | NH₂ | CF₃ | Me | CH | CH | O | H | 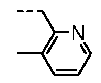 | log P |
| I-A-Q2-030 | 3-Cl | H | 5-Cl | NH₂ | CF₃ | Me | CH | CH | O | H | CH₂COOMe | log P |
| I-A-Q2-031 | 3-Cl | H | 5-Cl | NH₂ | CF₃ | Me | CH | CH | O | H | 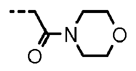 | log P |
| I-A-Q2-032 | 3-Cl | H | 5-Cl | NH₂ | CF₃ | Me | CH | CH | O | H | 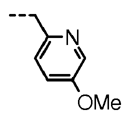 | log P |
| I-A-Q2-033 | 3-Cl | H | 5-Cl | NH₂ | CF₃ | Me | CH | CH | O | Me | CH₂CF₂CHF₂ | log P |
| I-A-Q2-034 | 3-Cl | H | 5-Cl | NH₂ | CF₃ | Me | CH | CH | O | H | 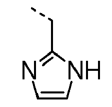 | log P |
| I-A-Q2-035 | 3-Cl | H | 5-Cl | NH₂ | CF₃ | Me | CH | CH | O | H | CH₂CF₂CHF₂ | log P |
| I-A-Q2-036 | 3-Cl | H | 5-Cl | NH₂ | CF₃ | Me | CH | CH | O | H | 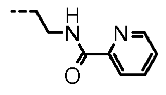 | log P |
| I-A-Q2-037 | 3-Cl | H | 5-Cl | NH₂ | CF₃ | Me | CH | CH | O | H | 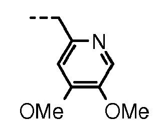 | log P |
| I-A-Q2-038 | 3-Cl | H | 5-Cl | NH₂ | CF₃ | Me | CH | CH | O | H | 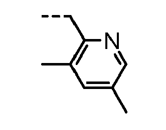 | log P |
| I-A-Q2-039 | 3-Cl | H | 5-Cl | NH₂ | CF₃ | Me | CH | CH | O | H | 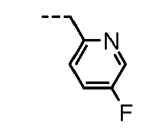 | log P |
| I-A-Q2-040 | 3-Cl | H | 5-Cl | NH₂ | CF₃ | Me | CH | CH | O | H | 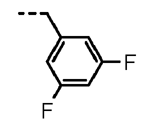 | log P |
| I-A-Q2-041 | 3-Cl | H | 5-Cl | NH₂ | CF₃ | Me | CH | CH | O | H | 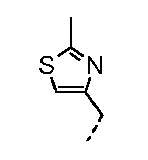 | log P |
| I-A-Q2-042 | 3-Cl | H | 5-Cl | NH₂ | CF₃ | Me | CH | CH | O | H | 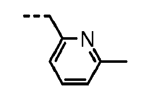 | log P |
| I-A-Q2-043 | 3-Cl | H | 5-Cl | NH₂ | CF₃ | Me | CH | CH | O | H | 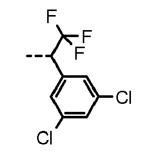 | log P |
| I-A-Q2-044 | 3-Cl | H | 5-Cl | NH₂ | CF₃ | Me | CH | CH | O | H | 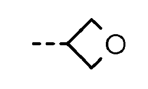 | log P |
| I-A-Q2-045 | 3-Cl | H | 5-Cl | NH₂ | CF₃ | Me | CH | CH | O | H | 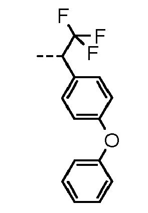 | log P |
| I-A-Q2-046 | 3-Cl | H | 5-Cl | NH₂ | CF₃ | Me | CH | CH | O | H | 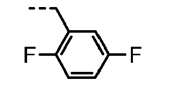 | log P |
| I-A-Q2-047 | 3-Cl | H | 5-Cl | NH₂ | CF₃ | Me | CH | CH | O | H | 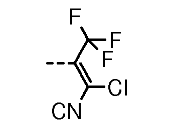 | log P |
| I-A-Q2-048 | 3-Cl | H | 5-Cl | NH₂ | CF₃ | Me | CH | CH | O | H | 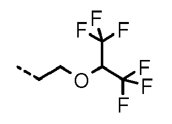 | log P |
| I-A-Q2-049 | 3-Cl | H | 5-Cl | NH₂ | CF₃ | Me | CH | CH | O | H | 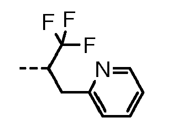 | log P |
| I-A-Q2-050 | 3-Cl | H | 5-Cl | NH₂ | CF₃ | Me | CH | CH | O | H | 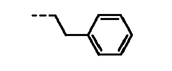 | log P |
| I-A-Q2-051 | 3-Cl | H | 5-Cl | NH₂ | CF₃ | Me | CH | CH | O | H | 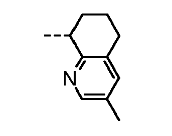 | log P |
| I-A-Q2-052 | 3-Cl | H | 5-Cl | NH₂ | CF₃ | Me | CH | CH | O | H | 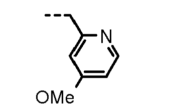 | log P |
| I-A-Q2-053 | 3-Cl | H | 5-Cl | NH₂ | CF₃ | Me | CH | CH | O | H | 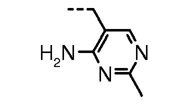 | log P |
| I-A-Q2-054 | 3-Cl | H | 5-Cl | NH₂ | CF₃ | Me | CH | CH | O | H | 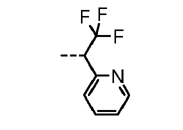 | log P |
| I-A-Q2-055 | 3-Cl | H | 5-Cl | NH₂ | CF₃ | Me | CH | CH | O | H | 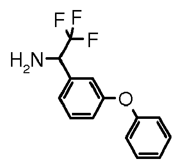 | log P |
| I-A-Q2-056 | 3-Cl | H | 5-Cl | NH₂ | CF₃ | Me | CH | CH | O | Me | Me | log P |
| I-A-Q2-057 | 3-Cl | H | 5-Cl | NH₂ | CF₃ | Me | CH | CH | O | H | 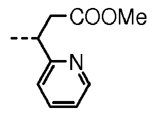 | log P |
| I-A-Q2-058 | 3-Cl | H | 5-Cl | NH₂ | CF₃ | Me | CH | CH | O | H | 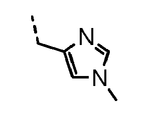 | log P |
| I-A-Q2-059 | 3-Cl | H | 5-Cl | NH₂ | CF₃ | Me | CH | CH | O | H | 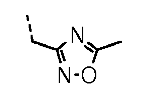 | log P |
| I-A-Q2-060 | 3-Cl | H | 5-Cl | NH₂ | CF₃ | Me | CH | CH | O | H | 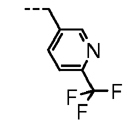 | log P |
| I-A-Q2-061 | 3-Cl | H | 5-Cl | NH₂ | CF₃ | Me | CH | CH | O | H | 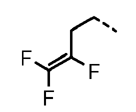 | log P |
| I-A-Q2-062 | 3-Cl | H | 5-Cl | NH₂ | CF₃ | Me | CH | CH | O | H | CH₂CHCH₂ | log P |
| I-A-Q2-063 | 3-Cl | H | 5-Cl | SOM e | CF₃ | Me | CH | CH | O | H | CH₂-2-Pyr | log P |
| | | | | | | | | | | | | ¹H-NMR |
| I-A-Q2-064 | 3-Cl | H | 5-Cl | N=C HN Me₂ | CF₃ | Me | CH | CH | O | H | 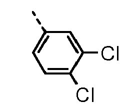 | log P |
| I-A-Q2-065 | 3-Cl | H | 5-Cl | NH₂ | CF₃ | Me | CH | CH | O | H | 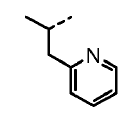 | log P |
| I-A-Q2-066 | 3-Cl | H | 5-Cl | NH₂ | CF₃ | Me | CH | CH | O | H | 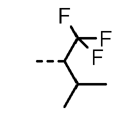 | log P |
| I-A-Q2-067 | 3-Cl | H | 5-Cl | NH₂ | CF₃ | H | CH | CH | O | H | 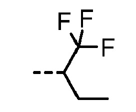 | log P |
| I-A-Q2-068 | 3-Cl | H | 5-CF₃ | SMe | CF₃ | H | CH | CH | O | H | TFiPr | log P |
| I-A-Q2-069 | 3-Cl | H | 5-Cl | NH₂ | CF₃ | H | CH | CH | O | H | CMe₂CH₂CF₃ | log P |
| I-A-Q2-070 | 3-Cl | H | 5-Cl | NH₂ | CF₃ | H | CH | CH | O | H | 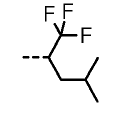 | log P |
| I-A-Q2-071 | 3-Cl | H | 5-Cl | NH₂ | CF₃ | H | CH | CH | O | H | 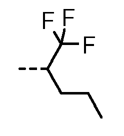 | log P |
| I-A-Q2-072 | 3-Cl | H | 5-Cl | NH₂ | CF₃ | H | CH | CH | O | H | 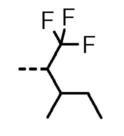 | log P |
| I-A-Q2-073 | 3-Cl | H | 5-Cl | NH₂ | CF₃ | H | CH | CH | O | H | 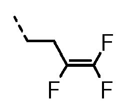 | log P |
| I-A-Q2-074 | H | 4-Cl | H | NH₂ | iPr | H | CH | CH | O | H | Me | log P |
| I-A-Q2-075 | H | 4-Cl | H | NH₂ | Ph | H | CH | CH | O | H | TFiPr | log P |
| I-A-Q2-076 | H | 4-Cl | H | NH₂ | Ph | H | CH | CH | O | H | CH₂-2-Pyrm | log P |
| I-A-Q2-077 | H | 4-Cl | H | NH₂ | Ph | H | CH | CH | O | H | CHMePh | log P |
| I-A-Q2-078 | H | 4-Cl | H | NH₂ | Ph | H | CH | CH | O | H | Me | log P |
| I-A-Q2-079 | 2-Cl | H | H | NH₂ | CF₃ | H | CH | CH | O | H | TFiPr | log P |
| I-A-Q2-080 | 2-Cl | H | H | NH₂ | CF₃ | H | CH | CH | O | H | CH₂-2-Pyrm | log P |
| I-A-Q2-081 | 2-Cl | H | H | NH₂ | CF₃ | H | CH | CH | O | H | CHMePh | log P |
| I-A-Q2-082 | 2-Cl | H | H | NH₂ | CF₃ | H | CH | CH | O | H | Me | log P |
| I-A-Q2-083 | 3-Cl | H | H | NH₂ | CF₃ | H | CH | CH | O | H | TFiPr | log P |
| I-A-Q2-084 | 3-F | H | 5-Cl | NH₂ | CF₃ | H | CH | CH | O | H | TFiPr | log P |
| I-A-Q2-085 | 3-Cl | H | H | NH₂ | CF₃ | H | CH | CH | O | H | CH₂CF₃ | log P |
| I-A-Q2-086 | 3-F | H | 5-Cl | NH₂ | CF₃ | H | CH | CH | O | H | CH₂CF₃ | log P |
| I-A-Q2-087 | 3-Cl | H | H | NH₂ | CF₃ | H | CH | CH | O | H | CHMe-2-Pyr | log P |
| I-A-Q2-088 | 3-F | H | 5-Cl | NH₂ | CF₃ | H | CH | CH | O | H | CHMe-2-Pyr | log P |
| I-A-Q2-089 | 3-Cl | H | H | NH₂ | CF₃ | H | CH | CH | O | H | CHMePh | log P |
| I-A-Q2-090 | 3-F | H | 5-Cl | NH₂ | CF₃ | H | CH | CH | O | H | CHMePh | log P |
| I-A-Q2-091 | 3-Cl | H | H | NH₂ | CF₃ | H | CH | CH | O | H | Cypr | log P |
| I-A-Q2-092 | 3-F | H | 5-Cl | NH₂ | CF₃ | H | CH | CH | O | H | Cypr | log P |
| I-A-Q2-093 | 3-Cl | H | H | NH₂ | CF₃ | H | CH | CH | O | H | Cyhex | log P |
| I-A-Q2-094 | 3-Cl | H | H | NH₂ | CF₃ | H | CH | CH | O | H | Me | log P |
| I-A-Q2-095 | 3-Cl | H | 5-CF₃ | NH₂ | CF₃ | H | CH | CH | O | H | TFiPr | log P |
| I-A-Q2-096 | 3-Cl | H | 5-Cl | Br | CF₃ | H | CH | CH | O | H | TFiPr | log P |
| I-A-Q2-097 | 3-Cl | H | 5-Cl | NH₂ | CF₃ | H | N | CH | O | H | iPr | log P |
| I-A-Q2-098 | 3-Cl | H | H | Br | Me | H | CH | CH | O | H | TFiPr | log P |
| I-A-Q2-099 | 3-Cl | H | 5-Cl | NH₂ | CF₃ | H | N | CH | O | H | CMe₂CH₂CF₃ | log P |
| I-A-Q2-100 | 3-Cl | H | 5-Cl | NH₂ | CF₃ | H | N | CH | O | H | TFiPr | log P |
| I-A-Q2-101 | 3-Cl | H | 5-Cl | NH₂ | CF₃ | H | N | CH | O | H | CH₂CF₃ | log P |
| I-A-Q2-102 | 3-Cl | H | 5-Cl | NH₂ | CF₃ | H | N | CH | O | H | CHMe-2-Pyr | log P |
| I-A-Q2-103 | 3-Cl | H | 5-Cl | NH₂ | CF₃ | H | N | CH | O | H | CHMePh | log P |
| I-A-Q2-104 | 3-Cl | H | 5-Cl | NH₂ | CF₃ | H | N | CH | O | H | CH₂CHCH₂ | log P |
| I-A-Q2-105 | 3-Cl | H | 5-Cl | NH₂ | CF₃ | H | N | CH | O | H | CH₂-2-Pyr | log P |
| I-A-Q2-106 | 3-Cl | H | 5-Cl | NH₂ | CF₃ | H | N | CH | O | H | Cypr | log P |
| I-A-Q2-107 | 3-Cl | H | 5-Cl | NH₂ | CF₃ | H | N | CH | O | H | CH₂-3-Pyr | log P |
| I-A-Q2-108 | 3-Cl | H | 5-Cl | NH₂ | CF₃ | H | N | CH | O | H | CH₂-2-Pyrm | log P |
| I-A-Q2-109 | 3-Cl | H | 5-Cl | Br | CF₃ | H | N | CH | O | H | Me | log P |
| I-A-Q2-110 | 3-Cl | H | 5-Cl | NH₂ | CF₃ | Me | CH | CH | O | Me | CH₂-2-Pyr | log P |
| I-A-Q2-111 | 3-Cl | H | 5-Cl | NH₂ | CF₃ | Me | CH | CH | O | Cypr | CH₂-2-Pyr | log P |
| I-A-Q2-112 | 3-Cl | H | 5-Cl | NH₂ | CF₃ | Me | CH | CH | O | H | 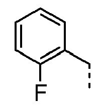 | log P |
| I-A-Q2-113 | 3-Cl | H | 5-Cl | H | CF₃ | Me | CH | CH | O | H | CH₂Cypr | |
| I-A-Q2-114 | 3-CF₃ | H | 5-CF₃ | H | CF₃ | CN | CH | CH | O | H | CH₂C₂F₅ | |
| I-A-Q2-115 | 3-CF₃ | H | H | NH₂ | CF₃ | NO₂ | CH | CH | O | H | CHMe₂ | |
| I-A-Q2-116 | 3-Br | H | 5-Br | NH₂ | CF₃ | CF₃ | CH | CH | O | H | CH₂CH₂SMe | |
| I-A-Q2-117 | 3-Cl | 4-Cl | 5-Cl | Cl | CF₃ | Br | CH | CH | O | H | TFiPr | |
| I-A-Q2-118 | 3-Br | 4-Cl | 5-Br | Cl | C₂F₅ | Cl | CH | CH | O | H | HFiPr | |
| I-A-Q2-119 | 3-CF₃ | 4-Cl | 5-Cl | Br | C₂F₅ | CH₂O Me | CH | CH | O | H | CH₂Ph | |
| I-A-Q2-120 | 3-CF₃ | 4-F | H | Br | C₂F₅ | CH₂N HMe | CH | CH | O | H | CH₂-2-Pyr | |
| I-A-Q2-121 | 3-Cl | 4-CF₃ | 5-Cl | I | C₂F₅ | Me | CH | CH | O | H | CH₂-2-Pyr | |
| I-A-Q2-122 | 3-CF₃ | H | 5-Cl | I | CF₃ | CN | CH | CH | O | H | CHMe-2-Pyr | |
| I-A-Q2-123 | 3-Cl | 4-CF₃ | 5-Cl | CN | CF₃ | NO₂ | N | N | O | H | CHMe-2-Pyr | |
| I-A-Q2-124 | 3-Cl | 4-Br | 5-Cl | CN | CF₃ | CF₃ | N | N | O | H | CH₂-3-Pyr | |
| I-A-Q2-125 | 3-F | 4-F | 5-F | NHC H₂Ph | Cl | Br | CH | CH | O | H | CH₂-3-Pyr | |
| I-A-Q2-126 | 2-Cl | 3-CF₃ | 4-Cl | NHA c | Br | Cl | CH | CH | s | H | CHMe-3-Pyr | |
| I-A-Q2-127 | 3-Cl | 4-CF₃ | H | NAc₂ | CF₃ | CH₂O Ac | CH | CH | s | H | CHMe-3-Pyr | |
| I-A-Q2-128 | 3-Br | 4-NH₂ | 5-Br | NH Me | CF₂Ph | CH₂N HAc | CH | CH | O | Me | CH₂-4-Pyr | |
| I-A-Q2-129 | 3-Br | H | H | NMe 2 | CF₂O Me | Me | CH | CH | O | CH₂CF₃ | CH₂-4-Pyr | |
| I-A-Q2-130 | 3-Cl | 4-Cl | H | SMe | CF₂OP h | CN | CH | CH | O | CH₂Ph | CHMe-4-Pyr | |
| I-A-Q2-131 | 2-Cl | 3-Cl | 4-Cl | SOM e | CF₂(2-Pyr) | NO2 | N | CH | O | CH₂-4-Pyr | CHMe-4-Pyr | |
| I-A-Q2-132 | 3-NO₂ | H | H | SO₂ Me | CF₂O( 2-Pyr) | CF₃ | CH | N | O | Cypr | CH₂-2-Pyrm | |
| I-A-Q2-133 | 3-Cl | H | 5-Cl | NH₂ | CF₃ | H | CH | CH | O | H | TFiPr | log P |
| I-A-Q2-134 | 3-Cl | H | 5-Cl | NH₂ | CF₃ | H | CH | CH | O | H | CH₂-2-Pyr | log P |
| I-A-Q2-135 | 3-Cl | H | 5-Cl | NH₂ | CF₃ | H | CH | CH | O | H | iPr | log P |
| I-A-Q2-136 | 3-Cl | H | 5-Cl | NH₂ | CF₃ | H | CH | CH | O | H | Cypr | log P |
| I-A-Q2-137 | 3-Cl | H | 5-Cl | NH₂ | CF₃ | H | CH | CH | O | H | CH₂-2-Pyrm | log P |
| I-A-Q2-138 | 3-Cl | H | 5-Cl | NH₂ | CF₃ | H | CH | CH | O | H | CH₂-CF₃ | log P |
| I-A-Q2-139 | 3-Cl | H | 5-Cl | NH₂ | CF₃ | H | CH | CH | O | H | CH₂-CH₂-CF₃ | log P |
| I-A-Q2-140 | 3-Cl | H | 5-Cl | NH₂ | CF₃ | H | CH | CH | O | H | CH₂-CHF₂ | log P |
| I-A-Q2-141 | 3-Cl | H | 5-Cl | NH₂ | CF₃ | H | CH | CH | O | H | 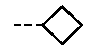 | log P |
| I-A-Q2-142 | 3-Cl | H | 5-Cl | NH₂ | CF₃ | H | CH | CH | O | H | Et | log P |
| I-A-Q2-143 | 3-Cl | H | 5-Cl | NH₂ | CF₃ | H | CH | CH | O | H | CH₂-3-Pyr | log P |
| I-A-Q2-144 | 3-Cl | H | 5-Cl | NH₂ | CF₃ | H | CH | CH | O | H | CH₂-CH=CH₂ | log P |
| I-A-Q2-145 | 3-Cl | H | 5-Cl | NH₂ | CF₃ | H | CH | CH | O | H | CH(Me)Et | log P |
| I-A-Q2-146 | 3-Cl | H | 5-Cl | NH₂ | CF₃ | H | CH | CH | O | H | tBu | log P |
| I-A-Q2-147 | 3-Cl | H | 5-Cl | NH₂ | CF₃ | H | CH | CH | O | H | CHMe-2-Pyr | log P |
| I-A-Q2-148 | 3-Cl | H | 5-Cl | NH₂ | CF₃ | H | CH | CH | O | H | 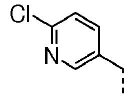 | log P |
| I-A-Q2-149 | 3-Cl | H | 5-Cl | NH₂ | CF₃ | H | CH | CH | O | H | CH₂-Cypr | log P |
| I-A-Q2-150 | 3-Cl | H | 5-Cl | NH₂ | CF₃ | H | CH | CH | O | H | CHMe-Ph | log P |
| I-A-Q2-151 | 3-Cl | H | 5-Cl | NH₂ | CF₃ | H | CH | CH | O | H | Pr | log P |
| I-A-Q2-152 | 3-Cl | H | 5-Cl | NH₂ | CF₃ | H | CH | CH | O | H | CH₂-Ph | log P |
| I-A-Q2-153 | H | 4-Cl | H | NH₂ | CF₃ | H | CH | CH | O | H | TFiPr | log P |
| I-A-Q2-154 | 3-Cl | 4-Cl | H | NH₂ | CF₃ | H | CH | CH | O | H | iPr | log P |
| I-A-Q2-155 | 3-Cl | 4-Cl | H | NH₂ | CF₃ | H | CH | CH | O | H | TFiPr | log P |
| I-A-Q2-156 | 3-Cl | 4-Cl | H | NH₂ | CF₃ | H | CH | CH | O | H | CH₂-CF₃ | log P |
| I-A-Q2-157 | 3-Cl | 4-Cl | H | NH₂ | CF₃ | H | CH | CH | O | H | Cypr | log P |
| I-A-Q2-158 | 3-Cl | 4-Cl | H | NH₂ | CF₃ | H | CH | CH | O | H | CH₂-2-Pyr | log P |
| I-A-Q2-159 | 3-Cl | 4-Cl | H | NH₂ | CF₃ | H | CH | CH | O | H | CH₂-CHF₂ | log P |
| I-A-Q2-160 | 3-Cl | 4-Cl | H | NH₂ | CF₃ | H | CH | CH | O | H | CHMe-2-Pyr | log P |
| I-A-Q2-161 | H | 4-Cl | H | NH₂ | CF₃ | H | CH | CH | O | H | iPr | log P |
| I-A-Q2-162 | H | 4-Cl | H | NH₂ | CF₃ | H | CH | CH | O | H | CH₂-CF₃ | log P |
| I-A-Q2-163 | H | 4-Cl | H | NH₂ | CF₃ | H | CH | CH | O | H | Cypr | log P |
| I-A-Q2-164 | H | 4-Cl | H | NH₂ | CF₃ | H | CH | CH | O | H | CH₂-CHF₂ | log P |
| I-A-Q2-165 | H | 4-Cl | H | NH₂ | CF₃ | H | CH | CH | O | H | CHMe-2-Pyr | log P |
| I-A-Q2-166 | 3-F | 4-Cl | H | NH₂ | CF₃ | H | CH | CH | O | H | TFiPr | log P |
| I-A-Q2-167 | 3-F | 4-Cl | H | NH₂ | CF₃ | H | CH | CH | O | H | Cypr | log P |
| I-A-Q2-168 | 3-F | 4-Cl | H | NH₂ | CF₃ | H | CH | CH | O | H | CH₂-2-Pyr | log P |
| I-A-Q2-169 | 4-Cl | 3-F | H | NH₂ | CF₃ | H | CH | CH | O | H | TFiPr | log P |
| I-A-Q2-170 | 4-Cl | 3-F | H | NH₂ | CF₃ | H | CH | CH | O | H | Cypr | log P |
| I-A-Q2-171 | 4-Cl | 3-F | H | NH₂ | CF₃ | H | CH | CH | O | H | CH₂-2-Pyr | log P |
| I-A-Q2-172 | 3-Br | H | H | NH₂ | CF₃ | H | CH | CH | O | H | TFiPr | log P |
| I-A-Q2-173 | 3-Br | H | H | NH₂ | CF₃ | H | CH | CH | O | H | Cypr | log P |
| I-A-Q2-174 | 3-Br | H | H | NH₂ | CF₃ | H | CH | CH | O | H | CH₂-2-Pyr | log P |
| I-A-Q2-175 | 3-F | 4-F | H | NH₂ | CF₃ | H | CH | CH | O | H | TFiPr | log P |
| I-A-Q2-176 | 3-F | 4-F | H | NH₂ | CF₃ | H | CH | CH | O | H | Cypr | log P |
| I-A-Q2-177 | 3-F | 4-F | H | NH₂ | CF₃ | H | CH | CH | O | H | CH₂-2-Pyr | log P |
| I-A-Q2-178 | 3-Me | H | 5-Me | NH₂ | CF₃ | H | CH | CH | O | H | TFiPr | log P |
| I-A-Q2-179 | 3-Me | H | 5-Me | NH₂ | CF₃ | H | CH | CH | O | H | Cypr | log P |
| I-A-Q2-180 | 3-Me | H | 5-Me | NH₂ | CF₃ | H | CH | CH | O | H | CH₂-2-Pyr | log P |
| I-A-Q2-181 | 3-F | H | H | NH₂ | CF₃ | H | CH | CH | O | H | iPr | log P |
| I-A-Q2-182 | 3-F | H | H | NH₂ | CF₃ | H | CH | CH | O | H | TFiPr | log P |
| I-A-Q2-183 | 3-F | H | H | NH₂ | CF₃ | H | CH | CH | O | H | CH₂-CF₃ | log P |
| I-A-Q2-184 | 3-F | H | H | NH₂ | CF₃ | H | CH | CH | O | H | Cypr | log P |
| I-A-Q2-185 | 3-F | H | H | NH₂ | CF₃ | H | CH | CH | O | H | CH₂-2-Pyr | log P |
| I-A-Q2-186 | 3-F | H | H | NH₂ | CF₃ | H | CH | CH | O | H | CHMe-2-Pyr | log P |
| I-A-Q2-187 | H | 4-Br | H | NH₂ | CF₃ | H | CH | CH | O | H | iPr | log P |
| I-A-Q2-188 | H | 4-Br | H | NH₂ | CF₃ | H | CH | CH | O | H | TFiPr | log P |
| I-A-Q2-189 | H | 4-Br | H | NH₂ | CF₃ | H | CH | CH | O | H | CH₂-CF₃ | log P |
| I-A-Q2-190 | H | 4-Br | H | NH₂ | CF₃ | H | CH | CH | O | H | Cypr | log P |
| I-A-Q2-191 | H | 4-Br | H | NH₂ | CF₃ | H | CH | CH | O | H | CH₂-2-Pyr | log P |
| I-A-Q2-192 | H | 4-Br | H | NH₂ | CF₃ | H | CH | CH | O | H | CHMe-2-Pyr | log P |
| I-A-Q2-193 | 3-Cl | H | 5-Cl | NH₂ | CF₃ | H | CH | CH | O | H | Me | log P |
| I-A-Q2-194 | 3-Cl | 4-Cl | H | NH₂ | CF₃ | H | CH | CH | O | H | Me | log P |
| I-A-Q2-195 | H | 4-Cl | H | NH₂ | CF₃ | H | CH | CH | O | H | Me | log P |
| I-A-Q2-196 | 3-F | 4-F | 5-F | NH₂ | CF₃ | H | CH | CH | O | H | CHMe-2-Pyr | log P |
| I-A-Q2-197 | 3-Cl | H | 5-Cl | NHA c | CF₃ | Me | CH | CH | O | H | CH₂-2-Pyr | log P |
| I-A-Q2-198 | 3-Cl | H | 5-Cl | NH₂ | CF₃ | Me | CH | CH | O | H | CH(CF₃)(CH₂iPr) | log P |
| I-A-Q2-199 | 3-Cl | H | 5-Cl | NH₂ | CF₃ | Me | CH | CH | O | CH₂-2-Pyr | CH₂-2-Pyr | log P |
| I-A-Q2-200 | 3-Cl | H | 5-Cl | NH₂ | CF₃ | Me | CH | CH | O | H | CH(CF₃)(Et) | log P |
| I-A-Q2-201 | 3-Cl | H | 5-Cl | NH₂ | CF₃ | Me | CH | CH | O | H | CH(CF₃)(Pr) | log P |
| I-A-Q2-202 | 3-Cl | H | 5-Cl | NH₂ | CF₃ | Me | CH | CH | O | H | Pr | log P |
| I-A-Q2-203 | 3-Cl | H | 5-Cl | NH₂ | CF₃ | Me | CH | CH | O | H | 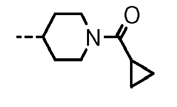 | log P |
| I-A-Q2-204 | 3-Cl | H | 5-Cl | NH₂ | CF₃ | Me | CH | CH | O | H | 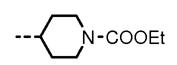 | log P |
| I-A-Q2-205 | 3-Cl | H | 5-Cl | NH₂ | CF₃ | Me | CH | CH | O | H | CH₂CH₂SMe | log P |
| I-A-Q2-206 | 3-Cl | H | 5-Cl | NH₂ | CF₃ | Me | CH | CH | O | H | 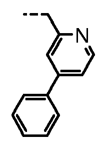 | log P |
| I-A-Q2-207 | 3-Cl | H | 5-Cl | NH₂ | CF₃ | Me | CH | CH | O | H | 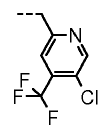 | log P |
| I-A-Q2-208 | 3-Cl | H | 5-Cl | NH₂ | CF₃ | Me | CH | CH | O | Me | CHMe-3-Pyr | log P |
| I-A-Q2-209 | 3-Cl | H | 5-Cl | NH₂ | CF₃ | Me | CH | CH | O | Me | CH₂-3-Pyr | log P |
| I-A-Q2-210 | 3-Cl | H | 5-Cl | NH₂ | CF₃ | Me | CH | CH | O | Me | CHMe-2-Pyr | log P |
| I-A-Q2-211 | 3-Cl | H | 5-Cl | NH₂ | CF₃ | Me | CH | CH | O | Me | CHMe-4-Pyr | log P |
| I-A-Q2-212 | 3-Cl | H | 5-Cl | NH₂ | CF₃ | Me | CH | CH | O | H | CH₂-4-Pyr | log P |
| I-A-Q2-213 | 3-Cl | H | 5-Cl | NH₂ | CF₃ | Me | CH | CH | O | H | CHMe-(4-Cl-Ph) | log P |
| I-A-Q2-214 | 3-Cl | H | 5-Cl | NH₂ | CF₃ | Me | CH | CH | O | H | CH₂-(3-F-Ph) | log P |
| I-A-Q2-215 | 3-Cl | H | 5-Cl | NH₂ | CF₃ | Me | CH | CH | O | H | CH₂-(3-Cl-Ph) | log P |
| I-A-Q2-216 | 3-Cl | H | 5-Cl | NH₂ | CF₃ | Me | CH | CH | O | H | CH₂-3-Pyr | log P |
| I-A-Q2-217 | 3-Cl | H | 5-Cl | NH₂ | CF₃ | Me | CH | CH | O | H | 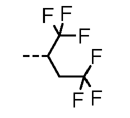 | log P |
| I-A-Q2-218 | 3-Cl | H | 5-Cl | NH₂ | CF₃ | Me | CH | CH | O | H | 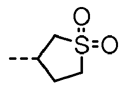 | log P |
| I-A-Q2-219 | 3-Cl | H | 5-Cl | NH₂ | CF₃ | Me | CH | CH | O | H | 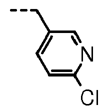 | log P |
| I-A-Q2-220 | 3-Cl | H | 5-Cl | NH₂ | CF₃ | Me | CH | CH | O | H | CH₂-Cypr | log P |
| I-A-Q2-221 | 3-Cl | H | 5-Cl | NH₂ | CF₃ | Me | CH | CH | O | H | tBu | log P |
| I-A-Q2-222 | 3-Cl | H | 5-Cl | NH₂ | CF₃ | Me | CH | CH | O | H | Cyhex | log P |
| I-A-Q2-223 | 3-Cl | H | 5-Cl | NH₂ | CF₃ | Me | CH | CH | O | H | Cypen | log P |
| I-A-Q2-224 | 3-Cl | H | 5-Cl | NH₂ | CF₃ | Me | CH | CH | O | H | CH₂CH(OMe)₂ | log P |
| I-A-Q2-225 | 3-Cl | H | 5-Cl | NH₂ | CF₃ | Me | CH | CH | O | H | CHEt₂ | log P |
| I-A-Q2-226 | 3-Cl | H | 5-Cl | NH₂ | CF₃ | Me | CH | CH | O | H | CH₂CH₂-2-Pyr | log P |
| I-A-Q2-227 | 3-Cl | H | 5-Cl | NH₂ | CF₃ | Me | CH | CH | O | H | CH₂-iPr | log P |
| I-A-Q2-228 | 3-Cl | H | 5-Cl | NH₂ | CF₃ | Me | CH | CH | O | H | Cypr | log P |
| I-A-Q2-229 | 3-Cl | H | 5-Cl | NH₂ | CF₃ | Me | CH | CH | O | H | iPr | log P |
| I-A-Q2-230 | 3-Cl | H | 5-Cl | NH₂ | CF₃ | Me | CH | CH | O | H | 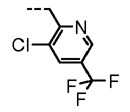 | log P |
| I-A-Q2-231 | 3-Cl | H | 5-Cl | NH₂ | CF₃ | Me | CH | CH | O | H | CHMe-3-Pyr | log P |
| I-A-Q2-232 | 3-Cl | H | 5-Cl | NH₂ | CF₃ | Me | CH | CH | O | H | 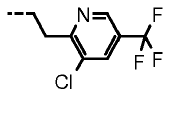 | log P |
| I-A-Q2-233 | 3-Cl | H | 5-Cl | NH₂ | CF₃ | Me | CH | CH | O | H | 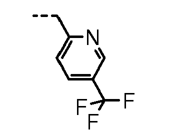 | log P |
| I-A-Q2-234 | 3-Cl | H | 5-Cl | NH₂ | CF₃ | Me | CH | CH | O | H | CHMe-4-Pyr | log P |
| I-A-Q2-235 | 3-Cl | H | 5-Cl | NH₂ | CF₃ | Me | CH | CH | O | H | 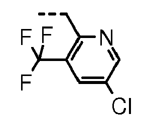 | log P |
| I-A-Q2-236 | 3-Cl | H | 5-Cl | NH₂ | CF₃ | Me | CH | CH | O | Me | CH₂CF₃ | log P |
| I-A-Q2-237 | 3-Cl | H | 5-Cl | NH₂ | CF₃ | Me | CH | CH | O | H | Pen | log P |
| I-A-Q2-238 | 3-Cl | H | 5-C1 | NH₂ | CF₃ | Me | CH | CH | O | H | CH₂CCH | log P |
| I-A-Q2-239 | 3-Cl | H | 5-Cl | NH₂ | CF₃ | Me | CH | CH | O | H | Cybu | log P |
| I-A-Q2-240 | 3-Cl | H | 5-Cl | NH₂ | CF₃ | Me | CH | CH | O | H | 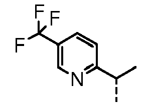 | log P |
| I-A-Q2-241 | 3-Cl | H | 5-Cl | NH₂ | CF₃ | Me | CH | CH | O | H | CH(CF₃)Ph | log P |
| I-A-Q2-242 | 3-Cl | H | 5-Cl | NH₂ | CF₃ | Me | CH | CH | O | H | CH(CF₃)(4-CF₃Ph) | log P |
| I-A-Q2-243 | 3-Cl | H | 5-Cl | NH₂ | CF₃ | Me | CH | CH | O | H | 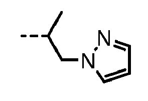 | log P |
| I-A-Q2-244 | 3-Cl | H | 5-Cl | NH₂ | CF₃ | Me | CH | CH | O | H | 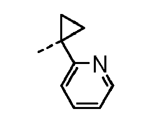 | log P |
| I-A-Q2-245 | 3-Cl | H | 5-Cl | NH₂ | CF₃ | Me | CH | CH | O | H | 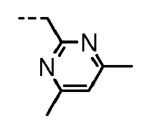 | log P |
| I-A-Q2-246 | 3-Cl | H | 5-Cl | NH₂ | CF₃ | Me | CH | CH | O | H | CH(CF₃)(4-NMe₂- | log P |
| | | | | | | | | | | | Ph) | |
| I-A-Q2-247 | 3-Cl | H | 5-Cl | NH₂ | CF₃ | Me | CH | CH | O | H | CHMe-(4-F-Ph) | log P |
| I-A-Q2-248 | 3-Cl | H | 5-Cl | NH₂ | CF₃ | Me | CH | CH | O | H | 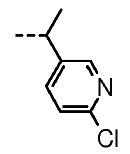 | log P |
| I-A-Q2-249 | 3-Cl | H | 5-Cl | NH₂ | CF₃ | Me | CH | CH | O | H | CH₂-(4-NO₂-Ph) | log P |
| I-A-Q2-250 | 3-Cl | H | 5-Cl | NH₂ | CF₃ | Me | CH | CH | O | H | 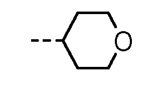 | log P |
| I-A-Q2-251 | 3-Cl | H | 5-Cl | NH₂ | CF₃ | Me | CH | CH | O | H | 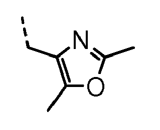 | log P |
| I-A-Q2-252 | 3-Cl | H | 5-Cl | NH₂ | CF₃ | Me | CH | CH | O | H | 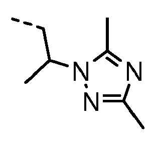 | log P |
| I-A-Q2-253 | 3-Cl | H | 5-Cl | NH₂ | CF₃ | Me | CH | CH | O | H | 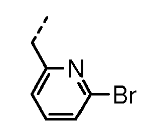 | log P |
| I-A-Q2-254 | 3-Cl | H | 5-Cl | NH₂ | CF₃ | Me | CH | CH | O | H | 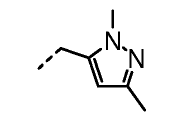 | log P |
| I-A-Q2-255 | 3-Cl | H | 5-Cl | NH₂ | CF₃ | Me | CH | CH | O | H | 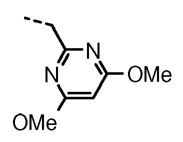 | log P |
| I-A-Q2-256 | 3-Cl | H | 5-Cl | NH₂ | CF₃ | Me | CH | CH | O | H | 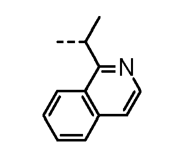 | log P |
| I-A-Q2-257 | 3-Cl | H | 5-Cl | NH₂ | CF₃ | Me | CH | CH | O | H | CH(CF₃)(3-CF₃-Ph) | log P |
| I-A-Q2-258 | 3-Cl | H | 5-Cl | NH₂ | CF₃ | Me | CH | CH | O | H | 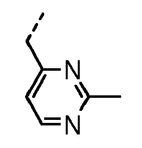 | log P |
| I-A-Q2-259 | 3-Cl | H | 5-Cl | NH₂ | CF₃ | Me | CH | CH | O | H | 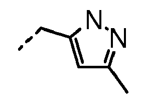 | log P |
| I-A-Q2-260 | 3-Cl | H | 5-Cl | NH₂ | CF₃ | Me | CH | CH | O | H | 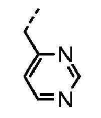 | log P |
| I-A-Q2-261 | 3-Cl | H | 5-Cl | NH₂ | CF₃ | Me | CH | CH | O | H | 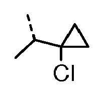 | log P |
| I-A-Q2-262 | 3-Cl | H | 5-Cl | NH₂ | CF₃ | Me | CH | CH | O | H | CH₂CHMe(OH) | log P |
| I-A-Q2-263 | 3-Cl | H | 5-Cl | NH₂ | CF₃ | Me | CH | CH | O | H | CH₂-Cyhex | log P |
| I-A-Q2-264 | 3-Cl | H | 5-Cl | NH₂ | CF₃ | Me | CH | CH | O | H | 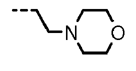 | log P |
| I-A-Q2-265 | 3-Cl | H | 5-Cl | NH₂ | CF₃ | Me | CH | CH | O | H | 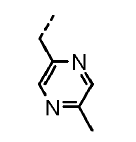 | log P |
| I-A-Q2-266 | 3-Cl | H | 5-Cl | NH₂ | CF₃ | Me | CH | CH | O | H | CH₂CF₂CF₂CF₃ | log P |
| I-A-Q2-267 | 3-Cl | H | 5-Cl | NH₂ | CF₃ | Me | CH | CH | O | H | CH₂(CF₂)₆CF₃ | log P |
| I-A-Q2-268 | 3-Cl | H | 5-Cl | NH₂ | CF₃ | Me | CH | CH | O | H | CH₂CF₂Br | log P |
| I-A-Q2-269 | 3-Cl | H | 5-Cl | NH₂ | CF₃ | Me | CH | CH | O | H | CH₂CF₂CF₃ | log P |
| I-A-Q2-270 | 3-Cl | H | 5-Cl | NH₂ | CF₃ | Me | CH | CH | O | H | Et | log P |
| I-A-Q2-271 | 3-Cl | H | 5-Cl | NH₂ | CF₃ | Me | CH | CH | O | H | CH₂Ph | log P |
| I-A-Q2-272 | 3-Cl | H | 5-Cl | NH₂ | CF₃ | Me | CH | CH | O | H | CH₂CH₂OH | log P |
| I-A-Q2-273 | 3-Cl | H | 5-Cl | NH₂ | CF₃ | Me | CH | CH | O | H | CH₂CH₂S(O)Me | log P |
| I-A-Q2-274 | 3-Cl | H | 5-Cl | NH₂ | CF₃ | Me | CH | CH | O | H | 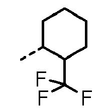 | log P |
| I-A-Q2-275 | 3-Cl | H | 5-Cl | I | CF₃ | Me | CH | CH | O | H | CH₂-2-Pyrm | log P |
| I-A-Q2-276 | 3-Cl | H | 5-Cl | SMe | CF₃ | Me | CH | CH | O | H | CH₂-2-Pyrm | log P |
| I-A-Q2-277 | 3-Cl | H | 5-Cl | Cl | CF₃ | Me | CH | CH | O | H | CH₂-2-Pyrm | log P |
| I-A-Q2-278 | 3-Cl | H | 5-Cl | NH₂ | CF₃ | Me | CH | CH | O | H | 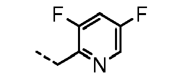 | log P |
| I-A-Q2-279 | 3-Cl | H | 5-Cl | NH₂ | CF₃ | Me | CH | CH | O | H | 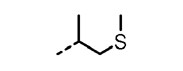 | log P |
| I-A-Q2-280 | 3-Cl | H | 5-Cl | NH₂ | CF₃ | Me | CH | CH | O | H | 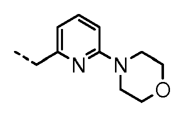 | log P |
| I-A-Q2-281 | 3-Cl | H | 5-Cl | NH₂ | CF₃ | Me | CH | CH | O | H | 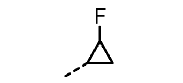 | log P |
| I-A-Q2-282 | 3-Cl | H | 5-Cl | SMe | CF₃ | Me | CH | CH | O | H | CH₂CF₂CF₂H | log P |
| I-A-Q2-283 | 3-Cl | H | 5-Cl | SOM e | CF₃ | Me | CH | CH | O | H | CH₂CF₂CF₂H | log P |
| I-A-Q2-284 | 3-Cl | H | 5-Cl | SOM e | CF₃ | Me | CH | CH | O | H | CH₂CF₂CF₂H | log P |
| I-A-Q2-285 | 3-Cl | H | 5-CF₃ | NH₂ | (3-BrC₆H 4)CF2 | Me | CH | CH | O | H | CH₂CF₂CF₂H | log P |
| I-A-Q2-286 | 3-Cl | H | 5-CF₃ | NH₂ | (3-CF₃C₆ H₄)CF₂ | Me | CH | CH | O | H | CH₂CF₂CF₂H | log P |
| I-A-Q2-287 | 3-Cl | H | 5-CF₃ | NH₂ | CF₃OC F₂CF₂ OCF₂ | Me | CH | CH | O | H | CH₂CF₂CF₂H | log P |
| I-A-Q2-288 | 3-Cl | H | 5-CF₃ | NH₂ | (3-MeOC ₆H₄)CF 2 | Me | CH | CH | O | H | CH₂CF₂CF₂H | log P |
| I-A-Q2-289 | 3-Cl | H | 5-CF₃ | NH₂ | (4-CF₃C₆ H₄)CF₂ | Me | CH | CH | O | H | CH₂CF₂CF₂H | log P |
| I-A-Q2-290 | 3-Cl | H | 5-CF₃ | NH₂ | 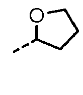 | Me | CH | CH | O | H | CH₂CF₂CF₂H | log P |
| I-A-Q2-291 | 3-Cl | H | 5-CF₃ | NH₂ | 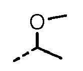 | Me | CH | CH | O | H | CH₂CF₂CF₂H | log P |
| I-A-Q2-292 | 3-Cl | 4-MeO | 5-MeO | NH₂ | CF₃ | Me | CH | CH | O | H | CH₂CF₂CF₂H | log P |
| I-A-Q2-293 | 3,4 | -OCH₂O- | 5-Cl | NH₂ | CF3 | Me | CH | CH | O | H | CH₂CF₂CF₂H | log P |
| I-A-Q2-294 | 3-Cl | H | 5-CF₃ | NH₂ | (4-Cl-C₆H₄) OCF₂ | Me | CH | CH | O | H | CH₂CF₂CF₂H | log P |
| I-A-Q2-295 | 3-Cl | H | 5-CF₃ | NH₂ | (2,4-Cl,Cl-C₆H₄) OCF₂ | Me | CH | CH | O | H | CH₂CF₂CF₂H | log P |
| I-A-Q2-296 | 3-Cl | H | 5-CF₃ | NH₂ | (2,5-Cl,Cl-C₆H₄) OCF₂ | Me | CH | CH | O | H | CH₂CF₂CF₂H | log P |
| I-A-Q2-297 | 3-Cl | H | 5-CF₃ | NH₂ | (3-Cl-C₆H₄) | Me | CH | CH | O | H | CH₂CF₂CF₂H | log P |
| | | | | | OCF₂ | | | | | | | |
| I-A-Q2-298 | 3-Cl | H | 5-CF₃ | NH₂ | (2-Cl-C₆H₄) OCF₂ | Me | CH | CH | O | H | CH₂CF₂CF₂H | log P |
| I-A-Q2-299 | 3-Cl | H | 5-CF₃ | NH₂ | PhOC F₂ | Me | CH | CH | O | H | CH₂CF₂CF₂H | log P |
| I-A-Q2-300 | 3-Cl | H | 5-CF₃ | NH₂ | (3,4-Cl,Cl-C₆H₄) OCF₂ | Me | CH | CH | O | H | CH₂CF₂CF₂H | log P |
| I-A-Q2-301 | 3-Cl | H | 5-F | NH₂ | CF₃ | Me | CH | CH | O | H | CH₂CF₂CF₂H | log P |
| I-A-Q2-302 | H | 4-Cl | H | NH₂ | CF₃ | Me | CH | CH | O | H | CH₂CF₂CF₂H | log P |
| I-A-Q2-303 | H | 4-Br | H | NH₂ | CF₃ | Me | CH | CH | O | H | CH₂CF₂CF₂H | log P |
| I-A-Q2-304 | 3-Cl | 4-Cl | H | NH₂ | CF₃ | Me | CH | CH | O | H | CH₂CF₂CF₂H | log P |
| I-A-Q2-305 | 2-Cl | H | 4-Cl | NH₂ | CF₃ | Me | CH | CH | O | H | CH₂CF₂CF₂H | log P |
| I-A-Q2-306 | H | 4-CN | H | NH₂ | CF₃ | Me | CH | CH | O | H | CH₂CF₂CF₂H | log P |
| I-A-Q2-307 | 2-F | 4-F | 6-F | NH₂ | CF₃ | Me | CH | CH | O | H | CH₂CF₂CF₂H | log P |
| I-A-Q2-308 | 2-Cl | H | H | NH₂ | CF₃ | Me | CH | CH | O | H | CH₂CF₂CF₂H | log P |
| I-A-Q2-309 | 3-Cl | 4-F | H | NH₂ | CF₃ | Me | CH | CH | O | H | CH₂CF₂CF₂H | log P |
| I-A-Q2-310 | 3-F | 4-Cl | H | NH₂ | CF₃ | Me | CH | CH | O | H | CH₂CF₂CF₂H | log P |
| I-A-Q2-311 | 3-F | H | H | NH₂ | CF₃ | Me | CH | CH | O | H | CH₂CF₂CF₂H | log P |
| I-A-Q2-312 | 3-F | 4-F | H | NH₂ | CF₃ | Me | CH | CH | O | H | CH₂CF₂CF₂H | log P |
| I-A-Q2-313 | H | 4-I | H | NH₂ | CF₃ | Me | CH | CH | O | H | CH₂CF₂CF₂H | log P |
| I-A-Q2-314 | H | 3-Br | H | NH₂ | CF₃ | Me | CH | CH | O | H | CH₂CF₂CF₂H | log P |
| I-A-Q2-315 | 3-F | H | 5-F | NH₂ | CF₃ | Me | CH | CH | O | H | CH₂CF₂CF₂H | log P |
| I-A-Q2-316 | 3-F | 4-F | 5-F | NH₂ | CF₃ | Me | CH | CH | O | H | CH₂CF₂CF₂H | log P |
| I-A-Q2-317 | 2-Me | H | H | NH₂ | CF₃ | Me | CH | CH | O | H | CH₂CF₂CF₂H | log P |
| I-A-Q2-318 | 3-CF₃ | H | 5-CF₃ | NH₂ | CF₃ | Me | CH | CH | O | H | CH₂CF₂CF₂H | log P |
| I-A-Q2-319 | 2-F | H | 4-F | NH₂ | CF₂H | Me | CH | CH | O | H | CH₂CF₂CF₂H | log P |
| I-A-Q2-320 | H | 4-Cl | H | NH₂ | CF₂H | Me | CH | CH | O | H | CH₂CF₂CF₂H | log P |
| I-A-Q2-321 | H | 4-Cl | H | NH₂ | CF₂CF 3 | Me | CH | CH | O | H | CH₂CF₂CF₂H | log P |
| I-A-Q2-322 | 3-Cl | H | 5-CF₃ | NH₂ | CF₂Cl | Me | CH | CH | O | H | CH₂CF₂CF₂H | log P |
| I-A-Q2-323 | 3-Cl | 4-Cl | H | NH₂ | Me | Me | CH | CH | O | H | CH₂CF₂CF₂H | log P |
| I-A-Q2-324 | H | 4-MeO | H | NH₂ | Me | Me | CH | CH | O | H | CH₂CF₂CF₂H | log P |
| I-A-Q2-325 | H | 4-Cl | H | NH₂ | Me | Me | CH | CH | O | H | CH₂CF₂CF₂H | log P |
| I-A-Q2-326 | H | 4-F | H | NH₂ | Me | Me | CH | CH | O | H | CH₂CF₂CF₂H | log P |
| I-A-Q2-327 | 2-Cl | 4-F | H | NH₂ | Me | Me | CH | CH | O | H | CH₂CF₂CF₂H | log P |
| I-A-Q2-328 | 2-F | H | 6-F | NH₂ | Me | Me | CH | CH | O | H | CH₂CF₂CF₂H | log P |
| I-A-Q2-329 | 2-F | 4-F | H | NH₂ | Me | Me | CH | CH | O | H | CH₂CF₂CF₂H | log P |
| I-A-Q2-330 | H | 4-Cl, | H | NH₂ | Me | Me | CH | CH | O | H | CH₂CF₂CF₂H | log P |
| I-A-Q2-331 | 3-Cl | H | H | NH₂ | Me | Me | CH | CH | O | H | CH₂CF₂CF₂H | log P |
| I-A-Q2-332 | 2-F | 5-F | H | NH₂ | Me | Me | CH | CH | O | H | CH₂CF₂CF₂H | log P |
| I-A-Q2-333 | 2-Cl | H | H | NH₂ | Me | Me | CH | CH | O | H | CH₂CF₂CF₂H | log P |
| I-A-Q2-334 | 2-Cl | H | 6-F | NH₂ | Me | Me | CH | CH | O | H | CH₂CF₂CF₂H | log P |
| I-A-Q2-335 | 2-Cl | H | 5-Cl | NH₂ | Me | Me | CH | CH | O | H | CH₂CF₂CF₂H | log P |
| I-A-Q2-336 | 2-Cl | H | 6-Cl | NH₂ | Me | Me | CH | CH | O | H | CH₂CF₂CF₂H | log P |
| I-A-Q2-337 | H | 4-Cl | H | NH₂ | Ph | Me | CH | CH | O | H | CH₂CF₂CF₂H | log P |
| I-A-Q2-338 | H | 4-Cl | H | NH₂ | iPr | Me | CH | CH | O | H | CH₂CF₂CF₂H | log P |
| I-A-Q2-339 | H | 4-Cl | H | NH₂ | Et | Me | CH | CH | O | H | CH₂CF₂CF₂H | log P |
| I-A-Q2-340 | H | 4-Cl | H | NH₂ | Pr | Me | CH | CH | O | H | CH₂CF₂CF₂H | log P |
| I-A-Q2-341 | H | 4-Cl | H | NH₂ | Cypr | Me | CH | CH | O | H | CH₂CF₂CF₂H | log P |
| I-A-Q2-342 | H | 4-Cl | H | NH₂ | iPrCH 2 | Me | CH | CH | O | H | CH₂CF₂CF₂H | log P |
| I-A-Q2-343 | 3-CF₃ | H | 5-CF₃ | NH₂ | CF₂CF 3 | Me | CH | CH | O | H | CH₂CF₂CF₂H | log P |
| I-A-Q2-344 | 3-F | H | 5-Cl | NH₂ | CF₃ | Me | CH | CH | O | H | CH₂-2-Pyr | log P |
| I-A-Q2-345 | H | 4-Cl | H | NH₂ | CF₃ | Me | CH | CH | O | H | CH₂-2-Pyr | log P |
| I-A-Q2-346 | H | 4-Br | H | NH₂ | CF₃ | Me | CH | CH | O | H | CH₂-2-Pyr | log P |
| I-A-Q2-347 | 3-Cl | 4-Cl | H | NH₂ | CF₃ | Me | CH | CH | O | H | CH₂-2-Pyr | log P |
| I-A-Q2-348 | 2-Cl | 4-Cl | H | NH₂ | CF₃ | Me | CH | CH | O | H | CH₂-2-Pyr | log P |
| I-A-Q2-349 | H | 4-CN | H | NH₂ | CF₃ | Me | CH | CH | O | H | CH₂-2-Pyr | log P |
| I-A-Q2-350 | 2-F | 4-F | 6-F | NH₂ | CF₃ | Me | CH | CH | O | H | CH₂-2-Pyr | log P |
| I-A-Q2-351 | 2-Cl | H | H | NH₂ | CF₃ | Me | CH | CH | O | H | CH₂-2-Pyr | log P |
| I-A-Q2-352 | 3-Cl | 4-F | H | NH₂ | CF₃ | Me | CH | CH | O | H | CH₂-2-Pyr | log P |
| I-A-Q2-353 | 3-F | 4-Cl | H | NH₂ | CF₃ | Me | CH | CH | O | H | CH₂-2-Pyr | log P |
| I-A-Q2-354 | 3-F | H | H | NH₂ | CF₃ | Me | CH | CH | O | H | CH₂-2-Pyr | log P |
| I-A-Q2-355 | 3-F | 4-F | H | NH₂ | CF₃ | Me | CH | CH | O | H | CH₂-2-Pyr | log P |
| I-A-Q2-356 | H | 4-I | H | NH₂ | CF₃ | Me | CH | CH | O | H | CH₂-2-Pyr | log P |
| I-A-Q2-357 | 3-Br | H | H | NH₂ | CF₃ | Me | CH | CH | O | H | CH₂-2-Pyr | log P |
| I-A-Q2-358 | 3-F | 4-F | 5-F | NH₂ | CF₃ | Me | CH | CH | O | H | CH₂-2-Pyr | log P |
| I-A-Q2-359 | 2-Me | H | H | NH₂ | CF₃ | Me | CH | CH | O | H | CH₂-2-Pyr | log P |
| I-A-Q2-360 | 3-CF₃ | H | 5-CF₃ | NH₂ | CF₃ | Me | CH | CH | O | H | CH₂-2-Pyr | log P |
| I-A-Q2-361 | 2-F | H | 4-F | NH₂ | CF₂H | Me | CH | CH | O | H | CH₂-2-Pyr | log P |
| I-A-Q2-362 | H | 4-Cl | H | NH₂ | CF₂H | Me | CH | CH | O | H | CH₂-2-Pyr | log P |
| I-A-Q2-363 | H | 4-Cl | H | NH₂ | CF₂CF 3 | Me | CH | CH | O | H | CH₂-2-Pyr | log P |
| I-A-Q2-364 | 3-Cl | H | 5-CF₃ | NH₂ | CF₂Cl | Me | CH | CH | O | H | CH₂-2-Pyr | log P |
| I-A-Q2-365 | 3-Cl | 4-Cl | H | NH₂ | Me | Me | CH | CH | O | H | CH₂-2-Pyr | log P |
| I-A-Q2-366 | H | 4-Cl | H | NH₂ | Me | Me | CH | CH | O | H | CH₂-2-Pyr | log P |
| I-A-Q2-367 | H | 4-F | H | NH₂ | Me | Me | CH | CH | O | H | CH₂-2-Pyr | log P |
| I-A-Q2-368 | 2-Cl | 4-F | H | NH₂ | Me | Me | CH | CH | O | H | CH₂-2-Pyr | log P |
| I-A-Q2-369 | 2-F | H | 6-F | NH₂ | Me | Me | CH | CH | O | H | CH₂-2-Pyr | log P |
| I-A-Q2-370 | 2-F | 4-F | H | NH₂ | Me | Me | CH | CH | O | H | CH₂-2-Pyr | log P |
| I-A-Q2-371 | 3-Cl | H | H | NH₂ | Me | Me | CH | CH | O | H | CH₂-2-Pyr | log P |
| I-A-Q2-372 | 2-F | H | 5-F | NH₂ | Me | Me | CH | CH | O | H | CH₂-2-Pyr | log P |
| I-A-Q2-373 | 2-Cl | H | H | NH₂ | Me | Me | CH | CH | O | H | CH₂-2-Pyr | log P |
| I-A-Q2-374 | 2-F | H | 6-Cl | NH₂ | Me | Me | CH | CH | O | H | CH₂-2-Pyr | log P |
| I-A-Q2-375 | 2-Cl | H | 5-Cl | NH₂ | Me | Me | CH | CH | O | H | CH₂-2-Pyr | log P |
| I-A-Q2-376 | 2-Cl | H | 6-Cl | NH₂ | Me | Me | CH | CH | O | H | CH₂-2-Pyr | log P |
| I-A-Q2-377 | H | 4-Cl | H | NH₂ | Ph | Me | CH | CH | O | H | CH₂-2-Pyr | log P |
| I-A-Q2-378 | H | 4-Cl | H | NH₂ | iPr | Me | CH | CH | O | H | CH₂-2-Pyr | log P |
| I-A-Q2-379 | H | 4-Cl | H | NH₂ | Et | Me | CH | CH | O | H | CH₂-2-Pyr | log P |
| I-A-Q2-380 | H | 4-Cl | H | NH₂ | Pr | Me | CH | CH | O | H | CH₂-2-Pyr | log P |
| I-A-Q2-381 | H | 4-Cl | H | NH₂ | Cypr | Me | CH | CH | O | H | CH₂-2-Pyr | log P |
| I-A-Q2-382 | H | 4-Cl | H | NH₂ | iPrCH₂ | Me | CH | CH | O | H | CH₂-2-Pyr | log P |
| I-A-Q2-383 | 3-CF₃ | H | 5-CF₃ | NH₂ | CF₂CF 3 | Me | CH | CH | O | H | CH₂-2-Pyr | log P |
| I-A-Q2-384 | 3-Cl | H | 5-Cl | NH₂ | CF₃ | Cl | CH | CH | O | H | CH₂CF₂CF₂H | log P |
| I-A-Q2-385 | 3-Cl | H | 5-Cl | NH₂ | CF₃ | CF₃ | CH | CH | O | H | CH₂CF₂CF₂H | log P |
| I-A-Q2-386 | 3-Cl | H | 5-Cl | NH₂ | CF₃ | CF₃ | CH | CH | O | H | CH₂-2-Pyr | log P |
| I-A-Q2-387 | 3-Cl | H | 5-Cl | NH₂ | CF₃ | Me | CF | CH | O | H | CH₂CF₂CF₂H | log P |
| I-A-Q2-388 | 3-Cl | H | 5-Cl | NH₂ | CF₃ | Me | CF | CH | O | H | CH₂-2-Pyr | log P |
| I-A-Q2-389 | 3-Cl | H | 5-Cl | NH₂ | CF₃ | Me | CH | CF | O | H | CH₂CF₂CF₂H | log P |
| I-A-Q2-390 | 3-Cl | H | 5-Cl | NH₂ | CF₃ | Me | CH | CF | O | H | CH₂-2-Pyr | log P |
| I-A-Q2-391 | 3-Cl | H | 5-Cl | NH₂ | CF₃ | Cl | CH | CH | O | H | CH₂-2-Pyr | log P |
| I-A-Q2-392 | 3-Cl | H | 5-Cl | NH₂ | CF₃ | Br | CH | CH | O | H | CH₂-2-Pyr | log P |
| I-A-Q2-393 | 3-Cl | H | 5-Cl | NH₂ | CF₃ | NMe₂ | CH | CH | O | H | CH₂CF₂CF₂H | log P |
| I-A-Q2-394 | 3-Cl | H | 5-Cl | NH₂ | CF₃ | NMe₂ | CH | CH | O | H | CH₂-2-Pyr | log P |
| I-A-Q2-395 | 3-Cl | H | 5-Cl | NH₂ | CF₃ | SMe | CH | CH | O | H | CH₂CF₂CF₂H | log P |
| I-A-Q2-396 | 3-Cl | H | 5-Cl | SMe | CF₃ | CF3 | CH | CH | O | H | CH₂CF₂CF₂H | log P |
| I-A-Q2-397 | 3-Cl | H | 5-Cl | NH₂ | CF₃ | NHM e | CH | CH | O | H | CH₂CF₂CF₂H | log P |
| I-A-Q2-398 | 3-Cl | H | 5-Cl | NH₂ | CF₃ | NHM e | CH | CH | O | H | CH₂-2-Pyr | log P |
| I-A-Q2-399 | 3-Cl | H | 5-Cl | Br | CF₃ | Me | CH | CH | O | H | CH₂CF₂CF₂H | log P |
| I-A-Q2-400 | 3-Cl | H | 5-Cl | 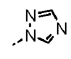 | CF₃ | Me | CH | CH | O | H | CH₂CF₂CF₂H | log P |
| I-A-Q2-401 | 3-Cl | H | 5-Cl | CN | CF₃ | Me | CH | CH | O | H | CH₂CF₂CF₂H | log P |
| I-A-Q2-402 | 3-Cl | H | 5-Cl | NH₂ | CF₃ | Me | CH | N | O | H | CH₂-2-Pyr | log P |
| I-A-Q2-403 | 3-Cl | H | 5-Cl | NH₂ | CF₃ | Me | CH | N | O | H | CHMe-2-Pyr | log P |
| I-A-Q2-404 | 3-Cl | H | 5-Cl | NH₂ | CF₃ | Me | CH | N | O | H | CH₂Cypr | log P |
| I-A-Q2-405 | 3-Cl | H | 5-Cl | NH₂ | CF₃ | Me | CH | N | O | H | CH₂CF₂CF₂H | log P |
| I-A-Q2-407 | 3-Cl | H | 5-Cl | H | CF₃ | SMe | CH | CH | O | H | CH₂CF₂CF₂H | log P |
| I-A-Q2-408 | 3-Cl | H | 5-Cl | NH₂ | tBu | SMe | CH | CH | O | H | CH₂-2-Pyr | log P |
| I-A-Q2-409 | 3-Cl | H | 5-Cl | NH₂ | CF₃ | SMe | CH | CH | O | H | CH₂-2-Pyr | log P |
| 1-A-Q2-410 | 2-Cl | 4-Cl | H | NH₂ | CF₃ | Me | CH | CH | O | H | CH₂Cypr | log P |
| I-A-Q2-411 | 2-Cl | 4-Cl | H | NH₂ | CF₃ | Me | CH | CH | O | H | CH₂-2-Pyrm | log P |
| I-A-Q2-412 | 2-Cl | 4-Cl | H | NH₂ | CF₃ | Me | CH | CH | O | H | CHMe-2-Pyr | log P |
| I-A-Q2-413 | 2-Cl | H | H | NH₂ | CF₃ | Me | CH | CH | O | H | CH₂Cypr | log P |
| I-A-Q2-414 | 2-Cl | H | H | NH₂ | CF₃ | Me | CH | CH | O | H | CH₂-2-Pyrm | log P |
| I-A-Q2-415 | 2-Cl | H | H | NH₂ | CF₃ | Me | CH | CH | O | H | CHMe-2-Pyr | log P |
| I-A-Q2-416 | 3-Br | H | H | NH₂ | CF₃ | Me | CH | CH | O | H | CH₂Cypr | log P |
| I-A-Q2-417 | 3-Br | H | H | NH₂ | CF₃ | Me | CH | CH | O | H | CH₂-2-Pyrm | log P |
| I-A-Q2-418 | 3-Br | H | H | NH₂ | CF₃ | Me | CH | CH | O | H | CHMe-2-Pyr | log P |
| I-A-Q2-419 | 2-Br | H | H | NH₂ | CF₃ | Me | CH | CH | O | H | CH₂-2-Pyrm | log P |
| I-A-Q2-420 | 2-Br | H | H | NH₂ | CF₃ | Me | CH | CH | O | H | CHMe-2-Pyr | log P |
| I-A-Q2-421 | 2-Br | H | H | NH₂ | CF₃ | Me | CH | CH | O | H | CH₂-2-Pyr | log P |
| I-A-Q2-422 | 2-Br | H | H | NH₂ | CF₃ | Me | CH | CH | O | H | CH₂Cypr | log P |
| I-A-Q2-423 | 2-Br | H | H | NH₂ | CF₃ | Me | CH | CH | O | H | CH₂CF₂CF₂H | log P |
| I-A-Q2-424 | 3-Cl | H | 5-Cl | NH₂ | CF₃ | S(O) Me | CH | CH | O | H | CH₂-2-Pyr | log P |
| I-A-Q2-425 | 3-Cl | H | 5-Cl | NH₂ | CF₃ | S(O)₂ | CH | CH | O | H | CH₂-2-Pyr | log P |
| | | | | | | Me | | | | | | |

### Charakteristische Daten für Synthesebeispiele:

| | | | | | |
|---|---|---|---|---|---|
| **I-A-Q2-001:** | log P (pH2): 4,02 | | | | |
| | | | | | |
| | log P (pH7,5): 4,74 | | | | |
| | | | | | |
| | 1H NMR (CDCl3): 4,78 (d, 2 H, J = 4,6 Hz), 7,22-7,42 (m, 6 H), 7,72-7,78 (m, 2 H), 8,00-8,02 (m, 1 H), 8,11-8,13 (m, 2 H), 8,53 (d, 1 H, J = 4,8 Hz) | | | | |
| | | | | | |
| **I-A-Q2-002:** | spektroskopische Daten siehe Protokoll unter allgemeinen Synthesevorschriften | | | | |
| | | | | | |
| **I-A-Q2-003:** | 1H NMR (CDCl3): 4,81 (d, 2 H, J = 4,6 Hz), 7,28-7,39 (m, 7 H), 7,73-7,78 (m, 2 H), 8,11-8,17 (m, 2 H), 8,45-8,54 (m, 2 H) | | | | |
| | | | | | |
| **I-A-Q2-004:** | 1H NMR (CDCl3): 3,86-4,16 (m, 2 H), 7,34-7,42 (m, 3 H), 7,86-8,02 (m, 3 H), 8,15-8,17 (m, 1 H) | | | | |
| | | | | | |
| **I-A-Q2-005:** | 1H NMR (CDCl3): 1,43 (d, 3 H, J = 3,5 Hz), 4,86-4,91 (m, 1 H), 6,13-6,20 (m, 1 H), 7,34-7,40 (m, 3 H), 7,59-7,67 (m, 1 H), 7,86-8,15 (m, 3 H) | | | | |
| | | | | | |
| **I-A-Q2-006:** | 1H NMR (CDCl3): 1,45 (d, 3 H, J = 7,1 Hz), 4,88-4,93 (m, 1 H), 7,36 (t, 1 H, J = 1,8 Hz), 7,74-7,76 (m, 3 H), 8,23-8,29 (m, 4 H), 9,17 (s, 1 H) | | | | |
| | | | | | |
| **I-A-Q2-008:** | 1H NMR (CDCl3): 4,79 (d, 2 H, J = 4,9 Hz), 7,43-7,77 (m, 9 H), 7,95-8,54 (m, 4 H) | | | | |
| | | | | | |
| **I-A-Q2-009:** | 1H NMR (CDCl3): H), 8,27-8,31 4,77 (d, 2 H, J = 4,8 (m, 1 H), 8,52 (d, 1 H, J = Hz), 7,22-7,42 (m, 3 H), 4,6 Hz) 7,72-7,78 (m, 2 H), 7,93-7,95 (m, 3 H), 8,03-8,05 (m, 1 H), 8,14-8,14 (m, 1 | | | | |
| **I-A-Q2-010:** | 1H NMR (CDCl3): 4,10-4,14 (m, 2 H), 6,33-6,35 (m, 1 H), 7,71 (d, 1 H, J = 8,4 Hz), 7,92-7,95 (m, 3 H), 8,03-8,05 (m, 1 H), 8,14-8,14 (m, 1 H), 8,27 (s, 1 H) | | | | |
| | | | | | |
| **I-A-Q2-011:** | log P (HCOOH): 4,54 | | | | |
| | | | | | |
| | 1H NMR (DMSO-d6): 8,77 (d, 1 H, J = 8.8 Hz), 7,95 (s, 1 H), 7,53 (t, 1 H, J = 2,0 Hz), 7,52-7,49 (m, 2 H), 7,34 (d, 2 H, J = 2,0 Hz), 5,58 (s, 2 H), 4,84-4,78 (m, 1 H), 2,42 (s, 3 H), 1,34 (d, 3 H, J = 7,2 Hz) | | | | |
| | | | | | |
| **I-A-Q2-012:** | spektroskopische Daten siehe Protokoll unter allgemeinen Synthesevorschriften | | | | |
| | | | | | |
| **I-A-Q2-013:** | log P (HCOOH): 3,59 | | | | |
| | | | | | |
| **I-A-Q2-014:** | log P (HCOOH): 4,34 | | | | |
| | | | | | |
| | 1H NMR (DMSO-d6): 8,86 (t, 1 H, J = 6,4 Hz), 7,54-7,49 (m, 4 H), 7,34 (d, 2 H, J = 2,0 Hz), 5,59 (s, 2 H), 4,12-4,04 (m, 1 H), 2,43 (s, 3 H) | | | | |
| | | | | | |
| **I-A-Q2-015:** | log P (HCOOH): 3,70 | | | | |
| | | | | | |
| | 1H NMR (DMSO-d6): 8,55-8,52 (m, 2 H), 7,77 (td, 1 H, J = 7,6 und 2,0 Hz), 7,54 (d, 1 H, J = 8,8 Hz), 7,53 (t, 1 H, J = 2,0 Hz), 7,49-7,47 (m, 2 H), 7,46 (d, 1 H, J = 7,6 Hz), 7,34 (d, 2 H, J = 2,0 Hz), 7,25 (dd, 1 H, J = 7,6 und 4,8 Hz), 5,58 (s, 2 H), 5,22-5,12 (m, 1 H), 2,41 (s, 3 H), 1,51 (d, 3 H, J = 6,8 Hz) | | | | |
| | | | | | |
| **I-A-Q2-016:** | log P (HCOOH): 4,79 | | | | |
| | | | | | |
| | 1H NMR 1,48 (d, 3 H, (DMSO-d6): 8.59 (d, 1 J = 6,8 Hz) H, J = 7,6 Hz), 7,53 (t, 1 H, J = 2,0 Hz), 7,49-7,21 (m, 10 H), 5,54 (s, 2 H), 5,19-5,14 (m, 1 H), 2,38 (s, 3 H), | | | | |
| **I-A-Q2-017:** | log P (HCOOH): 4,04 | | | | |
| | | | | | |
| | ¹H NMR (DMSO-d6): 8,55 (t, 1 H, J = 5,8 Hz), 7,53 (t, 1 H, J = 2,0 Hz), 7,53-7,47 (m, 3 H), 7,34 (d, 2 H, J = 2,0 Hz), 6,12 (tt, 1 H, J = 56,0 und 3,8 Hz), 5,59 (s, 2 H), 3,73-3,63 (m, 2 H), 2,43 (s, 3 H) | | | | |
| | | | | | |
| **I-A-Q2-018:** | log P (HCOOH): 3,56 | | | | |
| | | | | | |
| | ¹H NMR (DMSO-d6): 8,77 (d, 2 H, J = 5,2 Hz), 8,59 (t, 1 H, J = 5,8 Hz), 7,62 (d, 1 H, J = 8,8 Hz), 7,53 (t, 1 H, J = 2,0 Hz), 7,52-7,48 (m, 2 H), 7,40 (t, 1 H, J = 4,8 Hz), 7,35 (d, 2 H, J = 1,6 Hz), 5,59 (s, 2 H), 4,66 (d, 2 H, J = 6,0 Hz), Me unter DMSO-Signal | | | | |
| | | | | | |
| **I-A-Q2-019:** | log P (HCOOH): 4,57 | | | | |
| | | | | | |
| | ¹H NMR (DMSO-d6): 1.36 (d, 3 H); 2.42 (s, 3 H); 4.81 (m, 1 H); 5.51 (br. s, 2 H); 7.32 (dd, 1 H); 7.47-7.57 (m, 4 H); 7.67 (d, 1 H); 7.70 (br. d, 1 H) | | | | |
| | | | | | |
| **I-A-Q2-020:** | spektroskopische Daten siehe Protokoll unter allgemeinen Synthesevorschriften | | | | |
| | | | | | |
| **I-A-Q2-021:** | log P (HCOOH): 3,48 | | | | |
| | | | | | |
| | ¹H NMR (DMSO-d6): 8,85 (d, 1 H, J = 8,8 Hz), 8,69 (s, 1 H), 8,51 (d, 1 H, J = 4,8 Hz), 7,75 (td, 1 H, J = 7,6 und 1,6 Hz), 7,54 (s, 1 H), 7,40 (d, 1 H, J = 7,6 Hz), 7,31 (d, 2 H, J = 2,0 Hz), 7,25 (dd, 1 H, J = 7,6 und 5,6 Hz), 5,14-5,09 (dd, 1 H), 1,45 (d, 3 H, J = 6,8 Hz) | | | | |
| | | | | | |
| **I-A-Q2-022:** | spektroskopische Daten siehe Protokoll unter allgemeinen Synthesevorschriften | | | | |
| | | | | | |
| **I-A-Q2-023:** | log P (HCOOH): 4,91 | | | | |
| | 1H NMR (DMSO-d6): 8,85 (t, 1 H, J = 5,6 Hz), 8,52 (d, 1 H, J = 4,0 Hz), 7,79 (td, 1 H, J = 7,6 und 1,6 Hz), 7,72 (t, 1 H, J = 1,8 Hz), 7,64 (d, 1 H, J = 8,4 Hz), 7,58-7,53 (m, 2 H), 7,50 (d, 2 H, J = 2,0 Hz), 7,42 (d, 1 H, J = 8,0 Hz), 7,28 (dd, 1 H, J = 6,8 und 5,6 Hz), 4,58 (d, 2 H, J = 6,0 Hz), 2,47 (s, 3 H) | | | | |
| | | | | | |
| **I-A-Q2-024:** | spektroskopische Daten siehe Protokoll unter allgemeinen Synthesevorschriften | | | | |
| | | | | | |
| **I-A-Q2-025:** | spektroskopische Daten siehe Protokoll unter allgemeinen Synthesevorschriften | | | | |
| | | | | | |
| **I-A-Q2-026:** | log P (HCOOH): 5,44 | | | | |
| | | | | | |
| | 1H NMR (DMSO-d6): 8.90 (d, 1 H, J = 8,8 Hz), 7,71 (t, 1 H, J = 2,0 Hz), 7,54-7,52 (m, 3 H), 7,45 (d, 2 H, J = 2,0 Hz), 4,85-4,79 (m, 1 H), 2,43 (s, 3 H), 1,34 (d, 3 H, J = 7,2 Hz) | | | | |
| | | | | | |
| **I-A-Q2-027:** | spektroskopische Daten siehe Protokoll unter allgemeinen Synthesevorschriften | | | | |
| | | | | | |
| **I-A-Q2-028:** | log P (HCOOH): 4,35 | | | | |
| | | | | | |
| | 1H NMR (DMSO-d6): 8,83 (t, 1 H, J = 5,6 Hz), 8,52 (d, 1 H, J = 4,0 Hz), 7,78 (td, 1 H, J = 7,6 und 2,0 Hz), 7,69 (t, 1 H, J = 2,0 Hz), 7,63 (d, 1 H, J = 8,0 Hz), 7,58-7,55 (m, 2 H), 7,50 (d, 2 H, J = 2,0 Hz), 7,41 (d, 1 H, J = 8,0 Hz), 7,27 (dd, 1 H, J = 6,8 und 5,2 Hz), 4,58 (d, 2 H, J = 5,6 Hz), 2,47 (s, 3 H), 2,04 (s, 3 H) | | | | |
| | | | | | |
| **I-A-Q2-029:** | log P (HCOOH): 3,24 | **I-A-Q2-030:** | log P (HCOOH): 3,68 | **I-A-Q2-031:** | log P (HCOOH): 3,33 |
| | | | | | |
| **I-A-Q2-032:** | log P (HCOOH): 3,63 | **I-A-Q2-033:** | log P (HCOOH): 4,51 | **I-A-Q2-034:** | log P (HCOOH): 1,94 |
| | | | | | |
| **I-A-Q2-035:** | log P (HCOOH): 4,23 | **I-A-Q2-036:** | log P (HCOOH): 3,68 | **I-A-Q2-037:** | log P (HCOOH): 2,42 |
| **I-A-Q2-038:** | log P (HCOOH): 3,11 | **I-A-Q2-039:** | log P (HCOOH): 3,94 | **I-A-Q2-040:** | log P (HCOOH): 4,57 |
| | | | | | |
| **I-A-Q2-041:** | log P (HCOOH): 3,94 | **I-A-Q2-042:** | log P (HCOOH): 2,93 | **I-A-Q2-043:** | log P (HCOOH): 5,65 |
| | | | | | |
| **I-A-Q2-044:** | log P (HCOOH): 3,42 | **I-A-Q2-045:** | log P (HCOOH): 5,85 | **I-A-Q2-046:** | log P (HCOOH): 4,51 |
| | | | | | |
| **I-A-Q2-047:** | log P (HCOOH): 4,94 | **I-A-Q2-048:** | log P (HCOOH): 4,70 | **I-A-Q2-049:** | log P (HCOOH): 4,28 |
| | | | | | |
| **I-A-Q2-050:** | log P (HCOOH): 4,64 | **I-A-Q2-051:** | log P (HCOOH): 3,06 | **I-A-Q2-052:** | log P (HCOOH): 2,34 |
| | | | | | |
| **I-A-Q2-053:** | log P (HCOOH): 2,04 | **I-A-Q2-054:** | log P (HCOOH): 2,31 | **I-A-Q2-055:** | log P (HCOOH): 5,79 |
| | | | | | |
| **I-A-Q2-056:** | log P (HCOOH): 3,93 | **I-A-Q2-057:** | log P (HCOOH): 2,72 | **I-A-Q2-058:** | log P (HCOOH): 1,94 |
| | | | | | |
| **I-A-Q2-059:** | log P (HCOOH): 3,63 | **I-A-Q2-060:** | log P (HCOOH): 4,34 | **I-A-Q2-061:** | log P (HCOOH): 4,45 |
| | | | | | |
| **I-A-Q2-062:** | log P (HCOOH): 3,94 | **I-A-Q2-063:** | log P (HCOOH): 3,11 | **I-A-Q2-064:** | log P (HCOOH): 6,44 |
| | | | | | |
| **I-A-Q2-065:** | log P (HCOOH): 2,72 | **I-A-Q2-066:** | log P (HCOOH): 5,15 | **I-A-Q2-067:** | log P (HCOOH): 4,65 |
| | | | | | |
| **I-A-Q2-068:** | log P (HCOOH): 5,35 | **I-A-Q2-069:** | log P (HCOOH): 4,83 | **I-A-Q2-070:** | log P (HCOOH): 5,25 |
| | | | | | |
| **I-A-Q2-071:** | log P (HCOOH): 4,95 | **I-A-Q2-072:** | log P (HCOOH): 5,22 | **I-A-Q2-073:** | log P (HCOOH): 4,35 |
| | | | | | |
| **I-A-Q2-074:** | log P (HCOOH): 2,76 | **I-A-Q2-075:** | log P (HCOOH): 3,94 | **I-A-Q2-076:** | log P (HCOOH): 2,89 |
| | | | | | |
| **I-A-Q2-077:** | log P (HCOOH): 4,17 | **I-A-Q2-078:** | log P (HCOOH): 2,94 | **I-A-Q2-079:** | log P (HCOOH): 3,37 |
| **I-A-Q2-080:** | log P (HCOOH): 2,38 | **I-A-Q2-081:** | log P (HCOOH): 3,58 | **I-A-Q2-082:** | log P (HCOOH): 2,46 |
| | | | | | |
| **I-A-Q2-083:** | log P (HCOOH): 3,68 | **I-A-Q2-084:** | log P (HCOOH): 3,89 | **I-A-Q2-085:** | log P (HCOOH): 3,47 |
| | | | | | |
| **I-A-Q2-086:** | log P (HCOOH): 3,68 | **I-A-Q2-087:** | log P (HCOOH): 2,80 | **I-A-Q2-088:** | log P (HCOOH): 2,99 |
| | | | | | |
| **I-A-Q2-089:** | log P (HCOOH): 3,89 | **I-A-Q2-090:** | log P (HCOOH): 4,11 | **I-A-Q2-091:** | log P (HCOOH): 3,09 |
| | | | | | |
| **I-A-Q2-092:** | log P (HCOOH): 3,27 | **I-A-Q2-093:** | log P (HCOOH): 4,00 | **I-A-Q2-094:** | log P (HCOOH): 2,76 |
| | | | | | |
| **I-A-Q2-095:** | log P (HCOOH): 4,51 | **I-A-Q2-096:** | log P (HCOOH): 5,35 | **I-A-Q2-097:** | log P (HCOOH): 4,64 |
| | | | | | |
| **I-A-Q2-098:** | log P (HCOOH): 4,36 | **I-A-Q2-099:** | log P (HCOOH): 5,42 | **I-A-Q2-100:** | log P (HCOOH): 4,96 |
| | | | | | |
| **I-A-Q2-101:** | log P (HCOOH): 4,71 | **I-A-Q2-102:** | log P (HCOOH): 4,06 | **I-A-Q2-103:** | log P (HCOOH): 5,19 |
| | | | | | |
| **I-A-Q2-104:** | log P (HCOOH): 4,46 | **I-A-Q2-105:** | log P (HCOOH): 3,61 | **I-A-Q2-106:** | log P (HCOOH): 4,30 |
| | | | | | |
| **I-A-Q2-107:** | log P (HCOOH): 2,95 | **I-A-Q2-108:** | log P (HCOOH): 3,80 | **I-A-Q2-109:** | log P (HCOOH): 4,35 |
| | | | | | |
| **I-A-Q2-110:** | log P (HCOOH): 2,69 | **I-A-Q2-111:** | log P (HCOOH): 4,17 | **I-A-Q2-112:** | log P (HCOOH): 4,34 |
| | | | | | |
| **I-A-Q2-133:** | log P (HCOOH): 4,44 | **I-A-Q2-134:** | log P (HCOOH): 3,11 | **I-A-Q2-135:** | log P (HCOOH): 4,06 |
| | | | | | |
| **I-A-Q2-136:** | log P (HCOOH): 3,80 | **I-A-Q2-137:** | log P (HCOOH): 3,40 | **I-A-Q2-138:** | log P (HCOOH): 4,21 |
| | | | | | |
| **I-A-Q2-139:** | log P (HCOOH): 4,25 | **I-A-Q2-140:** | log P (HCOOH): 3,93 | **I-A-Q2-141:** | log P (HCOOH): 4,21 |
| **I-A-Q2-142:** | log P (HCOOH): 3,77 | **I-A-Q2-143:** | log P (HCOOH): 2,59 | **I-A-Q2-144:** | log P (HCOOH): 3,93 |
| | | | | | |
| **I-A-Q2-145:** | log P (HCOOH): 4,34 | **I-A-Q2-146:** | log P (HCOOH): 4,54 | **I-A-Q2-147:** | log P (HCOOH): 3,47 |
| | | | | | |
| **I-A-Q2-148:** | log P (HCOOH): 4,12 | **I-A-Q2-149:** | log P (HCOOH): 4,15 | **I-A-Q2-150:** | log P (HCOOH): 4,65 |
| | | | | | |
| **I-A-Q2-151:** | log P (HCOOH): 4,06 | **I-A-Q2-152:** | log P (HCOOH): 4,45 | **I-A-Q2-153:** | log P (HCOOH): 3,89 |
| | | | | | |
| **I-A-Q2-154:** | log P (HCOOH): 3,75 | **I-A-Q2-155:** | log P (HCOOH): 4,11 | **I-A-Q2-156:** | log P (HCOOH): 3,89 |
| | | | | | |
| **I-A-Q2-157:** | log P (HCOOH): 3,51 | **I-A-Q2-158:** | log P (HCOOH): 2,85 | **I-A-Q2-159:** | log P (HCOOH): 3,65 |
| | | | | | |
| **I-A-Q2-160:** | log P (HCOOH): 3,23 | **I-A-Q2-161:** | log P (HCOOH): 3,37 | **I-A-Q2-162:** | log P (HCOOH): 3,51 |
| | | | | | |
| **I-A-Q2-163:** | log P (HCOOH): 3,13 | **I-A-Q2-164:** | log P (HCOOH): 3,27 | **I-A-Q2-165:** | log P (HCOOH): 2,72 |
| | | | | | |
| **I-A-Q2-166:** | log P (HCOOH): 3,84 | **I-A-Q2-167:** | log P (HCOOH): 3,19 | **I-A-Q2-168:** | log P (HCOOH): 2,61 |
| | | | | | |
| **I-A-Q2-169:** | log P (HCOOH): 3,78 | **I-A-Q2-170:** | log P (HCOOH): 3,15 | **I-A-Q2-171:** | log P (HCOOH): 2,53 |
| | | | | | |
| **I-A-Q2-172:** | log P (HCOOH): 3,73 | **I-A-Q2-173:** | log P (HCOOH): 3,11 | **I-A-Q2-174:** | log P (HCOOH): 2,50 |
| | | | | | |
| **I-A-Q2-175:** | log P (HCOOH): 3,47 | **I-A-Q2-176:** | log P (HCOOH): 2,89 | **I-A-Q2-177:** | log P (HCOOH): 2,27 |
| | | | | | |
| **I-A-Q2-178:** | log P (HCOOH): 3,89 | **I-A-Q2-179:** | log P (HCOOH): 3,24 | **I-A-Q2-180:** | log P (HCOOH): 2,61 |
| | | | | | |
| **I-A-Q2-181:** | log P (HCOOH): 2,98 | **I-A-Q2-182:** | log P (HCOOH): 3,33 | **I-A-Q2-183:** | log P (HCOOH): 3,11 |
| **I-A-Q2-184:** | log P (HCOOH): 2,76 | **I-A-Q2-185:** | log P (HCOOH): 2,14 | **I-A-Q2-186:** | log P (HCOOH): 2,42 |
| | | | | | |
| **I-A-Q2-187:** | log P (HCOOH): 3,42 | **I-A-Q2-188:** | log P (HCOOH): 3,78 | **I-A-Q2-189:** | log P (HCOOH): 3,53 |
| | | | | | |
| **I-A-Q2-190:** | log P (HCOOH): 3,15 | **I-A-Q2-191:** | log P (HCOOH): 2,57 | **I-A-Q2-192:** | log P (HCOOH): 2,89 |
| | | | | | |
| **I-A-Q2-193:** | log P (HCOOH): 3,42 | **I-A-Q2-194:** | log P (HCOOH): 3,37 | **I-A-Q2-195:** | log P (HCOOH): 2,96 |
| | | | | | |
| **I-A-Q2-196:** | log P (HCOOH): 2,84 | **I-A-Q2-197:** | log P (HCOOH): 2,76 | **I-A-Q2-198:** | log P (HCOOH): 5,40 |
| | | | | | |
| **I-A-Q2-199:** | log P (HCOOH): 3,48 | **I-A-Q2-200:** | log P (HCOOH): 4,82 | **I-A-Q2-201:** | log P (HCOOH): 5,11 |
| | | | | | |
| **I-A-Q2-202:** | log P (HCOOH): 4,22 | **I-A-Q2-203:** | log P (HCOOH): 3,77 | **I-A-Q2-204:** | log P (HCOOH): 4,22 |
| | | | | | |
| **I-A-Q2-205:** | log P (HCOOH): 4,17 | **I-A-Q2-206:** | log P (HCOOH): 4,29 | **I-A-Q2-207:** | log P (HCOOH): 4,83 |
| | | | | | |
| **I-A-Q2-208:** | log P (HCOOH): 3,19 | **I-A-Q2-209:** | log P (HCOOH): 3,02 | **I-A-Q2-210:** | log P (HCOOH): 4,28 |
| | | | | | |
| **I-A-Q2-211:** | log P (HCOOH): 2,89 | **I-A-Q2-212:** | log P (HCOOH): 2,27 | **I-A-Q2-213:** | log P (HCOOH): 4,89 |
| | | | | | |
| **I-A-Q2-214:** | log P (HCOOH): 4,45 | **I-A-Q2-215:** | log P (HCOOH): 4,76 | **I-A-Q2-216:** | log P (HCOOH): 2,57 |
| | | | | | |
| **I-A-Q2-217:** | log P (HCOOH): 4,70 | **I-A-Q2-218:** | log P (HCOOH): 3,33 | **I-A-Q2-219:** | log P (HCOOH): 4,05 |
| | | | | | |
| **I-A-Q2-220:** | log P (HCOOH): 4,11 | **I-A-Q2-221:** | log P (HCOOH): 4,51 | **I-A-Q2-222:** | log P (HCOOH): 4,76 |
| | | | | | |
| **I-A-Q2-223:** | log P (HCOOH): 4,45 | **I-A-Q2-224:** | log P (HCOOH): 3,78 | **I-A-Q2-225:** | log P (HCOOH): 4,57 |
| **I-A-Q2-226:** | log P (HCOOH): 2,50 | **I-A-Q2-227:** | log P (HCOOH): 4,40 | **I-A-Q2-228:** | log P (HCOOH): 3,78 |
| | | | | | |
| **I-A-Q2-229:** | log P (HCOOH): 4,05 | **I-A-Q2-230:** | log P (HCOOH): 4,89 | **I-A-Q2-231:** | log P (HCOOH): 2,69 |
| | | | | | |
| **I-A-Q2-232:** | log P (HCOOH): 4,95 | **I-A-Q2-233:** | log P (HCOOH): 4,40 | **I-A-Q2-234:** | log P (HCOOH): 2,38 |
| | | | | | |
| **I-A-Q2-235:** | log P (HCOOH): 4,95 | **I-A-Q2-236:** | log P (HCOOH): 4,51 | **I-A-Q2-237:** | log P (HCOOH): 4,76 |
| | | | | | |
| **I-A-Q2-238:** | log P (HCOOH): 3,78 | **I-A-Q2-239:** | log P (HCOOH): 4,17 | **I-A-Q2-240:** | log P (HCOOH): 4,70 |
| | | | | | |
| **I-A-Q2-241:** | log P (HCOOH): 4,89 | **I-A-Q2-242:** | log P (HCOOH): 5,29 | **I-A-Q2-243:** | log P (HCOOH): 3,78 |
| | | | | | |
| **I-A-Q2-244:** | log P (HCOOH): 3,68 | **I-A-Q2-245:** | log P (HCOOH): 3,89 | **I-A-Q2-246:** | log P (HCOOH): 5,02 |
| | | | | | |
| **I-A-Q2-247:** | log P (HCOOH): 4,57 | **I-A-Q2-248:** | log P (HCOOH): 4,34 | **I-A-Q2-249:** | log P (HCOOH): 4,28 |
| | | | | | |
| **I-A-Q2-250:** | log P (HCOOH): 3,68 | **I-A-Q2-251:** | log P (HCOOH): 3,84 | **I-A-Q2-252:** | log P (HCOOH): 3,02 |
| | | | | | |
| **I-A-Q2-253:** | log P (HCOOH): 4,40 | **I-A-Q2-254:** | log P (HCOOH): 3,58 | **I-A-Q2-255:** | log P (HCOOH): 4,40 |
| | | | | | |
| **I-A-Q2-256:** | log P (HCOOH): 4,64 | **I-A-Q2-257:** | log P (HCOOH): 5,22 | **I-A-Q2-258:** | log P (HCOOH): 3,47 |
| | | | | | |
| **I-A-Q2-259:** | log P (HCOOH): 3,33 | **I-A-Q2-260:** | log P (HCOOH): 3,33 | **I-A-Q2-261:** | log P (HCOOH): 4,76 |
| | | | | | |
| **I-A-Q2-262:** | log P (HCOOH): 3,28 | **I-A-Q2-263:** | log P (HCOOH): 5,08 | **I-A-Q2-264:** | log P (HCOOH): 1,99 |
| | | | | | |
| **I-A-Q2-265:** | log P (HCOOH): 3,63 | **I-A-Q2-266:** | log P (HCOOH): 4,89 | **I-A-Q2-267:** | log P (HCOOH): 6,14 |
| **I-A-Q2-268:** | log P (HCOOH): 4,45 | **I-A-Q2-269:** | log P (HCOOH): 4,57 | **I-A-Q2-270:** | log P (HCOOH): 3,84 |
| | | | | | |
| **I-A-Q2-271:** | log P (HCOOH): 4,45 | **I-A-Q2-272:** | log P (HCOOH): 3,24 | **I-A-Q2-273:** | log P (HCOOH): 2,98 |
| | | | | | |
| **I-A-Q2-274:** | log P (HCOOH): 4,95 | **I-A-Q2-275:** | log P (HCOOH): 4,47 | **I-A-Q2-276:** | log P (HCOOH): 4,50 |
| | | | | | |
| **I-A-Q2-277:** | log P (HCOOH): 4,56 | **I-A-Q2-278:** | log P (HCOOH): 4,30 | **I-A-Q2-279:** | log P (HCOOH): 4,43 |
| | | | | | |
| **I-A-Q2-280:** | log P (HCOOH): 3,96 | **I-A-Q2-281:** | log P (HCOOH): 3,97 | **I-A-Q2-282:** | log P (HCOOH): 5,25 |
| | | | | | |
| **I-A-Q2-283:** | log P (HCOOH): 4,12 | **I-A-Q2-284:** | log P (HCOOH): 4,32 | **I-A-Q2-285:** | log P (HCOOH): 4,95 |
| | | | | | |
| **I-A-Q2-286:** | log P (HCOOH): 4,89 | **I-A-Q2-287:** | log P (HCOOH): 5,33 | **I-A-Q2-288:** | log P (HCOOH): 4,40 |
| | | | | | |
| **I-A-Q2-289:** | log P (HCOOH): 4,95 | **I-A-Q2-290:** | log P (HCOOH): 4,00 | **I-A-Q2-291:** | log P (HCOOH): 3,84 |
| | | | | | |
| **I-A-Q2-292:** | log P (HCOOH): 3,53 | **I-A-Q2-293:** | log P (HCOOH): 3,11 | **I-A-Q2-294:** | log P (HCOOH): 4,95 |
| | | | | | |
| **I-A-Q2-295:** | log P (HCOOH): 5,27 | **I-A-Q2-296:** | log P (HCOOH): 5,14 | **I-A-Q2-297:** | log P (HCOOH): 4,95 |
| | | | | | |
| **I-A-Q2-298:** | log P (HCOOH): 4,64 | **I-A-Q2-299:** | log P (HCOOH): 4,51 | **I-A-Q2-300:** | log P (HCOOH): 5,40 |
| | | | | | |
| **I-A-Q2-301:** | log P (HCOOH): 3,75 | **I-A-Q2-302:** | log P (HCOOH): 3,61 | **I-A-Q2-303:** | log P (HCOOH): 3,70 |
| | | | | | |
| **I-A-Q2-304:** | log P (HCOOH): 4,00 | **I-A-Q2-305:** | log P (HCOOH): 3,75 | **I-A-Q2-306:** | log P (HCOOH): 3,24 |
| | | | | | |
| **I-A-Q2-307:** | log P (HCOOH): 3,27 | **I-A-Q2-308:** | log P (HCOOH): 3,23 | **I-A-Q2-309:** | log P (HCOOH): 3,65 |
| **I-A-Q2-310:** | log P (HCOOH): 3,70 | **I-A-Q2-311:** | log P (HCOOH): 3,27 | **I-A-Q2-312:** | log P (HCOOH): 3,42 |
| | | | | | |
| **I-A-Q2-313:** | log P (HCOOH): 3,85 | **I-A-Q2-314:** | log P (HCOOH): 3,65 | **I-A-Q2-315:** | log P (HCOOH): 3,37 |
| | | | | | |
| **I-A-Q2-316:** | log P (HCOOH): 3,65 | **I-A-Q2-317:** | log P (HCOOH): 3,32 | **I-A-Q2-318:** | log P (HCOOH): 4,23 |
| | | | | | |
| **I-A-Q2-319:** | log P (HCOOH): 2,80 | **I-A-Q2-320:** | log P (HCOOH): 3,23 | **I-A-Q2-321:** | log P (HCOOH): 4,00 |
| | | | | | |
| **I-A-Q2-322:** | log P (HCOOH): 4,34 | **I-A-Q2-323:** | log P (HCOOH): 4,32 | **I-A-Q2-324:** | log P (HCOOH): 2,30 |
| | | | | | |
| **I-A-Q2-325:** | log P (HCOOH): 2,85 | **I-A-Q2-326:** | log P (HCOOH): 2,51 | **I-A-Q2-327:** | log P (HCOOH): 2,73 |
| | | | | | |
| **I-A-Q2-328:** | log P (HCOOH): 2,48 | **I-A-Q2-329:** | log P (HCOOH): 2,59 | **I-A-Q2-330:** | log P (HCOOH): 3,12 |
| | | | | | |
| **I-A-Q2-331:** | log P (HCOOH): 2,85 | **I-A-Q2-332:** | log P (HCOOH): 2,59 | **I-A-Q2-333:** | log P (HCOOH): 2,59 |
| | | | | | |
| **I-A-Q2-334:** | log P (HCOOH): 2,59 | **I-A-Q2-335:** | log P (HCOOH): 3,04 | **I-A-Q2-336:** | log P (HCOOH): 2,76 |
| | | | | | |
| **I-A-Q2-337:** | log P (HCOOH): 3,80 | **I-A-Q2-338:** | log P (HCOOH): 3,65 | **I-A-Q2-339:** | log P (HCOOH): 3,27 |
| | | | | | |
| **I-A-Q2-340:** | log P (HCOOH): 3,56 | **I-A-Q2-341:** | log P (HCOOH): 3,46 | **I-A-Q2-342:** | log P (HCOOH): 3,85 |
| | | | | | |
| **I-A-Q2-343:** | log P (HCOOH): 4,57 | **I-A-Q2-344:** | log P (HCOOH): 2,84 | **I-A-Q2-345:** | log P (HCOOH): 2,69 |
| | | | | | |
| **I-A-Q2-346:** | log P (HCOOH): 2,80 | **I-A-Q2-347:** | log P (HCOOH): 3,11 | **I-A-Q2-348:** | log P (HCOOH): 2,84 |
| | | | | | |
| **I-A-Q2-349:** | log P (HCOOH): 2,04 | **I-A-Q2-350:** | log P (HCOOH): 2,34 | **I-A-Q2-351:** | log P (HCOOH): 2,27 |
| **I-A-Q2-352:** | log P (HCOOH): 2,76 | **I-A-Q2-353:** | log P (HCOOH): 2,84 | **I-A-Q2-354:** | log P (HCOOH): 2,31 |
| | | | | | |
| **I-A-Q2-355:** | log P (HCOOH): 2,50 | **I-A-Q2-356:** | log P (HCOOH): 2,93 | **I-A-Q2-357:** | log P (HCOOH): 2,72 |
| | | | | | |
| **I-A-Q2-358:** | log P (HCOOH): 2,80 | **I-A-Q2-359:** | log P (HCOOH): 2,34 | **I-A-Q2-360:** | log P (HCOOH): 3,47 |
| | | | | | |
| **I-A-Q2-361:** | log P (HCOOH): 1,91 | **I-A-Q2-362:** | log P (HCOOH): 2,27 | **I-A-Q2-363:** | log P (HCOOH): 3,19 |
| | | | | | |
| **I-A-Q2-364:** | log P (HCOOH): 3,53 | **I-A-Q2-365:** | log P (HCOOH): 2,27 | **I-A-Q2-366:** | log P (HCOOH): 1,91 |
| | | | | | |
| **I-A-Q2-367:** | log P (HCOOH): 1,59 | **I-A-Q2-368:** | log P (HCOOH): 1,79 | **I-A-Q2-369:** | log P (HCOOH): 1,52 |
| | | | | | |
| **I-A-Q2-370:** | log P (HCOOH): 1,64 | **I-A-Q2-371:** | log P (HCOOH): 1,88 | **I-A-Q2-372:** | log P (HCOOH): 1,61 |
| | | | | | |
| **I-A-Q2-373:** | log P (HCOOH): 1,64 | **I-A-Q2-374:** | log P (HCOOH): 1,66 | **I-A-Q2-375:** | log P (HCOOH): 2,04 |
| | | | | | |
| **I-A-Q2-376:** | log P (HCOOH): 1,79 | **I-A-Q2-377:** | log P (HCOOH): 2,80 | **I-A-Q2-378** | log P (HCOOH): 2,61 |
| | | | | | |
| **I-A-Q2-379:** | log P (HCOOH): 2,20 | **I-A-Q2-380:** | log P (HCOOH): 2,53 | **I-A-Q2-381:** | log P (HCOOH): 2,46 |
| | | | | | |
| **I-A-Q2-382:** | log P (HCOOH): 2,84 | **I-A-Q2-383:** | log P (HCOOH): 3,94 | **I-A-Q2-384:** | log P (HCOOH): 4,53 |
| | | | | | |
| **I-A-Q2-385:** | log P (HCOOH): 4,60 | **I-A-Q2-386:** | log P (HCOOH): 3,77 | **I-A-Q2-387:** | log P (HCOOH): 4,32 |
| | | | | | |
| **I-A-Q2-388:** | log P (HCOOH): 3,37 | **I-A-Q2-389:** | log P (HCOOH): 4,35 | **I-A-Q2-390:** | log P (HCOOH): 3,41 |
| | | | | | |
| **I-A-Q2-391:** | log P (HCOOH): 3,57 | **I-A-Q2-392:** | log P (HCOOH): 3,51 | **I-A-Q2-393:** | log P (HCOOH): 4,82 |
| **I-A-Q2-394:** | log P (HCOOH): 3,70 | **I-A-Q2-395:** | log P (HCOOH): 4,43 | **I-A-Q2-396:** | log P (HCOOH): 4,59 |
| | | | | | |
| **I-A-Q2-397:** | log P (HCOOH): 4,69 | **I-A-Q2-398:** | log P (HCOOH): 3,59 | **I-A-Q2-399:** | log P (HCOOH): 5,26 |
| | | | | | |
| **I-A-Q2-400:** | log P (HCOOH): 4,36 | **I-A-Q2-401:** | log P (HCOOH): 4,93 | **I-A-Q2-402:** | log P (HCOOH): 3,89 |
| | | | | | |
| **I-A-Q2-403:** | log P (HCOOH): 4,34 | **I-A-Q2-404:** | log P (HCOOH): 4,81 | **I-A-Q2-405:** | log P (HCOOH): 4,84 |
| | | | | | |
| **I-A-Q2-407:** | log P (HCOOH): 5,15 | **I-A-Q2-408:** | log P (HCOOH): 3,68 | **I-A-Q2-409:** | log P (HCOOH): 3,38 |
| | | | | | |
| **I-A-Q2-410:** | log P (HCOOH): 3,94 | **I-A-Q2-411:** | log P (HCOOH): 3,17 | **I-A-Q2-412:** | log P (HCOOH): 3,29 |
| | | | | | |
| **I-A-Q2-413:** | log P (HCOOH): 3,37 | **I-A-Q2-414:** | log P (HCOOH): 2,72 | **I-A-Q2-415:** | log P (HCOOH): 2,71 |
| | | | | | |
| **I-A-Q2-416:** | log P (HCOOH): 3,81 | **I-A-Q2-417:** | log P (HCOOH): 3,13 | **I-A-Q2-418:** | log P (HCOOH): 3,22 |
| | | | | | |
| **I-A-Q2-419:** | log P (HCOOH): 2,68 | **I-A-Q2-420:** | log P (HCOOH): 2,81 | **I-A-Q2-421:** | log P (HCOOH): 2,53 |
| | | | | | |
| **I-A-Q2-422:** | log P (HCOOH): 3,41 | **I-A-Q2-423:** | log P (HCOOH): 3,55 | **I-A-Q2-424:** | log P (HCOOH): 2,75 |
| | | | | | |
| **I-A-Q2-425:** | log P (HCOOH): 2,99 | | | | |

**Tabelle 3**

| | | | | | | | | | | | | | |
|---|---|---|---|---|---|---|---|---|---|---|---|---|---|
| Gemäß den oben angegebenen allgemeinen Methoden lassen sich die in der folgenden Tabelle angegebenen Verbindungen der Formel (I-A mit Q = Q³) herstellen. | | | | | | | | | | | | | |
| 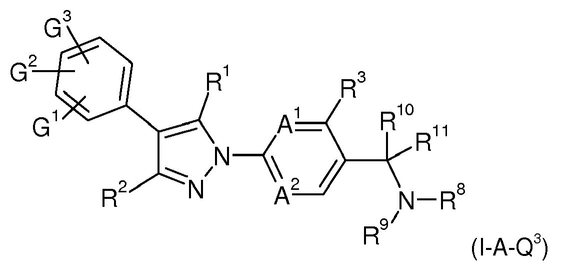 | | | | | | | | | | | | | |

| **Tabelle 3 Beispiel Nr.** | **G¹** | **G²** | **G³** | **R¹** | **R²** | **R³** | **A**¹ | **A²** | **R⁸** | **R⁹** | **R¹⁰** | **R¹¹** | **Phys. Chem. Daten** |
|---|---|---|---|---|---|---|---|---|---|---|---|---|---|
| I-A-Q3-001 | 3-Cl | H | 5-Cl | H | CF₃ | Me | CH | CH | Ac | H | H | H | |
| I-A-Q3-002 | 3-CF₃ | H | 5-CF₃ | H | CF₃ | CN | CH | CH | CSMe | H | H | H | |
| I-A-Q3-003 | 3-CF₃ | H | H | NH₂ | CF₃ | NO₂ | CH | CH | COEt | H | H | H | |
| I-A-Q3-004 | 3-Br | H | 5-Br | NH₂ | CF₃ | CF₃ | CH | CH | CH₂-2-Pyr | H | H | H | |
| I-A-Q3-005 | 3-Cl | 4-Cl | 5-Cl | Cl | CF₃ | Br | CH | CH | COiPr | H | H | H | |
| I-A-Q3-006 | 3-Br | 4-Cl | 5-Br | Cl | C₂F₅ | Cl | CH | CH | COCyclpr | H | H | H | |
| I-A-Q3-007 | 3-CF₃ | 4-Cl | 5-Cl | Br | C₂F₅ | CH₂OMe | CH | CH | SO₂Me | H | H | H | |
| I-A-Q3-008 | 3-CF₃ | 4-F | H | Br | C₂F₅ | CH₂NHMe | CH | CH | CONMe₂ | H | H | H | |
| I-A-Q3-009 | 3-Cl | 4-CF₃ | 5-Cl | I | C₂F₅ | Me | CH | CH | CHO | H | H | H | |
| I-A-Q3-010 | 3-CF₃ | H | 5-Cl | I | CF₃ | CN | CH | CH | CONHCH₂CF₃ | H | H | H | |
| I-A-Q3-011 | 3-Cl | 4-CF₃ | 5-Cl | CN | CF₃ | NO₂ | N | N | COOMe | H | H | H | |
| I-A-Q3-012 | 3-Cl | 4-Br | 5-Cl | CN | CF₃ | CF₃ | N | N | Ac | Ac | H | H | |
| I-A-Q3-013 | 3-F | 4-F | 5-F | NHCH₂Ph | C₂F₅ | Br | CH | CH | Ac | Ac | Me | Me | |
| I-A-Q3-014 | 2-Cl | 3-CF₃ | 4-Cl | NHAc | CF₃ | Cl | CH | CH | COOMe | COOMe | H | H | |
| I-A-Q3-015 | 3-Cl | 4-CF₃ | H | NAC₂ | CF₃ | CH₂OAc | CH | CH | COOMe | COOMe | Me | Me | |
| I-A-Q3-016 | 3-Br | 4-NH₂ | 5-Br | NHMe | CF₂Ph | CH₂NHAc | CH | CH | SO2Me | H | Me | Me | |
| I-A-Q3-017 | 3-Br | H | H | NMe₂ | CF₂OMe | Me | CH | CH | CH₂-2-Pyr | H | Me | Me | |
| I-A-Q3-018 | 3-Cl | 4-Cl | H | SMe | CF₂OPh | CN | CH | CH | CH₂-2-Pyr | Ac | Me | Me | |
| I-A-Q3-019 | 2-Cl | 3-Cl | 4-Cl | SOMe | CF₂(2-Pyr) | NO2 | N | CH | Me | H | H | H | |
| I-A-Q3-020 | 3-NO₂ | H | H | SO₂Me | CF₂O(2-Pyr) | CF₃ | CH | N | Ph | H | H | H | |
| I-A-Q3-021 | 3-Cl | H | 5-Cl | NH₂ | CF₃ | Cl | CH | CH | COCH₂CF₃ | H | H | H | log P |
| I-A-Q3-022 | 3-Cl | H | 5-Cl | NH₂ | CF₃ | Cl | CH | CH | COCypr₃ | H | H | H | log P |
| I-A-Q3-023 | 3-Cl | H | 5-Cl | NH₂ | CF₃ | Cl | CH | CH | COCH₃ | H | H | H | log P |
| I-A-Q3-024 | 3-Cl | H | 5-Cl | NH₂ | CF₃ | Cl | CH | CH | CONHEt | H | H | H | log P |

| | | | | | |
|---|---|---|---|---|---|
| **I-A-Q3-021:** | log P (HCOOH): 4,48 | **I-A-Q3-022:** | log P (HCOOH): 4,12 | **I-A-Q3-023:** | log P (HCOOH): 3,70 |
| | | | | | |
| **I-A-Q3-024:** | log P (HCOOH): 3,85 | | | | |

**Tabelle 4**

| | | | | | | | | | | |
|---|---|---|---|---|---|---|---|---|---|---|
| Gemäß den oben angegebenen allgemeinen Methoden lassen sich die in der folgenden Tabelle angegebenen Verbindungen der Formel (I-A mit Q = Q⁴) herstellen. | | | | | | | | | | |
| 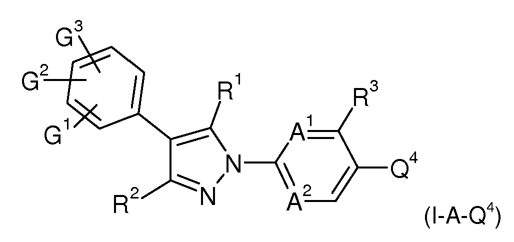 | | | | | | | | | | |

| **Tabelle 4 Beispiel Nr.** | **G¹** | **G²** | **G³** | **R¹** | **R²** | **R³** | **A¹** | **A²** | **Q⁴** | **Phys. Chem. Daten** |
|---|---|---|---|---|---|---|---|---|---|---|
| I-A-Q4-001 | 3-Cl | H | 5-Cl | H | CF₃ | CF₃ | CH | CH | COOEt | ¹H-NMR |
| I-A-Q4-002 | 3-Cl | H | 5-Cl | H | CF₃ | CF₃ | CH | CH | COOH | Feststoff |
| I-A-Q4-003 | 3-CF₃ | H | 5-CF₃ | H | CF₃ | CF₃ | CH | CH | COOEt | ¹H-NMR |
| I-A-Q4-004 | 3-CF₃ | H | 5-CF₃ | H | CF₃ | CF₃ | CH | CH | COOH | |
| I-A-Q4-005 | 3-Cl | H | 5-Cl | H | CF₃ | NO₂ | CH | CH | COOtBu | ¹H-NMR |
| I-A-Q4-006 | 3-Cl | H | 5-Cl | H | CF₃ | CF₃ | CH | CH | NO₂ | ¹H-NMR |
| I-A-Q4-007 | 3-Cl | H | 5-Cl | H | CF₃ | CF₃ | CH | CH | NH₂ | ¹H-NMR |
| I-A-Q4-008 | 3-Cl | H | 5-Cl | H | H | CF₃ | CH | CH | COOEt | ¹H-NMR |
| I-A-Q4-009 | 3-Cl | H | 5-CF₃ | NH₂ | CF₃ | CN | CH | CH | F | log P |
| | | | | | | | | | | ¹H-NMR |
| I-A-Q4-010 | 3-Cl | H | 5-Cl | NH₂ | CF₃ | Me | CH | CH | COOH | log P |
| | | | | | | | | | | ¹H-NMR |
| I-A-Q4-011 | 3-Cl | H | 5-Cl | NH₂ | CF₃ | Me | CH | CH | COOMe | log P |
| | | | | | | | | | | ¹H-NMR |
| I-A-Q4-012 | 3-Cl | 4-Cl | H | NH₂ | CF₃ | Me | CH | CH | COOH | log P |
| | | | | | | | | | | ¹H-NMR |
| I-A-Q4-013 | 3-Cl | 4-Cl | H | NH₂ | CF₃ | Me | CH | CH | COOEt | log P |
| | | | | | | | | | | ¹H-NMR |
| I-A-Q4-014 | 3-Cl | H | 5-Cl | NH₂ | CF₃ | H | N | CH | Cl | log P |
| I-A-Q4-015 | H | 4-Cl | H | NCHNMe₂ | CF₃ | H | N | N | Cl | log P |
| I-A-Q4-016 | 3-Cl | H | 5-CF₃ | NH₂ | CF₃ | H | N | CH | Cl | log P |
| I-A-Q4-017 | 3-Cl | H | 5-Cl | Br | CF₃ | H | CH | CH | I | log P |
| I-A-Q4-018 | 3-Cl | H | 5-Cl | I | CF₃ | H | CH | CH | I | log P |
| I-A-Q4-019 | 3-Cl | H | 5-Cl | NH₂ | CF₃ | H | CF | CH | SO₂Et | log P |
| I-A-Q4-020 | 3-Cl | 4-Cl | H | SMe | CF₃ | H | N | N | Cl | log P |
| I-A-Q4-021 | 2-Cl | 4-Cl | H | NH₂ | Me | H | N | CH | Cl | log P |
| I-A-Q4-022 | 3-Me | H | 5-Me | NH₂ | CF₃ | H | N | CH | Cl | log P |
| I-A-Q4-023 | 2-Cl | H | H | NH₂ | CF₃ | H | N | CH | Cl | log P |
| I-A-Q4-024 | H | 4-Cl | H | NH₂ | Cypr | H | N | CH | Cl | log P |
| I-A-Q4-025 | H | 4-Cl | H | NH₂ | Pr | H | N | CH | Cl | log P |
| I-A-Q4-026 | H | 4-Cl | H | NH₂ | Et | H | N | CH | Cl | log P |
| I-A-Q4-027 | H | 4-Cl | H | NH₂ | iPr | H | N | CH | Cl | log P |
| I-A-Q4-028 | H | 4-Cl | H | NH₂ | Ph | H | N | CH | Cl | log P |
| I-A-Q4-029 | 2-Cl | H | 6-Cl | NH₂ | Me | H | N | CH | Cl | log P |
| I-A-Q4-030 | 2-Cl | H | 5-Cl | NH₂ | Me | H | N | CH | Cl | log P |
| I-A-Q4-031 | 2-F | H | 6-Cl | NH₂ | Me | H | N | CH | Cl | log P |
| I-A-Q4-032 | 2-Cl | H | H | NH₂ | Me | H | N | CH | Cl | log P |
| I-A-Q4-033 | 2-F | H | 5-F | NH₂ | Me | H | N | CH | Cl | log P |
| I-A-Q4-034 | 3-Cl | H | H | NH₂ | Me | H | N | CH | Cl | log P |
| I-A-Q4-035 | 2-F | 4-F | H | NH₂ | Me | H | N | CH | Cl | log P |
| I-A-Q4-036 | 2-F | H | 6-F | NH₂ | Me | H | N | CH | Cl | log P |
| I-A-Q4-037 | 2-Cl | 4-F | H | NH₂ | Me | H | N | CH | Cl | log P |
| I-A-Q4-038 | H | 4-F | H | NH₂ | Me | H | N | CH | Cl | log P |
| I-A-Q4-039 | H | 4-Cl | H | NH₂ | Me | H | N | CH | Cl | log P |
| I-A-Q4-040 | 3-F | H | 5-Cl | NH₂ | CF₃ | H | N | CH | Cl | log P |
| I-A-Q4-041 | H | 4-Cl | H | NH₂ | CF₃ | H | N | CH | Cl | log P |
| I-A-Q4-042 | H | 4-Br | H | NH₂ | CF₃ | H | N | CH | Cl | log P |
| I-A-Q4-043 | 3-Cl | 4-Cl | H | NH₂ | CF₃ | H | N | CH | Cl | log P |
| I-A-Q4-044 | 2-Cl | 4-Cl | H | NH₂ | CF₃ | H | N | CH | Cl | log P |
| I-A-Q4-045 | H | 4-CN | H | NH₂ | CF₃ | H | N | CH | Cl | log P |
| I-A-Q4-046 | 2-F | 4-F | 6-F | NH₂ | CF₃ | H | N | CH | Cl | log P |
| I-A-Q4-047 | 3-Cl | 4-F | H | NH₂ | CF₃ | H | N | CH | Cl | log P |
| I-A-Q4-048 | 3-F | 4-Cl | H | NH₂ | CF₃ | H | N | CH | Cl | log P |
| I-A-Q4-049 | 3-F | H | H | NH₂ | CF₃ | H | N | CH | Cl | log P |
| I-A-Q4-050 | 3-F | 4-F | H | NH₂ | CF₃ | H | N | CH | Cl | log P |
| I-A-Q4-051 | H | 4-I | H | NH₂ | CF₃ | H | N | CH | Cl | log P |
| I-A-Q4-052 | 3-Br | H | H | NH₂ | CF₃ | H | N | CH | Cl | log P |
| I-A-Q4-053 | 3-F | 4-F | 5-F | NH₂ | CF₃ | H | N | CH | Cl | log P |
| I-A-Q4-054 | 2-Me | H | H | NH₂ | CF₃ | H | N | CH | Cl | log P |
| I-A-Q4-055 | H | 4-Me | H | NH₂ | CF₃ | H | N | CH | Cl | log P |
| I-A-Q4-056 | 2-F | 4-F | H | NH₂ | CHF₂ | H | N | CH | Cl | log P |
| I-A-Q4-057 | H | 4-Cl | H | NH₂ | CHF₂ | H | N | CH | Cl | log P |
| I-A-Q4-058 | H | 4-Cl | H | NH₂ | C₂F₅ | H | N | CH | Cl | log P |
| I-A-Q4-059 | 3-Cl | H | 5-CF₃ | NH₂ | CClF₂ | H | N | CH | Cl | log P |
| I-A-Q4-060 | 3-Cl | 4-Cl | H | NH₂ | Me | H | N | CH | Cl | log P |
| I-A-Q4-061 | H | 4-OMe | H | NH₂ | Me | H | N | CH | Cl | log P |
| I-A-Q4-062 | H | 4-Cl | H | NH₂ | CH₂-iPr | H | N | CH | Cl | log P |
| I-A-Q4-063 | 3-Cl | H | 5-Cl | NH₂ | CF₃ | CF₃ | N | N | COOMe | log P |
| I-A-Q4-064 | 3-Cl | H | 5-Cl | NH₂ | CF₃ | H | N | CH | COOH | log P |
| I-A-Q4-065 | 3-Cl | H | 5-Cl | H | CF₃ | Me | CH | CH | F | |
| I-A-Q4-066 | 3-CF₃ | H | 5-CF₃ | H | CF₃ | CN | CH | CH | Cl | |
| I-A-Q4-067 | 3-CF₃ | H | H | NH₂ | CF₃ | NO₂ | CH | CH | Br | |
| I-A-Q4-068 | 3-Br | H | 5-Br | NH₂ | CF₃ | CF₃ | CH | CH | I | |
| I-A-Q4-069 | 3-Cl | 4-Cl | 5-Cl | Cl | CF₃ | Br | CH | CH | CN | |
| I-A-Q4-070 | 3-Br | 4-Cl | 5-Br | Cl | C₂F₅ | Cl | CH | CH | NO₂ | |
| I-A-Q4-071 | 3-CF₃ | 4-Cl | 5-Cl | Br | C₂F₅ | CH₂O Me | CH | CH | SMe | |
| I-A-Q4-072 | 3-CF₃ | 4-F | H | Br | C₂F₅ | CH₂N HMe | CH | CH | SCF₃ | |
| I-A-Q4-073 | 3-Cl | 4-CF₃ | 5-Cl | I | C₂F₅ | Me | CH | CH | SOMe | |
| I-A-Q4-074 | 3-CF₃ | H | 5-Cl | I | CF₃ | CN | CH | CH | SOCF₃ | |
| I-A-Q4-075 | 3-Cl | 4-CF₃ | 5-Cl | CN | CF₃ | NO₂ | N | N | SO₂Me | |
| I-A-Q4-076 | 3-Cl | 4-Br | 5-Cl | CN | CF₃ | CF₃ | N | N | SO₂CF₃ | |
| I-A-Q4-077 | 3-F | 4-F | 5-F | NHCH₂Ph | C₂F₅ | Br | CH | CH | CN | |
| I-A-Q4-078 | 2-Cl | 3-CF₃ | 4-Cl | NHAc | CF₃ | Cl | CH | CH | NO₂ | |
| I-A-Q4-079 | 3-Cl | 4-CF₃ | H | NAc₂ | CF₃ | CH₂O Ac | CH | CH | F | |
| I-A-Q4-080 | 3-Br | 4-NH₂ | 5-Br | NHMe | CF₂Ph | CH₂N HAc | CH | CH | Cl | |
| I-A-Q4-081 | 3-Br | H | H | NMe₂ | CF₂OMe | Me | CH | CH | Br | |
| I-A-Q4-082 | 3-Cl | 4-Cl | H | SMe | CF₂OPh | CN | CH | CH | I | |
| I-A-Q4-083 | 2-Cl | 3-Cl | 4-Cl | SOMe | CF₂(2-Pyr) | NO2 | N | CH | COOH | |
| I-A-Q4-084 | 3-NO₂ | H | H | SO₂Me | CF₂O(2-Pyr) | CF₃ | CH | N | COOEt | |
| I-A-Q4-085 | 2-Cl | 4-Cl | H | NH₂ | CF₃ | H | CH | CH | COOEt | log P |
| I-A-Q4-086 | 3-Cl | H | 5-Cl | NH₂ | CF₃ | H | CH | CH | COOEt | log P |
| I-A-Q4-087 | 3-Cl | H | 5-Cl | NH₂ | CF₃ | H | CH | CH | COOH | log P |
| I-A-Q4-08 | H | 4-Cl | H | NH₂ | CF₃ | H | CH | CH | COOH | log P |
| I-A-Q4-089 | 3-Cl | 4-Cl | H | NH₂ | CF₃ | H | CH | CH | COOH | log P |
| I-A-Q4-090 | 3-Me | H | 5-Me | NH₂ | CF₃ | H | CH | CH | COOH | log P |
| I-A-Q4-091 | 3-Br | H | H | NH₂ | CF₃ | H | CH | CH | COOH | log P |
| I-A-Q4-092 | 3-F | 4-F | H | NH₂ | CF₃ | H | CH | CH | COOH | log P |
| I-A-Q4-093 | 3-F | 4-Cl | H | NH₂ | CF₃ | H | CH | CH | COOH | log P |
| I-A-Q4-094 | 3-Cl | 4-F | H | NH₂ | CF₃ | H | CH | CH | COOH | log P |
| I-A-Q4-095 | 2-Cl | 4-Cl | H | NH₂ | CF₃ | H | CH | CH | COOH | log P |
| I-A-Q4-096 | 3-Cl | H | H | NH₂ | CF₃ | H | CH | CH | COOH | log P |
| I-A-Q4-097 | 3-F | H | H | NH₂ | CF₃ | H | CH | CH | COOH | log P |
| I-A-Q4-098 | H | 4-Br | H | NH₂ | CF₃ | H | CH | CH | COOH | log P |
| I-A-Q4-099 | 3-F | 4-F | 5-F | NH₂ | CF₃ | H | CH | CH | COOH | log P |
| I-A-Q4-100 | 3-F | H | 5-Cl | NH₂ | CF₃ | H | CH | CH | COOH | log P |
| I-A-Q4-101 | 2-Me | H | H | NH₂ | CF₃ | H | CH | CH | COOH | log P |
| I-A-Q4-102 | 3-Cl | H | 5-Cl | NH₂ | CF₃ | CN | CH | CH | I | log P |
| I-A-Q4-103 | 3-Cl | H | 5-Cl | NH₂ | CF₃ | CN | CH | CH | F | log P |
| I-A-Q4-104 | 3-Cl | H | 5-Cl | NH₂ | CF₃ | Cl | CH | CH | COOMe | log P |
| I-A-Q4-105 | 3-Cl | H | 5-Cl | NH₂ | CF₃ | CF₃ | CH | CH | F | log P |
| I-A-Q4-106 | 3-Cl | H | 5-Cl | NH₂ | CF₃ | H | CCF₃ | CH | F | log P |
| I-A-Q4-107 | 3-Cl | H | 5-Cl | NH₂ | CF₃ | H | CF | CF | F | log P |
| I-A-Q4-108 | 3-Cl | H | 5-CF₃ | NH₂ | CF₃ | CF₃ | CH | CH | COOMe | |
| I-A-Q4-109 | 3-Cl | H | 5-Cl | NH₂ | CF₃ | F | CH | CH | F | log P |
| I-A-Q4-110 | 3-Cl | H | 5-Cl | NH₂ | CF₃ | Cl | CH | CH | SO₂NHCy pr | log P |
| I-A-Q4-111 | 3-Cl | H | 5-Cl | NH₂ | CF₃ | Br | CH | CH | SO₂NHCy pr | log P |
| I-A-Q4-112 | 3-Cl | H | 5-Cl | NH₂ | CF₃ | CF₃ | CH | CH | COOMe | log P |
| I-A-Q4-113 | 3-Cl | H | 5-Cl | NH₂ | CF₃ | Me | CF | CH | COOMe | log P |
| I-A-Q4-114 | 3-Cl | H | 5-Cl | NH₂ | CF₃ | Me | CH | CF | COOMe | log P |
| I-A-Q4-115 | 3-Cl | H | 5-Cl | NH₂ | CF₃ | CF₃ | CH | CH | COOH | log P |
| I-A-Q4-116 | 3-Cl | H | 5-Cl | NH₂ | CF₃ | Me | CF | CH | COOH | log P |
| I-A-Q4-117 | 3-Cl | H | 5-Cl | NH₂ | CF₃ | Me | CH | CF | COOH | log P |
| I-A-Q4-118 | 3-Cl | H | 5-Cl | NH₂ | CF₃ | SMe | CH | CH | COOMe | log P |
| I-A-Q4-119 | 3-Cl | H | 5-Cl | NH₂ | CF₃ | NMe₂ | CH | CH | COOMe | log P |
| I-A-Q4-120 | 3-Cl | H | 5-Cl | NH₂ | CF₃ | NHMe | CH | CH | COOH | log P |
| I-A-Q4-121 | 3-Cl | H | 5-Cl | NH₂ | CF₃ | NMe2 | CH | CH | COOH | log P |
| I-A-Q4-122 | 3-Cl | H | 5-Cl | NH₂ | CF₃ | CN | CH | CH | S(O)₂NHC ybu | log P |
| I-A-Q4-123 | 3-Cl | H | 5-Cl | NH₂ | CF₃ | CN | CH | CH | S(O)₂NHC H₂CF₃ | log P |

### Charakteristische Daten für Synthesebeispiele:

| | | | | | |
|---|---|---|---|---|---|
| **I-A-Q4-001:** | spektroskopische Daten siehe Protokoll unter allgemeinen Synthesevorschriften | | | | |
| | | | | | |
| **I-A-Q4-002:** | spektroskopische Daten siehe Protokoll unter allgemeinen Synthesevorschriften | | | | |
| | | | | | |
| **I-A-Q4-003:** | 1H NMR (CDCl3): 8,26-8,18 (m, 2 H), 8,04-7,97 (m, 5 H), 4,44 (q, 2 H, J = 7,1 Hz), 1,42 (t, 3 H, J = 7,1 Hz) | | | | |
| | | | | | |
| **I-A-Q4-005:** | 1H NMR (CDCl3): 8,22 (d, 1H, J = 2,2 Hz), 8,15 (d, 1 H, J = 0,9 Hz), 8,03 (dd, 1 H, J = 8,4 und 2,2 Hz), 7,94-7,91 (m, 1 H), 7,42-7,36 (m, 3 H), 1,58 (s, 9 H) | | | | |
| | | | | | |
| **I-A-Q4-006:** | 1H NMR (CDCl3): 8,27-8,23 (m, 1 H), 8,20-8,17 (m, 1 H), 8,15-8,11 (m, 2 H), 7,44-7,43 (m, 1 H), 7,37-7,37 (m, 2 H) | | | | |
| | | | | | |
| **I-A-Q4-007:** | 1H NMR (CDCl3): 7,92-7,87 (m, 1 H), 7,75-7,73 (m, 1 H), 7,66-7,61 (m, 1 H), 7,34-7,29 (m, 3 H), 6,84 (t, 1 H, J = 8,7 Hz), 4,37-4,25 (m, 2 H) | | | | |
| | | | | | |
| **I-A-Q4-008:** | 1H NMR (CDCl3): 8,25 (d, 1 H, J = 0,5 Hz), 8,16 (s, 1 H), 8,02 (s, 1 H), 7,97 (d, 2 H, J = 1,3 Hz), 7,44 (d, 2 H, J = 1,8 Hz), 7,32-7,30 (m, 1 H), 4,43 (q, 2 H J = 7,1 Hz), 1,41 (t, 3 H, J = 8,6 Hz) | | | | |
| **I-A-Q4-009:** | spektroskopische Daten siehe Protokoll unter allgemeinen Synthesevorschriften | | | | |
| | | | | | |
| **I-A-Q4-010:** | spektroskopische Daten siehe Protokoll unter allgemeinen Synthesevorschriften | | | | |
| | | | | | |
| **I-A-Q4-011:** | spektroskopische Daten siehe Protokoll unter allgemeinen Synthesevorschriften | | | | |
| | | | | | |
| **I-A-Q4-012:** | spektroskopische Daten siehe Protokoll unter allgemeinen Synthesevorschriften | | | | |
| | | | | | |
| **I-A-Q4-013:** | spektroskopische Daten siehe Protokoll unter allgemeinen Synthesevorschriften | | | | |
| | | | | | |
| **I-A-Q4-014:** | log P (HCOOH): 5,76 | **I-A-Q4-015:** | log P (HCOOH): 3,47 | **I-A-Q4-016:** | log P (HCOOH): 5,76 |
| | | | | | |
| **I-A-Q4-017:** | log P (HCOOH): 6,71 | **I-A-Q4-018:** | log P (HCOOH): 6,50 | **I-A-Q4-019:** | log P (HCOOH): 3,94 |
| | | | | | |
| **I-A-Q4-020:** | log P (HCOOH): 5,22 | **I-A-Q4-021:** | log P (HCOOH): 4,70 | **I-A-Q4-022:** | log P (HCOOH): 5,22 |
| | | | | | |
| **I-A-Q4-023:** | log P (HCOOH): 4,57 | **I-A-Q4-024:** | log P (HCOOH): 5,36 | **I-A-Q4-025:** | log P (HCOOH): 5,51 |
| | | | | | |
| **I-A-Q4-026:** | log P (HCOOH): 5,02 | **I-A-Q4-027:** | log P (HCOOH): 5,73 | **I-A-Q4-028:** | log P (HCOOH): 5,64 |
| | | | | | |
| **I-A-Q4-029:** | log P (HCOOH): 4,05 | **I-A-Q4-030:** | log P (HCOOH): 4,57 | **I-A-Q4-031:** | log P (HCOOH): 3,78 |
| | | | | | |
| **I-A-Q4-032:** | log P (HCOOH): 3,89 | **I-A-Q4-033:** | log P (HCOOH): 3,84 | **I-A-Q4-034:** | log P (HCOOH): 4,40 |
| | | | | | |
| **I-A-Q4-035:** | log P (HCOOH): 3,78 | **I-A-Q4-036:** | log P (HCOOH): 3,58 | **I-A-Q4-037:** | log P (HCOOH): 4,05 |
| | | | | | |
| **I-A-Q4-038:** | log P (HCOOH): 3,84 | **I-A-Q4-039:** | log P (HCOOH): 4,40 | **I-A-Q4-040:** | log P (HCOOH): 5,29 |
| **I-A-Q4-041:** | log P (HCOOH): 4,95 | **I-A-Q4-042:** | log P (HCOOH): 5,22 | **I-A-Q4-043:** | log P (HCOOH): 5,44 |
| | | | | | |
| **I-A-Q4-044:** | log P (HCOOH): 5,02 | **I-A-Q4-045:** | log P (HCOOH): 4,11 | **I-A-Q4-046:** | log P (HCOOH): 4,46 |
| | | | | | |
| **I-A-Q4-047:** | log P (HCOOH): 4,95 | **I-A-Q4-048:** | log P (HCOOH): 5,02 | **I-A-Q4-049:** | log P (HCOOH): 4,51 |
| | | | | | |
| **I-A-Q4-050:** | log P (HCOOH): 4,64 | **I-A-Q4-051:** | log P (HCOOH): 5,29 | **I-A-Q4-052:** | log P (HCOOH): 5,02 |
| | | | | | |
| **I-A-Q4-053:** | log P (HCOOH): 4,89 | **I-A-Q4-054:** | log P (HCOOH): 4,64 | **I-A-Q4-055:** | log P (HCOOH): 4,83 |
| | | | | | |
| **I-A-Q4-056:** | log P (HCOOH): 3,84 | **I-A-Q4-057:** | log P (HCOOH): 4,45 | **I-A-Q4-058:** | log P (HCOOH): 5,44 |
| | | | | | |
| **I-A-Q4-059:** | log P (HCOOH): 5,81 | **I-A-Q4-060:** | log P (HCOOH): 5,02 | **I-A-Q4-061:** | log P (HCOOH): 3,58 |
| | | | | | |
| **I-A-Q4-062:** | log P (HCOOH): 5,97 | **I-A-Q4-063:** | log P (HCOOH): 4,66 | **I-A-Q4-064:** | log P (HCOOH): 4,23 |
| | | | | | |
| **I-A-Q4-085:** | log P (HCOOH): 4,57 | **I-A-Q4-086:** | log P (HCOOH): 4,93 | **I-A-Q4-087:** | log P (HCOOH): 3,79 |
| | | | | | |
| **I-A-Q4-088:** | log P (HCOOH): 3,28 | **I-A-Q4-089:** | log P (HCOOH): 3,69 | **I-A-Q4-090:** | log P (HCOOH): 3,42 |
| | | | | | |
| **I-A-Q4-091:** | log P (HCOOH): 3,29 | **I-A-Q4-092:** | log P (HCOOH): 3,06 | **I-A-Q4-093:** | log P (HCOOH): 3,39 |
| | | | | | |
| **I-A-Q4-094:** | log P (HCOOH): 3,31 | **I-A-Q4-095:** | log P (HCOOH): 3,44 | **I-A-Q4-096:** | log P (HCOOH): 3,20 |
| | | | | | |
| **I-A-Q4-097:** | log P (HCOOH): 2,88 | **I-A-Q4-098:** | log P (HCOOH): 3,35 | **I-A-Q4-099:** | log P (HCOOH): 3,32 |
| | | | | | |
| **I-A-Q4-100:** | log P (HCOOH): 3,32 | **I-A-Q4-101:** | log P (HCOOH): 2,94 | **I-A-Q4-102:** | log P (HCOOH): 5,94 |
| **I-A-Q4-103:** | log P (HCOOH): 4,44 | **I-A-Q4-104:** | log P (HCOOH): 5,07 | **I-A-Q4-105:** | log P (HCOOH): 5,25 |
| | | | | | |
| **I-A-Q4-106:** | log P (HCOOH): 4,68 | **I-A-Q4-107:** | log P (HCOOH): 4,45 | **I-A-Q4-109:** | log P (HCOOH): 4,77 |
| | | | | | |
| **I-A-Q4-110:** | log P (HCOOH): 4,49 | **I-A-Q4-111:** | log P (HCOOH): 4,77 | **I-A-Q4-112:** | log P (HCOOH): 5,21 |
| | | | | | |
| **I-A-Q4-113:** | log P (HCOOH): 4,78 | **I-A-Q4-114:** | log P (HCOOH): 4,84 | **I-A-Q4-115:** | log P (HCOOH): 4,22 |
| | | | | | |
| **I-A-Q4-116:** | log P (HCOOH): 3,91 | **I-A-Q4-117:** | log P (HCOOH): 3,96 | **I-A-Q4-118:** | log P (HCOOH): 4,93 |
| | | | | | |
| **I-A-Q4-119:** | log P (HCOOH): 4,71 | **I-A-Q4-120:** | log P (HCOOH): 4,11 | **I-A-Q4-121:** | log P (HCOOH): 3,07 |
| | | | | | |
| **I-A-Q4-122:** | log P (HCOOH): 4,69 | **I-A-Q4-123:** | log P (HCOOH): 4,41 | | |

**Tabelle 5**

| | | | | | | | | |
|---|---|---|---|---|---|---|---|---|
| Gemäß den oben angegebenen allgemeinen Methoden lassen sich die in der folgenden Tabelle angegebenen Verbindungen der Formel (I-B mit Q = Q¹) herstellen | | | | | | | | |
| 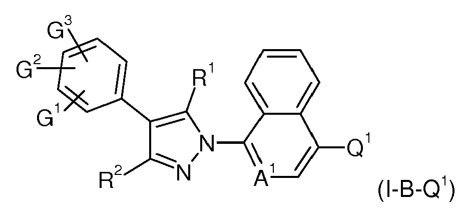 | | | | | | | | |

| **Tabelle 5 Beispiel Nr.** | **G¹** | **G²** | **G³** | **R¹** | **R²** | **A¹** | **Q¹** | **Phys. Chem. Daten** |
|---|---|---|---|---|---|---|---|---|
| I-B-Q1-001 | 3-Cl | H | 5-Cl | H | CF₃ | CH | 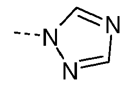 | |
| I-B-Q1-002 | 3-CF₃ | H | 5-CF₃ | H | CF₃ | CH | 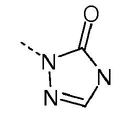 | |
| I-B-Q1-003 | 3-CF₃ | H | H | NH₂ | CF₃ | CH | 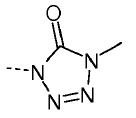 | |
| I-B-Q1-004 | 3-Br | H | 5-Br | NH₂ | CF₃ | CH | 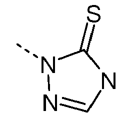 | |
| I-B-Q1-005 | 3-Cl | 4-Cl | 5-Cl | Cl | CF₃ | CH | 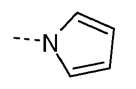 | |
| I-B-Q1-006 | 3-Br | 4-Cl | 5-Br | Cl | C₂F₅ | CH | 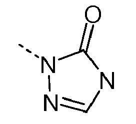 | |
| I-B-QI-007 | 3-CF₃ | 4-Cl | 5-Cl | Br | C₂F₅ | CH | 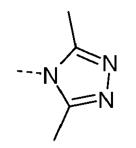 | |
| I-B-Q1-008 | 3-CF₃ | 4-F | H | Br | C₂F₅ | CH | 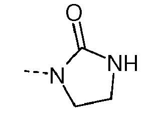 | |
| I-B-Q1-009 | 3-Cl | 4-CF₃ | 5-Cl | I | C₂F₅ | CH | 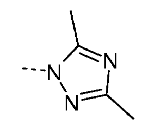 | |
| I-B-Q1-010 | 3-CF₃ | H | 5-Cl | I | CF₃ | CH | 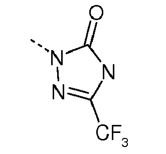 | |
| I-B-Q1-011 | 3-Cl | 4-CF₃ | 5-Cl | CN | CF₃ | N | 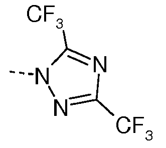 | |
| I-B-Q1-012 | 3-Cl | 4-Br | 5-Cl | CN | CF₃ | N | 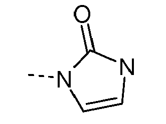 | |
| I-B-Q1-013 | 3-F | 4-F | 5-F | NHCH₂Ph | C₂F₅ | CH | 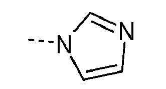 | |
| I-B-Q1-014 | 2-Cl | 3-CF₃ | 4-Cl | NHAc | CF₃ | CH | 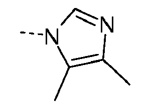 | |
| I-B-Q1-015 | 3-Cl | 4-CF₃ | H | NAc₂ | CF₃ | CH | 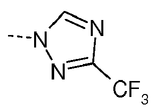 | |
| I-B-Q1-016 | 3-Br | 4-NH₂ | 5-Br | NHMe | CF₂Ph | CH | 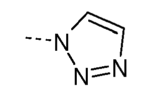 | |
| I-B-Q1-017 | 3-Br | H | H | NMe₂ | CF₂OMe | CH | 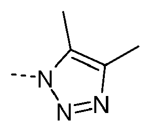 | |
| I-B-Q1-018 | 3-Cl | 4-Cl | H | SMe | CF₂OPh | CH | 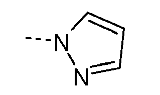 | |
| I-B-Q1-019 | 2-Cl | 3-Cl | 4-Cl | SOMe | CF₂(2-Pyr) | N | 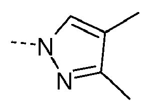 | |
| I-B-Q1-020 | 3-NO₂ | H | H | SO₂Me | CF₂O(2-Pyr) | CH | 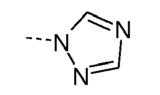 | |

**Tabelle 6**

| | | | | | | | | | | |
|---|---|---|---|---|---|---|---|---|---|---|
| Gemäß den oben angegebenen allgemeinen Methoden lassen sich die in der folgenden Tabelle angegebenen Verbindungen der Formel (I-B mit Q = Q²) herstellen | | | | | | | | | | |
| 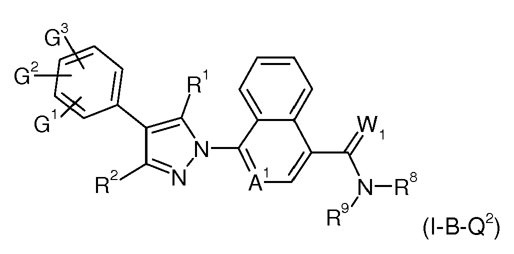 | | | | | | | | | | |

| **Beispiel Nr.** | **G¹** | **G²** | **G³** | **R¹** | **R²** | **A¹** | **W¹** | **R⁸** | **R⁹** | **Phys. Chem. Daten** |
|---|---|---|---|---|---|---|---|---|---|---|
| I-B-Q2-001 | 3-Cl | H | 5-Cl | H | CF₃ | CH | O | H | TFiPr | log P |
| I-B-Q2-002 | 3-CF₃ | H | 5-CF₃ | H | CF₃ | CH | O | H | HFiPr | |
| I-B-Q2-003 | 3-CF₃ | H | H | NH₂ | CF₃ | CH | O | H | CH₂-2-Pyr | |
| I-B-Q2-004 | 3-Br | H | 5-Br | NH₂ | CF₃ | CH | O | Me | CH₂-2-Pyr | |
| I-B-Q2-005 | 3-Cl | 4-Cl | 5-Cl | Cl | CF₃ | CH | O | H | CHMe-2-Pyr | |
| I-B-Q2-006 | 3-Br | 4-Cl | 5-Br | Cl | C₂F₅ | CH | O | Me | CHMe-2-Pyr | |
| I-B-Q2-007 | 3-CF₃ | 4-Cl | 5-Cl | Br | C₂F₅ | CH | O | H | CH₂-3-Pyr | |
| I-B-Q2-008 | 3-CF₃ | 4-F | H | Br | C₂F₅ | CH | O | Me | CH₂-3-Pyr | |
| I-B-Q2-009 | 3-Cl | 4-CF₃ | 5-Cl | I | C₂F₅ | CH | O | H | CHMe-3-Pyr | |
| I-B-Q2-010 | 3-CF₃ | H | 5-Cl | I | CF₃ | CH | O | Me | CHMe-3-Pyr | |
| I-B-Q2-011 | 3-Cl | 4-CF₃ | 5-Cl | CN | CF₃ | N | O | H | CH₂-4-Pyr | |
| I-B-Q2-012 | 3-Cl | 4-Br | 5-Cl | CN | CF₃ | N | O | Me | CH₂-4-Pyr | |
| I-B-Q2-013 | 3-F | 4-F | 5-F | NHCH₂Ph | C₂F₅ | CH | O | H | CHMe-4-Pyr | |
| I-B-Q2-014 | 2-Cl | 3-CF₃ | 4-Cl | NHAc | CF₃ | CH | O | Me | CHMe-4-Pyr | |
| I-B-Q2-015 | 3-Cl | 4-CF₃ | H | NAc₂ | CF₃ | CH | O | H | CH₂-2-Pyrm | |
| I-B-Q2-016 | 3-Br | 4-NH₂ | 5-Br | NHMe | CF₂Ph | CH | O | H | CH₂-2-Pyrz | |
| I-B-Q2-017 | 3-Br | H | H | NMe₂ | CF₂OMe | CH | O | H | Me | |
| I-B-Q2-018 | 3-Cl | 4-Cl | H | SMe | CF₂OPh | CH | O | Me | Me | |
| I-B-Q2-019 | 2-Cl | 3-Cl | 4-Cl | SOMe | CF₂(2-Pyr) | N | O | H | 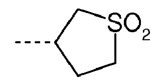 | |
| I-B-Q2-020 | 3-NO₂ | H | H | SO₂Me | CF₂O(2-Pyr) | CH | O | H | CH₂COOMe | |
| I-B-Q2-021 | 3-Cl | H | 5-Cl | H | CF₃ | CH | O | H | CH₂CF₂CF₂H | log P |
| I-B-Q2-022 | 3-Cl | H | 5-Cl | H | CF₃ | CH | O | H | CH₂-2-Pyr | log P |
| I-B-Q2-023 | 3-Cl | H | 5-Cl | H | CF₃ | CH | O | H | CyBu | log P |
| I-B-Q2-024 | 3-Cl | H | 5-Cl | H | CF₃ | CH | O | H | 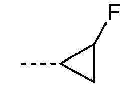 | log P |

| | | | | | |
|---|---|---|---|---|---|
| **I-B-Q2-001:** | log P (HCOOH): 4,74 | **I-B-Q2-021:** | log P (HCOOH): 4,58 | **I-B-Q2-022:** | log P (HCOOH): 3,54 |
| | | | | | |
| **I-B-Q2-023:** | log P (HCOOH): 4,57 | **I-B-Q2-024:** | log P (HCOOH): 4,20 | | |

**Tabelle 7**

| | | | | | | | | | | | |
|---|---|---|---|---|---|---|---|---|---|---|---|
| Gemäß den oben angegebenen allgemeinen Methoden lassen sich die in der folgenden Tabelle angegebenen Verbindungen der Formel (I-B mit Q = Q³) herstellen. | | | | | | | | | | | |
| 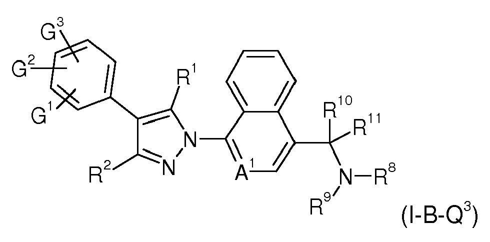 | | | | | | | | | | | |

| **Tabelle 7 Beispiel Nr.** | **G¹** | **G²** | **G³** | **R¹** | **R³** | **A¹** | **R⁸** | **R⁹** | **R¹⁰** | **R¹¹** | **Phys. Chem. Daten** |
|---|---|---|---|---|---|---|---|---|---|---|---|
| I-B-Q3-001 | 3-Cl | H | 5-Cl | H | CF₃ | CH | Ac | H | H | H | |
| I-B-Q3-002 | 3-CF₃ | H | 5-CF₃ | H | CF₃ | CH | CSMe | H | H | H | |
| I-B-Q3-003 | 3-CF₃ | H | H | NH₂ | CF₃ | CH | COEt | H | H | H | |
| I-B-Q3-004 | 3-Br | H | 5-Br | NH₂ | CF₃ | CH | CH₂-2-Pyr | H | H | H | |
| I-B-Q3-005 | 3-Cl | 4-Cl | 5-Cl | Cl | CF₃ | CH | COiPr | H | H | H | |
| I-B-Q3-006 | 3-Br | 4-Cl | 5-Br | Cl | C₂F₅ | CH | COCyclpr | H | H | H | |
| I-B-Q3-007 | 3-CF₃ | 4-Cl | 5-Cl | Br | C₂F₅ | CH | SO₂Me | H | H | H | |
| I-B-Q3-008 | 3-CF₃ | 4-F | H | Br | C₂F₅ | CH | CONMe₂ | H | H | H | |
| I-B-Q3-009 | 3-Cl | 4-CF₃ | 5-Cl | I | C₂F₅ | CH | CHO | H | H | H | |
| I-B-Q3-010 | 3-CF₃ | H | 5-Cl | I | CF₃ | CH | CONHCH₂CF₃ | H | H | H | |
| I-B-Q3-011 | 3-Cl | 4-CF₃ | 5-Cl | CN | CF₃ | N | COOMe | H | H | H | |
| I-B-Q3-012 | 3-Cl | 4-Br | 5-Cl | CN | CF₃ | N | Ac | Ac | H | H | |
| I-B-Q3-013 | 3-F | 4-F | 5-F | NHCH₂Ph | C₂F₅ | CH | Ac | Ac | Me | Me | |
| I-B-Q3-014 | 2-Cl | 3-CF₃ | 4-Cl | NHAc | CF₃ | CH | COOMe | COOMe | H | H | |
| I-B-Q3-015 | 3-Cl | 4-CF₃ | H | NAc₂ | CF₃ | CH | COOMe | COOMe | Me | Me | |
| I-B-Q3-016 | 3-Br | 4-NH₂ | 5-Br | NHMe | CF₂Ph | CH | SO2Me | H | Me | Me | |
| I-B-Q3-017 | 3-Br | H | H | NMe₂ | CF₂OMe | CH | CH₂-2-Pyr | H | Me | Me | |
| I-B-Q3-018 | 3-Cl | 4-Cl | H | SMe | CF₂OPh | CH | CH₂-2-Pyr | Ac | Me | Me | |
| I-B-Q3-019 | 2-Cl | 3-Cl | 4-Cl | SOMe | CF₂(2-Pyr) | N | Me | H | H | H | |
| I-B-Q3-020 | 3-NO₂ | H | H | SO₂Me | CF₂O(2-Pyr) | CH | Ph | H | H | H | |

**Tabelle 8**

| | | | | | | | | |
|---|---|---|---|---|---|---|---|---|
| Gemäß den oben angegebenen allgemeinen Methoden lassen sich die in der folgenden Tabelle angegebenen Verbindungen der Formel (I-B mit Q = Q⁴) herstellen. | | | | | | | | |
| 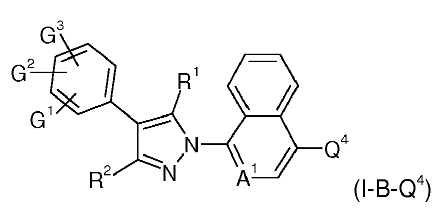 | | | | | | | | |

| **Tabelle 8 Beispiel Nr.** | **G¹** | **G²** | **G³** | **R¹** | **R³** | **A¹** | **Q⁴** | **Phys Chem. Daten** |
|---|---|---|---|---|---|---|---|---|
| I-B-Q4-001 | 3-Cl | H | 5-Cl | H | CF₃ | CH | F | |
| I-B-Q4-002 | 3-CF₃ | H | 5-CF₃ | H | CF₃ | CH | Cl | |
| I-B-Q4-003 | 3-CF₃ | H | H | NH₂ | CF₃ | CH | Br | |
| I-B-Q4-004 | 3-Br | H | 5-Br | NH₂ | CF₃ | CH | I | |
| I-B-Q4-005 | 3-Cl | 4-Cl | 5-Cl | Cl | CF₃ | CH | CN | |
| I-B-Q4-006 | 3-Br | 4-Cl | 5-Br | Cl | C₂F₅ | CH | NO₂ | |
| I-B-Q4-007 | 3-CF₃ | 4-Cl | 5-Cl | Br | C₂F₅ | CH | SMe | |
| I-B-Q4-008 | 3-CF₃ | 4-F | H | Br | C₂F₅ | CH | SCF₃ | |
| I-B-Q4-009 | 3-Cl | 4-CF₃ | 5-Cl | I | C₂F₅ | CH | SOMe | |
| I-B-Q4-010 | 3-CF₃ | H | 5-Cl | I | CF₃ | CH | SOCF₃ | |
| I-B-Q4-011 | 3-Cl | 4-CF₃ | 5-Cl | CN | CF₃ | N | SO₂Me | |
| I-B-Q4-012 | 3-Cl | 4-Br | 5-Cl | CN | CF₃ | N | SO₂CF₃ | |
| I-B-Q4-013 | 3-F | 4-F | 5-F | NHCH₂Ph | C₂F₅ | CH | CN | |
| I-B-Q4-014 | 2-Cl | 3-CF₃ | 4-Cl | NHAc | CF₃ | CH | NO₂ | |
| I-B-Q4-015 | 3-Cl | 4-CF₃ | H | NAc₂ | CF₃ | CH | F | |
| I-B-Q4-016 | 3-Br | 4-NH₂ | 5-Br | NHMe | CF₂Ph | CH | Cl | |
| I-B-Q4-017 | 3-Br | H | H | NMe₂ | CF₂OMe | CH | Br | |
| I-B-Q4-018 | 3-Cl | 4-Cl | H | SMe | CF₂OPh | CH | I | |
| I-B-Q4-019 | 2-Cl | 3-Cl | 4-Cl | SOMe | CF₂(2-Pyr) | N | COOH | |
| I-B-Q4-020 | 3-NO₂ | H | H | SO₂Me | CF₂O(2-Pyr) | CH | COOEt | |
| I-B-Q4-021 | 3-Cl | H | 5-Cl | NH₂ | CF₃ | CH | COOH | log P |

| | |
|---|---|
| **I-B-Q4-021:** | log P (HCOOH): 4,12 |

**Tabelle 9**

| | | | | | |
|---|---|---|---|---|---|
| Gemäß den oben angegebenen allgemeinen Methoden lassen sich die in der folgenden Tabelle angegebenen Hydrazine der Formel (VII-A) herstellen. | | | | | |
| 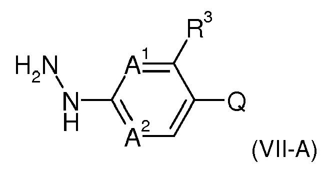 | | | | | |

| **Tabelle 9 Beispiel Nr.** | **A¹** | **A**² | **R³** | **Q** | **Phys. Chem. Daten** |
|---|---|---|---|---|---|
| VII-A-001 | CH | CH | Me | COOH | |
| VII-A-002 | N | CH | Me | COOH | |
| VII-A-003 | CH | N | Me | COOH | |
| VII-A-004 | CH | CH | Me | COOEt | |
| VII-A-005 | N | N | Me | COOMe | |
| VII-A-006 | N | CH | Me | COOMe | |
| VII-A-007 | CH | N | Me | COOMe | |
| VII-A-008 | CH | CH | Me | COOMe | log P |
| | | | | | ¹H-NMR |
| VII-A-009 | N | CH | Me | COOEt | |
| VII-A-010 | CH | N | Me | COOEt | |
| VII-A-011 | CH | CH | CN | COOH | |
| VII-A-012 | N | N | CN | COOH | |
| VII-A-013 | N | CH | CN | COOH | |
| VII-A-014 | CH | N | CN | COOH | |
| VII-A-015 | CH | CH | CN | COOMe | |
| VII-A-016 | N | N | CN | COOMe | |
| VII-A-017 | N | CH | CN | COOMe | |
| VII-A-018 | CH | N | CN | COOMe | |
| VII-A-019 | CH | CH | CN | COOEt | |
| VII-A-020 | N | N | CN | COOEt | |
| VII-A-021 | N | CH | CN | COOEt | |
| VII-A-022 | CH | N | CN | COOEt | |
| VII-A-023 | CH | CH | CF₃ | COOH | |
| VII-A-024 | N | N | CF₃ | COOH | |
| VII-A-025 | N | CH | CF₃ | COOH | |
| VII-A-026 | CH | N | CF₃ | COOH | |
| VII-A-027 | CH | CH | CF₃ | COOMe | |
| VII-A-028 | N | CH | CF₃ | COOMe | |
| VII-A-029 | CH | N | CF₃ | COOMe | |
| VII-A-030 | CH | CH | CF₃ | COOEt | |
| VII-A-031 | N | CH | CF₃ | COOEt | |
| VII-A-032 | CH | N | CF₃ | COOEt | |
| VII-A-033 | N | N | NO₂ | COOH | |
| VII-A-034 | N | CH | NO₂ | COOH | |
| VII-A-035 | CH | N | NO₂ | COOH | |
| VII-A-036 | CH | CH | NO₂ | COOMe | |
| VII-A-037 | N | N | NO₂ | COOMe | |
| VII-A-038 | N | CH | NO₂ | COOMe | |
| VII-A-039 | CH | N | NO₂ | COOMe | |
| VII-A-040 | CH | CH | NO₂ | COOEt | |
| VII-A-041 | N | N | NO₂ | COOEt | |
| VII-A-042 | N | CH | NO₂ | COOEt | |
| VII-A-043 | CH | N | NO₂ | COOEt | |
| VII-A-044 | CH | CH | Br | COOH | |
| VII-A-045 | N | N | Br | COOH | |
| VII-A-046 | N | CH | Br | COOH | |
| VII-A-047 | CH | N | Br | COOH | |
| VII-A-048 | CH | CH | Br | COOMe | |
| VII-A-049 | N | N | Br | COOMe | |
| VII-A-050 | N | CH | Br | COOMe | |
| VII-A-051 | CH | N | Br | COOMe | |
| VII-A-052 | CH | CH | Br | COOEt | |
| VII-A-053 | N | N | Br | COOEt | |
| VII-A-054 | N | CH | Br | COOEt | |
| VII-A-055 | CH | N | Br | COOEt | |
| VII-A-056 | N | N | Cl | COOH | |
| VII-A-057 | N | CH | Cl | COOH | |
| VII-A-058 | CH | N | Cl | COOH | |
| VII-A-059 | N | N | Cl | COOMe | |
| VII-A-060 | N | CH | Cl | COOMe | |
| VII-A-061 | CH | N | Cl | COOMe | |
| VII-A-062 | CH | CH | Cl | COOEt | |
| VII-A-063 | N | N | Cl | COOEt | |
| VII-A-064 | N | CH | Cl | COOEt | |
| VII-A-065 | CH | N | Cl | COOEt | |
| VII-A-066 | CH | CH | I | COOH | |
| VII-A-067 | N | N | I | COOH | |
| VII-A-068 | N | CH | I | COOH | |
| VII-A-069 | CH | N | I | COOH | |
| VII-A-070 | CH | CH | I | COOMe | |
| VII-A-071 | N | N | I | COOMe | |
| VII-A-072 | N | CH | I | COOMe | |
| VII-A-073 | CH | N | I | COOMe | |
| VII-A-074 | CH | CH | I | COOEt | |
| VII-A-075 | N | N | I | COOEt | |
| VII-A-076 | N | CH | I | COOEt | |
| VII-A-077 | CH | N | I | COOEt | |
| VII-A-078 | CH | CH | OCF₃ | COOH | |
| VII-A-079 | N | N | OCF₃ | COOH | |
| VII-A-080 | N | CH | OCF₃ | COOH | |
| VII-A-081 | CH | N | OCF₃ | COOH | |
| VII-A-082 | CH | CH | OCF₃ | COOMe | |
| VII-A-083 | N | N | OCF₃ | COOMe | |
| VII-A-084 | N | CH | OCF₃ | COOMe | |
| VII-A-085 | CH | N | OCF₃ | COOMe | |
| VII-A-086 | CH | CH | OCF₃ | COOEt | |
| VII-A-087 | N | N | OCF₃ | COOEt | |
| VII-A-088 | N | CH | OCF₃ | COOEt | |
| VII-A-089 | CH | N | OCF₃ | COOEt | |
| VII-A-090 | CH | CH | CH₂OMe | COOH | |
| VII-A-091 | N | N | CH₂OMe | COOH | |
| VII-A-092 | N | CH | CH₂OMe | COOH | |
| VII-A-093 | CH | N | CH₂OMe | COOH | |
| VII-A-094 | CH | CH | CH₂OMe | COOMe | |
| VII-A-095 | N | N | CH₂OMe | COOMe | |
| VII-A-096 | N | CH | CH₂OMe | COOMe | |
| VII-A-097 | CH | N | CH₂OMe | COOMe | |
| VII-A-098 | CH | CH | CH₂OMe | COOEt | |
| VII-A-099 | N | N | CH₂OMe | COOEt | |
| VII-A-100 | N | CH | CH₂OMe | COOEt | |
| VII-A-101 | CH | N | CH₂OMe | COOEt | |
| VII-A-102 | CH | CH | Me | 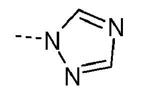 | |
| VII-A-103 | CH | CH | CN | 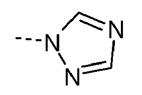 | ¹H-NMR |
| VII-A-104 | CH | CH | CF₃ | 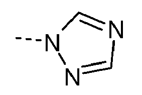 | |
| VII-A-105 | CH | CH | NO₂ | 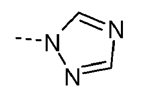 | |
| VII-A-106 | CH | CH | Br | 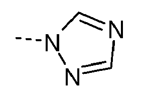 | |
| VII-A-107 | CH | CH | Cl | 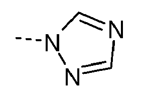 | |
| VII-A-108 | CH | CH | I | 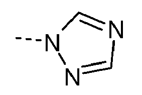 | |
| VII-A-109 | CH | CH | OCF₃ | 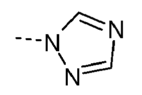 | |
| VII-A-110 | CH | CH | CH₂OMe | 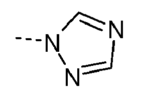 | |
| VII-A-111 | CH | CH | Me | CONH-CH₂-2-Pyr | |
| VII-A-112 | CH | CH | CN | CONH-CH₂-2-Pyr | |
| VII-A-113 | CH | **A²** CH | CF₃ | **Q** CONH-CH₂-2-Pyr | |
| VII-A-114 | CH | CH | NO₂ | CONH-CH₂-2-Pyr | |
| VII-A-115 | CH | CH | Br | CONH-CH₂-2-Pyr | |
| VII-A-116 | CH | CH | Cl | CONH-CH₂-2-Pyr | |
| VII-A-117 | CH | CH | I | CONH-CH₂-2-Pyr | |
| VII-A-118 | CH | CH | OCF₃ | CONH-CH₂-2-Pyr | |
| VII-A-119 | CH | CH | CH₂OMe | CONH-CH₂-2-Pyr | |
| VII-A-120 | CH | CH | Me | CONH-CHMe-2-Pyr | |
| VII-A-121 | CH | CH | CN | CONH-CHMe-2-Pyr | |
| VII-A-122 | CH | CH | CF₃ | CONH-CHMe-2-Pyr | |
| VII-A-123 | CH | CH | NO₂ | CONH-CHMe-2-Pyr | |
| VII-A-124 | CH | CH | Br | CONH-CHMe-2-Pyr | |
| VII-A-125 | CH | CH | Cl | CONH-CHMe-2-Pyr | |
| VII-A-126 | CH | CH | I | CONH-CHMe-2-Pyr | |
| VII-A-127 | CH | CH | OCF₃ | CONH-CHMe-2-Pyr | |
| VII-A-128 | CH | CH | CH₂OMe | CONH-CHMe-2-Pyr | |
| VII-A-129 | CH | CH | Me | CONH-TFiPr | |
| VII-A-130 | CH | CH | CN | CONH-TFiPr | |
| VII-A-131 | CH | CH | CF₃ | CONH-TFiPr | |
| VII-A-132 | CH | CH | NO₂ | CONH-TFiPr | |
| VII-A-133 | CH | CH | Br | CONH-TFiPr | |
| VII-A-134 | CH | CH | Cl | CONH-TFiPr | |
| VII-A-135 | CH | CH | I | CONH-TFiPr | |
| VII-A-136 | CH | CH | OCF₃ | CONH-TFiPr | |
| VII-A-137 | CH | CH | CH₂OMe | CONH-TFiPr | |

### Charakteristische Daten für Synthesebeispiele:

**VII-A-008:** spektroskopische Daten siehe Protokoll unter allgemeinen Synthesevorschriften
**VII-A-103:** spektroskopische Daten siehe Protokoll unter allgemeinen Synthesevorschriften

**Tabelle 10**

| | | | |
|---|---|---|---|
| Gemäß den oben angegebenen allgemeinen Methoden lassen sich die in der folgenden Tabelle angegebenen Hydrazine der Formel (VII-B) herstellen. | | | |
| 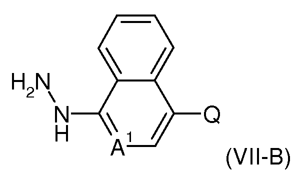 | | | |

| **Tabelle 10 Beispiel Nr.** | **A¹** | **Q** | **Phys. Chem. Daten** |
|---|---|---|---|
| VII-B-001 | N | COOH | |
| VII-B-002 | CH | COOMe | |
| VII-B-003 | CH | COOEt | |
| VII-B-004 | N | COOEt | |
| VII-B-005 | CH | COOH | |

**Tabelle 11**

| | | | | | | |
|---|---|---|---|---|---|---|
| Gemäß den oben angegebenen allgemeinen Methoden lassen sich die in der folgenden Tabelle angegebenen Verbindungen der Formel (IX) herstellen. | | | | | | |
| 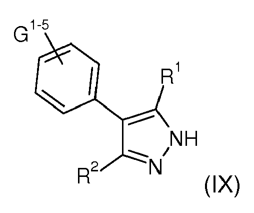 | | | | | | |

| **Tabelle 11 Beispiel Nr.** | **G¹** | **G²** | **G³** | **R¹** | **R²** | **Phys. Chem. Daten** |
|---|---|---|---|---|---|---|
| IX-001 | 3-Cl | H | 5-Cl | H | CF₃ | |
| IX-002 | 3-CF₃ | H | 5-CF₃ | H | CF₃ | |
| IX-003 | 3-CF₃ | H | H | H | CF₃ | |
| IX-004 | 3-Br | H | 5-Br | H | CF₃ | |
| IX-005 | 3-Cl | 4-Cl | 5-Cl | H | CF₃ | |
| IX-006 | 3-Br | 4-Cl | 5-Br | H | C₂F₅ | |
| IX-007 | 3-CF₃ | 4-Cl | 5-Cl | H | C₂F₅ | |
| IX-008 | 3-CF₃ | 4-F | H | H | C₂F₅ | |
| IX-009 | 3-Cl | 4-CF₃ | 5-Cl | H | C₂F₅ | |
| IX-010 | 3-CF₃ | H | 5-Cl | H | CF₃ | |
| IX-011 | 3-Cl | 4-CF₃ | 5-Cl | H | CF₃ | |
| IX-012 | 3-Cl | 4-Br | 5-Cl | H | CF₃ | |
| IX-013 | 3-F | 4-F | 5-F | H | C₂F₅ | |
| IX-014 | 2-Cl | 3-CF₃ | 4-Cl | H | CF₃ | |
| IX-015 | 3-Cl | 4-CF₃ | H | H | CF₃ | |
| IX-016 | 3-Br | 4-NH₂ | 5-Br | H | CF₂Ph | |
| IX-017 | 3-Br | H | H | H | CF₂OMe | |
| IX-018 | 3-Cl | 4-Cl | H | H | CF₂OPh | |
| IX-019 | 2-Cl | 3-Cl | 4-Cl | H | CF₂(2-Pyr) | |
| IX-020 | 3-NO₂ | H | H | H | CF₂O(2-Pyr) | |
| IX-021 | 3-Cl | H | 5-Cl | NH₂ | CF₃ | log P |
| | | | | | | ¹H-NMR |
| IX-022 | 3-CF₃ | H | 5-CF₃ | NH₂ | CF₃ | |
| IX-023 | 3-CF3 | H | H | NH₂ | CF₃ | |
| IX-024 | 3-Br | H | 5-Br | NH₂ | CF₃ | |
| IX-025 | 3-Cl | 4-Cl | 5-Cl | NH₂ | CF₃ | |
| IX-026 | 3-Br | 4-Cl | 5-Br | NH₂ | CF₃ | |
| IX-027 | 3-CF₃ | 4-Cl | 5-Cl | NH₂ | CF₃ | |
| IX-028 | 3-CF₃ | 4-F | H | NH₂ | CF₃ | |
| IX-029 | 3-Cl | 4-CF₃ | 5-Cl | NH₂ | CF₃ | |
| IX-030 | 3-CF₃ | H | 5-Cl | NH₂ | CF₃ | |
| IX-031 | 3-Cl | 4-CF₃ | 5-Cl | NH₂ | CF₃ | |
| IX-032 | 3-Cl | 4-Br | 5-Cl | NH₂ | CF₃ | |
| IX-033 | 3-F | 4-F | 5-F | NH₂ | CF₃ | |
| IX-034 | 2-Cl | 3-CF₃ | 4-Cl | NH₂ | CF₃ | |
| IX-035 | 3-Cl | 4-CF₃ | H | NH₂ | CF₃ | |
| IX-036 | 3-Br | 4-NH₂ | 5-Br | NH₂ | CF₃ | |
| IX-037 | 3-Br | H | H | NH₂ | CF₃ | |
| IX-038 | 3-Cl | 4-Cl | H | NH₂ | CF₃ | log P |
| | | | | | | ¹H-NMR |
| IX-039 | 2-Cl | 3-Cl | 4-Cl | NH₂ | CF₃ | |
| IX-040 | 3-NO₂ | H | H | NH₂ | CF₃ | |
| IX-041 | 3-Cl | H | 5-Cl | NH₂ | C₂F₅ | |
| IX-042 | 3-CF₃ | H | 5-CF₃ | NH₂ | C₂F₅ | |
| IX-043 | 3-CF₃ | H | H | NH₂ | C₂F₅ | |
| IX-044 | 3-Br | H | 5-Br | NH₂ | C₂F₅ | |
| IX-045 | 3-Cl | 4-Cl | 5-Cl | NH₂ | C₂F₅ | |
| IX-046 | 3-Br | 4-Cl | 5-Br | NH₂ | C₂F₅ | |
| IX-047 | 3-CF₃ | 4-Cl | 5-Cl | NH₂ | CF₂Ph | |
| IX-048 | 3-CF₃ | 4-F | H | NH₂ | CF₂OMe | |
| IX-049 | 3-Cl | 4-CF₃ | 5-Cl | NH₂ | CF₂OPh | |
| IX-050 | 3-CF₃ | H | 5-Cl | NH₂ | CF₂(2-Pyr) | |
| IX-051 | 3-Cl | 4-CF₃ | 5-Cl | NH₂ | CF₂O(2-Pyr) | |
| IX-052 | 3-Cl | 4-Br | 5-Cl | NH₂ | C₂F₅ | |
| IX-053 | 3-F | 4-F | 5-F | NH₂ | C₂F₅ | |
| IX-054 | 2-Cl | 3-CF₃ | 4-Cl | NH₂ | C₂F₅ | |
| IX-055 | 3-Cl | 4-CF₃ | H | NH₂ | C₂F₅ | |
| IX-056 | 3-Br | 4-NH₂ | 5-Br | NH₂ | CF₂Ph | |
| IX-057 | 3-Br | H | H | NH₂ | CF₂OMe | |
| IX-058 | 3-Cl | 4-Cl | H | NH₂ | CF₂OPh | |
| IX-059 | 2-Cl | 3-Cl | 4-Cl | NH₂ | CF₂(2-Pyr) | |
| IX-060 | 3-NO₂ | H | H | NH₂ | CF₂O(2-Pyr) | |
| IX-061 | 3-Cl | H | 5-Cl | NHAc | CF₃ | |
| IX-062 | 3-CF₃ | H | 5-CF₃ | NAc₂ | CF₃ | |
| IX-063 | 3-CF₃ | H | H | NH(C(O)OMe) | CF₃ | |
| IX-064 | 3-Br | H | 5-Br | N(C(O)OMe)₂ | CF₃ | |
| IX-065 | 3-Cl | 4-Cl | 5-Cl | NHMe | CF₃ | |
| IX-066 | 3-Br | 4-Cl | 5-Br | NMe₂ | C₂F₅ | |
| IX-067 | 3-CF₃ | 4-Cl | 5-Cl | NH(CH₂Ph) | C₂F₅ | |
| IX-068 | 3-CF₃ | 4-F | H | N(CH₂Ph)₂ | C₂F₅ | |
| IX-069 | 3-Cl | 4-CF₃ | 5-Cl | NH(CH₂-2-Pyr) | C₂F₅ | |
| IX-070 | 3-CF₃ | H | 5-Cl | N(CH₂-2-Pyr)₂ | CF₃ | |
| IX-071 | 3-Cl | 4-CF₃ | 5-Cl | NH(SO₂Me) | CF₃ | |
| IX-072 | 3-Cl | 4-Br | 5-Cl | NH(SO₂CF₃) | CF₃ | |
| IX-073 | 3-F | 4-F | 5-F | NHCypr | C₂F₅ | |
| IX-074 | 2-Cl | 3-CF₃ | 4-Cl | NHEt | CF₃ | |
| IX-075 | 3-Cl | 4-CF₃ | H | NHPh | CF₃ | |
| IX-076 | 3-Br | 4-NH₂ | 5-Br | NH(C(O)OtBu) | CF₂Ph | |
| IX-077 | 3-Br | H | H | NH(C(O)tBu | CF₂OMe | |
| IX-078 | 3-Cl | 4-Cl | H | NHtBu | CF₂OPh | |
| IX-079 | 2-Cl | 3-Cl | 4-Cl | NH(CH₂-2-Pyrim) | CF₂(2-Pyr) | |
| IX-080 | 3-NO₂ | H | H | NH(CH₂-2-Pyraz) | CF₂O(2-Pyr) | |
| IX-081 | 3-Cl | H | 5-Cl | NHAc | CF₃ | |
| IX-082 | 3-CF₃ | H | 5-CF₃ | NAc₂ | CF₃ | |
| IX-083 | 3-CF₃ | H | H | NH(C(O)OMe) | CF₃ | |
| IX-084 | 3-Br | H | 5-Br | N(C(O)OMe)₂ | CF₃ | |
| IX-085 | 3-Cl | 4-Cl | 5-Cl | NHMe | CF₃ | |
| IX-086 | 3-Br | 4-Cl | 5-Br | NMe₂ | C₂F₅ | |
| IX-087 | 3-CF₃ | 4-Cl | 5-Cl | NH(CH₂Ph) | C₂F₅ | |
| IX-088 | 3-CF₃ | 4-F | H | N(CH₂Ph)₂ | C₂F₅ | |
| IX-089 | 3-Cl | 4-CF₃ | 5-Cl | NH(CH₂-2-Pyr) | C₂F₅ | |
| IX-090 | 3-CF₃ | H | 5-Cl | N(CH₂-2-Pyr)₂ | CF₃ | |
| IX-091 | 3-Cl | 4-CF₃ | 5-Cl | NH(SO₂Me) | CF₃ | |
| IX-092 | 3-Cl | 4-Br | 5-Cl | NH(SO₂CF₃) | CF₃ | |
| IX-093 | 3-F | 4-F | 5-F | NHCypr | C₂F₅ | |
| IX-094 | 2-Cl | 3-CF₃ | 4-Cl | NHEt | CF₃ | |
| IX-095 | 3-Cl | 4-CF₃ | H | NHPh | CF₃ | |
| IX-096 | 3-Br | 4-NH₂ | 5-Br | NH(C(O)OtBu) | CF₂Ph | |
| IX-097 | 3-Br | H | H | NH(C(O)tBu | CF₂OMe | |
| IX-098 | 3-Cl | 4-Cl | H | NHiPr | CF₂OPh | |
| IX-099 | 2-Cl | 3-Cl | 4-Cl | NH(CH₂-2-Pyrim) | CF₂(2-Pyr) | |
| IX-100 | 3-NO₂ | H | H | NH(CH₂-2-Pyraz) | CF₂O(2-Pyr) | |
| IX-101 | 3-Cl | H | 5-Cl | Cl | CF₃ | |
| IX-102 | 3-CF₃ | H | 5-CF₃ | Cl | CF₃ | |
| IX-103 | 3-CF₃ | H | H | Cl | CF₃ | |
| IX-104 | 3-Br | H | 5-Br | Cl | CF₃ | |
| IX-105 | 3-Cl | 4-Cl | 5-Cl | Cl | CF₃ | |
| IX-106 | 3-Br | 4-Cl | 5-Br | Cl | C₂F₅ | |
| IX-107 | 3-CF₃ | 4-Cl | 5-Cl | Cl | C₂F₅ | |
| IX-108 | 3-CF₃ | 4-F | H | Cl | C₂F₅ | |
| IX-109 | 3-Cl | 4-CF₃ | 5-Cl | Cl | C₂F₅ | |
| IX-110 | 3-CF₃ | H | 5-Cl | Cl | CF₃ | |
| IX-111 | 3-Cl | 4-CF₃ | 5-Cl | Cl | CF₃ | |
| IX-112 | 3-Cl | 4-Br | 5-Cl | Cl | CF₃ | |
| IX-113 | 3-F | 4-F | 5-F | Cl | C₂F₅ | |
| IX-114 | 2-Cl | 3-CF₃ | 4-Cl | Cl | CF₃ | |
| IX-115 | 3-Cl | 4-CF₃ | H | Cl | CF₃ | |
| IX-116 | 3-Br | 4-NH₂ | 5-Br | Cl | CF₂Ph | |
| IX-117 | 3-Br | H | H | Cl | CF₂OMe | |
| IX-118 | 3-Cl | 4-Cl | H | Cl | CF₂OPh | |
| IX-119 | 2-Cl | 3-Cl | 4-Cl | Cl | CF₂(2-Pyr) | |
| IX-120 | 3-NO₂ | H | H | Cl | CF₂O(2-Pyr) | |
| IX-121 | 3-Cl | H | 5-Cl | Br | CF₃ | |
| IX-122 | 3-CF₃ | H | 5-CF₃ | Br | CF₃ | |
| IX-123 | 3-CF₃ | H | H | Br | CF₃ | |
| IX-124 | 3-Br | H | 5-Br | Br | CF₃ | |
| IX-125 | 3-Cl | 4-Cl | 5-Cl | Br | CF₃ | |
| IX-126 | 3-Br | 4-Cl | 5-Br | Br | C₂F₅ | |
| IX-127 | 3-CF₃ | 4-Cl | 5-Cl | Br | C₂F₅ | |
| IX-128 | 3-CF₃ | 4-F | H | Br | C₂F₅ | |
| IX-129 | 3-Cl | 4-CF₃ | 5-Cl | Br | C₂F₅ | |
| IX-130 | 3-CF₃ | H | 5-Cl | Br | CF₃ | |
| IX-131 | 3-Cl | 4-CF₃ | 5-Cl | Br | CF₃ | |
| IX-132 | 3-Cl | 4-Br | 5-Cl | Br | CF₃ | |
| IX-133 | 3-F | 4-F | 5-F | Br | C₂F₅ | |
| IX-134 | 2-Cl | 3-CF₃ | 4-Cl | Br | CF₃ | |
| IX-135 | 3-Cl | 4-CF₃ | H | Br | CF₃ | |
| IX-136 | 3-Br | 4-NH₂ | 5-Br | Br | CF₂Ph | |
| IX-137 | 3-Br | H | H | Br | CF₂OMe | |
| IX-138 | 3-Cl | 4-Cl | H | Br | CF₂OPh | |
| IX-139 | 2-Cl | 3-Cl | 4-Cl | Br | CF₂(2-Pyr) | |
| IX-140 | 3-NO₂ | H | H | Br | CF₂O(2-Pyr) | |
| IX-141 | 3-Cl | H | 5-Cl | I | CF₃ | |
| IX-142 | 3-CF₃ | H | 5-CF₃ | I | CF₃ | |
| IX-143 | 3-CF₃ | H | H | I | CF₃ | |
| IX-144 | 3-Br | H | 5-Br | I | CF₃ | |
| IX-145 | 3-Cl | 4-Cl | 5-Cl | I | CF₃ | |
| IX-146 | 3-Br | 4-Cl | 5-Br | I | C₂F₅ | |
| IX-147 | 3-CF₃ | 4-Cl | 5-Cl | I | C₂F₅ | |
| IX-148 | 3-CF₃ | 4-F | H | I | C₂F₅ | |
| IX-149 | 3-Cl | 4-CF₃ | 5-Cl | I | C₂F₅ | |
| IX-150 | 3-CF₃ | H | 5-Cl | I | CF₃ | |
| IX-151 | 3-Cl | 4-CF₃ | 5-Cl | I | CF₃ | |
| IX-152 | 3-Cl | 4-Br | 5-Cl | I | CF₃ | |
| IX-153 | 3-F | 4-F | 5-F | I | C₂F₅ | |
| IX-154 | 2-Cl | 3-CF₃ | 4-Cl | I | CF₃ | |
| IX-155 | 3-Cl | 4-CF₃ | H | I | CF₃ | |
| IX-156 | 3-Br | 4-NH₂ | 5-Br | I | CF₂Ph | |
| IX-157 | 3-Br | H | H | I | CF₂OMe | |
| IX-158 | 3-Cl | 4-Cl | H | I | CF₂OPh | |
| IX-159 | 2-Cl | 3-Cl | 4-Cl | I | CF₂(2-Pyr) | |
| IX-160 | 3-NO₂ | H | H | I | CF₂O(2-Pyr) | |
| IX-161 | 3-Cl | H | 5-Cl | CN | CF₃ | |
| IX-162 | 3-CF₃ | H | 5-CF₃ | CN | CF₃ | |
| IX-163 | 3-CF₃ | H | H | CN | CF₃ | |
| IX-164 | 3-Br | H | 5-Br | CN | CF₃ | |
| IX-165 | 3-Cl | 4-Cl | 5-Cl | CN | CF₃ | |
| IX-166 | 3-Br | 4-Cl | 5-Br | CN | C₂F₅ | |
| IX-167 | 3-CF₃ | 4-Cl | 5-Cl | CN | C₂F₅ | |
| IX-168 | 3-CF₃ | 4-F | H | CN | C₂F₅ | |
| IX-169 | 3-Cl | 4-CF₃ | 5-Cl | CN | C₂F₅ | |
| IX-170 | 3-CF₃ | H | 5-Cl | CN | CF₃ | |
| IX-171 | 3-Cl | 4-CF₃ | 5-Cl | CN | CF₃ | |
| IX-172 | 3-Cl | 4-Br | 5-Cl | CN | CF₃ | |
| IX-173 | 3-F | 4-F | 5-F | CN | C₂F₅ | |
| IX-174 | 2-Cl | 3-CF₃ | 4-Cl | CN | CF₃ | |
| IX-175 | 3-Cl | 4-CF₃ | H | CN | CF₃ | |
| IX-176 | 3-Br | 4-NH₂ | 5-Br | CN | CF₂Ph | |
| IX-177 | 3-Br | H | H | CN | CF₂OMe | |
| IX-178 | 3-Cl | 4-Cl | H | CN | CF₂OPh | |
| IX-179 | 2-Cl | 3-Cl | 4-Cl | CN | CF₂(2-Pyr) | |
| IX-180 | 3-NO₂ | H | H | CN | CF₂O(2-Pyr) | |
| IX-181 | 3-Cl | H | 5-Cl | SMe | CF₃ | |
| IX-182 | 3-CF₃ | H | 5-CF₃ | SOMe | CF₃ | |
| IX-183 | 3-CF₃ | H | H | SO₂Me | CF₃ | |
| IX-184 | 3-Br | H | 5-Br | SMe | CF₃ | |
| IX-185 | 3-Cl | 4-Cl | 5-Cl | SOMe | CF₃ | |
| IX-186 | 3-Br | 4-Cl | 5-Br | SO₂Me | C₂F₅ | |
| IX-187 | 3-CF₃ | 4-Cl | 5-Cl | SMe | C₂F₅ | |
| IX-188 | 3-CF₃ | 4-F | H | SOMe | C₂F₅ | |
| IX-189 | 3-Cl | 4-CF₃ | 5-Cl | SO₂Me | C₂F₅ | |
| IX-190 | 3-CF₃ | H | 5-Cl | SMe | CF₃ | |
| IX-191 | 3-Cl | 4-CF₃ | 5-Cl | SOMe | CF₃ | |
| IX-192 | 3-Cl | 4-Br | 5-Cl | SO₂Me | CF₃ | |
| IX-193 | 3-F | 4-F | 5-F | SMe | C₂F₅ | |
| IX-194 | 2-Cl | 3-CF₃ | 4-Cl | SOMe | CF₃ | |
| IX-195 | 3-Cl | 4-CF₃ | H | SO₂Me | CF₃ | |
| IX-196 | 3-Br | 4-NH₂ | 5-Br | SMe | CF₂Ph | |
| IX-197 | 3-Br | H | H | SOMe | CF₂OMe | |
| IX-198 | 3-Cl | 4-Cl | H | SO₂Me | CF₂OPh | |
| IX-199 | 2-Cl | 3-Cl | 4-Cl | SMe | CF₂(2-Pyr) | |
| IX-200 | 3-NO₂ | H | H | SOMe | CF₂O(2-Pyr) | |
| IX-201 | 3-F | 4-F | H | NH₂ | CF₃ | log P |
| IX-202 | H | 4-F | H | NH₂ | CF₃ | log P |
| IX-203 | 3-Cl | H | 5-Cl | NHAc | CF₃ | log P |
| IX-204 | 2-Cl | 4-Cl | H | NHAc | CF₃ | log P |
| | | | | | | ¹H-NMR |

### Charakteristische Daten für Synthesebeispiele:

| | |
|---|---|
| **IX-021:** | log P (HCOOH): 2,91 |
| | |
| | ¹H NMR (DMSO-d6): δ = 12,32 (s, 1 H), 7,45 (s, 1 H), 7,25 (s, 2), 5,32 (s, 2 H) ppm |
| | |
| **IX-38:** | log P (HCOOH): 2.80 |
| | |
| | ¹H NMR (DMSO-d6): δ = 5.27 (br. s, 2H); 7.25 (dd, 1H); 7.46 (d, 1H); 7.61 (d, 1H) ppm |
| | |
| **IX-201:** | log P (HCOOH): 2.33 |
| | |
| **IX-202:** | log P (HCOOH): 2.16 |
| | |
| **IX-203:** | log P (HCOOH): 2.98 |
| **IX-204:** | log P (HCOOH): 2.79 |
| | |
| | ¹H NMR (DMSO-d6): δ = 1.99 (s, 3H); 7.32 (d, 1H); 7.47 (d, 1H); 7.67 (s, 1H); 9.98 (s, 1H, NH); 13.53 (s, 1H, NH) ppm |

**Tabelle 12**

| | | | | | | | | |
|---|---|---|---|---|---|---|---|---|
| Gemäß den oben angegebenen allgemeinen Methoden lassen sich die in der folgenden Tabelle angegebenen Verbindungen der Formel (XI-A) herstellen. | | | | | | | | |
| 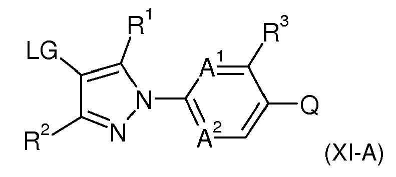 | | | | | | | | |

| **Tabelle 12 Beispiel Nr.** | **LG** | **R¹** | **R²** | **R³** | **A¹** | **A²** | **Q** | **Phys. Chem. Daten** |
|---|---|---|---|---|---|---|---|---|
| XI-A-001 | I | H | CF₃ | CF₃ | CH | CH | COOEt | ¹H-NMR |
| XI-A-002 | Br | H | CF₃ | Me | CH | CH | COOEt | |
| XI-A-003 | Br | H | CF₃ | CN | CH | CH | COOH | |
| XI-A-004 | Br | NH₂ | CF₃ | NO₂ | CH | CH | Br | |
| XI-A-005 | Br | NH₂ | CF₃ | CF₃ | CH | CH | I | |
| XI-A-006 | Br | Cl | CF₃ | Br | CH | CH | CN | |
| XI-A-007 | Br | Cl | C₂F₅ | Cl | CH | CH | NO₂ | |
| XI-A-008 | Br | Br | C₂F₅ | CH₂OM | CH | CH | SMe | |
| XI-A-009 | Br | Br | C₂F₅ | CH₂NHMe | CH | CH | SCF₃ | |
| XI-A-010 | Br | I | C₂F₅ | Me | CH | CH | SOMe | |
| XI-A-011 | Br | I | CF₃ | CN | CH | CH | SOCF₃ | |
| XI-A-012 | Br | CN | CF₃ | NO₂ | N | N | SO₂Me | |
| XI-A-013 | Br | CN | CF₃ | CF₃ | N | N | SO₂CF₃ | |
| XI-A-014 | Br | NHCH₂Ph | C₂F₅ | Br | CH | CH | CN | |
| XI-A-015 | Br | NHAc | CF₃ | Cl | CH | CH | NO₂ | |
| XI-A-016 | Br | NAc₂ | CF₃ | CH₂OAc | CH | CH | F | |
| XI-A-017 | Br | NHMe | CF₂Ph | CH₂NHAc | CH | CH | Cl | |
| XI-A-018 | Br | NH₂ | CF₃ | H | CH | CH | COOEt | log P |
| | | | | | | | | ¹H-NMR |
| XI-A-019 | Br | SMe | CF₂Oph | CN | CH | CH | CONH-CH₂-Pyr | |
| XI-A-020 | Br | SOMe | CF₂(2-Pyr) | NO2 | N | CH | CONH-CHMe-2-Pyr | |
| XI-A-021 | Br | SO₂Me | CF₂O(2-Pyr) | CF₃ | CH | N | CONH-TfiPr | |
| XI-A-022 | I | H | CF₃ | Me | CH | CH | COOEt | |
| XI-A-023 | I | H | CF₃ | CN | CH | CH | COOH | |
| XI-A-024 | I | NH₂ | CF₃ | NO₂ | CH | CH | Br | |
| XI-A-025 | I | NH₂ | CF₃ | CF₃ | CH | CH | I | |
| XI-A-026 | I | Cl | CF₃ | Br | CH | CH | CN | |
| XI-A-027 | I | Cl | C₂F₅ | Cl | CH | CH | NO₂ | |
| XI-A-028 | I | Br | C₂F₅ | CH₂OMe | CH | CH | SMe | |
| XI-A-029 | I | Br | C₂F₅ | CH₂NHMe | CH | CH | SCF₃ | |
| XI-A-030 | I | I | C₂F₅ | Me | CH | CH | SOMe | |
| XI-A-031 | I | I | CF₃ | CN | CH | CH | SOCF₃ | |
| XI-A-032 | I | CN | CF₃ | NO₂ | N | N | SO₂Me | |
| XI-A-033 | I | CN | CF₃ | CF₃ | N | N | SO₂CF₃ | |
| XI-A-034 | I | NHCH₂Ph | C₂F₅ | Br | CH | CH | CN | |
| XI-A-035 | I | NHAc | CF₃ | Cl | CH | CH | NO₂ | |
| XI-A-036 | I | NAc₂ | CF₃ | CH₂OAc | CH | CH | F | |
| XI-A-037 | I | NHMe | CF₂Ph | CH₂NHAc | CH | CH | Cl | |
| XI-A-038 | Br | NH₂ | CF₃ | H | CH | CH | COOH | log P |
| | | | | | | | | ¹H-NMR |
| XI-A-039 | I | SMe | CF₂Oph | CN | CH | CH | CONH-CH₂-2-Pyr | |
| XI-A-040 | I | SOMe | CF₂(2-Pyr) | NO2 | N | CH | CONH-CHMe-2-Pyr | |
| XI-A-041 | I | SO₂Me | CF₂O(2-Pyr) | CF₃ | CH | N | CONH-TfiPr | |
| XI-A-042 | Cl | NH₂ | CF₃ | H | CH | CH | COOEt | log P |
| | | | | | | | | ¹H-NMR |
| XI-A-043 | Cl | NH₂ | CF₃ | H | CH | CH | COOH | log P |
| | | | | | | | | ¹H-NMR |
| XI-A-044 | I | NH₂ | CF₃ | H | CH | CH | COOEt | log P |
| | | | | | | | | ¹H-NMR |
| XI-A-045 | I | NH₂ | CF₃ | H | CH | CH | COOH | log P |
| | | | | | | | | ¹H-NMR |
| XI-A-046 | Cl | NH₂ | CF₃ | H | CH | CH | CO-NH-Me | ¹H-NMR |
| XI-A-047 | Cl | NH₂ | CF₃ | H | CH | CH | CO-NMe₂ | ¹H-NMR |
| XI-A-048 | Cl | NH₂ | CF₃ | H | CH | CH | CONH-Et | ¹H-NMR |
| XI-A-049 | Cl | NH₂ | CF₃ | H | CH | CH | CONH-Pr | ¹H-NMR |
| XI-A-050 | Cl | NH₂ | CF₃ | H | CH | CH | CONH-iPr | ¹H-NMR |
| XI-A-051 | Cl | NH₂ | CF₃ | H | CH | CH | CONH-Cypr | ¹H-NMR |
| XI-A-052 | Cl | NH₂ | CF₃ | H | CH | CH | CONH-TFiPr | ¹H-NMR |
| XI-A-053 | Cl | NH₂ | CF₃ | H | CH | CH | CONH-tBu | ¹H-NMR |
| XI-A-054 | Cl | NH₂ | CF₃ | H | CH | CH | CONH-CHMe-tBu | ¹H-NMR |
| XI-A-055 | Cl | NH₂ | CF₃ | H | CH | CH | CONH-3-Pyr | ¹H-NMR |
| XI-A-056 | Cl | NH₂ | CF₃ | H | CH | CH | CONH-CH₂-2-Pyr | ¹H-NMR |
| XI-A-057 | Br | NH₂ | CF₃ | H | CH | CH | CO-NH-Me | ¹H-NMR |
| XI-A-058 | Br | NH₂ | CF₃ | H | CH | CH | CO-NMe₂ | ¹H-NMR |
| XI-A-059 | Br | NH₂ | CF₃ | H | CH | CH | CONH-Et | ¹H-NMR |
| XI-A-060 | Br | NH₂ | CF₃ | H | CH | CH | CONH-Pr | ¹H-NMR |
| XI-A-061 | Br | NH₂ | CF₃ | H | CH | CH | CONH-iPr | ¹H-NMR |
| XI-A-062 | Br | NH₂ | CF₃ | H | CH | CH | CONH-Cypr | ¹H-NMR |
| XI-A-063 | Br | NH₂ | CF₃ | H | CH | CH | CONH-TFiPr | ¹H-NMR |
| XI-A-064 | Br | NH₂ | CF₃ | H | CH | CH | CONH-tBu | ¹H-NMR |
| XI-A-065 | Br | NH₂ | CF₃ | H | CH | CH | CONH-CHMe-tBu | ¹H-NMR |
| XI-A-066 | Br | NH₂ | CF₃ | H | CH | CH | CONH-3-Pyr | ¹H-NMR |
| XI-A-067 | Br | NH₂ | CF₃ | H | CH | CH | CONH-CH₂-2-Pyr | ¹H-NMR |
| XI-A-068 | I | NH₂ | CF₃ | H | CH | CH | CO-NH-Me | ¹H-NMR |
| XI-A-069 | I | NH₂ | CF₃ | H | CH | CH | CO-NMe₂ | ¹H-NMR |
| XI-A-070 | I | NH₂ | CF₃ | H | CH | CH | CONH-Et | ¹H-NMR |
| XI-A-071 | I | NH₂ | CF₃ | H | CH | CH | CONH-Pr | ¹H-NMR |
| XI-A-072 | I | NH₂ | CF₃ | H | CH | CH | CONH-iPr | ¹H-NMR |
| XI-A-073 | I | NH₂ | CF₃ | H | CH | CH | CONH-Cypr | ¹H-NMR |
| XI-A-074 | I | NH₂ | CF₃ | H | CH | CH | CONH-TFiPr | ¹H-NMR |
| XI-A-075 | I | NH₂ | CF₃ | H | CH | CH | CONH-tBu | ¹H-NMR |
| XI-A-076 | I | NH₂ | CF₃ | H | CH | CH | CONH-CHMe-tBu | ¹H-NMR |
| XI-A-077 | I | NH₂ | CF₃ | H | CH | CH | CONH-3-Pyr | ¹H-NMR |
| XI-A-078 | I | NH₂ | CF₃ | H | CH | CH | CONH-CH₂-2-Pyr | ¹H-NMR |
| XI-A-079 | H | NH₂ | CF₃ | CN | CH | CH | 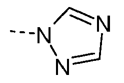 | log P |
| XI-A-080 | H | NH₂ | CF₃ | Me | CH | CH | COOMe | log P |
| XI-A-081 | Cl | NH₂ | CF₃ | Me | CH | CH | COOMe | log P |
| XI-A-082 | Br | NH₂ | CF₃ | Me | CH | CH | COOMe | log P |
| XI-A-083 | I | NH₂ | CF₃ | Me | CH | CH | COOMe | log P |
| XI-A-084 | H | NH₂ | CF₃ | Me | CH | CH | COOH | log P |
| XI-A-085 | Cl | NH₂ | CF₃ | Me | CH | CH | COOH | log P |
| XI-A-086 | Br | NH₂ | CF₃ | Me | CH | CH | COOH | log P |
| XI-A-087 | I | NH₂ | CF₃ | Me | CH | CH | COOH | log P |
| XI-A-08 | H | NH₂ | CF₃ | Me | CH | CH | CONHCH₂CF₂CF₂H | log P |
| XI-A-089 | H | NH₂ | CF₃ | Me | CH | CH | CONHCH₂-2-Pyr | log P |
| XI-A-090 | Cl | NH₂ | CF₃ | Me | CH | CH | CONHCH₂CF₂CF₂H | log P |
| XI-A-091 | Cl | NH₂ | CF₃ | Me | CH | CH | CONHCH₂-2-Pyr | log P |
| XI-A-092 | Br | NH₂ | CF₃ | Me | CH | CH | CONHCH₂CF₂CF₂H | log P |
| XI-A-093 | Br | NH₂ | CF₃ | Me | CH | CH | CONHCH₂-2-Pyr | log P |
| XI-A-094 | I | NH₂ | CF₃ | Me | CH | CH | CONHCH₂CF₂CF₂H | log P |
| XI-A-095 | I | NH₂ | CF₃ | Me | CH | CH | CONHCH₂-2-Pyr | log P |

### Charakteristische Daten für Synthesebeispiele:

¹H-NMR-Daten (400 MHz., interner Standard: Tetramethylsilan δ = 0.00 ppm; s = Singulett, br. s = breites Singulett, d = Dublett, dd = Doppeldublett, m = Multiplett, q = Quartett, t = Triplett)

| | | | | | |
|---|---|---|---|---|---|
| **XI-A-001:** | spektroskopische Daten siehe Protokoll unter allgemeinen Synthesevorschriften | | | | |
| | | | | | |
| **XI-A-018:** | log P (HCOOH): 3.60 | | | | |
| | | | | | |
| | ¹H NMR (DMSO-d6): δ = 1.35 (t, 3H); 4.36 (q, 2H); 5.88 (br. s, 2H); 7.74 (d, 2H); 8.10 (d, 2H) ppm | | | | |
| | | | | | |
| **XI-A-038:** | log P (HCOOH): 2,38 | | | | |
| | | | | | |
| | ¹H NMR (DMSO-d6): δ = 4.70 (br. s, 2H); 7.68 (d, 2H); 8.15 (d, 2H) ppm | | | | |
| | | | | | |
| **XI-A-042:** | log P (HCOOH): 3.52 | | | | |
| | | | | | |
| | ¹H NMR (CD₃CN): δ = 1.37 (t, 3H); 4.38 (q, 2H); 4.71 (br.s, 2H); 7.68 (d, 2H); 8.14 (d, 2H) ppm | | | | |
| | | | | | |
| **XI-A-043:** | log P (HCOOH): 2.34 | | | | |
| | | | | | |
| | ¹H NMR (CD₃CN): δ = 4.70 (br.s, 2H); 7.68 (d, 2H); 8.15 (d, 2H) ppm | | | | |
| | | | | | |
| **XI-A-044:** | log P (HCOOH): 3.66 | | | | |
| | | | | | |
| | ¹H NMR (DMSO-d6): δ = 1.35 (t, 3H); 4.34 (q, 2H); 5.70 (br.s, 2H); 7.73 (d, 2H); 8.09 (d, 2H) ppm | | | | |
| **XI-A-045:** | log P (HCOOH): 2.47 | | | | |
| | | | | | |
| | ¹H NMR (DMSO-d6): δ = 5.69 (br.s, 2H); 7.70 (d, 2H); 8.07 (d, 2H); 12.9 (br. s, 1H) ppm | | | | |
| | | | | | |
| **XI-A-046:** | ¹H NMR (CD₃CN): δ = 2.89 (d,3H); 4.66 (br. s, 2H); 6.95 (br. S, 1H); 7.63 (d, 2H); 7.92 (d, 2H) ppm | | | | |
| | | | | | |
| **XI-A-047:** | ¹H NMR (CD₃CN): δ = 2.99 (br. s, 6H); 4.66 (br. s, 2H); 7.53-7.60 (m, 4H) ppm | | | | |
| | | | | | |
| **XI-A-048:** | ¹H NMR (CD₃CN): δ = 1.20 (t, 3H); 3.40 (m, 2H); 4.66 (br.s, 2H); 6.99 (br. s, 1H); 7.62 (d, 2H); 7.93 (d, 2H) ppm | | | | |
| | | | | | |
| **XI-A-049:** | ¹H NMR (CD₃CN): δ = 0.96 (t, 3H); 1.62 (m, 2H); 3.33 (m, 2H); 4.66 (br. s, 2H); 6.99 (br. s, 1H); 7.62 (d, 2H); 7.93 (d, 2H) ppm | | | | |
| | | | | | |
| **XI-A-050:** | ¹H NMR (CD₃CN): δ = 1.24 (d, 6H); 4.19 (m, 1H); 4.66 (br.s, 2H); 6.80 (br. s, 1H); 7.62 (d, 2H); 7.93 (d, 2H) ppm | | | | |
| | | | | | |
| **XI-A-051:** | ¹H NMR (CD₃CN): δ = 0.62 (m, 2H); 0.76 (m, 2H); 2.86 (m, 1H); 4.68 (br. s, 2H); 7.06 (br. s, 1H); 7.62 (d, 2H); 7.90 (d, 2H) ppm | | | | |
| | | | | | |
| **XI-A-052:** | ¹H NMR (CD₃CN): δ = 1.43 (d, 3H); 4.82 (br. s, 2H); 4.93 (m, 1H); 7.40 (br. d, 1H); 7.68 (d, 2H); 7.99 (d, 2H) ppm | | | | |
| | | | | | |
| **XI-A-053:** | ¹H NMR (CD₃CN): δ = 1.44 (s, 9H); 4.80 (br. s, 2H); 6.70 (br. s, 1H); 7.60 (d, 2H); 7.91 (d, 2H) ppm | | | | |
| | | | | | |
| **XI-A-054:** | ¹H NMR (CD₃CN): δ = 0.96 (s, 9H); 1.15 (d, 3H); 4.05 (m, 1H); 4.80 (br. s, 2H); 6.78 (br. d, 1H); 7.63 (d, 2H); 7.94 (d, 2H) ppm | | | | |
| | | | | | |
| **XI-A-055:** | ¹H NMR (CD₃CN): δ = 4.71 (br. s, 2H); 7.35 (m, 1H); 7.73 (d, 2H); 8.10 (d, 2H); 8.14 (m, 1H); 8.34 (m, 1H); 8.85 (br., 2H) ppm | | | | |
| | | | | | |
| **XI-A-056:** | ¹H NMR (CD₃CN): δ = 4.68 (d, 2H); 4.82 (br. s, 2H); 7.29 (m, 1H); 7.40 (d, 1H); 7.67 (d, 2H); 7.77 (m, 1H); 7.88 (br. m, 1H); 8.02 (d, 2H); 8.53 (m, 1H) ppm | | | | |
| **XI-A-057:** | ¹H NMR (DMSO-d6): δ = 2.82 (d, 3H); 5.80 (br. s, 2H); 7.66 (d, 2H); 7.98 (d, 2H); 8.37 (br. s, 1H) ppm | | | | |
| | | | | | |
| **XI-A-058:** | ¹H NMR (DMSO-d6): δ = 2.98 (s, 6H); 5.80 (br. s, 2H); 7.54-7.63 (m, 4H) ppm | | | | |
| | | | | | |
| **XI-A-059:** | 1H NMR (DMSO-d6): δ = 1.15 (t, 3H); 3.32 (m, 2H); 5.79 (br. s, 2H); 7.65 (d, 2H); 7.99 (d, 2H); 8.40 (br. t, 1H) ppm | | | | |
| | | | | | |
| **XI-A-060:** | ¹H NMR (DMSO-d6): δ = 0.91 (t, 3H); 1.57 (m, 2H); 3.25 (m, 2H); 5.79 (br. s, 2H); 7.65 (d, 2H); 8.00 (d, 2H); 8.38 (br. t, 1H) ppm | | | | |
| | | | | | |
| **XI-A-061:** | ¹H NMR (DMSO-d6): δ = 1.20 (d, 6H); 4.12 (m, 1H); 5.78 (br. s, 2H); 7.64 (d, 2H); 8.00 (d, 2H); 8.15 (br. d, 1H) ppm | | | | |
| | | | | | |
| **XI-A-062:** | ¹H NMR (DMSO-d6): δ = 0.60 (m, 2H); 0.71 (m, 2H); 2.90 (m, 1H); 5.79 (br. s, 2H); 7.64 (d, 2H); 7.97 (d, 2H); 8.38 (br. s, 1H) ppm | | | | |
| | | | | | |
| **XI-A-063:** | ¹H NMR (DMSO-d6): δ = 1.39 (d, 3H); 4.88 (m, 1H); 5.81 (br. s, 2H); 7.70 (d, 2H); 8.05 (d, 2H); 8.82 (br. d, 1H) ppm | | | | |
| | | | | | |
| **XI-A-064:** | ¹H NMR (DMSO-d6): δ = 1.40 (s, 9H); 5.76 (br. s, 2H); 7.62 (d, 2H); 7.67 (br. s, 1H); 7.96 (d, 2H) ppm | | | | |
| | | | | | |
| **XI-A-065:** | ¹H NMR (DMSO-d6): δ = 0.92 (s, 9H); 1.11 (d, 3H); 4.01 (m, 1H); 5.78 (br. s, 2H); 7.64 (d, 2H); 7.87 (br. d, 1H); 8.01 (d, 2H) ppm | | | | |
| | | | | | |
| **XI-A-066:** | ¹H NMR (DMSO-d6): δ = 5.7-6.0 (br. Signal, 2H); 7.55 (m, 1H); 7.78 (d, 2H); 8.17 (d, 2H); 8.34 (m, 1H); 8.40 (m, 1H); 9.06 (br. s, 1H); 10.62 (s, 1H) ppm | | | | |
| | | | | | |
| **XI-A-067:** | ¹H NMR (DMSO-d6): δ = 4.61 (d, 2H); 5.82 (br. s, 2H); 7.25 (m, 1H); 7.34 (m, 1H); 7.67 (d, 2H); ~7.5 (m, 1H); 8.08 (d, 2H); 8.51 (m, 1H); 9.03 (br. t, 1H) ppm | | | | |
| | | | | | |
| **XI-A-068:** | ¹H NMR (DMSO-d6): δ = 2.82 (d, 3H); 5.62 (br. s, 2H); 7.64 (d, 2H); 7.98 (d, 2H); 8.36 (br. s, 1H) ppm | | | | |
| | | | | | |
| **XI-A-069:** | ¹H NMR (DMSO-d6): δ = 2.98 (s, 6H); 5.63 (br. s, 2H); 7.53-7.73 (m, 4H) ppm | | | | |
| **XI-A-070:** | ¹H NMR (DMSO-d6): δ = 1.15 (t, 3H); 3.32 (m, 2H); 5.61 (br. s, 2H); 7.64 (d, 2H); 7.99 (d, 2H); 8.39 (br. s, 1H) ppm | | | | |
| | | | | | |
| **XI-A-071:** | ¹H NMR (DMSO-d6): δ = 0.91 (t, 3H); 1.55 (m, 2H); 3.26 (m, 2H); 5.61 (br. s, 2H); 7.64 (d, 2H); 7.99 (d, 2H); 8.38 (br. t, 1H) ppm | | | | |
| | | | | | |
| **XI-A-072:** | ¹H NMR (DMSO-d6): δ = 1.19 (d, 6H); 4.12 (m, 1H); 5.60 (br. s, 2H); 7.63 (d, 2H); 8.00 (d, 2H); 8.15 (br. d, 1H) ppm | | | | |
| | | | | | |
| **XI-A-073:** | ¹H NMR (DMSO-d6): δ = 0.61 (m, 2H); 0.71 (m, 2H); 2.89 (m, 1H); 5.61 (br. s, 2H); 7.63 (d, 2H); 7.97 (d, 2H); 8.37 (br. s, 1H) ppm | | | | |
| | | | | | |
| **XI-A-074:** | ¹H NMR (DMSO-d6): δ = 1.39 (d, 3H); 4.87 (m, 1H); 5.64 (br. s, 2H); 7.68 (d, 2H); 8.05 (d, 2H); 8.82 (br. s, 1H) ppm | | | | |
| | | | | | |
| **XI-A-075:** | ¹H NMR (DMSO-d6): δ = 1.40 (s, 9H); 5.58 (br. s, 2H); 7.60 (d, 2H); 7.66 (br. s, 1H); 7.96 (d, 2H) ppm | | | | |
| | | | | | |
| **XI-A-076:** | ¹H NMR (DMSO-d6): δ = 0.92 (s, 9H); 1.11 (d, 3H); 4.01 (m, 1H); 7.62 (d, 2H); 7.86 (br. d, 1H); 8.00 (d, 2H) ppm | | | | |
| | | | | | |
| **XI-A-077:** | ¹H NMR (DMSO-d6): δ = 5.68 (br. s, 2H); 6.9 (m, 1H); 6.98 (m, 1H); 7.39 (m, 1H); 7.75 (d, 2H); 8.15 (d, 2H); 8.33 (m, 1H); 10.40 (s, 1H) ppm | | | | |
| | | | | | |
| **XI-A-078:** | ¹H NMR (DMSO-d6): δ = 4.60 (d, 2H); 5.64 (br. d, 2H); 7.25 (m, 1H); 7.34 (m, 1H); 7.67 (d, 2H); 7.74 (m, 1H); 8.07 (d, 2H); 8.50 (m, 1H); 9.03 (br. t, 1H) ppm | | | | |
| | | | | | |
| **XI-A-079:** | log P (HCOOH): 2.80 | **XI-A-080:** | log P (HCOOH): 1,97 | **XI-A-081:** | log P (HCOOH): 2,76 |
| | | | | | |
| **XI-A-082:** | log P (HCOOH): 3,52 | **XI-A-083:** | log P (HCOOH): 3,48 | **XI-A-084:** | log P (HCOOH): 3,53 |
| | | | | | |
| **XI-A-085:** | log P (HCOOH): 1,95 | **XI-A-086:** | log P (HCOOH): 2,54 | **XI-A-087:** | log P (HCOOH): 2,58 |
| | | | | | |
| **XI-A-088:** | log P (HCOOH): 2,63 | **XI-A-089:** | log P (HCOOH): 2,45 | **XI-A-090:** | log P (HCOOH): 1,42 |
| **XI-A-090:** | log P (HCOOH): 2,99 | **XI-A-091:** | log P (HCOOH): 1,94 | **XI-A-092:** | log P (HCOOH): 3,03 |
| | | | | | |
| **XI-A-093:** | log P (HCOOH): 1,98 | **XI-A-094:** | log P (HCOOH): 3,08 | **XI-A-095:** | log P (HCOOH): 2,03 |

**Tabelle 15**

| | | | | | | |
|---|---|---|---|---|---|---|
| Gemäß den oben angegebenen allgemeinen Methoden lassen sich die in der folgenden Tabelle angegebenen Verbindungen der Formel (XI-B) herstellen. | | | | | | |
| 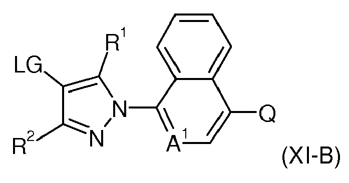 | | | | | | |

| **Tabelle 15 Beispiel Nr.** | **LG** | **R¹** | **R²** | **A¹** | **Q** | **Phys. Chem. Daten** |
|---|---|---|---|---|---|---|
| XI-B-001 | Br | H | CF₃ | CH | COOEt | |
| XI-B-002 | Br | H | CF₃ | CH | COOH | |
| XI-B-003 | Br | NH₂ | CF₃ | CH | Br | |
| XI-B-004 | Br | NH₂ | CF₃ | CH | I | |
| XI-B-005 | Br | Cl | CF₃ | CH | CN | |
| XI-B-006 | Br | Cl | C₂F₅ | CH | NO₂ | |
| XI-B-007 | Br | Br | C₂F₅ | CH | SMe | |
| XI-B-008 | Br | Br | C₂F₅ | CH | SCF₃ | |
| XI-B-009 | Br | I | C₂F₅ | CH | SOMe | |
| XI-B-010 | Br | I | CF₃ | CH | SOCF₃ | |
| XI-B-011 | Br | CN | CF₃ | N | SO₂Me | |
| XI-B-012 | Br | CN | CF₃ | N | SO₂CF₃ | |
| XI-B-013 | Br | NHCH₂Ph | C₂F₅ | CH | CN | |
| XI-B-014 | Br | NHAc | CF₃ | CH | NO₂ | |
| XI-B-015 | Br | NAc₂ | CF₃ | CH | F | |
| XI-B-016 | Br | NHMe | CF₂Ph | CH | Cl | |
| XI-B-017 | Br | NMe₂ | CF₂OMe | CH | 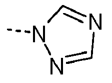 | |
| XI-B-018 | Br | SMe | CF₂OPh | CH | CONH-CH₂-Pyr | |
| XI-B-019 | Br | SOMe | CF₂(2-Pyr) | CH | CONH-CHMe-2-Pyr | |
| XI-B-020 | Br | SO₂Me | CF₂O(2-Pyr) | N | CONH-TFiPr | |
| XI-B-021 | I | H | CF₃ | CH | COOEt | |
| XI-B-022 | I | H | CF₃ | CH | COOH | |
| XI-B-023 | I | NH₂ | CF₃ | CH | Br | |
| XI-B-024 | I | NH₂ | CF₃ | CH | I | |
| XI-B-025 | I | Cl | CF₃ | CH | CN | |
| XI-B-026 | I | Cl | C₂F₅ | CH | NO₂ | |
| XI-B-027 | I | Br | C₂F₅ | CH | SMe | |
| XI-B-028 | I | Br | C₂F₅ | CH | SCF₃ | |
| XI-B-029 | I | I | C₂F₅ | CH | SOMe | |
| XI-B-030 | I | I | CF₃ | CH | SOCF₃ | |
| XI-B-031 | I | CN | CF₃ | N | SO₂Me | |
| XI-B-032 | I | CN | CF₃ | N | SO₂CF₃ | |
| XI-B-033 | I | NHCH₂Ph | C₂F₅ | CH | CN | |
| XI-B-034 | I | NHAc | CF₃ | CH | NO₂ | |
| XI-B-035 | I | NAc₂ | CF₃ | CH | F | |
| XI-B-036 | I | NHMe | CF₂Ph | CH | Cl | |
| XI-B-037 | I | NMe₂ | CF₂OMe | CH | 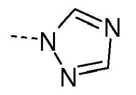 | |
| XI-B-038 | I | SMe | CF₂OPh | CH | CONH-CH₂-Pyr | |
| XI-B-039 | I | SOMe | CF₂(2-Pyr) | CH | CONH-CHMe-2-Pyr | |
| XI-B-040 | I | SO₂Me | CF₂O(2-Pyr) | N | CONH-TFiPr | |

### Biologische Beispiele

### Beispiel A

### Phaedon Cochleariae-Test (PHAECO Spritzbehandlung)

| | |
|---|---|
| Lösungsmittel: | 78,0 Gewichtsteile Aceton |
| | |
| | 1,5 Gewichtsteile Dimethylformamid |
| | |
| Emulgator: | 0,5 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigten z. B. die folgenden Verbindungen der Herstellungsbeispiele eine Wirkung von ≥ 80 % bei einer Aufwandmenge von 500 g/ha: I-A-Q1-001, I-A-Q1-002, I-A-Q2-011, I-A-Q2-012, I-A-Q2-013, I-A-Q2-014, I-A-Q2-015, I-A-Q2-016, I-A-Q2-017, I-A-Q2-018, I-A-Q2-019, I-A-Q2-020, I-A-Q2-022, I-A-Q2-023, I-A-Q2-024, I-A-Q2-025, I-A-Q2-026, I-A-Q2-027,I-A-Q2-028, I-A-Q2-029, I-A-Q2-030, I-A-Q2-032, I-A-Q2-033, I-A-Q2-034, I-A-Q2-035, I-A-Q2-037, I-A-Q2-039, I-A-Q2-040, I-A-Q2-041, I-A-Q2-042, I-A-Q2-044, I-A-Q2-046, I-A-Q2-047, I-A-Q2-048, I-A-Q2-049, I-A-Q2-050, I-A-Q2-051, I-A-Q2-052, I-A-Q2-053, I-A-Q2-054, I-A-Q2-055, I-A-Q2-056, I-A-Q2-057, I-A-Q2-058, I-A-Q2-059, I-A-Q2-060, I-A-Q2-061, I-A-Q2-062, I-A-Q2-065, I-A-Q2-066, I-A-Q2-069, I-A-Q2-073, I-A-Q2-079, I-A-Q2-080, I-A-Q2-083, I-A-Q2-084, I-A-Q2-085, I-A-Q2-086, I-A-Q2-087, I-A-Q2-088, I-A-Q2-089, I-A-Q2-090, I-A-Q2-092, I-A-Q2-095, I-A-Q2-096, I-A-Q2-098, I-A-Q2-109, I-A-Q2-110, I-A-Q2-111, I-A-Q2-112, I-A-Q2-133, I-A-Q2-134, I-A-Q2-135, I-A-Q2-136, I-A-Q2-137, I-A-Q2-138, I-A-Q2-139, I-A-Q2-140, I-A-Q2-141, I-A-Q2-144, I-A-Q2-145, I-A-Q2-147, I-A-Q2-149, I-A-Q2-150, I-A-Q2-151, I-A-Q2-152, I-A-Q2-153, I-A-Q2-154, I-A-Q2-155, I-A-Q2-157, I-A-Q2-159, I-A-Q2-160, I-A-Q2-162, I-A-Q2-165, I-A-Q2-166, I-A-Q2-167, I-A-Q2-169, I-A-Q2-170, I-A-Q2-171, I-A-Q2-172, I-A-Q2-174, I-A-Q2-175, I-A-Q2-176, I-A-Q2-180, I-A-Q2-182, I-A-Q2-183, I-A-Q2-188, I-A-Q2-202, I-A-Q2-203, I-A-Q2-205, I-A-Q2-207, I-A-Q2-209, I-A-Q2-210,I-A-Q2-211, I-A-Q2-213, I-A-Q2-214, I-A-Q2-215, I-A-Q2-216, I-A-Q2-217, I-A-Q2-218, I-A-Q2-219, I-A-Q2-220, I-A-Q2-221, I-A-Q2-222, I-A-Q2-223, I-A-Q2-224, I-A-Q2-225, I-A-Q2-226, I-A-Q2-227, I-A-Q2-228, I-A-Q2-229, I-A-Q2-230, I-A-Q2-233, I-A-Q2-234, I-A-Q2-236, I-A-Q2-237, I-A-Q2-238, I-A-Q2-239, I-A-Q2-240, I-A-Q2-241, I-A-Q2-242, I-A-Q2-244, I-A-Q2-246, I-A-Q2-247, I-A-Q4-009, I-A-Q4-011, I-A-Q4-040, I-A-Q4-042, I-A-Q4-048, I-A-Q4-058, I-A-Q4-102.

Eine Wirkung von 83 % bei einer Aufwandmenge von 500 g/ha zeigte bei diesem Test z. B. die folgende Verbindung der Herstellungsbeispiele: I-A-Q1-089.

Eine Wirkung von 100 % bei einer Aufwandmenge von 500 g/ha zeigten bei diesem Test z. B. die folgenden Verbindungen der Herstellungsbeispiele: I-A-Q1-087, I-A-Q1-090, I-A-Q1-091, I-A-Q2-407, I-A-Q2-412, I-A-Q2-414, I-A-Q2-415, I-A-Q2-417, I-A-Q2-419, I-A-Q2-420, I-A-Q2-421.

### Beispiel B

### Spodoptera frugiperda-Test (SPODFR Spritzbehandlung)

| | | |
|---|---|---|
| Lösungsmittel: | 78,0 | Gewichtsteile Aceton |
| | | |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigten z. B. die folgenden Verbindungen der Herstellungsbeispiele eine Wirkung von ≥ 80 % bei einer Aufwandmenge von 500 g/ha: I-A-Q1-001, I-A-Q2-011, I-A-Q2-012, I-A-Q2-014, I-A-Q2-015, I-A-Q2-017, I-A-Q2-018, I-A-Q2-019, I-A-Q2-020, I-A-Q2-023, I-A-Q2-030, I-A-Q2-033, I-A-Q2-035, I-A-Q2-039, I-A-Q2-044, I-A-Q2-048, I-A-Q2-052, I-A-Q2-053, I-A-Q2-054, I-A-Q2-055, I-A-Q2-058, I-A-Q2-060, I-A-Q2-061, I-A-Q2-062, I-A-Q2-107, I-A-Q2-110, I-A-Q2-133, I-A-Q2-141, I-A-Q2-147, I-A-Q2-155, I-A-Q2-160, I-A-Q2-166, I-A-Q2-202, I-A-Q2-207, I-A-Q2-217, I-A-Q2-218, I-A-Q2-219, I-A-Q2-220, I-A-Q2-221, I-A-Q2-223, I-A-Q2-224, I-A-Q2-227, I-A-Q2-230, I-A-Q2-231, I-A-Q2-236, I-A-Q2-238, I-A-Q2-239, I-A-Q2-240, I-A-Q2-242, I-A-Q2-244, I-A-Q2-246, I-A-Q4-009, I-A-Q4-014, I-A-Q4-016, I-A-Q4-040, I-A-Q4-041, I-A-Q4-042, I-A-Q4-043, I-A-Q4-048, I-A-Q4-051, I-A-Q4-053, I-A-Q4-058, I-A-Q4-102, IX-021, IX-038.

Eine Wirkung von 83 % bei einer Aufwandmenge von 500 g/ha zeigte bei diesem Test z. B. die folgende Verbindung der Herstellungsbeispiele: I-A-Q2-409.

Eine Wirkung von 100 % bei einer Aufwandmenge von 500 g/ha zeigten bei diesem Test z. B. die folgenden Verbindungen der Herstellungsbeispiele: I-A-QI-091, I-A-Q2-407.

### Beispiel C

### Myzus persicae-Test (MYZUPE Spritzbehandlung)

| | | |
|---|---|---|
| Lösungsmittel: | 78,0 | Gewichtsteile Aceton |
| | | |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Chinakohlblattscheiben *(Brassica pekinensis),* die von allen Stadien der Grünen Pfirsichblattlaus *(Myzus persicae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigten z. B. die folgenden Verbindungen der Herstellungsbeispiele eine Wirkung von ≥ 80 % bei einer Aufwandmenge von 500 g/ha: I-A-Q2-053, I-A-Q2-133, I-A-Q2-134, I-A-Q2-0192, I-A-Q4-088, I-A-Q4-089.

Eine Wirkung von 90 % bei einer Aufwandmenge von 500 g/ha zeigte bei diesem Test z. B. die folgende Verbindung der Herstellungsbeispiele: I-A-Q1-089.

### Beispiel D

### Tetranychus-Test, OP-resistent (TETRUR Spritzbehandlung)

| | | |
|---|---|---|
| Lösungsmittel: | 78,0 | Gewichtsteile Aceton |
| | | |
| | 1,5 | Gewichtsteile Dimethylformamid |
| | | |
| Emulgator: | 0,5 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit emulgatorhaltigem Wasser auf die gewünschte Konzentration.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigten z. B. die folgenden Verbindungen der Herstellungsbeispiele eine Wirkung von ≥ 80 % bei einer Aufwandmenge von 500 g/ha: I-A-Q2-012, I-A-Q2-015, I-A-Q2-020, I-A-Q2-035, I-A-Q2-040, I-A-Q2-041, I-A-Q2-048, I-A-Q2-051, I-A-Q2-052, I-A-Q2-055, I-A-Q2-060, I-A-Q2-112,I-A-Q4-058.

Bei diesem Test zeigten z. B. die folgenden Verbindungen der Herstellungsbeispiele eine Wirkung von ≥ 80 % bei einer Aufwandmenge von 100 g/ha: Nr. I-A-Q2-046, I-A-Q2-110, I-A-Q2-202, I-A-Q2-214, I-A-Q2-216, I-A-Q2-219, I-A-Q2-220, I-A-Q2-223, I-A-Q2-224, I-A-Q2-227, I-A-Q2-229, I-A-Q2-231, I-A-Q2-239, I-A-Q2-240, I-A-Q2-247

### Beispiel E

### Lucilia cuprina-Test (LUCICU)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße, die Pferdefleisch enthalten, das mit der Wirkstoffzubereitung der gewünschten Konzentration behandelt wurde, werden mit *Lucilia cuprina* Larven besetzt.

Nach der gewünschten Zeit wird die Abtötung in % bestimmt. Dabei bedeutet 100 %, dass alle Larven abgetötet wurden; 0 % bedeutet, dass keine Larven abgetötet wurden.

Bei diesem Test zeigten z. B. die folgenden Verbindungen der Herstellungsbeispiele eine Wirkung von ≥ 80 % bei einer Aufwandmenge von 100 ppm: I-A-Q1-001, I-A-Q2-002, I-A-Q2-005, I-A-Q2-010, I-A-Q2-011, I-A-Q2-012, I-A-Q2-014, I-A-Q2-015, I-A-Q2-017, I-A-Q2-018, I-A-Q2-019, I-A-Q2-023, I-A-Q2-134, I-A-Q2-141, I-A-Q2-147, I-A-Q2-149, I-A-Q2-152.

### Beispiel F

### Musca domestica-Test (MUSCDO)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße, die einen Schwamm enthalten, der mit der Wirkstoffzubereitung der gewünschten Konzentration behandelt wurde, werden mit *Musca domestica Adulten* besetzt.

Nach der gewünschten Zeit wird die Abtötung in % bestimmt. Dabei bedeutet 100 %, dass alle Fliegen abgetötet wurden; 0 % bedeutet, dass keine Fliegen abgetötet wurden.

Bei diesem Test zeigten z. B. die folgenden Verbindungen der Herstellungsbeispiele eine Wirkung von ≥ 80 % bei einer Aufwandmenge von 100 ppm: I-A-Q2-002, I-A-Q2-005, I-A-Q2-011, I-A-Q2-014, I-A-Q2-017, I-A-Q2-018.

### Beispiel G

### Ctenocephalides felis; oral (CTECFE)

| | |
|---|---|
| Lösungsmittel: | 1 Gewichtsteil Dimethylsulfoxid |

Zwecks Herstellung einer zweckmäßigen Wirkstoffzubereitung vermischt man 2 Gewichtsteile Wirkstoff mit der angegebenen Menge Lösungsmittel. Ein Teil des Konzentrats wird mit citiriertem Rinderblut verdünnt und die gewünschte Konzentration hergestellt.

20 nüchterne adulte Flöhe *(Ctenocephalides felis)* werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach der gewünschten Zeit wird die Abtötung in % bestimmt. Dabei bedeutet 100 %, dass alle Flöhe abgetötet wurden; 0 % bedeutet, dass kein Floh abgetötet wurde.

Bei diesem Test zeigten z. B. die folgenden Verbindungen der Herstellungsbeispiele eine Wirkung von ≥ 80 % bei einer Aufwandmenge von 100 ppm: I-A-Q1-001, I-A-Q2-011, I-A-Q2-012, I-A-Q2-014, I-A-Q2-015, I-A-Q2-016, I-A-Q2-017, I-A-Q2-018, I-A-Q2-023, I-A-Q2-134

### Beispiel H

### Boophilus microplus -Test (BOOPMI Injektion)

| | |
|---|---|
| Lösungsmittel: | Dimethylsulfoxid |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Die Wirkstofflösung wird in das Abdomen *(Boophilus microplus)* injiziert, die Tiere werden in Schalen überführt und in einem klimatisierten Raum aufbewahrt. Die Wirkungskontrolle erfolgt auf Ablage fertiler Eier.

Nach der gewünschten Zeit wird die Wirkung in % bestimmt. Dabei bedeutet 100%, dass keine Zecke fertile Eier gelegt hat.

Bei diesem Test zeigten z. B. die folgenden Verbindungen der Herstellungsbeispiele eine Wirkung von ≥ 80 % bei einer Aufwandmenge von 20 µg/Tier: I-A-Q1-001, I-A-Q2-002, I-A-Q2-005, I-A-Q2-010, I-A-Q2-011, I-A-Q2-012, I-A-Q2-014, I-A-Q2-015, I-A-Q2-016, I-A-Q2-017, I-A-Q2-018, I-A-Q2-019, I-A-Q2-022, I-A-Q2-023, I-A-Q2-024, I-A-Q2-026, I-A-Q2-134, I-A-Q2-141, I-A-Q2-147, I-A-Q2-149, I-A-Q2-150, I-A-Q2-152, I-A-Q2-195.

### Beispiel I

### Spodoptera litura Larven - Test

| | |
|---|---|
| Lösungsmittel: | 3 Gewichtsanteile Dimethylformamid |
| | |
| Emulgator: | 1 Gewichtsanteil Polyoxyethylenalkylphenylether |

Zur Herstellung eines geeigneten Wirkstoffs wird ein Gewichtsanteil Wirkstoff mit der oben angegebenen Menge Lösungsmittel enthaltend die oben angegeben Menge Emulgator gemischt und die Mischung wird mit Wasser auf die vorgeschriebene Konzentration verdünnt.

Blätter der Süßkartoffel werden in die mit Wasser auf die vorgeschriebene Konzentration verdünnte Probenlösung eingetaucht und, nachdem die sich auf den Blättern niedergeschlagene Lösung an der Luft getrocknet ist, werden die so behandelten Blätter in eine Laborschale von 9 cm Durchmesser gegeben, in die dann 10 sich im dritten Stadium befindlichen Spodoptera litura Larven übertragen werden. Die Schale wird dann in einen temperaturkontrollierten Raum bei 25°C gestellt, gefolgt von der Zugabe von Süßkartoffelblättern zu der Schale am zweiten und vierten Tag und der Ermittlung der Zahl der toten Insekten nach 7 Tagen, woraus das insektizide Verhältnis berechnet wird.

Die Ergebnisse sind die Mittelwerte von zwei Laborschalen pro Gruppe in diesem Test.

Verbindungen I-A-Q4-001 und I-A-Q4-006 zeigten bei einer Wirkstoffkonzentration von 500 ppm eine 80 %ige Abtötung der Insektenlarven.

Verbindungen I-A-Q2-001, I-A-Q2-002, I-A-Q2-003, I-A-Q2-005, I-A-Q2-006, und I-A-Q2-010 zeigten bei einer Wirkstoffkonzentration von 500 ppm eine 100 %ige Abtötung der Insektenlarven.

### Beispiel J

### Aulacophora femoralis - Test (Sprühtest)

| | |
|---|---|
| Lösungsmittel: | 3 Gewichtsanteile Dimethylformamid |
| | |
| Emulgator: | 1 Gewichtsanteil Polyoxyethylenalkylphenylether |

Zur Herstellung einer geeigneten Formulierung eines Wirkstoffs wird ein Gewichtsanteil des Wirkstoffs mit der genannten Menge an Lösungsmittel enthaltend die genannte Menge an Emulgator gemischt und die Mischung wird mit Wasser auf eine vorgegebene Konzentration verdünnt.

Gurkenblätter werden in eine verdünnte wässrige Lösung eines Wirkstoffs in der vorgegebenen Konzentration, die in gleicher Weise wie im oben beschriebenen Test hergestellt wurde, eingetaucht, luftgetrocknet und in eine Plastikschale mit sterilisierter schwarzer Erde gegeben. In diese Schale werden 5 Aulacophora femoralis Larven, die sich im zweiten Stadium befinden, übertragen. Die Schale wird dann in einen temperaturkontrollierten Raum bei 25°C gestellt. Nach 7 Tagen wird die Zahl der toten Larven gezählt, um die Sterberate zu berechnen.

Verbindungen I-A-Q2-002, I-A-Q2-005 und I-A-Q2-010 zeigten bei einer Konzentration der aktiven Verbindung von 500 ppm eine 100 %ige Abtötung der Insekten.

## Patentansprüche

1. Verbindungen der Formel (I) 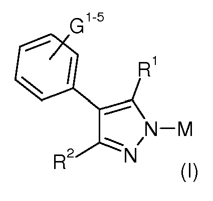 in welcher
M für eine der nachstehend aufgeführten Gruppierungen steht: 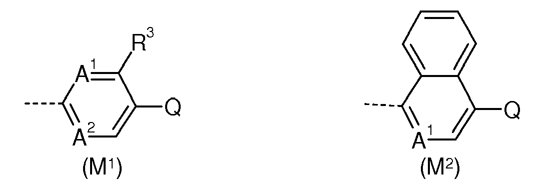 A¹ und A² unabhängig voneinander für Stickstoff oder C-R⁴ stehen;
G¹, G², G³, G⁴ und G⁵ für Wasserstoff oder einen Substituenten stehen, unabhängig voneinander ausgewählt aus: Halogen, Alkyl, Alkenyl, Alkinyl, Haloalkyl, SF₅, Hydroxy, Alkoxy, Alkoxyalkyl, Alkoxyalkoxy, Cycloalkyl, Alkenyloxy, Alkinyloxy, Cycloalkylalkoxy, Haloalkoxy, Haloalkoxyalkyl, Alkylsulfanyl, Haloalkylsulfanyl, Alkylsulfinyl, Haloalkylsulfinyl, Alkylsulfonyl, Haloalkylsulfonyl, Cyano, Alkylcarbonyl, Alkoxycarbonyl, Alkoxycarbonylalkyl, Carboxyl, Aminocarbonyl, Aminothiocarbonyl, Alkylaminocarbonyl, Dialkylaminocarbonyl, Trialkylsilyl, Nitro, Amino, Alkylamino, Dialkylamino, Alkylsulfonylamino, Dialkylsulfonylamino, -CR⁵=NO-R', -CR^{S}=NO-Haloalkyl oder Formyl, wobei vicinale Alkyl-, Haloalkyl, Alkoxy- und/oder Haloalkoxygruppen zusammen mit den Kohlenstoffatomen, an die sie gebunden sind, ein fünf- bis sechsgliedriges cyclisches System bilden können, das 0 bis 2 Sauerstoffatome enthält, und dessen Alkylanteil gegebenenfalls mit einem oder mehreren Fluoratomen substituiert sein kann;
Q für Q², Q³ oder Q⁴ steht;
Q² für C(W¹)NR⁸R⁹ steht;
Q³ für C(R¹⁰R¹¹)NR⁸R⁹ steht;
Q⁴ für Cyano (wobei R¹ nicht für Amino steht), Nitro, Amino, COOH, COOR¹², Fluor (falls R³ verschieden von Chlor ist), Chlor (falls R³ verschieden von Chlor, COOH, CH₂CH₂OMe und OMe ist), Brom Iod, SR¹² (wobei R¹ nicht für Amino steht, falls R¹² für Alkyl steht), S(O)R¹², S(O)₂R¹² oder S(O)₂NR⁸R⁹ steht;
R¹ für Wasserstoff, Halogen, Alkylsulfanyl, Alkylsulfinyl, Alkylsulfonyl, Cyano, Amino, Z¹⁶ oder NR¹³R¹⁴ steht;
R² für Wasserstoff, Halogen, Alkyl, Alkenyl, Alkinyl, Haloalkyl, Cycloalkyl, Hydroxyalkyl, Alkoxyalkyl, Haloalkoxyalkyl, Alkoxyhaloalkyl, Alkylsulfanylalkyl, Alkylsulfinylalkyl, Alkylsulfonylalkyl, Haloalkylsulfanylalkyl, Haloalkylsulfinylalkyl, Haloalkylsulfonylalkyl Cycloalkylalkyl, Phenylalkyl, Heteroarylalkyl, Heterocyclylalkyl, Arylhaloalkyl, Haloalkyloxyhaloalkyloxyhaloalkyl, Heterocyclyl, Alkylsulfanyl, Alkylsulfinyl, Alkylsulfonyl oder Phenyl steht;
wobei ein Phenyl- oder Heteroarylrest gegebenenfalls ein- oder mehrfach substituiert ist durch Halogen, Alkyl, Haloalkyl, Alkoxy, Nitro oder Cyano; ein Heterocyclylrest gegebenenfalls substituiert ist durch -OH / =O, -SH / =S, -NH₂, Halogen, Alkyl, Haloalkyl, Alkoxy, Alkylsulfanyl, Alkylsulfinyl, Alkylsulfonyl, Monoalkylamino, Dialkylamino, Nitro oder Cyano; und ein an Phenyl, Heteroaryl oder Heterocyclyl gebundener Alkandiyl-Rest gegebenenfalls ein- oder mehrfach substituiert ist durch Halogen, Alkyl, Haloalkyl, Alkoxy oder Cyano.
R³ für Halogen, Alkyl, Alkenyl, Alkinyl, Haloalkyl, Cycloalkyl, Alkoxyalkyl, Alkylcarbonyloxyalkyl, Alkylsulfanylalkyl, Alkylsulfinylalkyl, Alkylsulfonylalkyl, Hydroxy, Z¹⁶, Alkoxy, Acylamino, Alkoxycarbonylamino, Alkenyloxy, Alkinyloxy, Cycloalkylalkoxy, Haloalkoxy, Alkylsulfanyl, Haloalkylsulfanyl, Alkylsulfinyl, Haloalkylsulfinyl, Alkylsulfonyl, Haloalkylsulfonyl, Cyano, Alkylcarbonyl, Alkoxycarbonyl, Alkoxycarbonylalkyl, Carboxyl, Aminocarbonyl, Aminothiocarbonyl, Alkylaminocarbonyl, Dialkylaminocarbonyl, Alkylsulfonylaminocarbonyl, Trialkylsilyl, Nitro, Amino, Alkylamino, Dialkylamino, Alkylsulfonylamino, Dialkylsulfonylamino, -CR⁵=NO-R⁵, -CR⁵=NO-Haloalkyl oder Formyl steht;
R⁴ für Wasserstoff, Cyano, Nitro, Alkyl, Haloalkyl, Halogen oder Alkoxy steht;
R⁵ für Wasserstoff oder Alkyl steht;
R⁶, R^{6'}, R^{6"}, R^{6"'} unabhängig voneinander für Wasserstoff, Amino, Hydroxy, Mercapto, Nitro, Cyano, Carboxyl, Halogen, Alkyl, Haloalkyl, Alkenyl, Alkinyl, Cycloalkyl, Alkoxy, Haloalkoxy, Alkenyloxy, Alkinyloxy, Alkoxycarbonyl, Alkylsulfanyl, Haloalkylsulfanyl, Alkylsulfinyl, Haloalkylsulfinyl, Alkylsulfonyl, Haloalkylsulfonyl, Aminocarbonyl, Aminothiocarbonyl, CR⁵=NO-R⁵, -CR⁵=NO-Haloalkyl, Formyl, Alkylamino, Dialkylamino, Phenyl, Heteroaryl, Heteroarylalkyl oder Heterocyclylalkyl stehen;
wobei ein Phenyl- oder Heteroarylrest gegebenenfalls ein- oder mehrfach substituiert ist durch Halogen, Alkyl, Haloalkyl, Alkoxy, Nitro oder Cyano; ein Heterocyclylrest gegebenenfalls substituiert ist durch -OH / =O, -SH / =S, -NH₂, Halogen, Alkyl, Haloalkyl, Alkoxy, Alkylsulfanyl, Alkylsulfinyl, Alkylsulfonyl, Monoalkylamino, Dialkylamino, Nitro oder Cyano; und ein an Phenyl, Heteroaryl oder Heterocyclyl gebundener Alkandiylrest gegebenenfalls ein- oder mehrfach substituiert ist durch Halogen, Alkyl, Haloalkyl, Alkoxy oder Cyano;
R⁷ für Wasserstoff, Amino, Hydroxy, Cyano, Alkyl, Haloalkyl, Alkenyl, Alkinyl, Cycloalkyl, Alkoxy, Alkenyloxy, Alkinyloxy, Alkoxycarbonyl, Alkylcarbonyl, Alkylaminocarbonyl, Dialkylaminocarbonyl, Phenyl, Phenylalkyl, Heteroarylalkyl oder Heterocyclylalkyl steht;
wobei ein Phenyl- oder Heteroarylrest gegebenenfalls ein- oder mehrfach substituiert ist durch Halogen, Alkyl, Haloalkyl, Alkoxy, Nitro oder Cyano; ein Heterocyclylrest gegebenenfalls substituiert ist durch -OH / =0, -SH / =S, -NH₂, Halogen, Alkyl, Haloalkyl, Alkoxy, Alkylsulfanyl, Alkylsulfinyl, Alkylsulfonyl, Monoalkylamino, Dialkylamino, Nitro oder Cyano; und ein an Phenyl, Heteroaryl oder Heterocyclyl gebundener Alkandiyl-Rest gegebenenfalls ein- oder mehrfach substituiert ist durch Halogen, Alkyl, Haloalkyl, Alkoxy oder Cyano und
wobei gegebenenfalls zwei benachbarte Reste aus R⁶, R^{6'}, R^{6"}, R^{6"'} und R⁷ zusammen für gegebenenfalls substituiertes und/oder gegebenenfalls durch Sauerstoff oder Schwefel oder eine Gruppierung aus der Reihe -S(O)-, -SO₂-, -NH- oder -N-Alkyl- am Anfang (bzw. am Ende) oder innerhalb der Kohlenwasserstoffkette unterbrochenes Alkandiyl oder Alkendiyl mit jeweils 2 bis 5 Kohlenstoffatomen stehen;
R⁸ und R⁹ unabhängig voneinander für Wasserstoff, Alkyl, Haloalkyl, Alkenyl (gegebenenfalls substituiert durch Halogen, Alkyl, Haloalkyl oder Cyano), Alkinyl, Cycloalkyl (am Cyclus gegebenenfalls ein- oder mehrfach substituiert durch Halogen, Haloalkyl, Alkyl oder kondensiert an einen Aromaten oder Heteroaromaten), Cycloalkylalkyl (am Cyclus gegebenenfalls ein- oder mehrfach substituiert durch Halogen, Haloalkyl, Alkyl oder kondensiert an einen Aromaten oder Heteroaromaten, am Alkylteil gegebenenfalls ein- oder mehrfach substituiert durch Halogen, Alkyl, Haloalkyl, Alkoxy oder Cyano), Cycloalkylcarbonylheterocyclyl, Alkyloxycarbonylheterocyclyl, Alkylsulfinylalkyl, Alkylsulfanylalkyl, Alkylsulfonylalkyl, Hydroxyalkyl, Heterocyclylcarbonylalkyl, Heteroarylcarbonylaminoalkyl, Haloalkoxyalkyl, Alkylthiocarbonyl, Dialkylaminocarbonyl, Alkylaminocarbonyl, Haloalkylaminocarbonyl Alkoxy, Alkenyloxy, Alkinyloxy, Alkoxyalkyl, Alkylcarbonylalkyl, C(R¹⁰R¹¹)CR⁵=NO-R⁵, Alkylsulfonyl, Alkylcarbonyl, Haloalkylcarbonyl, Alkenylcarbonyl, Alkinylcarbonyl, Cycloalkylcarbonyl, Alkoxycarbonyl, Alkenyloxycarbonyl, Alkinyloxycarbonyl, Alkoxyalkylcarbonyl, Phenylsulfonyl, Phenyl, Heteroaryl, Heterocyclyl, Phenylalkyl, Heteroarylalkyl, Heterocyclylalkyl, Phenylcarbonyl, Heteroarylcarbonyl, Heterocyclylcarbonyl, Phenylalkylcarbonyl, Heteroarylalkylcarbonyl, Heterocyclylalkylcarbonyl, Phenoxycarbonyl oder Phenylalkoxycarbonyl stehen;
wobei ein Phenyl- oder Heteroarylrest gegebenenfalls ein- oder mehrfach substituiert ist durch Halogen, Alkyl, Haloalkyl, Alkoxy, Nitro oder Cyano; ein Heterocyclylrest gegebenenfalls substituiert ist durch -OH / =O, -SH / =S, -NH₂, Halogen, Alkyl, Haloalkyl, Alkoxy, Alkylsulfanyl, Alkylsulfinyl, Alkylsulfonyl, Monoalkylamino, Dialkylamino, Nitro oder Cyano; und ein an Phenyl, Heteroaryl oder Heterocyclyl gebundener Alkandiyl-Rest gegebenenfalls ein- oder mehrfach substituiert ist durch Halogen, Alkyl, Haloalkyl, Alkoxy oder Cyano und
wobei gegebenenfalls R⁸ / R⁹ zusammen für gegebenenfalls substituiertes und/oder gegebenenfalls durch Sauerstoff oder Schwefel oder eine Gruppierung aus der Reihe -S(O)-, -SO₂-, -NH- oder -N-Alkyl- am Anfang (bzw. am Ende) oder innerhalb der Kohlenwasserstoffkette unterbrochenes Alkandiyl oder Alkendiyl mit jeweils 2 bis 5 Kohlenstoffatomen stehen;
R¹⁰ und R¹¹ unabhängig voneinander für Wasserstoff, Alkyl, Haloalkyl oder Cyano stehen;
R¹² für Alkyl oder Haloalkyl steht;
R¹³ und R¹⁴ unabhängig voneinander für Wasserstoff, Alkyl, Haloalkyl, Alkenyl, Alkinyl, Alkoxyalkyl, Alkylcarbonylalkyl, C(R¹⁰R¹¹)CR⁵=NO-R⁵, Alkylsulfonyl, Alkylcarbonyl, Haloalkylcarbonyl, Alkenylcarbonyl, Alkinylcarbonyl, Cycloalkylcarbonyl, Alkoxycarbonyl, Alkenyloxycarbonyl, Alkinyloxycarbonyl, Alkoxyalkylcarbonyl, Phenylsulfonyl, Phenyl, Heteroaryl, Heterocyclyl, Phenylalkyl, Heteroarylalkyl, Heterocyclylalkyl, Phenylcarbonyl, Heteroarylcarbonyl, Heterocyclylcarbonyl, Phenylalkylcarbonyl, Heteroarylalkylcarbonyl, Heterocyclylalkylcarbonyl, Phenoxycarbonyl oder Phenylalkoxycarbonyl stehen;
wobei ein Phenyl- oder Heteroarylrest gegebenenfalls ein- oder mehrfach substituiert ist durch Halogen, Alkyl, Haloalkyl, Alkoxy, Nitro oder Cyano; ein Heterocyclylrest gegebenenfalls substituiert ist durch -OH / =O, -SH / =S, -NH₂, Halogen, Alkyl, Haloalkyl, Alkoxy, Alkylsulfanyl, Alkylsulfinyl, Alkylsulfonyl, Monoalkylamino, Dialkylamino, Nitro oder Cyano; und ein an Phenyl, Heteroaryl oder Heterocyclyl gebundener Alkandiyl-Rest gegebenenfalls ein- oder mehrfach substituiert ist durch Halogen, Alkyl, Haloalkyl, Alkoxy oder Cyano
oder
R¹³ und R¹⁴ als Imin die Gruppierung =CR⁵-NR¹⁵R¹⁶ oder =CR⁵-OR¹² bilden;
R¹⁵ und R¹⁶ unabhängig voneinander für Alkyl stehen oder
R¹⁵ und R¹⁶ zusammen für gegebenenfalls substituiertes und/oder gegebenenfalls durch Sauerstoff oder Schwefel oder eine Gruppierung aus der Reihe -S(O)-, -SO₂-, -NH-oder -N-Alkyl- am Anfang (bzw. am Ende) oder innerhalb der Kohlenwasserstoffkette unterbrochenes Alkandiyl oder Alkendiyl mit jeweils 2 bis 5 Kohlenstoffatomen stehen;
und die Verbindungen der allgemeinen Formel (I) außerdem N-Oxide, Salze, Tautomere, Diastereomere und optische Isomere umfassen.

2. Verbindungen der Formel (IX) 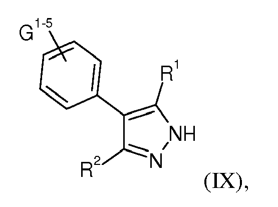 wobei G¹, G², G³, G⁴, G⁵, R¹ und R² wie in Anspruch 1 definiert sind, mit der Maßgabe, dass R¹ nicht für Amino steht.

3. Verbindungen der Formeln (XI-A) oder (XI-B) 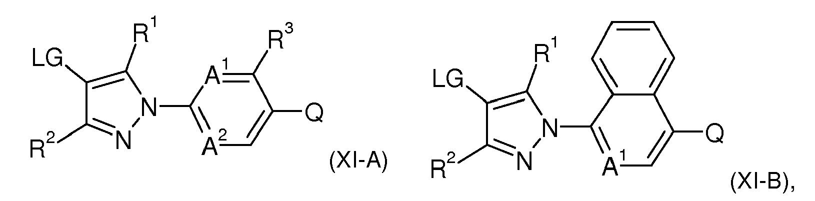 wobei R¹, R², R³, A¹, A² und Q wie in Anspruch 1 definiert sind und LG für Chlor, Brom, Iod oder Alkylsulfonyl steht.

4. Verbindungen der Formel (VII-A) oder (VII-B) 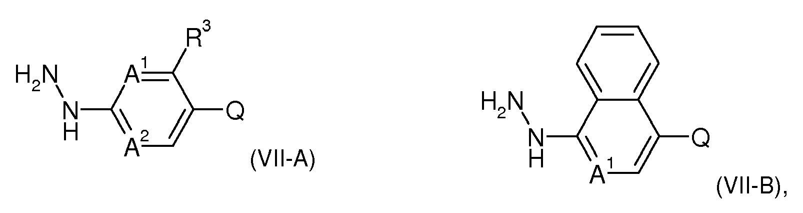 wobei A¹, A², R³ und Q wie in Anspruch 1 definiert sind, Q aber ungleich Z⁸ ist wenn A¹ gleich Stickstoff und A² gleich CH.

5. Verwendung von Verbindungen der Formel (I) gemäß Anspruch 1 zur Bekämpfung tierischer Schädlinge.

6. Verwendung von Verbindungen der Formel (I) zur Bekämpfung tierischer Schädlinge, wobei M für M¹ steht, R³ für Wasserstoff steht und alle anderen Reste wie oben definiert sind, mit der Maßgabe, dass A¹ und A² nicht gleichzeitig für Stickstoff stehen.

7. Verwendung von Verbindungen der Formeln (XI-A) oder (XI-B) gemäß Anspruch 3 zur Bekämpfung tierischer Schädlinge.

8. Verfahren zur Bekämpfung tierischer Schädlinge, **dadurch gekennzeichnet, dass** man Verbindungen der Formel (I) gemäß Anspruch 1 auf tierische Schädlinge und/oder deren Lebensraum und/oder Saatgut einwirken lässt.

9. Verfahren zur Herstellung agrochemischer Zusammensetzungen, **dadurch gekennzeichnet, dass** man Verbindungen der Formel (I) gemäß Anspruch 1 mit Streckmitteln und/oder oberflächenaktiven Stoffen vermischt.
